# EUROPEAN PATENT SPECIFICATION

(11) **EP 2 242 843 B1**
(45) Date of publication and mention of the grant of the patent: **27.05.2015**
(21) Application number: 08870397.0
(22) Date of filing: 31.12.2008
(51) Int. Cl.: C12N 15/10

(54) **METHODS AND MATERIALS FOR TARGETED MUTAGENESIS**
VERFAHREN UND MATERIAL FÜR GERICHTETE MUTAGENESE
PROCÉDÉS ET MATÉRIAUX POUR MUTAGENÈSE CIBLÉE

(30) Priority: 31.12.2007 US 18101 P; 31.12.2007 US 18105 P; 31.12.2007 US 18113 P
(43) Date of publication of application: 27.10.2010
(73) Proprietor: XOMA Technology Ltd., Berkeley, CA 94710 (US)
(72) Inventor: TAKEUCHI, Toshihiko, Berkeley,California 94710 (US)
(74) Representative: Huenges, Martin
(86) International application number: PCT/US2008/088651
(87) International publication number: WO 2009/088933

(56) References cited:
- WO-A-00/53744
- US-A1- 2006 024 308
- AKANUMA SATOSHI ET AL: "Combinatorial mutagenesis to restrict amino acid usage in an enzyme to a reduced set" PROCEEDINGS OF THE NATIONAL ACADEMY OF SCIENCES OF USA, NATIONAL ACADEMY OF SCIENCE, WASHINGTON, DC.; US, vol. 99, no. 21, 15 October 2002 (2002-10-15), pages 13549-13553, XP002437014 ISSN: 0027-8424
- TOMANDL D AND SCHWIENHORST A: ""Computer-assisted Design of Doped Libraries" in Evolutionary methods in biotechnology" 2004, WILEY-VCH VERLAG , WEINHEIM , XP002520940 ISBN: 3-527-30799-0 the whole document
- MAGLIERY, T: "Mixed Codons Worksheet"[Online] 23 July 2003 (2003-07-23), XP002520936 Retrieved from the Internet: URL:http://www.chemistry.ohio-state.edu/~m agliery/mixedcodons/mixed_codons_worksheet _v1.zip> [retrieved on 2009-03-23]
- MENA MARCO A ET AL: "Automated design of degenerate codon libraries" PROTEIN ENGINEERING DESIGN & SELECTION, vol. 18, no. 12, December 2005 (2005-12), pages 559-561, XP002520937 ISSN: 1741-0126
- WOLF ETHAN ET AL: "Combinatorial codons: A computer program to approximate amino acid probabilities with biased nucleotide usage" PROTEIN SCIENCE, vol. 8, no. 3, March 1999 (1999-03), pages 680-688, XP002520938 ISSN: 0961-8368
- LABEAN THOMAS H ET AL: "Design of synthetic gene libraries encoding random sequence proteins with desired ensemble characteristics" PROTEIN SCIENCE, vol. 2, no. 8, 1993, pages 1249-1254, XP000564520 ISSN: 0961-8368
- LARSSON OLA ET AL: "Quantitative codon optimisation of DNA libraries encoding sub-random peptides: design and characterisation of a novel library encoding transmembrane domain peptides." NUCLEIC ACIDS RESEARCH 1 DEC 2002, vol. 30, no. 23, 1 December 2002 (2002-12-01), page e133, XP002520939 ISSN: 1362-4962
- ARKIN A P ET AL: "OPTIMIZING NUCLEOTIDE MIXTURES TO ENCODE SPECIFIC SUBSETS OF AMINO ACIDS FOR SEMI-RANDOM MUTAGENESIS" NATURE BIOTECHNOLOGY, NATURE PUBLISHING GROUP, NEW YORK, NY, US, vol. 10, no. 3, 1 March 1999 (1999-03-01), pages 297-300, XP000942164 ISSN: 1087-0156
- HUGHES MARCUS D ET AL: "Removing the redundancy from randomised gene libraries." JOURNAL OF MOLECULAR BIOLOGY, vol. 331, no. 5, 29 August 2003 (2003-08-29), pages 973-979, XP004969255 ISSN: 0022-2836
- RAJPAL ARVIND ET AL: "A general method for greatly improving the affinity of antibodies by using combinatorial libraries" PROCEEDINGS OF THE NATIONAL ACADEMY OF SCIENCES OF THE UNITED STATES OF AMERICA, vol. 102, no. 24, June 2005 (2005-06), pages 8466-8471, XP002347095 ISSN: 0027-8424

## Description

### FIELD

The present disclosure relates to methods and materials for mutagenesis, including for the generation of novel or improved proteins and libraries or arrays of mutant proteins or nucleic acids encoding such mutant proteins.

### BACKGROUND

Mutagenesis is a powerful research tool whereby genetic information is deliberately changed in a stable manner (see, *e*.*g*., Hutchinson et al. (1978) J. Biol. Chem. 253:6551; and Razin et al. (1978) Proc. Natl. Acad. Sci. USA 75:4268). Mutagenesis may be performed experimentally by employing the use of recombinant DNA technology and used to introduce specific mutations in a gene to study the effects on its encoded protein. By comparing the properties of a wild-type protein and the mutants generated, it may be possible to identify individual amino acids or domains of amino acids that may be important for the structural integrity and/or biochemical function of the protein (*e*.*g*., binding and/or catalytic activity).

Methods of mutagenesis may be random (*e.g.,* error prone PCR) or deliberate (*e*.*g*., site directed mutagenesis). A common feature of many methods of mutagenesis is the use of synthetic primers (*e*.*g*., oligonucleotides) carrying desired changes in a nucleotide sequence at the site of mutagenesis. For example, saturation mutagenesis uses primers with one or more degenerate codons (*e*.*g*., a NNN, a NNK or a NNS codon) that codes for all possible amino acid substitutions at one or more sites in the parent nucleic sequence. Such degenerate codons may code for 32 to 64 unique codons which collectively may encode 20 amino acids in a redundant fashion (*e*.*g*., multiple codons for the same amino acid) and a stop codon. For example, given the degeneracy of the genetic code some amino acid residues may be overrepresented *(e.g.,* Arg, Leu, and Ser). This technique depends on screening a large number of variants and may require multiple large libraries using phage or ribosomal display to explore the variants. Another technique, error prone PCR uses polymerase to introduce mutations at random positions in a parent nucleic acid sequence. This technique may introduce mutations outside of an area of interest (*e*.*g*., a binding pocket such as a CDR) and necessitate backmutation to identify a productive mutation. Accordingly, methods for mutagenesis are desired that produce manageable libraries comprising targeted mutations at one or more positions in a parent nucleic acid sequence.

### SUMMARY

One aspect of the present invention refers to a method of mutagenesis of a parent nucleic acid encoding a protein to generate modified proteins, said method comprising:
(a.) obtaining a primer set comprising primers that each comprise at least one 2 to 12 fold degenerate codon, wherein each primer comprises at least two oligonucleotide sequences that are complementary to a sequence in the parent nucleic acid, and wherein the degenerate codons non-redundantly code for an equal representation of eighteen amino acid substitutions with the exception of cysteine or methionine at each of one or more amino acid position(s) encoded by the parent nucleic acid; and
(b.) mutating the parent nucleic acid by replication or polymerase based amplification using the primer set obtained in (a), wherein replication or amplification of the parent nucleic acid with the primers generate mutated nucleic acids that encode said equal representation of eighteen amino acid substitutions in modified proteins.

A second aspect of the present invention relates to a set of primers that each comprise at least one 2 to 12 fold degenerate codon, wherein each primer comprises at least two oligonucleotide sequences that are complementary to a sequence in a parent nucleic acid, and wherein the degenerate codons non-redundantly code for an equal representation of eighteen amino acid substitutions with the exception of cysteine or methionine at each of one or more amino acid position(s) encoded by the parent nucleic acid.

A further aspect of the invention is directed to a kit for mutagenesis of an amino acid residue encoded by a parent nucleic acid, the kit comprising: a set of primers that each comprise at least one 2 to 12 fold degenerate codon, wherein each primer comprises at least two oligonucleotide sequences that are complementary to a sequence in a parent nucleic acid, and wherein the degenerate codons non-redundantly code for an equal representation of eighteen amino acid substitutions with the exception of cysteine or methionine at each of one or more amino acid position(s) encoded by the parent nucleic acid.

The present disclosure relates to methods and materials for mutagenesis, including for the generation of novel or improved proteins and libraries or arrays of mutant proteins or nucleic acids encoding such mutant proteins. The present disclosure provides methods and materials for targeted mutagenesis of proteins, including by mutating one or more selected positions in a parent nucleic acid sequence. The proteins targeted for mutagenesis can be natural, synthetic or engineered proteins or variants (e.g., mutants of such proteins). The proteins can include binding proteins such as antibodies or their binding fragments and ligands or receptors. The proteins can also include enzymes or catalytic molecules.

The present disclosure provides methods of mutagenesis of a parent nucleic acid sequence encoding a protein by obtaining one or more primers that each comprise at least one 2 to 12 fold degenerate codon, wherein the primers are complementary to a sequence in the parent nucleic acid sequence and wherein the primers code for amino acid mutations at each of one or more amino acid positions encoded by the parent nucleic acid sequence; and subjecting the parent nucleic acid sequence to replication or polymerase based amplification using the obtained primers, wherein replication or amplification of the parent nucleic acid sequence with the primers generates variant nucleic acid sequences and wherein the variant nucleic acid sequences comprise amino acid mutations at the one or more positions in the parent nucleic acid sequence with the exception of cysteine and methionine.

The present disclosure provides methods of mutagenesis of a parent nucleic acid encoding a protein to generate modified proteins by obtaining one or more primers that each comprise at least one 2 to 12 fold degenerate codon, wherein each primer comprises at least two oligonucleotide sequences that are complementary to a sequence in the parent nucleic acid and code for an amino acid mutation with the exception of cysteine or methionine at one amino acid position encoded by the parent nucleic acid; and mutating the parent nucleic acid by replication or polymerase based amplification using the one or more obtained primers, wherein replication or amplification of the parent nucleic acid with the one or more primers generates mutated nucleic acids that encode modified proteins.

The present disclosure also provides libraries/arrays of mutated nucleic acid sequences generated by the methods of the present disclosure.

The present disclosure also provides methods for mutagenesis of a protein to obtain modified proteins with mutated amino acid sequences by identifying one or more amino acid positions in the protein for mutagenesis; substituting one or more of the identified amino acid residues in the protein with other amino acid residues excluding cysteine and methionine to generate a library or an array of modified proteins with mutated amino acid sequences; screening the library or array of modified proteins in an assay for a biological activity of the protein; and obtaining modified proteins having the biological activity of the protein.

The present disclosure also provides methods for generating an array of nucleic acids encoding modified proteins by obtaining a collection of nucleic acids encoding modified proteins containing amino acid mutations other than cysteine and methionine at amino acid residues of a parent protein sequence by mutagenesis of a nucleic acid encoding the protein sequence using primers that each comprise at least one 2 to 12 fold degenerate codon; sequencing the collection of nucleic acids encoding the modified proteins; and arranging each sequenced nucleic acid encoding a modified protein to generate an array of nucleic acid sequences each encoding a modified protein.

The present disclosure also provides methods for generating an array of nucleic acid sequences encoding modified proteins by preparing a plurality of nucleic acid sequences by mutagenesis that encode a plurality of modified proteins that vary from a parent protein sequence at one or more amino acid positions and contain one of eighteen different amino acids excluding cysteine and methionine at each position mutated from the parent protein sequence; and arranging each prepared nucleic acid sequence to generate an array of nucleic acid sequences each encoding a modified protein.

The present disclosure also provides libraries/arrays of mutated nucleic acid sequences generated by the method of the present disclosure.

The present disclosure also provides methods for generating an array of clones comprising nucleic acids encoding modified proteins by preparing a plurality of nucleic acids by mutagenesis that encode a plurality of modified proteins that vary from a parent protein sequence at one or more amino acid positions and contain one of eighteen different amino acids excluding cysteine and methionine at each position varied from the parent protein sequence; transfecting the prepared nucleic acids prepared into host cells and selecting clones comprising the transfected nucleic acids; and arranging each selected clone to generate an array of clones with each arrayed clone capable of expressing a modified protein.

The present disclosure also provides methods of producing a nucleic acid library with an equal representation of non-redundant amino acid changes at an amino acid position encoded by a parent nucleic acids by providing a set of primers that each comprise at least one degenerate codon, wherein each primer comprises at least two oligonucleotide sequence that are complementary to a sequence in the parent nucleic acid and code for an amino acid mutation with the exception of cysteine and methionine at one amino acid position encoded by the parent nucleic acid, wherein the primers code for an equal representation of non-redundant amino acid changes at the one position; hybridizing a primer from the set to the parent nucleic acid; replicating or amplifying the parent nucleic acid molecule with the primer to generate nucleic acids that code for amino acid changes at the one position; repeating the hybridizing and replicating steps with each remaining primer from the set; pooling the nucleic acids produced with each primer; and obtaining a library of nucleic acids coding for an equal representation of amino acid changes at the one position.

The present disclosure also provides methods for obtaining a nucleic acid sequence with an improvement in comparison to a parent nucleic acid sequence with respect to at least one molecular or biological property of interest by obtaining a set of primers that each comprise at least one 2 to 12 fold degenerate codon that does not code for cysteine and methionine, wherein the primers are complementary to a sequence in the parent nucleic acid sequence and wherein the primers code for non-redundant amino acid mutations at one amino acid position encoded by the parent nucleic acid sequence; mutating the parent nucleic acid sequence by replication or polymerase based amplification using the obtained set of primers to generate variant nucleic acid sequences; producing a library or array of variant nucleic acid sequences from (b) coding for amino acid mutations at the one position in the parent nucleic acid sequence; and screening the library or array of variant nucleic acid sequences to identify nucleic acid sequences that have a desirable improvement in comparison with the parent nucleic acid sequence with respect to at least one molecular or biological property of interest.

The present disclosure also provides methods of making modified proteins with mutated amino acid sequences by modifying the amino acid sequence of a protein to produce amino acid mutations at an amino acid residue in the protein to generate a library or an array of modified proteins with mutated amino acid sequences, wherein the amino acid mutations exclude cysteine and methionine; and selecting modified proteins from the library or the array that have a biological activity of an unmodified protein.

The present disclosure also provides methods for selecting modified proteins with mutated amino acid sequences by obtaining a library or an array of modified proteins comprising amino acid mutations at one amino acid residues in an amino acid sequence of a protein, wherein the amino acid mutations exclude cysteine and methionine; assaying the modified proteins for a biological activity of an unmodified protein; and selecting the modified proteins that have a biological activity of the unmodified protein.

The present disclosure also provides a set of primers that each comprise at least one 2 to 12 fold degenerate codon, wherein each primer comprises at least two oligonucleotide sequences that are complementary to a sequence in a parent nucleic acid and code for an amino acid mutation with the exception of cysteine or methionine at one amino acid position encoded by the parent nucleic acid.

The present disclosure also provides a kit for mutagenesis of an amino acid residue encoded by a parent nucleic acid comprising a set of primers that each comprise at least one 2 to 12 fold degenerate codon, wherein each primer comprises at least two oligonucleotide sequences that are complementary to a sequence in a parent nucleic acid and code for an amino acid mutation with the exception of cysteine or methionine at one amino acid position encoded by the parent nucleic acid.

The present disclosure also provides libraries/arrays comprising variants of a protein sequence, wherein the variants each comprise an amino acid mutation at one amino acid position in the protein sequence of a parent protein and wherein the amino acid mutations are not cysteine or methionine.

The present disclosure also provides methods for obtaining a nucleic acid sequence with an improvement in comparison to a parent nucleic acid sequence with respect to at least one molecular or biological property of interest by mutating the parent nucleic acid by polymerase based amplification using one or more primers that each comprise at least one 2 to 12 fold degenerate codon to generate mutated nucleic acid sequences, wherein each primer comprises at least two oligonucleotide sequences that are complementary to a sequence in the parent nucleic acid and code for an amino acid mutation with the exception of cysteine or methionine at one amino acid position encoded by the parent nucleic acid; sequencing the mutated nucleic acid sequences; arranging each sequenced mutated nucleic acid sequence comprising one amino acid mutation to generate an array of mutated nucleic acid sequences; and screening the array of variant nucleic acid sequences to identify nucleic acid sequences that have a desirable improvement in comparison with the parent nucleic acid sequence with respect to at least one molecular or biological property of interest.

In some embodiments, the amino acid mutations are selected from the group consisting of: alanine, arginine, asparagine, aspartic acid, glutamine, glutamine acid, glycine, histidine, isoleucine, leucine, lysine, phenylalanine, proline, serine, threonine, tryptophan, tyrosine and valine at each position.

In some embodiments, the primers code for eighteen amino acid mutations at the one amino acid position encoded by the parent nucleic acid.

In some embodiments, three primers that each comprise at least one 2 to 12 fold degenerate codon are obtained. In some embodiments, seven primers that each comprise at least one 2 to 12 fold degenerate codon are obtained. In some embodiments, the degenerate codons are selected from the group consisting of: NHT or NHC (where N=A/G/C/T, H=A/C/T), VAG or VAA (where V=A/C/G) and BGG or DGG (where B=C/G/T, D=A/G/T). In some embodiments, the degenerate codons are selected from the group consisting of: ARG (where R=A/G), WMC (where W=A/T and M=A/C), CAS (where S=C/G), GAS (where S=C/G), NTC (where N=A/G/C/T), KGG (where K=G/T) and SCG (where S=C/G).

In some embodiments, the primers code for basic amino acid mutations at the one amino acid position encoded by the parent nucleic acid. In some embodiments, one primer that comprises at least one 2 to 12 fold degenerate codon is obtained. In some embodiments, the one primer comprises a degenerate codon which codes for arginine and lysine. In some embodiments, the degenerate codon is represented by ARG (where, R=A/G).

In some embodiments, the primers code for polar amino acid mutations at the one amino acid position encoded by the parent nucleic acid. In some embodiments, two primers that comprise at least one 2 to 12 fold degenerate codon is obtained. In some embodiments, the two primers comprise degenerate codons that collectively code for serine, threonine, asparagine and tyrosine. In some embodiments, the degenerate codons are represented by WMC (where, W=A/T; M=A/C) and CAS (where S=C/G).

In some embodiments, the primers code for acidic amino acid mutations at the one amino acid position encoded by the parent nucleic acid. In some embodiments, one primer that comprises at least one 2 to 12 fold degenerate codon is obtained. In some embodiments, the one primer comprises a degenerate codon that codes for glutamic acid and aspartic acid. In some embodiments, the degenerate codon is represented by GAS (where S=C/G).

In some embodiments, the primers code for non-polar amino acid mutations at the one amino acid position encoded by the parent nucleic acid. In some embodiments, three primers that comprise at least one 2 to 12 fold degenerate codon are obtained. In some embodiments, the three primers comprise degenerate codons that collectively code for glutamic acid and aspartic acid. In some embodiments, the degenerate codons are represented by NTC (where, N=A/G/C/T), KGG (where, K=G/T), and SCG (where S=C/G).

In some embodiments, the parent nucleic acid encodes a binding molecule. In some embodiments, the binding molecule is an antibody or fragment thereof.

In some embodiments the methods may further comprise selecting the one or more positions in the parent nucleic acid sequence for mutation. In some embodiments the methods may further comprise transforming the mutated nucleic acid sequences into competent cells.

In some embodiments, the step of substituting is performed with one or more primers that each comprise at least one 2 to 12 fold degenerate codon, wherein each primer comprises at least two oligonucleotide sequences that are complementary to a sequence in a parent nucleic acid and code for an amino acid substitution with the exception of cysteine and methionine at one amino acid position encoded by the parent nucleic acid. In some embodiments, the step of modifying is performed with one or more primers that each comprise at least one 2 to 12 fold degenerate codon, wherein each primer comprises at least two oligonucleotide sequences that are complementary to a sequence in a parent nucleic acid and code for an amino acid substitution with the exception of cysteine and methionine at one amino acid position encoded by the parent nucleic acid.

In some embodiments, the biological property of interest is binding.

In some embodiments, modified proteins are selected that have increased activity as compared to the unmodified protein. In some embodiments, modified proteins are selected that have decreased activity as compared to the unmodified protein. In some embodiments, modified proteins are selected that have equal activity as compared to the unmodified protein.

Additional features and advantages are described herein, and will be apparent from, the following Detailed Description and the Figures.

### BRIEF DESCRIPTION OF THE FIGURES

The Figures relate to exemplary proteins, including those useful for mutagenesis according to methods and materials disclosed herein.

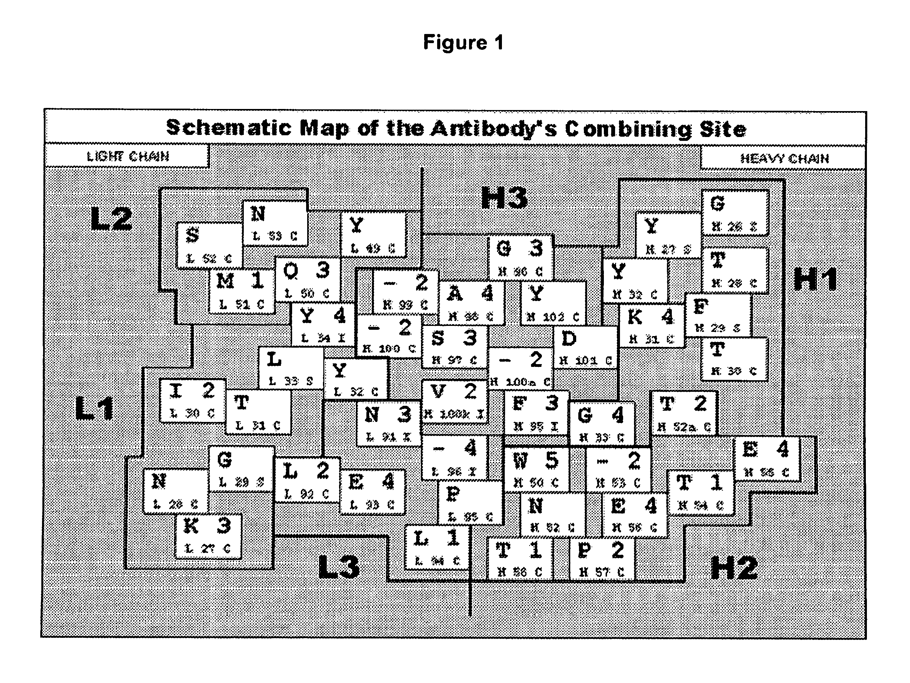

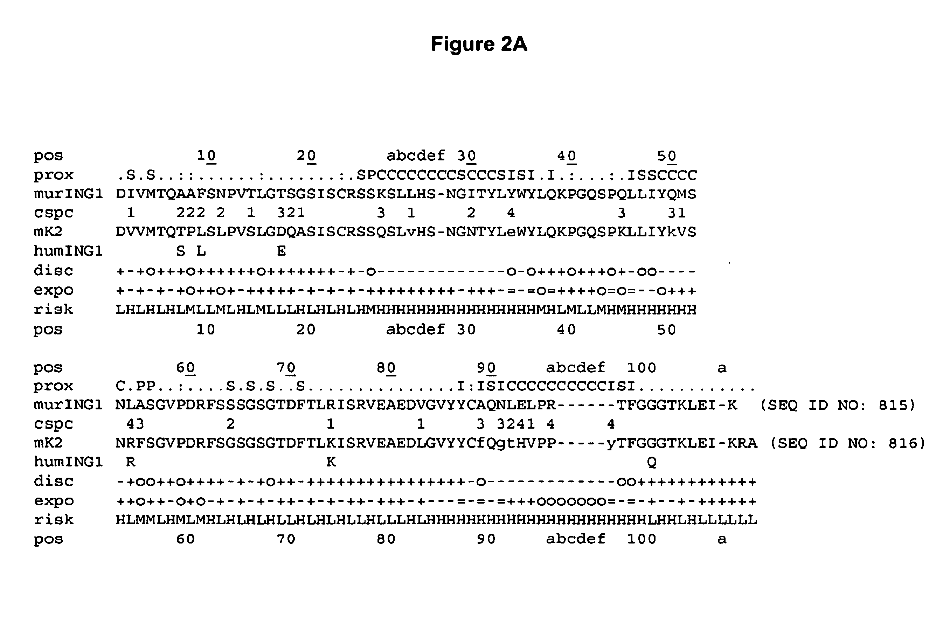

Figure 1 is a generalized schematic map of an exemplary antibody combining site as described herein, looking downward onto the "top" surface of a variable domain comprising a light chain variable region and a heavy chain variable region. It shows the six CDR loops (L1, L2, L3, H1, H2, H3) which are spatially located directly above the three-dimensional structure of the evolutionarily-conserved framework underneath. As shown and discussed herein, this map provides roughly approximate higher-order structural information, which is not available from the linear primary sequence alone, such as the identity of potential nearest neighbors in the space-filling model of a generic variable domain. Specific features of the murine ING1 monoclonal antibody have been added to this map, so that it can also call attention to localized domains of the antibody's combining site containing clusters of high-conspicuousness positions as described herein, which are likely to be in contact with sidechains on the antigen. In particular, each amino-acid position in the murine ING1 antibody is represented on this map by a white rectangle containing a group of symbols. The letter and number at the bottom-left of each rectangle (e.g., "H 98" in CDR-loop H3) is the Kabat-position number of the amino-acid residue on the antibody molecule within either chain (L=light, H=heavy). The small upper-case letter (e.g., "B") at the bottom-right is a designation for the residue's proximity as described herein (C=Contacting, P=Peripheral, S=Supporting, I=Interfacial) relative to the antibody's binding site (shown on the "prox" line in Figures 2A-2D). The large upper-case letter (*e.g*., "A") at the upper-left is the amino-acid code for the residue's sidechain (line "murING1" in Figures 2A-2D). The large single digit at the upper right (e.g., "3") in some rectangles is the non-zero conspicuousness-value as described herein of affinity enhancement for the sidechain (line "cspc" in Figures 2A-2D), calculated in reference to the appropriate human consensus sequence for light chain (hK2) or heavy chain (hH 1). Rectangles with no such value reflect a conspicuousness of zero.

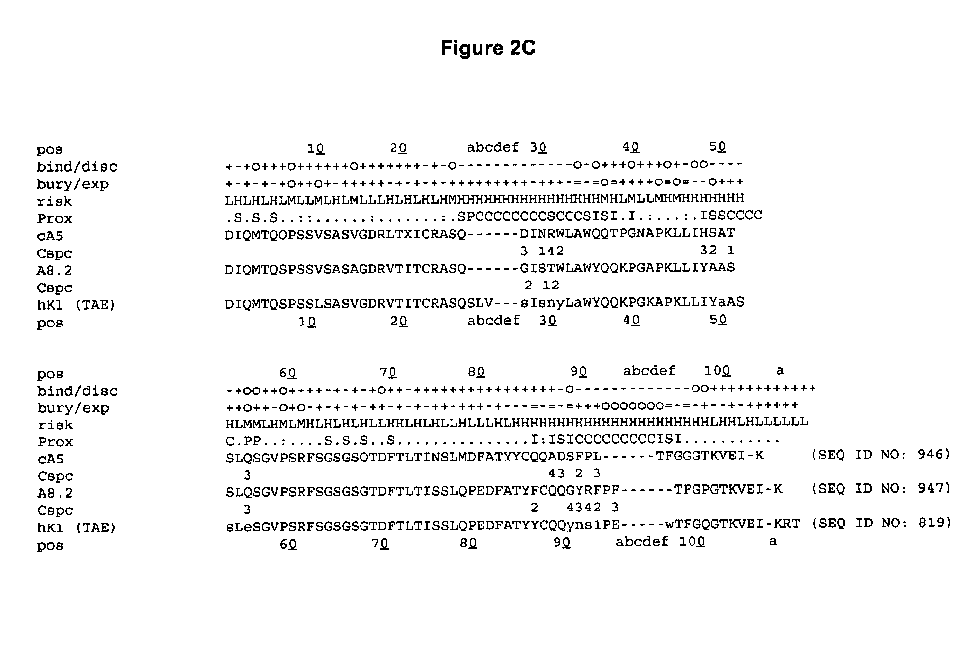

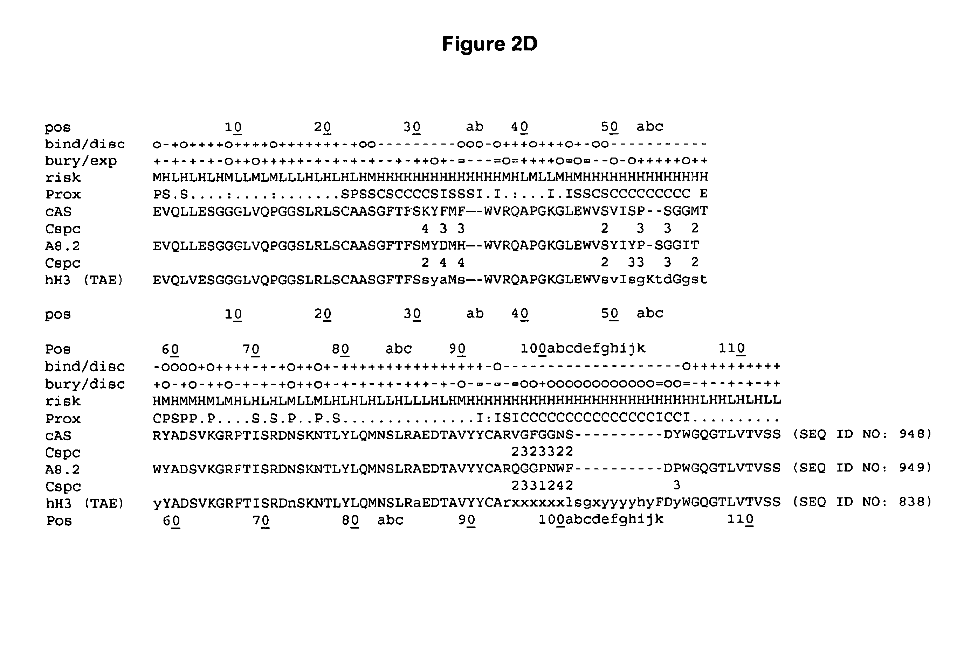

Figures 2A-2D: Alignments of sequences in the light chain and heavy chain, with lines (e.g., prox, cspc) relating to affinity enhancement and lines relating to human engineering (e.g., risk) are shown. In each set of lines, the top ones apply the present disclosure to the murine ING1 antibody (2A-2B), and the bottom ones relate the present disclosure to the general principles of human engineering (Studnicka et al., Protein Engineering, 7(6):805-814 (1994); U.S. Patent No. 5,766,886). Each set of lines shows the Kabat position numbers (pos), the general classification of proximity groups for each position of every antibody (prox), the murine ING1 monoclonal antibody sequence to be affinity-enhanced (murINGI), the conspicuousness value as described herein of each position for affinity-enhancement when the murine ING1 antibody is compared to murine consensus sequences (cspc), several murine consensus sequences to which ING1 is compared (mK2 or mH2a), the human ING1 residues which are introduced during the HUMAN ENGINEERING™ process (humING1), the degree of disconnection of the sidechain from the antibody's combining site (disc) as described herein, the degree of outward-orientation of the sidechain on the antibody's surface (outw) as described herein, the degree of risk for human engineering (risk), and the Kabat position numbers (pos) (2A-2B). Similarly, Figures 2C and 2D are alignments of sequences in the light chain and heavy chain of IL-1 antibody (also referred to as cA5 and/or XPA23), with lines (*e*.*g*., prox, cspc) relating to affinity enhancement and lines relating to human engineering (*e*.*g*., risk).
Figures 3A-3D are mutual alignments of consensus sequences (Kabat et al. (1991) (eds), Sequences of Proteins of Immunological Interest, 5th ed.) for major murine and human subgroups of the light chain and heavy chain. Each alignment relates them to the proximity groups as described herein for each position (prox), and the Kabat position numbers (pos).
Figure 4 shows a chart of the numerical components which can be added together to calculate each amino acid's affinity-enhancement conspicuousness value, including the components for changes in class-and-charge, for changes in physical size due to somatic mutation, and for repeated identical mutations at the same position in multiple homologous antibodies.
Figure 5 shows PCR mutagenesis of CDR3 utilizing the CDR-H3 oligonucleotide H3-3NP2 (SEQ ID NO: 267): 5'-GCTACATATTTCTGTGCAAGATTTG GCTCTKGGGTGGACTACTGGGGTCAAGG-3', which introduces an amino acid substitution into CDR3, and the reverse primer Notl-R (SEQ ID NO: 285): 5'-AGCGGCCGCACAAGATTTGGGCTCAACTCTC-3', which incorporates the *Not*I restriction site into the PCR product.
Figure 6 depicts the plasmid map of the pXOMA-gIII-Fab vector. The vector is 5,202 base pairs in length and has *Asc*I and *Not*I restriction sites flanking the heavy chain encoding sequences, and *Hind*III and *Asc*I restriction sites flanking the light chain encoding sequences. The heavy chain encoding sequences are fused to pIII encoding sequences in the vector. The pXOMA-Fab vector is similar but lacks the pIII encoding sequences.
Figure 7 depicts the strategy for creating the light chain combination variants.
Figure 8 depicts the strategy for creating the heavy chain combination variants.

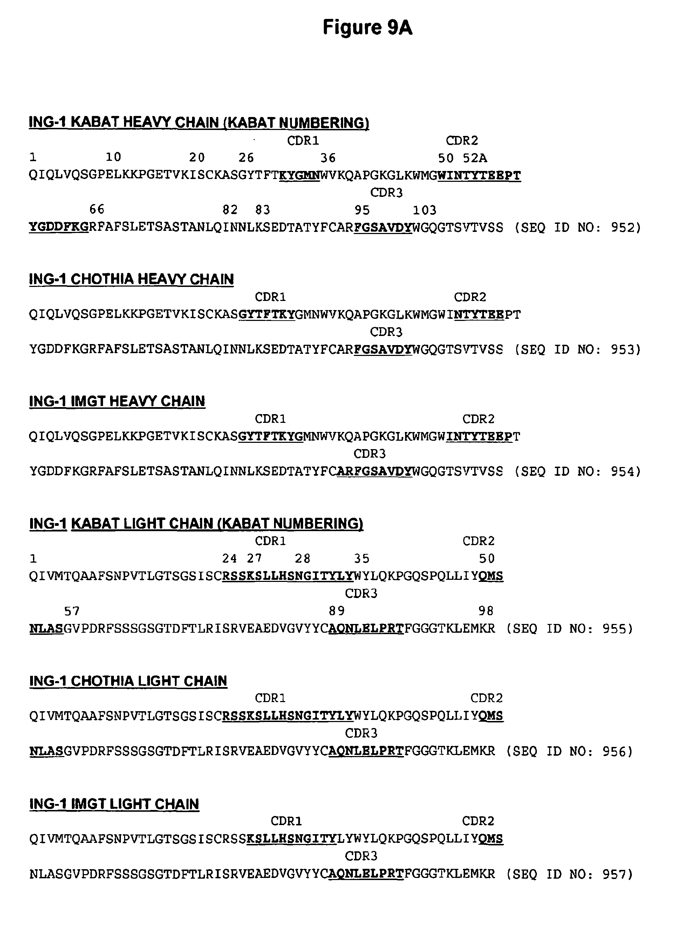

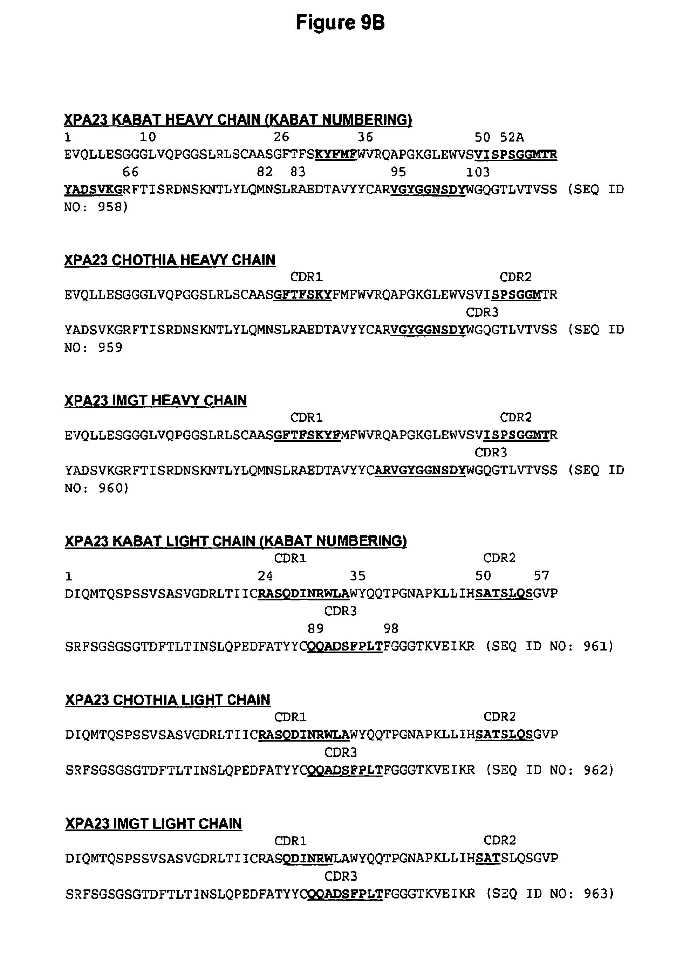

Figure 9A-9B shows CDR1, CDR2 and CDR3 as identified by the Kabat, Chothia and IMGT numbering scheme for ING-1 (9A) and XPA23 (9B).

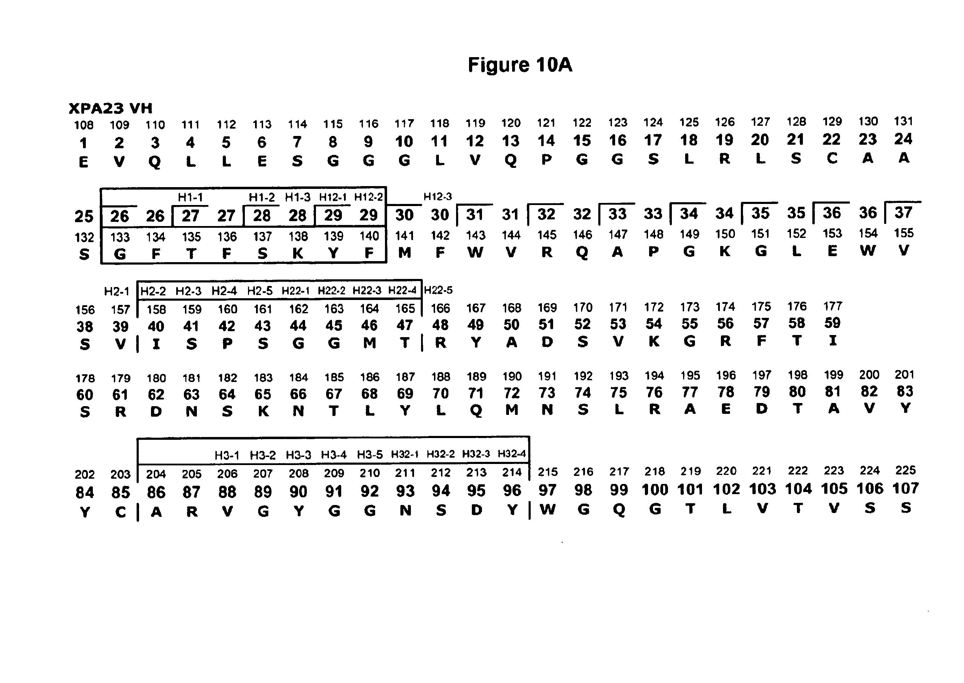

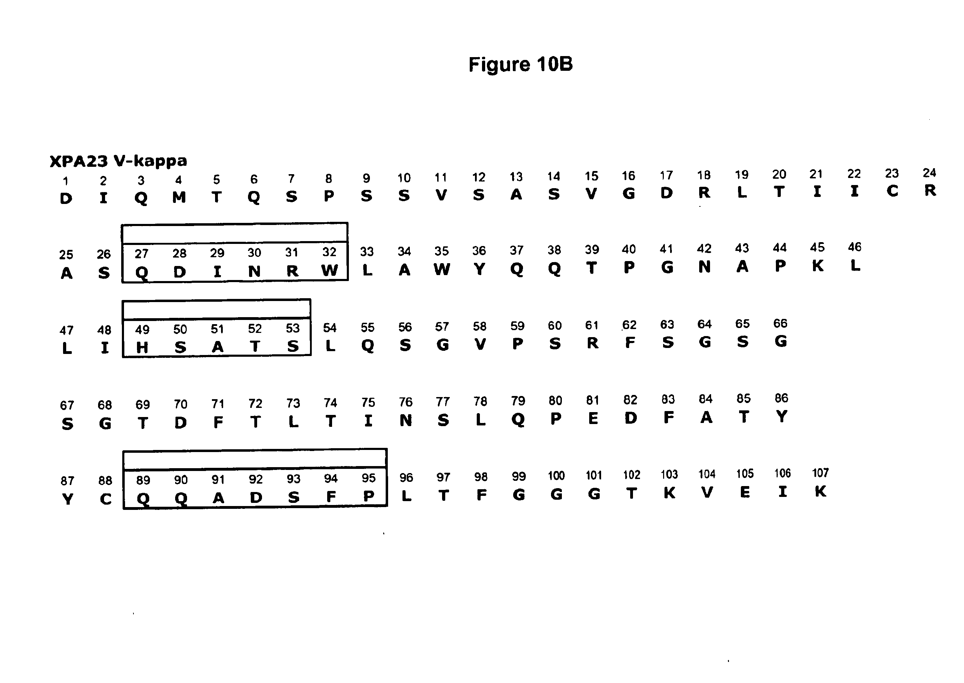

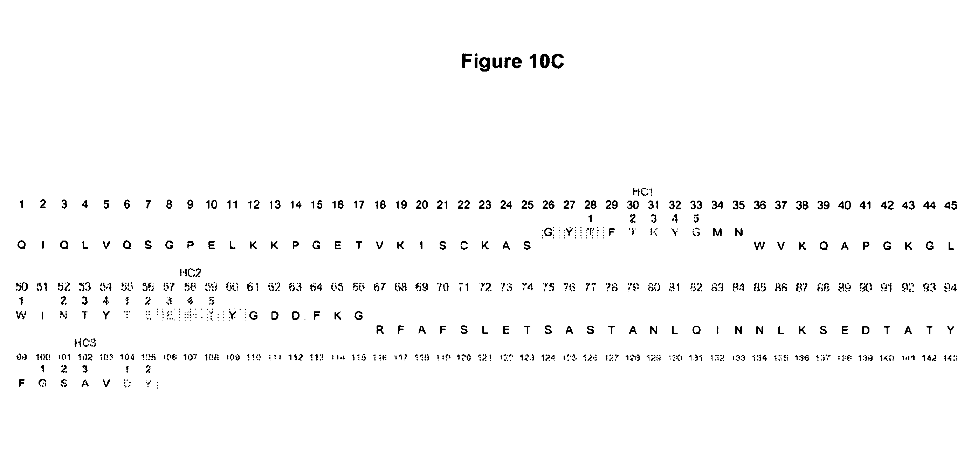

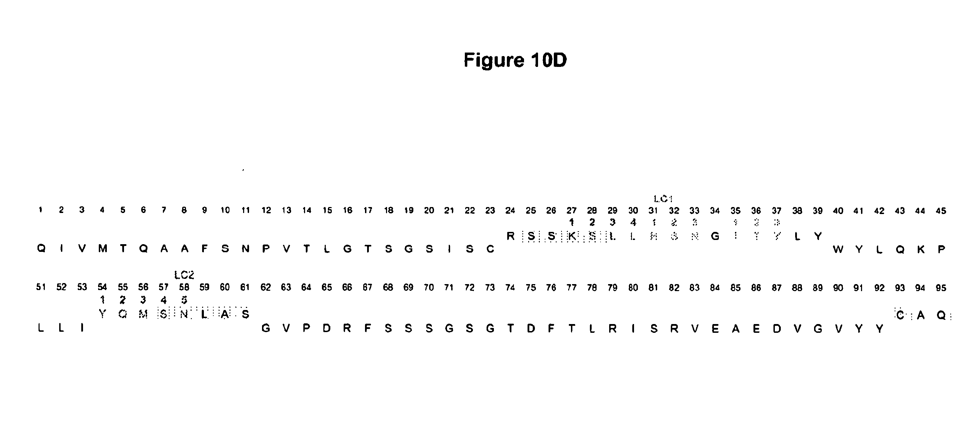

Figure 10A-10D depict a continuous numbering scheme for the heavy and light chain of XPA23 (10A and 10B, respectively). Consecutive numbering from position 1 in the light chain continues in the heavy chain such that position 1 in the heavy chain is also assigned number 108 since the light chain sequence ends at number 107. Boxed residues indicate CDRs identified by the IMGT method. Figures 10C and 10D show a continuous numbering scheme for the heavy and light chain of ING-1 (10C and 10D, respectively).
Figure 11: Periplasmic extracts of clones containing one of the eighteen preferred amino acid mutations at Heavy Chain contacting positions in ING-1 were tested on Biacore for improved off-rate (see example 7). Clones with greater than 1.9-fold decrease in off-rate are listed.
Figure 12: Periplasmic extracts of clones containing one of the eighteen preferred amino acid mutations at Light Chain contacting positions in ING-1 were tested on Biacore for improved off-rate (see example 7). Clones with greater than 1.9-fold decrease in off-rate are listed.
Figure 13: Periplasmic extracts of clones containing one of the eighteen preferred amino acid mutations at Heavy Chain contacting positions in XPA23 were tested on Biacore for improved off-rate (see example 7). Clones with greater than 1.9-fold decrease in off-rate are listed.
Figure 14: Periplasmic extracts of clones containing one of the eighteen preferred amino acid mutations at Light Chain contacting positions in XPA23 were tested on Biacore for improved off-rate (see example 7). Clones with greater than 1.9-fold decrease in off-rate are listed.
Figure 15A-15D depicts two modified IgGs with an A102F or 102G substitution that were prepared and evaluated by Biacore with improved affinity (15B-15C, respectively) as compared to the parental (15A) ING-1 antibody. 15D shows the affinity determination kinetics for both the modified and parental ING-1 antibodies.
Figure 16A-16C are sensogram profiles depicting ING-1 light chain binding to Ep-Cam.

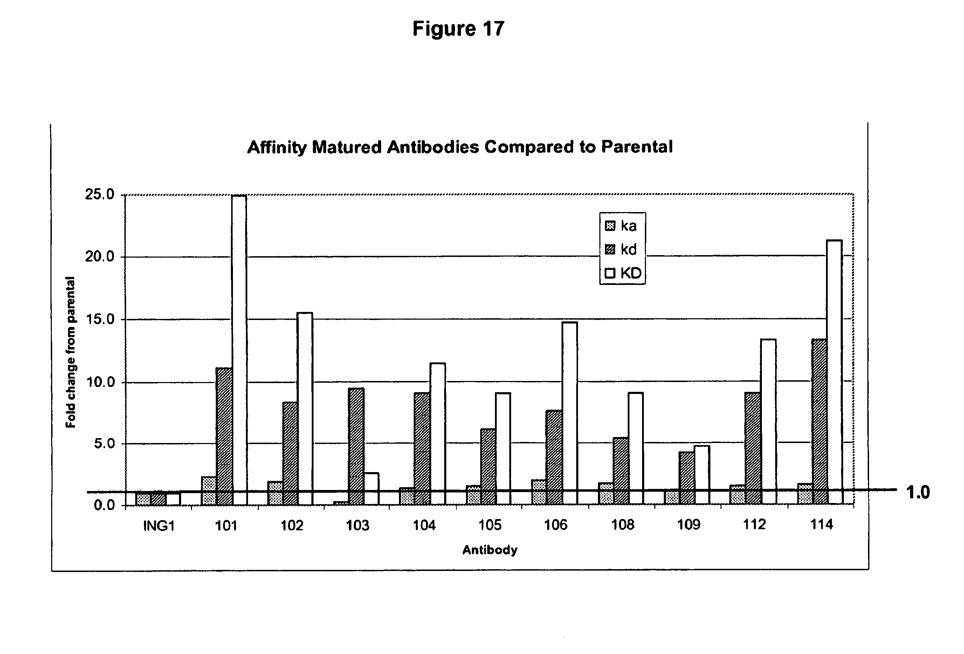

Figure 17 depicts modified ING-1 antibodies each comprising two or more heavy chain mutations as compared to the parental antibody. Combinations of heavy chain mutations yield affinity improvements up to 25-fold over the parental ING-1 antibody. Affinity improvements are driven largely by improvements in k_{off}.
Figure 18 shows amino acid substitutions at position 32 in the light chain variable region of XPA23. Generally the substitutions at position 30 decreased kd of the antibody-antigen interaction compared to the parental antibody.
Figure 19 shows amino acid substitutions at position 30 in the light chain variable region of XPA23. Generally the substitutions at position 30 resulted in a comparable kd of the antibody-antigen interaction compared to the parental antibody.
Figure 20 shows amino acid substitutions at position 45 in the heavy chain variable region of XPA23. Generally the substitutions improved kd of the antibody-antigen interaction at this position compared to the parental antibody.

### DETAILED DESCRIPTION

The present disclosure provides methods and materials for targeted mutagenesis of one or more selected positions in a parent nucleic acid sequence (e.g., a nucleotide sequence coding for an antibody or binding fragment thereof such as an IgG, Fab or ScFv). When the parent nucleic acid sequence encodes an antibody variable region, preferred positions for selection and mutagenesis are those encoding one of more CDR amino acid residues. Particularly preferred is mutation of each of the CDR residues in a heavy and/or light chain variable region. Techniques for site-directed mutagenesis of a nucleotide sequence rely on using degenerate codons including, for example, NNK or NNS. However, these degenerate codons may code for an overrepresentation of one or more amino acid residues which may result in the production of a large nucleotide library that does not contain all possible amino acid substitutions at a position of interest. The present disclosure provides novel degenerate codons that encode for an equal representation of eighteen amino acid residues including a stop codon. Notably, the degenerate codons may not code for cysteine and/or methionine. Additionally, the novel degenerate codons each collectively code for eighteen amino acid residues eliminating any redundancy which may result in an over-representation of one or more amino acid residues. As a result, the degenerate codons of the present disclosure allow for the generation of smaller, focused libraries that contain eighteen amino acid substitutions at a position of interest.

The present disclosure provides methods of mutagenesis of a parent nucleic acid sequence encoding a protein (e.g., an antibody or binding fragment thereof such as an IgG, Fab or scFv) by obtaining one or more primers that each comprise at least one 2 to 12 fold degenerate codon (e.g., NHT or NHC (where N=A/G/C/T, H=A/C/T), VAG or VAA (where V=A/C/G) and BGG or DGG (where B=C/G/T, D=A/G/T), ARG (where R=A/G), WMC (where W=A/T and M=A/C), CAS (where S=C/G), GAS (where S=C/G), NTC (where N=A/G/C/T), KGG (where K=G/T) and SCG (where S=C/G)), wherein the primers are complementary to a sequence in the parent nucleic acid sequence and wherein the primers code for amino acid mutations at each of one or more amino acid positions encoded by the parent nucleic acid sequence; and subjecting the parent nucleic acid sequence to replication or polymerase based amplification using the obtained primers, wherein replication or amplification of the parent nucleic acid sequence with the primers generates variant nucleic acid sequences and wherein the variant nucleic acid sequences comprise amino acid mutations at the one or more positions in the parent nucleic acid sequence with the exception of cysteine and methionine. Optionally, two or more mutations may be combined by recombinant DNA techniques into a single mutated protein.

The present disclosure provides methods of mutagenesis of a parent nucleic acid encoding a protein (*e*.*g*., an antibody or binding fragment thereof such as an IgG, Fab or scFv) to generate modified proteins by obtaining one or more primers that each comprise at least one 2 to 12 fold degenerate codon (*e*.*g*., NHT or NHC (where N=A/G/C/T, H=A/C/T), VAG or VAA (where V=A/C/G) and BGG or DGG (where B=C/G/T, D=A/G/T), ARG (where R=A/G), WMC (where W=A/T and M=A/C), CAS (where S=C/G), GAS (where S=C/G), NTC (where N=A/G/C/T), KGG (where K=G/T) and SCG (where S=C/G)), wherein each primer comprises at least two oligonucleotide sequences that are complementary to a sequence in the parent nucleic acid and code for an amino acid mutation with the exception of cysteine or methionine at one amino acid position encoded by the parent nucleic acid; and mutating the parent nucleic acid by replication or polymerase based amplification using the one or more obtained primers, wherein replication or amplification of the parent nucleic acid with the one or more primers generates mutated nucleic acids that encode modified proteins.

The present disclosure also provides libraries/arrays of mutated nucleic acid sequences generated by the methods of the present disclosure.

The present disclosure also provides methods for mutagenesis of a protein (*e*.*g*., an antibody or binding fragment thereof such as an IgG, Fab or scFv) to obtain modified proteins with mutated amino acid sequences by identifying one or more amino acid positions in the protein for mutagenesis; substituting one or more of the identified amino acid residues in the protein with other amino acid residues excluding cysteine and methionine to generate a library or an array of modified proteins with mutated amino acid sequences; screening the library or array of modified proteins in an assay for a biological activity of the protein; and obtaining modified proteins having the biological activity of the protein.

The present disclosure also provides methods for generating an array of nucleic acids encoding modified proteins (*e*.*g*., an antibody or binding fragment thereof such as an IgG, Fab or scFv) by obtaining a collection of nucleic acids encoding modified proteins containing amino acid mutations other than cysteine and methionine at amino acid residues of a parent protein sequence by mutagenesis of a nucleic acid encoding the protein sequence using primers that each comprise at least one 2 to 12 fold degenerate codon (*e*.*g*., NHT or NHC (where N=A/G/C/T, H=A/C/T), VAG or VAA (where V=A/C/G) and BGG or DGG (where B=C/G/T, D=A/G/T), ARG (where R=A/G), WMC (where W=A/T and M=A/C), CAS (where S=C/G), GAS (where S=C/G), NTC (where N=A/G/C/T), KGG (where K=G/T) and SCG (where S=C/G)); sequencing the collection of nucleic acids encoding the modified proteins; and arranging each sequenced nucleic acid encoding a modified protein to generate an array of nucleic acid sequences each encoding a modified protein.

The present disclosure also provides methods for generating an array of nucleic acid sequences encoding modified proteins (*e*.*g*., an antibody or binding fragment thereof such as an IgG, Fab or scFv) by preparing a plurality of nucleic acid sequences by mutagenesis that encode a plurality of modified proteins that vary from a parent protein sequence at one or more amino acid positions and contain one of eighteen different amino acids excluding cysteine and methionine at each position mutated from the parent protein sequence; and arranging each prepared nucleic acid sequence to generate an array of nucleic acid sequences each encoding a modified protein.

The present disclosure also provides libraries/arrays of mutated nucleic acid sequences generated by the method of the present disclosure.

The present disclosure also provides methods for generating an array of clones comprising nucleic acids encoding modified proteins (*e*.*g*., an antibody or binding fragment thereof such as an IgG, Fab or scFv) by preparing a plurality of nucleic acids by mutagenesis that encode a plurality of modified proteins that vary from a parent protein sequence at one or more amino acid positions and contain one of eighteen different amino acids excluding cysteine and methionine at each position varied from the parent protein sequence; transfecting the prepared nucleic acids prepared into host cells and selecting clones comprising the transfected nucleic acids; and arranging each selected clone to generate an array of clones with each arrayed clone capable of expressing a modified protein.

The present disclosure also provides methods of producing a nucleic acid library with an equal representation of non-redundant amino acid changes at an amino acid position encoded by a parent nucleic acids by providing a set of primers that each comprise at least one degenerate codon (e.g., NHT or NHC (where N=A/G/C/T, H=A/C/T), VAG or VAA (where V=A/C/G) and BGG or DGG (where B=C/G/T. D=A/G/T), ARG (where R=A/G), WMC (where W=A/T and M=A/C), CAS (where S=C/G), GAS (where S=C/G), NTC (where N=A/G/C/T), KGG (where K=G/T) and SCG (where S=C/G)), wherein each primer comprises at least two oligonucleotide sequence that are complementary to a sequence in the parent nucleic acid and code for an amino acid mutation with the exception of cysteine and methionine at one amino acid position encoded by the parent nucleic acid, wherein the primers code for an equal representation of non-redundant amino acid changes at the one position; hybridizing a primer from the set to the parent nucleic acid; replicating or amplifying the parent nucleic acid molecule with the primer to generate nucleic acids that code for amino acid changes at the one position; repeating the hybridizing and replicating steps with each remaining primer from the set; pooling the nucleic acids produced with each primer; and obtaining a library of nucleic acids coding for an equal representation of amino acid changes at the one position.

The present disclosure also provides methods for obtaining a nucleic acid sequence with an improvement in comparison to a parent nucleic acid sequence with respect to at least one molecular or biological property of interest by obtaining a set of primers that each comprise at least one 2 to 12 fold degenerate codon (*e*.*g*., NHT or NHC (where N=A/G/C/T, H=A/C/T), VAG or VAA (where V=A/C/G) and BGG or DGG (where B=C/G/T, D=A/G/T), ARG (where R=A/G), WMC (where W=A/T and M=A/C), CAS (where S=C/G), GAS (where S=C/G), NTC (where N=A/G/C/T), KGG

(where K=G/T) and SCG (where S=C/G)) that does not code for cysteine and methionine, wherein the primers are complementary to a sequence in the parent nucleic acid sequence and wherein the primers code for non-redundant amino acid mutations at one amino acid position encoded by the parent nucleic acid sequence; mutating the parent nucleic acid sequence by replication or polymerase based amplification using the obtained set of primers to generate variant nucleic acid sequences; producing a library or array of variant nucleic acid sequences from (b) coding for amino acid mutations at the one position in the parent nucleic acid sequence; and screening the library or array of variant nucleic acid sequences to identify nucleic acid sequences that have a desirable improvement in comparison with the parent nucleic acid sequence with respect to at least one molecular or biological property of interest.

The present disclosure also provides methods of making modified proteins (*e*.*g*., an antibody or binding fragment thereof such as an IgG, Fab or scFv) with mutated amino acid sequences by modifying the amino acid sequence of a protein to produce amino acid mutations at an amino acid residue in the protein to generate a library or an array of modified proteins with mutated amino acid sequences, wherein the amino acid mutations exclude cysteine and methionine; and selecting modified proteins from the library or the array that have a biological activity of an unmodified protein.

The present disclosure also provides methods for selecting modified proteins (*e*.*g*., an antibody or binding fragment thereof such as an IgG, Fab or scFv) with mutated amino acid sequences by obtaining a library or an array of modified proteins comprising amino acid mutations at one amino acid residues in an amino acid sequence of a protein, wherein the amino acid mutations exclude cysteine and methionine; assaying the modified proteins for a biological activity of an unmodified protein; and selecting the modified proteins that have a biological activity of the unmodified protein.

The present disclosure also provides a set of primers that each comprise at least one 2 to 12 fold degenerate codon (*e*.*g*., NHT or NHC (where N=A/G/C/T, H=A/C/T), VAG or VAA (where V=A/C/G) and BGG or DGG (where B=C/G/T, D=A/G/T), ARG (where R=A/G), WMC (where W=A/T and M=A/C), CAS (where S=C/G), GAS (where S=C/G), NTC (where N=A/G/C/T), KGG (where K=G/T) and SCG (where S=C/G)), wherein each primer comprises at least two oligonucleotide sequences that are complementary to a sequence in a parent nucleic acid and code for an amino acid mutation with the exception of cysteine or methionine at one amino acid position encoded by the parent nucleic acid.

The present disclosure also provides a kit for mutagenesis of an amino acid residue encoded by a parent nucleic acid comprising a set of primers that each comprise at least one 2 to 12 fold degenerate codon (*e*.*g*., NHT or NHC (where N=A/G/C/T, H=A/C/T), VAG or VAA (where V=A/C/G) and BGG or DGG (where B=C/G/T, D=A/G/T), ARG (where R=A/G), WMC (where W=A/T and M=A/C), CAS (where S=C/G), GAS (where S=C/G), NTC (where N=A/G/C/T), KGG (where K=G/T) and SCG (where S=C/G)), wherein each primer comprises at least two oligonucleotide sequences that are complementary to a sequence in a parent nucleic acid and code for an amino acid mutation with the exception of cysteine or methionine at one amino acid position encoded by the parent nucleic acid.

The present disclosure also provides libraries/arrays comprising variants of a protein sequence, wherein the variants each comprise an amino acid mutation at one amino acid position in the protein sequence of a parent protein and wherein the amino acid mutations are not cysteine or methionine.

### Selection of a Defined Region or Amino Acid Residue for Mutagenesis

A region(s) or a specific amino acid residue(s) in a protein may be subjected to the methods of mutagenesis as described herein. A region of a protein for mutagenesis may be deduced from comparing the region(s) to what is known from the study of other proteins, and may be aided by modeling information. For example, the region may be one that has a role in a functional site including, for example, in binding, catalysis, or another function. Regions involved in binding may include, for example, a hypervariable region or complementarity determining region (CDR) of an antigen binding molecule.

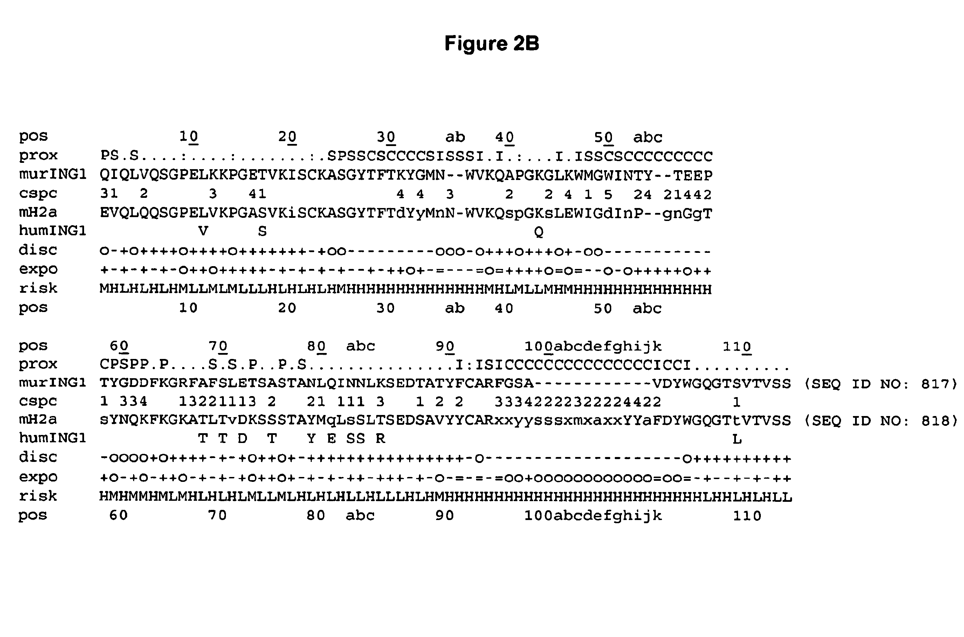

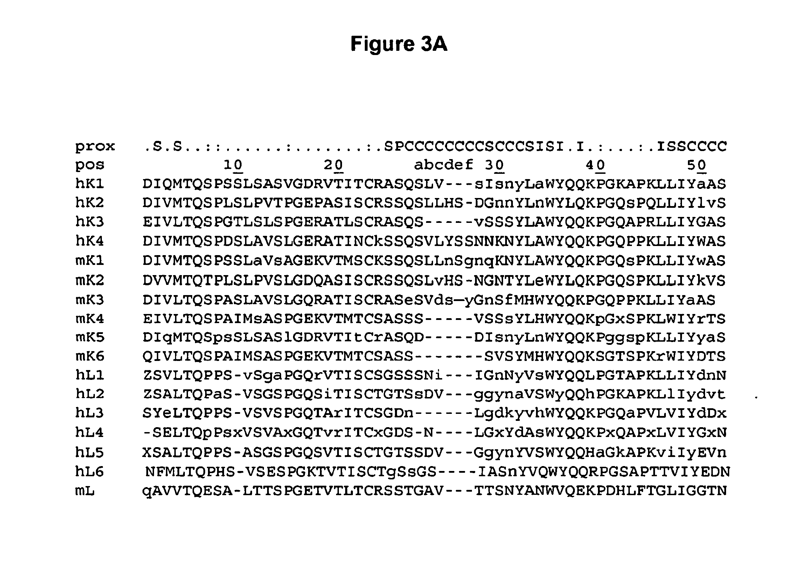

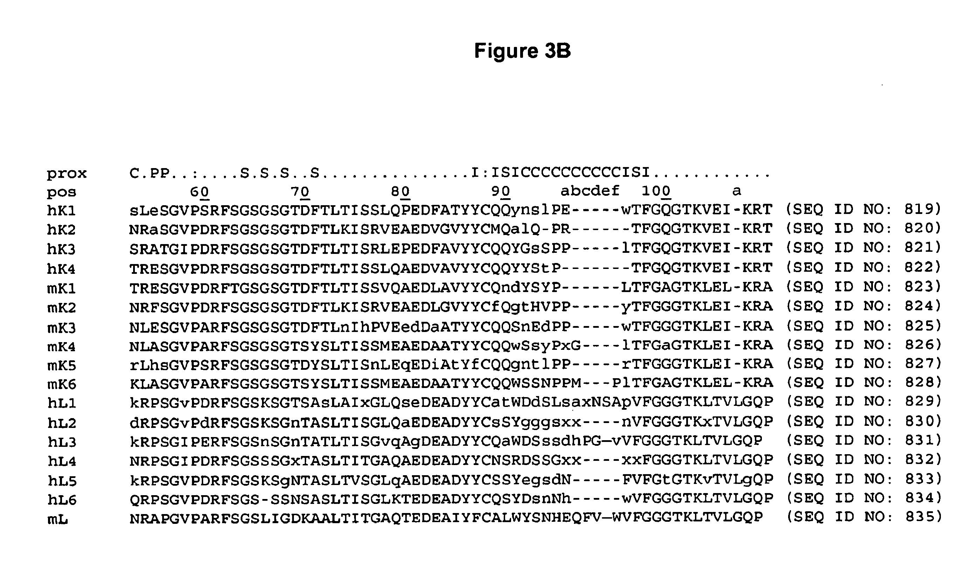

In an exemplary method to select amino acid residues in an antibody or binding fragment thereof for mutation, the amino acid residues may be assigned to a proximity group. For example, each amino acid in an antibody heavy and/or light chain variable region may be assigned to one of the following unique groups, which includes, contacting (C), peripheral (P), supporting (S), interfacial (I), or distant (D) residues, as shown, for example, on the "prox" lines of Figures 2A, 2B, 2C, 2D, 3A, and/or 3B. For example, each of the more-than-200 amino-acid positions in an antibody's variable light chain and heavy chain has been designated as a member of one of these five novel groups. The "prox" line as shown in Figure 3A or 3B is useful for any variable region sequence, irrespective of the present disclosure provides methods for mutagenesis of one or more defined region(s) within a protein. The region(s) may be important to a protein's structure or function. These region(s) can be deduced, for example, from what structural and/or functional aspects are known or specific amino acid sequence, such that residues can be selected as candidates for change (*e*.*g*., any and/or all contacting (C) residues). Additionally or alternatively, methods are provided that identify the presence of conspicuous amino-acid residues which may be candidates for change. Conspicuous amino acid changes may differ in charge or size or chemical functionality from the corresponding residues in the selected sequence (*e*.*g*., consensus or germline sequence) and represent positions where amino acid changes may enhance affinity.

Exemplary methods for characterization of amino acid residues in an antibody binding domain may include: a determination of each amino acid residue's proximity group as designated on the "prox" line of Figures 2A, 2B, 2C, 2D, 3A and / or 3B and additionally or alternatively a determination of each amino acid residue's conspicuousness as calculated by the methods provided in the present disclosure.

### A. Determination of Proximity Groups

The characterization process may determine the proximity group for each amino-acid position simply by inspecting the corresponding symbol ("CPSI.:") on the "prox" lines as shown, for example, in Figures 2A, 2B, 2C and/or 2D. In some embodiments, the antibody's light-chain and/or heavy-chain sequences are aligned with appropriate sequences (e.g., such as consensus or germline sequences) and also with the "prox" lines of the present methods (Figures 2A, 2B, 2C and/or 2D),

Each position in the light chain and heavy chain has been assigned to one of five novel groups designated as contacting (C), peripheral (P), supporting (S), interfacial (I), or distant (D) on the "prox" lines, for example, of Figures 2A, 2B, 2C, 2D, 3A, and/or 3B according to the methods disclosed herein. These Figures (*e*.*g*., 2A, 2B, 2C, 2D, 3A and/or 3B) contain a disc line to reflect disconnection from any significant effect upon an antibody's binding site, and an outw line to reflect outward-orientation on an antibody's surface.

Table 1 shows five proximity groups, as well as a novel designation of disconnection (as shown on a "disc" line, for example, in Figures 2A, 2B, 2C, 2D, 3A and/or 3B) and outward-orientation (shown as an "outw" line, for example, in Figures 2A, 2B, 2C, 2D, 3A and/or 3B) as defined for each group. The number of positions of each type of proximity group for an exemplary antibody (*e.g*., ING-1, as described herein) in a light chain, a heavy chain, and both chains together are shown in Table 2.

**Table 1.**

| Proximity | Abbr | Disc/Outw | | | | |
|---|---|---|---|---|---|---|
| Contacting | C | -/+ | -/o | | | |
| Peripheral | P | o/+ | o/o | | | |
| Supporting | S | -/- | o/- | | | |
| Interfacial | I | -/= | o/= | +/= | | |
| Distant | • | +/+ | +/o | +/- | p | c |

**Table 2.**

| Proximity | L | H | L+H |
|---|---|---|---|
| Contacting | 16 | 21 | 37 |
| Peripheral | 3 | 8 | 11 |
| Supporting | 14 | 16 | 30 |
| Interfacial | 9 | 10 | 19 |
| Distant | 70 | 63 | 133 |

Without being bound by a theory of the invention, it has been hypothesized that amino acid residues designated as contacting (C) are located within the combining site (see, *e*.*g*., "-" on the "disc" line of Figures 2A, 2B, 2C and/or 2D), and their sidechains are mostly outward-oriented (see, *e*.*g*., "+" or "o" on the outw line). It has been further hypothesized that these are generally surface-exposed residues in the CDR loops themselves, so their sidechains are very favorably situated for making direct contact with corresponding residues on a binding partner.

Without being bound by a theory of the invention, it has been hypothesized that amino acid residues designated as peripheral (P) are slightly disconnected from the binding site (see, *e*.*g*., "o" on the "disc" line), and their sidechains are mostly outward-oriented (see, *e*.*g*., "+" or "o" on the outw line). Many of these are framework residues with variable orientation, which are located at curves or twists in the protein chain not too far from CDR loops. Although they may normally not make direct contact with a binding partner, they may possibly make contact if a particular binding partner is bound preferentially toward one side of the binding site instead of being centered.

Without being bound by a theory of the invention, it has been hypothesized that amino acid residues designated as supporting (S) are either directly within or close to the combining site (see, *e*.*g*., "-" or "o" on the "disc" line), and their sidechains are inward-oriented (see, *e*.*g*., "-" on the outw line). It has been further hypothesized that many of these residues are buried in the Vernier-zone platform directly underneath a combining site, so that their nonpolar sidechains are able to act as conformation-stabilizing "anchors" for CDR loops which rest on top of them.

Without being bound by a theory of the invention, it has been hypothesized that amino acid residues designated as interfacial (I) may be located anywhere in relation to the binding site (see, *e*.*g*., "+" or "o" or "-" on the "disc" line), but their sidechains form the interface between the light and heavy subunits of the variable domain (see, *e*.*g*., "=" on the outw line). It has been further hypothesized that amino acid changes of these residues may cause the two subunits to pivot or rotate relative to one another along their shared hydrophobic interfacial surface, producing strong allosteric effects upon an entire binding site, for example, all six CDR loops may be forced to change their conformation in response.

Without being bound by a theory of the invention, it has been hypothesized that amino acid residues designated as distant (D) are of two different types, with those of the first type being disconnected from a combining site and its targeted epitope (see, *e*.*g*., "+" on the "disc" line), and their sidechains may have any orientation except interfacial (see, *e*.*g*., "+" or "o" or "-" but not "=" on the outw line). It is further hypothesized that amino acid changes at these positions generally will have little or no effect on enhanced affinity to a binding partner.

### B. Determination of Conspicuousness

In some embodiments, alternatively or additionally with determination of the proximity groups by inspection of the "prox" lines, the characterization process may involve a calculation of the conspicuousness value for each amino-acid position. The conspicuousness value of a sidechain at a particular antibody position is hypothesized to represent the degree to which it appears strikingly different or unusually outstanding in comparison with selected sequences (*e*.*g*., a consensus or germline sequence). Without being bound by a theory of the invention, this value indicates the likelihood that this particular residue may be a somatic mutation which was necessary to confer binding partner specific affinity upon an antibody. Consequently, the conspicuousness value also correlates with the hypothesis that a new engineered amino acid substitution at or near this position could possibly lead to forming or strengthening a bond with a residue on a binding partner surface.

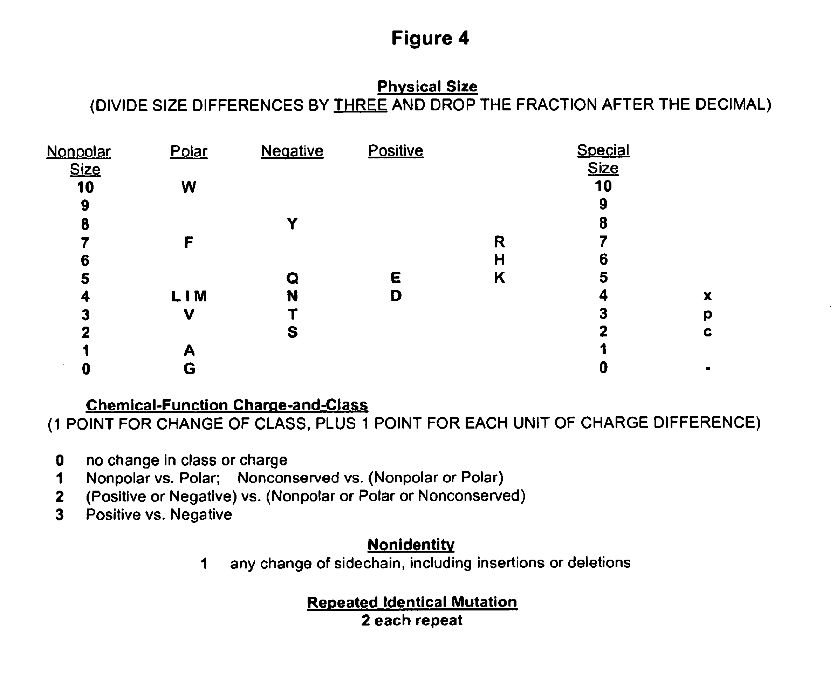

Conspicuousness values are calculated by comparing each sidechain of a candidate antibody with the corresponding sidechain of an appropriate consensus or germline sequence, for example, from a mutual alignment. For example, numerical values for conspicuousness can be calculated readily for each amino-acid position in a given antibody, according to the following formula: add 1 point for each three units of difference in size (*e*.*g*., divide the absolute value of the size-difference by 3 and drop the decimal without rounding); add 1 point for a shift from one sidechain class to another; add 1 point for each unit (absolute value) of difference in charge, and add 1 point for nonidentity (see, *e.g.,* Figure 4).

For example, where a single antibody sequence is aligned or compared with a single consensus or germline sequence, there is one "pair" of sequences being compared. The conspicuousness value for each amino-acid position in the alignment or comparison is the sum of the points for chemical function and physical size and nonidentity at that position. Where more than two sequences are aligned or compared together at the same time, each of the antibody sequences may form a separate "pair" with each of the consensus or germline sequences. The conspicuousness values are calculated as described (*e*.*g*., sum of function and size and nonidentity) for each pair of sequences being aligned or compared, and then the overall conspicuousness value for each amino acid position in the whole alignment is the sum of the values obtained from each pair at that position, while also adding in a value for repeated identical mutations.

It is hypothesized that nonidentity simply marks an amino-acid position as minimally conspicuous if it displays any kind of difference when compared with a corresponding consensus or germline position. Even a conservative mutation (*e*.*g*., from leucine to isoleucine or valine) may suggest a possible bond with a binding partner, especially if a slight change of size or shape was necessary to fine-tune steric relationships between the two molecules.

An exemplary calculation of conspicuousness is illustrated as follows. Four monoclonal antibodies to the same epitope were isolated, and portions of their heavy chains were mutually aligned with a germline sequence, between Kabat positions 25 and 57 [Mendez et al., Nature Genetics, 15:146-152 (1997)] (*see*, Table 3). Since this alignment contains more than two sequences, each of the four antibody sequences can separately form a "pair" with the one germline sequence. Thus, conspicuousness values are calculated separately for each of the four pairs, and then totaled at each amino-acid position, while also adding in the additional values for repeated identical mutations.

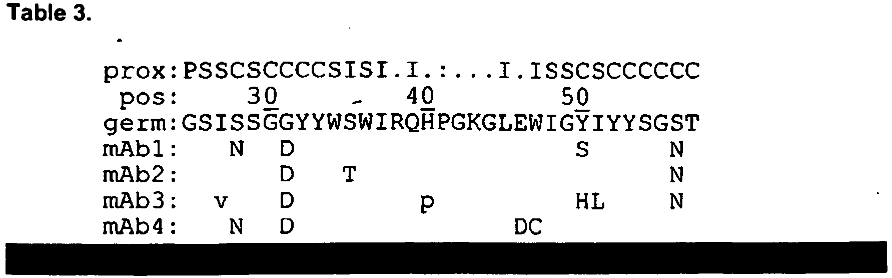

Three repetitions are shown in Table 3, at positions 28 and 31 and 56. In each of these cases, an identical amino acid (N or D) has appeared at the same location in more than one independently isolated antibody. Accordingly, as described herein, these positions are given very high conspicuousness in the affinity enhancement process. An additional 2 points are added for each repetition of an identical amino acid at a given position (*e*.*g*., four D's amount to three repetitions of the first D, so it is worth 3x2=6 points).

In an example, at position 50, the first pair (germ:mAb 1) gets 3 points (Y to S = 2 for size + 0 for class + 0 for charge + 1 for nonidentity), the second pair (germ:mAb2) gets 0 points (unmutated Y = 0+0+0+0), the third pair (germ:mAb3) gets 3 points (Y to H = 0 for size + 1 for class + 1 for charge + 1 for nonidentity), and the fourth pair (germ:mAb4) gets 0 points (unmutated Y = 0+0+0+0). The total conspicuousness for position 50 is the sum (3+0+3+0) of these, plus 0 extra points for no repeated identical mutations, which finally gives 6.

In another example, at position 28, the first pair gets 1 point (S to N = 0+0+0+1), the second and third pairs get 0 points, and the fourth pair gets I point. Since the somatic mutation N appears at position 28 twice, it is repeated once, and thus gets 2 extra points. The total conspicuousness for position 28 is the sum (1+0+0+1), plus 2 points for one repetition, which finally gives 4.

In another example, at position 31, each of the four pairs gets 4 points (G to D = 1 +1 +1+1). Since the somatic mutation D appears at position 31 four times, it is repeated three times, and thus gets 3x2=6 extra points. The total conspicuousness for position 28 is the sum (4+4+4+4), plus 6 points for three repetitions, which finally gives 22.

The conspicuousness points can be calculated (one pair at a time and then summed) for positions 28, 31, and 50 in the antibody sequence provided in Table 2.

### Degenerate Primers and Kits

The present disclosure provides primers and kits that may comprise one or more of the novel degenerate codons of the present disclosure. These degenerate codons that may be used to mutagenize a nucleotide sequence encoding a protein including, for example, an antibody such as an IgG, a Fab or a ScFv. The degenerate codons may code for an equal representation of eighteen amino acid substitutions including, for example, alanine (Ala, A), arginine (Arg, R), asparagine (Asn, N), aspartic acid (Asp, D), glutamine (Gln, Q), glutamic acid (Glu, E), glycine (Gly, G), histidine (His, H), isoleucine (Ile, I), leucine (Leu, L), lysine (Lys, K), phenylalanine (Phe, F), proline (Pro, P), serine (Ser, S), threonine (Thr, T), tryptophan (Trp, W), tyrosine (Tyr, Y) or valine (Val, V). The degenerate codons of the present disclosure do not code for cysteine and/or methionine. A degenerate primer may be between 2-fold degenerate (*e*.*g*., comprise 2 oligonucleotide sequences that collectively code for 2 different amino acid residues at the same position) and 12-fold degenerate (*e*.*g*., comprise 12 oligonucleotide sequence that collectively code for 12 different amino acid residues at the same position).

A set of primers is provided that comprise a set of primers that each comprise at least one 2 to 12 fold degenerate codon, wherein each primer comprises at least two oligonucleotide sequences that are complementary to a sequence in a parent nucleic acid and code for an amino acid mutation with the exception of cysteine or methionine at one amino acid position encoded by the parent nucleic acid. In some embodiments, the primer set codes for eighteen amino acid changes at each of one or more positions in the parent nucleic acid. In some embodiments, the set of primers each comprise a degenerate codon which collectively code for alanine, arginine, asparagine, aspartic acid, glutamine, glutamine acid, glycine, histidine, isoleucine, leucine, lysine, phenylalanine, proline, serine, threonine, tryptophan, tyrosine and valine at each position. A set of primers may comprise 2 or more primers (*e*.*g*., 3 or 7 primers) and contains a number of oligonucleotides equal to the sum of the degeneracy of each primer in the set. For example, a set of primers that comprises a 2-fold and a 4-fold degenerate primer contains six oligonucleotide sequences.

Kits are also provided for mutagenesis of a position in a parent nucleic acid that comprise a set of primers a set of primers that each comprise at least one 2 to 12 fold degenerate codon, wherein each primer comprises at least two oligonucleotide sequences that are complementary to a sequence in a parent nucleic acid and code for an amino acid mutation with the exception of cysteine or methionine at one amino acid position encoded by the parent nucleic acid.

A set of three degenerate codons may be used to mutagenize an amino acid position encoded by a parent nucleotide sequence. These degenerate codons may include, for example, NHT or NHC (where N=A/G/C/T, H=A/C/T), which codes for phenylalanine/serine/tyrosine/leucine/proline/histidine/isoleucine/threonine/ asparagine/valine/alanine/aspartic acid, VAG or VAA (where V=A/C/G), which codes for glutamine/lysine/glutamic acid, and BGG or DGG (where B=C/G/T, D=A/G/T), which codes for tryptophan/arginine/glycine. Alternatively, a set of seven degenerate codons may be used to mutagenize one or more selected positions in a parent nucleotide sequence. These degenerate codons may include, for example, ARG (where R=A/G), which codes for arginine/lysine, WMC (where W=A/T and M=A/C), which codes for serine/threonine/asparagine/tyrosine, CAS (where S=C/G), which codes for histidine/glutamine, GAS (where S=C/G), which codes for glutamic acid/aspartic acid, NTC (where N=A/G/C/T), which codes for leucine/phenylalanine/isoleucine/valine, KGG (where K=G/T), which codes for tryptophan/glycine, and SCG (where S=C/G), which codes for proline/alanine.

Alternate degenerate codons may be used to mutagenize one or more selected positions in a parent nucleotide sequence by modifying the degenerate codons described above. For example, ARG may be replaced with ARA, WMC may be replaced with WMT, CAS may be replaced with CAK (K=G,T), CAM (M=A or C), or CAW (W=A or T), NTC with NTT, SCG with SCA, SCC, or SCT. In addition, the primer listed as NTC or NTT may be replaced with two primers MTC, KTC (or MTT/KTT; MTC/KTT; MTT/KTC); STC, WTC (or STT/WTT; STT/WTC; STC/WTT); RTC, YTC (or RTT/YTT; RTC/YTT, RTT/YTC).

### Methods for Targeted Mutagenesis

The methods of the present disclosure may be used to mutagenize a nucleic acid sequence coding for a protein including, for example, an antibody or binding fragment thereof (*e*.*g*., an IgG, Fab or scFv). When the parent nucleic acid sequence encodes an antibody variable region, preferred positions for selection and mutagenesis are those encoding one of more CDR amino acid residues. Particularly preferred is mutation of each of the CDR residues in a heavy and/or light chain variable region. Methods for mutagenesis of the present disclosure may selectively target one or more regions of a protein including, for example, one or more amino acid residues. The region(s) mutagenized by the methods of the present disclosure may comprise a functional domain of a protein such as a binding or catalytic domain. For example, the region may be a hypervariable region (*e*.*g*., complementarity-determining region or CDR) of an immunoglobulin, the catalytic site of an enzyme, or a binding domain.

The CDRs (*e*.*g*., LCDR1, LCDR2 and LCDR3 for the light chain and HCDR1, HCDR2 and HCDR3 for the heavy chain) may be defined according to any known method in the art including, for example, Kabat, Chothia or IMGT. According to Kabat, LCDR1 comprises amino acid residues 24 to 34, LCDR2 comprises amino acid residues 50 to 56, LCDR3 comprises amino acid residues 89 to 97, HCDR1 comprises amino acid residues 31 to 35b, HCDR2 comprises amino acid residues 50 to 65 and HCDR3 comprises amino acid residues 95 to 102. According to Chothia, LCDR1 comprises amino acid residues 24 to 34, LCDR2 comprises amino acid residues 50 to 56, LCDR3 comprises amino acid residues 89 to 97, HCDR1 comprises amino acid residues 26 to 32, HCDR2 comprises amino acid residues 52 to 56 and HCDR3 comprises amino acid residues 95 to 102. According to IMGT, LCDR1 comprises amino acid residues 27 to 32, LCDR2 comprises amino acid residues 50 to 52, LCDR3 comprises amino acid residues 89 to 97, HCDR1 comprises amino acid residues 26 to 33, HCDR2 comprises amino acid residues 51 to 57 and HCDR3 comprises amino cid residues 93 to 102. Residues numbers for the Kabat, Chothia and IMGT CDRs are given as Kabat position numbers.

The present disclosure provides methods of mutagenesis of a parent nucleic acid sequence encoding a protein by obtaining one or more primers that each comprise at least one 2 to 12 fold degenerate codon, wherein the primers are complementary to a sequence in the parent nucleic acid sequence and wherein the primers code for amino acid mutations at one amino acid position encoded by the parent nucleic acid sequence; and subjecting the parent nucleic acid sequence to replication or polymerase based amplification using the obtained primers, wherein replication or amplification of the parent nucleic acid sequence with the primers generates variant nucleic acid sequences and wherein the variant nucleic acid sequences comprise amino acid mutations at the one position in the parent nucleic acid sequence with the exception of cysteine and methionine.

Several different regions of a protein may be mutagenized simultaneously. This approach may enable the evaluation of amino acid substitutions in conformationally related regions such as the regions which, upon folding of the protein, are associated to make up a functional site such as the catalytic site of an enzyme or the binding site of an antibody. For example, the six hypervariable regions of an immunoglobulin, which make up the unique aspects of the antigen binding site (e.g., Fv region), can be mutagenized simultaneously, or separately within the V_{H} or V_{L} chains, to study the three dimensional interrelationship of selected amino acids in this site.

Mutations may be introduced into a parent nucleic acid sequence by PCR mutagenesis using primers that comprise one or more degenerate codons. For example, basic amino acid changes may be introduced using the degenerate codon ARG (R=A/G), which codes for arginine/lysine. Polar amino acid changes may be introduced using the degenerate codons WMC (W=A/T; M=A/C), which codes for serine/threonine/asparagine/tyrosine and/or CAS (S=C/G), which codes for histidine/glutamine. Acidic amino acid changes may be introduced using the degenerate codon GAS (S=C/G), which codes for glutamic acid/aspartic acid. Non-polar changes may be introduced using the degenerate codons NTC (N=A/G/C/T), which codes for leucine/phenylalanine/isoleucine/valine, KGG (K=G/T), which codes for tryptophan/glycine and/or SCG (S=C/G), which codes for proline/alanine.

An oligonucleotide comprising one or more of the degenerate codons of the present disclosure may be synthesized by known methods for DNA synthesis. Such methods may involve the use of solid phase beta-cyanoethyl phosphoramidite chemistry (see, e.g., U.S. Pat. No. 4,725,677).

Methods are provided for making modified proteins that comprise changes at one or more positions in a parent protein in order to modify one or more biological properties of the parent protein. For example, one or more positions in a parent antibody may be modified in order to enhance the binding affinity of an antibody by means of producing targeted amino acid changes in the antibody's variable domain. Engineered amino acid changes may be introduced at positions likely to produce enhanced affinity based upon an amino acid residue's proximity group.

In an exemplary method, amino acid changes are engineered at one or more amino acid residues categorized as preferably contacting (C), peripheral (P), supporting (S) and/or interfacial on the "prox" lines of Figures 2A, 2B, 3A, and/or 3B. In other embodiments, amino acid residues categorized in more than one group may be selected for change. Less preferably one or more distant (D) amino acid residues may additionally or alternatively be changed.

Modified proteins are synthesized by mutating the nucleic acid encoding a parent protein, inserting the modified nucleic acid into an appropriate cloning vector and expressing the modified nucleic acid to produce modified proteins. Exemplary protocols are described below.

### 1. Masking Modified Nucleic Acid

Modified proteins that comprise one or more amino acid sequence changes (*e*.*g*., substitutions) relative to a parent protein sequence may be produced by methods known in the art using the degenerate primers of the present disclosure. For example, amino acids may be preferably incorporated into a position of interest by utilizing seven different degenerate codons. Basic amino acid changes can be produced with a single oligonucleotide that contains the codon mixture of ARG (R=A/G), encoding Arg/Lys. Polar amino acid changes can be produced with two oligonucleotides. For example, the first oligonucleotide contains the codon mixture WMC (W=A/T; M=A/C), encoding Ser/Thr/Asn/Tyr, while the second polar oligonucleotide utilizes the codon mixture CAS (S=C/G), encoding His/Gln. Acidic amino acid changes can be produced with a single codon mixture of GAS, encoding Glu/Asp. Non-polar functional amino acid changes can be produced with four oligonucleotide mixtures: NTC (N=A/G/C/T), encoding Leu/Phe/Ile/Val, KGG (K=G/T), encoding Trp/Gly, and SCG, encoding Pro/Ala.

In some embodiments, all seven of the degenerate primers are used to perform one PCR reaction. In other embodiments, each degenerate primer is used in a separate PCR reaction. Any combination of PCR primers may be used in a PCR reaction.

DNA encoding modified proteins may be prepared by a variety of methods known in the art. These methods include, but are not limited to, preparation by oligonucleotide-mediated (or site-directed) mutagenesis, PCR mutagenesis, and cassette mutagenesis of an earlier prepared modified protein or a parent protein. These techniques may utilize antibody nucleic acid (DNA or RNA), or nucleic acid complementary to the protein nucleic acid.

In an exemplary method, Kunkel mutagenesis may be performed by placing a plasmid that contains a protein to be mutated into an ung⁻ dut⁻ strain of *E*. *coli* bacteria. Dut ⁻ (lacking dUTPase) bacteria accumulate dUTP and ung⁻ (lacking uracil deglycosidase) bcteria cannot remove dUTP that gets incorporated into new DNA strands. The end result is that the plasmid is converted to DNA that lacks T's and contains U's instead. The U-containing target DNA may then be incubated with a mutagenic primer that base pairs with the target except at the location of the desired mutation. This mixture may then be incubated with Klenow, dNTP's and later Ligase and ATP to produce double-stranded plasmid with one strand containing U's and the new one containing only T's. Finally, the hybrid old/new double-stranded DNA may be transformed into bacteria that destroy the old U-containing DNA and produce a T-containing strand using the new and mutagenized DNA strand as a template.

DNA encoding a modified protein with more than one amino acid to be changed may be generated in one of several ways. If the amino acids are located close together in the protein chain, they may be mutated simultaneously using one oligonucleotide that codes for all of the desired amino acid changes. If, however, the amino acids are located some distance from each other (separated by more than about ten amino acids), it is more difficult to generate a single oligonucleotide that encodes all of the desired changes. Instead, one of two alternative methods may be employed.

In the first method, a separate oligonucleotide is generated for each amino acid to be changed. The oligonucleotides are then annealed to the single-stranded template DNA simultaneously, and the second strand of DNA that is synthesized from the template will encode all of the desired amino acid changes.

The alternative method involves two or more rounds of mutagenesis to produce the desired mutant antibody. The first round is as described for the modified variable domain which comprise one amino acid change: wild-type DNA is used for the template, an oligonucleotide encoding the first desired amino acid change(s) is annealed to this template, and the heteroduplex DNA molecule is then generated. The second round of mutagenesis utilizes the mutated DNA produced in the first round of mutagenesis as the template. Thus, this template already contains one or more mutations. The oligonucleotide encoding the additional desired amino acid change(s) is then annealed to this template, and the resulting strand of DNA now encodes mutations from both the first and second rounds of mutagenesis. This resultant DNA can be used as a template in a third round of mutagenesis, and so on.

### 2. Insertion of DNA into a Cloning Vehicle

The cDNA or genomic DNA encoding the modified protein may be inserted into a replicable vector for further cloning (amplification of the DNA) or for expression. Many vectors are available, and selection of the appropriate vector will depend on 1) whether it is to be used for DNA amplification or for DNA expression, 2) the size of the DNA to be inserted into the vector, and 3) the host cell to be transformed with the vector. Each vector contains various components depending on its function (amplification of DNA or expression of DNA) and the host cell for which it is compatible. The vector components generally include, but are not limited to, one or more of the following: a signal sequence, an origin of replication, one or more marker genes, an enhancer element, a promoter, and a transcription termination sequence.

For example, the cDNA or genomic DNA encoding the modified protein may be inserted into a modified phage vector (*i.e.* phagemid). Construction of phage display libraries exploits the bacteriophage's ability to display peptides and proteins on their surfaces, *i.e.,* on their capsids. Often, filamentous phage such as M13, f1 or fd are used. Filamentous phage contain single-stranded DNA surrounded by multiple copies of genes encoding major and minor coat proteins, *e*.*g*., pIII. Coat proteins are displayed on the capsid's outer surface. DNA sequences inserted in-frame with capsid protein genes are co-transcribed to generate fusion proteins or protein fragments displayed on the phage surface. Peptide phage libraries thus can display peptides representative of the diversity of the inserted genomic sequences. Significantly, these epitopes can be displayed in "natural" folded conformations. The peptides expressed on phage display libraries can then bind target molecules, *i.e.,* they can specifically interact with binding partner molecules such as antibodies (Petersen (1995) Mol. Gen. Genet. 249:425-31), cell surface receptors (Kay (1993) Gene 128:59-65), and extracellular and intracellular proteins (Gram (1993) J. Immunol. Methods 161:169-76).

The concept of using filamentous phages, such as M13, fd or fl, for displaying peptides on phage capsid surfaces was first introduced by Smith (1985) Science 228:1315-1317. Peptides have been displayed on phage surfaces to identify many potential ligands (see, e.g., Cwirla (1990) Proc. Natl. Acad. Sci. USA 87:6378-6382). There are numerous systems and methods for generating phage display libraries described in the scientific and patent literature (see, *e*.*g*., Sambrook and Russell, Molecule Cloning: A Laboratory Manual, 3rd edition, Cold Spring Harbor Laboratory Press, Chapter 18, 2001; "Phage Display of Peptides and Proteins: A Laboratory Manual, Academic Press, San Diego, 1996; Crameri (1994) Eur. J. Biochem. 226:53-58; de Kruif (1995) Proc. Natl. Acad. Sci. USA 92:3938-42; McGregor (1996) Mol. Biotechnol. 6:155-162; Jacobsson (1996) Biotechniques 20:1070-1076; Jespers (1996) Gene 173:179-181; Jacobsson (1997) Microbiol Res. 152:121-128; Fack (1997) J. Immunol. Methods 206:43-52; Rossenu (1997) J. Protein Chem. 16:499-503; Katz (1997) Annu. Rev. Biophys. Biomol. Struct. 26:27-45; Rader (1997) Curr. Opin. Biotechnol. 8:503-508; Griffiths (1998) Curr. Opin. Biotechnol. 9:102-108).

Typically, exogenous nucleic acid to be displayed are inserted into a coat protein gene, *e*.*g*. gene III or gene VIII of the phage. The resultant fusion proteins are displayed on the surface of the capsid. Protein VIII is present in approximately 2700 copies per phage, compared to 3 to 5 copies for protein III (Jacobsson (1996), supra). Multivalent expression vectors, such as phagemids, can be used for manipulation of exogenous genomic or antibody encoding inserts and production of phage particles in bacteria (see, *e.g*., Felici (1991) J. Mol. Biol. 222:301-310).

Phagemid vectors are often employed for constructing the phage library. These vectors include the origin of DNA replication from the genome of a single-stranded filamentous bacteriophage, *e*.*g*., M13, f1 or fd. A phagemid can be used in the same way as an orthodox plasmid vector, but can also be used to produce filamentous bacteriophage particle that contain single-stranded copies of cloned segments of DNA.

Other phage can also be used. For example, T7 vectors can be employed in which the displayed product on the mature phage particle is released by cell lysis.

In addition to phage epitope display libraries, analogous epitope display libraries can also be used. For example, the methods of the disclosure can also use yeast surface displayed epitope libraries (see, *e*.*g*., Boder (1997) Nat. Biotechnol. 15:553-557), which can be constructed using such vectors as the pYD1 yeast expression vector. Other potential display systems include mammalian display vectors and *E. coli* libraries.

An modified protein including, for example, an antibody or antibody fragment, e.g., a scFv, Fab or Fv may be displayed on the surface of a phage using phage display techniques. Exemplary antibody phage display methods are known to those skilled in the art and are described, *e*.*g*., in Hoogenboom, Overview of Antibody Phage-Display Technology and Its Applications, from Methods in Molecular Biology: Antibody Phase Display: Methods and Protocols (2002) 178:1-37 (O'Brien and Aitken, eds., Human Press, Totowa, N.J.). For example, a library of antibodies or antibody fragments (*e*.*g*., scFvs, Fabs, Fvs with an engineered intermolecular disulfide bond to stabilize the V_{H}-V_{L} pair, and diabodies) can be displayed on the surface of a filamentous phage, such as the nonlytic filamentous phage fd or M13. Antibodies or antibody fragments with the desired binding specificity can then be selected.

An antibody phage-display library can be prepared using methods known to those skilled in the art (see, *e.g.,* Hoogenboom, Overview of Antibody Phage-Display Technology and Its Applications, from Methods in Molecular Biology: Antibody Phage Display: Methods and Protocols (2002) 178:1-37 (O'Brien and Aitken, eds., Human Press, Totowa, N.J.).

In some embodiments, cDNA is cloned into a phage display vector, such as a phagemid vector (*e*.*g*., pCES1, p XOMA Fab or pXOMA Fab-gIII). In certain embodiments, cDNA encoding both heavy and light chains may be present on the same vector. In some embodiments, cDNA encoding scFvs are cloned in frame with all or a portion of gene III, which encodes the minor phage coat protein pIII. The phagemid directs the expression of the scFv-pIII fusion on the phage surface. In other embodiments, cDNA encoding heavy chain (or light chain) may be cloned in frame with all or a portion of gene III, and cDNA encoding light chain (or heavy chain) is cloned downstream of a signal sequence in the same vector. The signal sequence directs expression of the light chain (or heavy chain) into the periplasm of the host cell, where the heavy and light chains assemble into Fab fragments. Alternatively, in certain embodiments, cDNA encoding heavy chain and cDNA encoding light chain may be present on separate vectors. In certain embodiments, heavy chain and light chain cDNA may be cloned separately, one into a phagemid and the other into a phage vector, which both contain signals for in vivo recombination in the host cell.

The techniques for constructing and analyzing phage display libraries uses recombinant technology well known to those of skill in the art. General techniques, *e*.*g*., manipulation of nucleic encoding libraries, epitopes, antibodies, and vectors of interest, generating libraries, subcloning into expression vectors, labeling probes, sequencing DNA, DNA hybridization are described in the scientific and patent literature, see e.g., Sambrook and Russell, eds., Molecular Cloning: a Laboratory Manual (3rd), Vols. 1-3, Cold Spring Harbor Laboratory Press, (2001); Current Protocols in Molecular Biology, Ausubel, ed. John Wiley & Sons, Inc., New York (1997-2001) ("Ausubel"); and, Laboratory Techniques in Biochemistry and Molecular Biology: Hybridization with Nucleic Acid Probes, Part I. Theory and Nucleic Acid Preparation, Tijssen, ed. Elsevier, N.Y. (1993). Sequencing methods typically use dideoxy sequencing, however, other methodologies are available and well known to those of skill in the art.

### 3. Transformation of Host Cells

Suitable host cells for cloning or expressing the vectors herein may include prokaryote, yeast, or higher eukaryote cells. Suitable prokaryotes include eubacteria, such as Gram-negative or Gram-positive organisms, for example, *E. coli,* Bacilli such as *B. subtilis, Pseudomonas* species such as *P*. *aeruginosa, Salmonella typhimurium,* or *Serratia marcescens.*

For example, recombinant phagemid or phage vectors may be introduced into a suitable bacterial host, such as *E. coli.* In some embodiments using phagemid, the host may be infected with helper phage to supply phage structural proteins, thereby allowing expression of phage particles carrying the antibody-pIII fusion protein on the phage surface.

### Methods for Identifying a Modified Protein with Altered Activity as Compared to a Parent Protein

Methods are provided for identifying a modified protein with altered activity as compared to a parent protein. For example, a modified antibody variable domain having enhanced binding affinity for a binding partner may be identified by contacting a parent antibody variable domain with the binding partner under conditions that permit binding; contacting modified antibody variable domains made by the methods of the present disclosure with the binding partner under conditions that permit binding; and determining binding affinity of the modified antibody variable domains and the parent antibody variable domain for the binding partner, wherein modified antibody variable domains that have a binding affinity for the binding partner greater than the binding affinity of the parent antibody variable domain for the binding partner are identified as having enhanced binding affinity.

Isolated antibody variable domains may exhibit binding affinity as single chains, in the absence of assembly into a heteromeric structure with their respective V_{H} or V_{L} subunits. As such, populations of V_{H} and V_{L} altered antibody variable domains can be expressed alone and screened for binding affinity having substantially the same or greater binding affinity compared to the parent antibody V_{H} or V_{L} variable domain.

Alternatively, populations of antibody V_{H} and V_{L} altered variable domains proteins can be co-expressed so that they self-assemble into heteromeric altered antibody variable domain binding fragments. The heteromeric binding fragment population can then be screened for species exhibiting enhanced binding affinity to a binding partner compared to the binding affinity of the parent antibody variable domain.

The expressed population of modified antibody variable domains can be screened for the identification of one or more altered antibody variable domain species which exhibit enhanced binding affinity to a binding partner as compared with the parent antibody variable domain. Screening can be accomplished using various methods well known in the art for determining the binding affinity of a protein or compound. Additionally, methods based on determining the relative affinity of binding molecules to their partner by comparing the amount of binding between the modified antibody variable domain and the binding partner can similarly be used for the identification of species exhibiting binding affinity substantially the same or greater than the parent antibody variable domain to the binding partner. The above methods can be performed, for example, in solution or in solid phase. Moreover, various formats of binding assays are well known in the art and include, for example, immobilization to filters such as nylon or nitrocellulose; two-dimensional arrays, enzyme linked immunosorbant assay (ELISA), radioimmuno-assay (RIA), panning and plasmon resonance (see, *e.g.,* Sambrook *et al.,* supra, and Ansubel *et al.,* supra).

For the screening of populations of proteins such as the modified antibody variable domains produced by the methods of the disclosure, immobilization of the modified antibody variable domains to filters or other solid substrates is particularly advantageous because large numbers of different species can be efficiently screened for binding to a binding partner. Such filter lifts allow for the identification of modified antibody variable domains that exhibit enhanced binding affinity compared to the parent antibody variable domain to the binding partner. Alternatively, the modified antibody variable domains may be expressed on the surface of a cell or bacteriophage. For example, panning on an immobilized binding partner can be used to efficiently screen for the relative binding affinity of species within the population of modified antibody variable domains and for those which exhibit enhanced binding affinity to the binding partner than the parent antibody variable domain.

Another affinity method for screening populations of modified antibody variable domains is a capture lift assay that is useful for identifying a binding molecule having selective affinity for a ligand. This method employs the selective immobilization of modified antibody variable domains to a solid support and then screening of the selectively immobilized modified antibody variable domains for selective binding interactions against the binding partner. Selective immobilization functions to increase the sensitivity of the binding interaction being measured since initial immobilization of a population of modified antibody variable domains onto a solid support reduces nonspecific binding interactions with irrelevant molecules or contaminants which can be present in the reaction.

Another method for screening populations or for measuring the affinity of individual modified antibody variable domains is through surface plasmon resonance (SPR). This method is based on the phenomenon which occurs when surface plasmon waves are excited at a metal/liquid interface. Light is directed at, and reflected from, the side of the surface not in contact with sample, and SPR causes a reduction in the reflected light intensity at a specific combination of angle and wavelength. Biomolecular binding events cause changes in the refractive index at the surface layer, which are detected as changes in the SPR signal. The binding event can be either binding association or disassociation between a receptor-ligand pair. The changes in refractive index can be measured essentially instantaneously and therefore allows for determination of the individual components of an affinity constant. More specifically, the method enables accurate measurements of association rates (kₒₙ) and disassociation rates (koff).

Measurements of kₒₙ and k_{off} values can be advantageous because they can identify modified antibody variable domains with enhanced binding affinity for a binding partner. For example, a modified antibody variable domain can be more efficacious because it has, for example, a higher kₒₙ valued compared to the parent antibody variable domain. Increased efficacy is conferred because molecules with higher kₒₙ values can specifically bind and inhibit their binding partner at a faster rate. Similarly, a modified antibody variable domain can be more efficacious because it exhibits a lower k_{off} value compared to molecules having similar binding affinity. Increased efficacy observed with molecules having lower k_{off} rates can be observed because, once bound, the molecules are slower to dissociate from their binding partner.

Methods for measuring the affinity, including association and disassociation rates using surface plasmon resonance are well known in the arts and can be found described in, for example, Jonsson and Malmquist, Advances in Biosensors, 2:291-336 (1992) and Wu et al. Proc. Natl. Acad. Sci. USA, 95:6037-6042 (1998).

Using any of the above described screening methods, a modified antibody variable domain having binding affinity substantially the same or greater than the parent variable domain is identified by detecting the binding of at least one altered variable domain within the population to its binding partner.

Detection methods for identification of species within the population of modified variable domains can be direct or indirect and can include, for example, the measurement of light emission, radioisotopes, calorimetric dyes and fluorochromes. Direct detection includes methods that operate without intermediates or secondary measuring procedures to assess the amount of the binding partner bound by the modified antibody variable domain. Such methods generally employ ligands that are themselves labeled by, for example, radioactive, light emitting or fluorescent moieties. In contrast, indirect detection includes methods that operate through an intermediate or secondary measuring procedure. These methods generally employ molecules that specifically react with the binding partner and can themselves be directly labeled or detected by a secondary reagent. For example, a modified antibody variable domain specific for a binding partner can be detected using an antibody capable of interacting with the modified antibody variable domain, again using the detection methods described above for direct detection. Indirect methods can additionally employ detection by enzymatic labels. Moreover, for the specific example of screening for catalytic antibodies, the disappearance of a substrate or the appearance of a product can be used as an indirect measure of binding affinity or catalytic activity.

In some embodiments, the modified antibody variable domain has a binding affinity for the binding partner greater than the binding affinity of the parent variable domain for the binding partner and thus is identified as having enhanced binding affinity.

In some embodiments, a modified antibody variable domain exhibits enhanced binding affinity to a binding partner compared to the binding affinity between the parent variable domain and the binding partner. In some embodiments, a modified variable domain exhibits an at least 10%, at least 15%, at least 25%, at least 50%, at least 75%, at least 100% (or two-fold), at least 5-fold, at least 10-fold, at least 50-fold, at least 100-fold, or more, higher affinity to a binding partner than the corresponding parent antibody variable domain.

In other embodiments, the modified antibody variable domain has a binding affinity for the binding partner less than the binding affinity of the parent antibody variable domain for the binding partner and thus is identified as having reduced binding affinity for the binding partner.

This disclosure is further illustrated by the following examples which are provided to facilitate the practice of the disclosed methods. These examples are not intended to limit the scope of the disclosure in any way.

### EXAMPLES

### Example 1: Design of Primers for Synthesis of Nucleic Acid Encoding Modified Protein

Each amino acid residue in a parent protein may be changed with other amino acid residues (*e*.*g*., alanine, arginine, asparagine, aspartic acid, glutamine, glutamine acid, glycine, histidine, isoleucine, leucine, lysine, phenylalanine, proline, serine, threonine, tryptophan, tyrosine and valine) by performing PCR with an oligonucleotide containing one of seven different degenerate codons (*e*.*g*., ARG (where R=A/G), WMC (where W=A/T and M=A/C), CAS (where S=C/G), GAS (where S=C/G), NTC (where N=A/G/C/T), KGG (where K=G/T) and SCG (where S=C/G)). For example, contacting residues identified from the "prox" lines in Figure 1, 3A and/or 3B may be changed with other amino acid residues by performing PCR with an oligonucleotide containing one of seven different degenerate codons (*e*.*g*., ARG (where R=A/G), WMC (where W=A/T and M=A/C), CAS (where S=C/G), GAS (where S=C/G), NTC (where N=A/G/C/T), KGG (where K=G/T) and SCG (where S=C/G)).

In an exemplary substitution method, use of seven primers, each comprising one of the seven degenerate codons, may be employed to change one or more contacting (C) amino acid positions in a parent nucleic acid molecule to 18 other amino acid residues. An alternate substitution method may employ the use of three primers each comprising a different degenerate codon to produce eighteen amino acid changes at one or more contacting resides in a parent nucleic acid molecule. For example, the codons may include: NHT (where N=A/G/C/T, H=A/C/T), which codes for Phe/Ser/Tyr/Leu/Pro/His/Ile/Thr/Asn/Val/Ala/Asp; VAA (where V=A/C/G), which codes for Gln/Lys/Glu; and BGG (where B=C,G,T), which codes for Trp/Arg/Gly. This allows production of all eighteen amino acids at equal ratios if oligonucleotides comprising NHT is used at a 4:1:1 ratio with oligonucleotides comprising VAA and oligonucleotides comprising BGG, since NHT encodes twelve amino acids and VAA and BGG both encode three amino acids.

Primers containing one or more degenerate codons may be used to introduce a desired class of amino acid residue at a contacting (C) position by hybridizing to a parent nucleic acid (*e*.*g*., the nucleotide sequence encoding the degenerate codon pairs with a contacting (C) position to be changed). Basic amino acid changes can be produced at a contacting (C) position with a single oligonucleotide that contains the codon mixture of ARG (R=A/G), encoding Arg/Lys. Further, polar amino acid changes can be introduced at a contacting (C) position with two oligonucleotides. The first oligonucleotide contains the codon mixture WMC (W=A/T; M=A/C), encoding Ser/Thr/Asn/Tyr, while the second oligonucleotide utilizes the codon mixture CAS (S=C/G), encoding His/Gln. Additionally, acidic amino acid changes may be introduced at a contacting (C) position with a single codon mixture of GAS, encoding Glu/Asp. Last, non-polar amino acid changes may be introduced at a contacting (C) position with a mixture of three primers with degenerate codons: NTC (N=A/G/C/T), encoding Leu/Phe/Ile/Val, KGG (K=G/T), encoding Trp/Gly, and SCG, encoding Pro/Ala.

### Example 2: Construction of a Library Containing Modified Proteins

Modified proteins containing amino acid changes at one or more positions in a parent protein may be synthesized by PCR amplification from a parent nucleic acid molecule using synthetic oligonucleotides containing a degenerate codon. For example, modified antibody variable domains containing amino acid changes at one or more contacting (C) residues present within an exemplary antibody, for example, ING-1 (a mouse-human chimeric antibody containing the Br-1 mouse variable region domains and human constant regions domains which selectively binds to Ep-CAM (US Patent 5,576,184), heavy chain sequence represented by SEQ ID NO: 579, light chain sequence represented by SEQ ID NO: 580) may be synthesized by PCR amplification from a parent nucleic acid molecule using synthetic oligonucleotides containing a degenerate codon (SEQ ID NO: 1- 285 or SEQ ID NO: 583-699). Similarly, modified antibody variable domains containing amino acid changes at one or more contacting (C) residues present within an exemplary antibody, for example, IL-1 antibody (heavy chain sequence represented by SEQ ID NO: 581, kappa chain sequence represented by SEQ ID NO: 582) may be synthesized by PCR amplification from a parent nucleic acid molecule using synthetic oligonucleotides containing a degenerate codon (SEQ ID NO: 286- 578 or SEQ ID NO: 700-806).

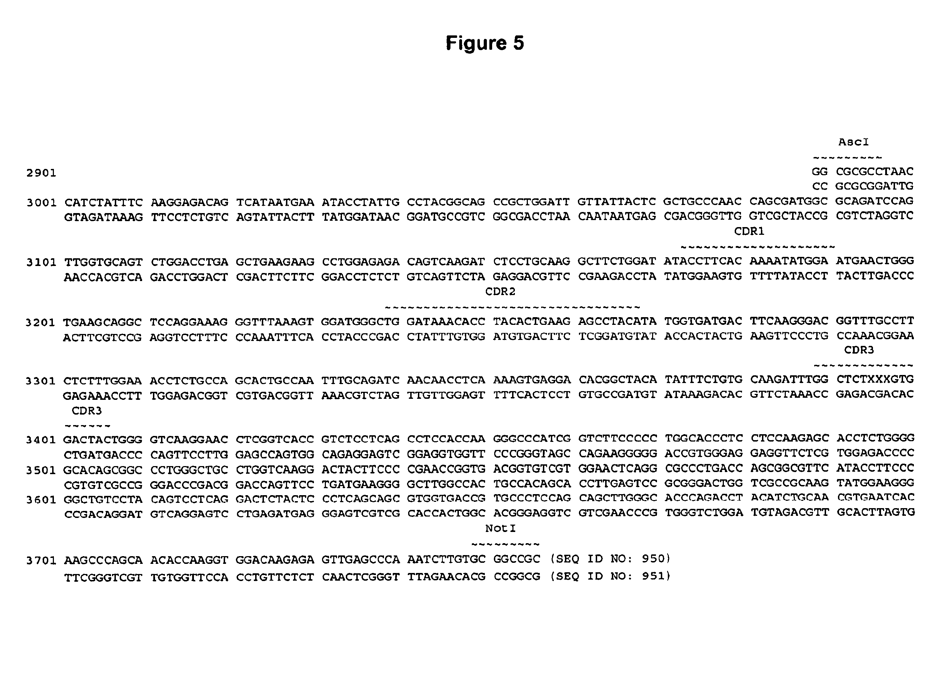

For example, each library oligonucleotide containing the degenerate codon described above for ING-1 may be used in a PCR reaction to synthesize a DNA fragment which incorporates an amino acid change and a 3' restriction site. In an exemplary method, PCR may be conducted at a contacting (C) position (e.g., H3-3) by utilizing the CDRH3 oligonucleotide H3-3NP2 (SEQ ID NO: 267): 5'-GCTACATATTTCTGTGCAAGATTTGGCTCTKGGGTGGACTACTGGGGTCAAGG-3', and the reverse primer *Not*I-R (SEQ ID NO: 285): 5'-AGCGGCCGCACAAGATTTGGGCTCAACTCTC-3') (see, Figure 5) under standard conditions (see, e.g., Sambrook and Russell, Molecule Cloning: A Laboratory Manual, 3rd edition, Cold Spring Harbor Laboratory Press, 2001). After PCR amplification, fragments are obtained which comprise either a tryptophan or glycine residue at the internal codon (underlined above). Further, six other PCR reactions may be performed at the H3-3 position, utilizing SEQ ID NO: 285 with one of SEQ ID NOs: 262-266 and 268 under the conditions described above to obtain other amino acid changes at the site. Next, the products from these reactions may be combined at equal mass, except for reactions which used SEQ ID NO: 263 and 266 as a primer (this mixture is termed the pooled H3-3 library). Due to the degeneracy of these primers, twice the mass of the sample obtained with SEQ ID NO: 263 and 266 is added to produce an equimolar ratio of encoded amino acids.

An additional PCR reaction may be performed to create a fragment (called the H3-R fragment) which contains a 5' restriction site and an overlapping complementary region to the library fragments described above. As an example, for the H3-3 position, a PCR reaction may be performed utilizing the Asc-F2 (SEQ ID NO: 284) and one of the H3R (SEQ ID NO: 247) primer. The 3' portion of this molecule contains a region that is identical to the 5' portion of the molecules created above which permits the use of a PCR reaction to create a contiguous molecule containing a 5' and 3' restriction site.

A PCR reaction may be performed to fuse the above PCR products together into a single molecule. Products from the two PCR reactions described above may be melted and re-annealed to allow for the region of overlap from the two molecules to hybridize. For example, an equal mass of the pooled H3-3 library (approximately two uL of each pooled PCR reaction) and the H3-R fragment may be annealed at their regions of overlap. Next, amplification of annealed molecules with both the Asc-F2 primer (SEQ ID NO: 284) and the NotI-R primer (SEQ ID NO: 247) allows for the synthesis of a single contiguous molecule (see, *e*.*g*., Sambrook and Russell, Molecule Cloning: A Laboratory Manual, 3rd edition, Cold Spring Harbor Laboratory Press, 2001).

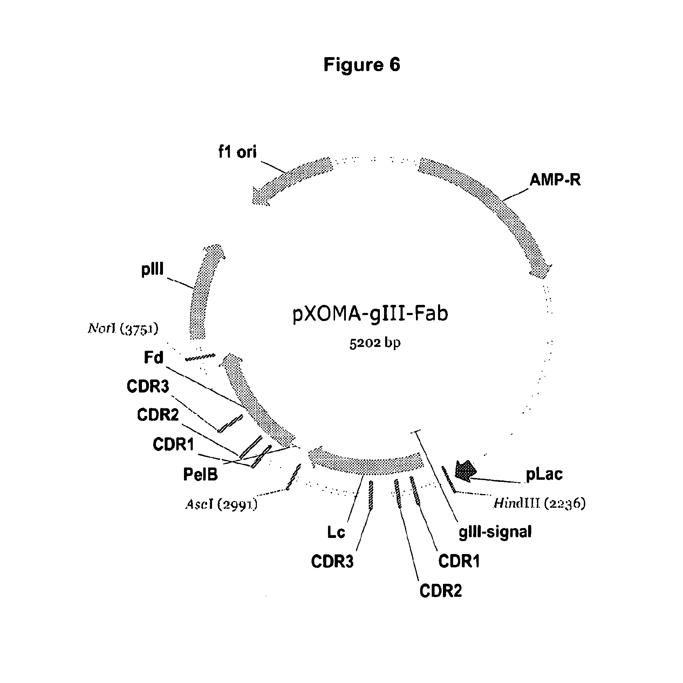

The DNA fragment synthesized by the methods above may be cloned into a pXOMA Fab or pXOMA Fab-glll vector. Briefly, the DNA fragment is purified by using the QIAGEN^{®} PCR purification kit and sequentially digesting the fragment with *Not*I (NEW ENGLAND BIOLABS^{®,} Ipswich, MA) and AscI (NEW ENGLAND BIOLABS^{®,} Ipswich, MA) (See, Methods in Molecular Biology, vol. 178: Antibody Phage Display: Methods and Protocols Edited by: P.M. O'Brien and R. Aitken, Humana Press, "Standard Protocols for the Construction of Fab Libraries, Clark, M. A., 39-58) (see, *e*.*g*., Figure 6). Next, the vectors may be ligated with the mutagenized insert using T4 Ligase (NEW ENGLAND BIOLABS^{®,} Ipswich, MA) and transformed into TG1 cells by electroporation.

### Example 3: Selection of High Affinity Binders

Phage containing a modified proteins including, for example, modified antibody variable domains that bind to an antigen (*e*.*g*., Ep-Cam or IL-1β) with high affinity may be selected by standard panning protocols (*see*, *e.g*., Methods in Molecular Biology, vol. 178: Antibody Phage Display: Methods and Protocols Edited by: P.M. O'Brien and R. Aitken, Humana Press, "Panning of Antibody Phage-Display Libraries", Coomber, D. W. J. pp133-145, and "Selection of Antibodies Against Biotinylated Antigens", Chames, P. et al. p.147-157).

In an exemplary method, library phage for the panning procedure are amplified by inoculating fifty milliliters of 2YT with library TG1 cells and grown to an OD₆₀₀ of 0.6-0.8. Helper phage VCSM13 are added to the inoculated 2YT culture at a multiplicity of infection (M.O.I.) of 10 (*e*.*g*., in 50 mL of cells with OD₆₀₀=0.6 there are 0.6x3⁸ x 50= 9x10⁹ cells, M.O.I. of 10 is therefore 9¹⁰ helper phage, which corresponds to about 10µl of 1¹³ stock phage). The helper phage are used to infect the TG1 cells by gently mixing the phage with the cells with no shaking for thirty minutes. The culture is then shaken for an additional thirty minutes at 180 rpm. Following infection, the culture is spun down at 2500 rpm for ten minutes. The resulting cell pellet is resuspended in fifty milliliters of 2TYAmpKan and grown overnight at 30°C and the supernatant is removed and discarded.

Exemplary methods of panning include coating one well of a NUNC^{®} MAXISORP plate with fifty µl of Ep-Cam or IL-1β at 0.1 µg/ml in DULBECCO'S^{®} PBS with Calcium and Magnesium chloride (Invitrogen, Carlsbad, CA) and incubating the plates overnight at 4°C. The wells are then blocked with 5% milk in PBS for one hour at room temperature. Separately 0.5 ml of phage supernatant from the overnight culture described above are blocked with 300 µL of 10% milk in PBS for one hour at room temperature. Blocked phage (*e*.*g*., approximately 200 µl) are added to the blocked wells in 3% BSA-PBS and incubated at room temperature with shaking for one to two hours. After incubation, the wells are emptied and washed five times with PBST quick wash (*e*.*g*., PBS + 0.05% Tween 20), then washed five times with PBST five minute wash, followed by five washes with PBS quick wash and lastly washed five times with PBS five minute wash. Phage bound to the wells are eluted by incubating with 200 µL/well of freshly prepared 100 mM TEA (prepared by adding 140 µL of 7.18 M Triethylamine stock to ten ml H₂O for 20 minutes at room temperature (see, *e*.*g*., Sambrook and Russell, Molecular Cloning: A Laboratory Manual, 3rd edition, Cold Spring Harbor Laboratory Press, 2001). The eluate is transferred to a Falcon tube containing 0.5 ml 2M TRIS-HCI pH 7.4. The pH of the eluate is checked with pH paper to ensure that it is about pH 7 and adjusted if necessary.

Eluted phage from the exemplary panning method are amplified by infecting TG1 cells. In an exemplary method, TG1 cells are grown to an OD₆₀₀=0.6 (*e*.*g*., mid log phase) and ten ml of the culture is added to the phage eluate from the panning method described above. The eluted phage are used to infect the TG1 cells at 37°C for thirty minutes without shaking and then continued for an additional thirty minutes at 37°C with shaking at 240 rpm. After the infection, the culture is centrifuged at 2500 rpm for five minutes. Next, the supernatant is removed and the cell pellet is resuspended in 700 µL of 2YTAG. The re-suspension is plated on two 15 cm 2YTAG agar plates and incubated at 30°C overnight. After the overnight incubation, the cells are scraped from the two plates using five to ten milliliters of 2YTAG per plate, and transferred to a fifty milliliter falcon tube where they are used to make a glycerol stock.

In an alternative exemplary method, panning may be performed with biotinylated Ep-Cam or IL-1β. Briefly, two hundred microliters of streptavidin beads (Dynal) are blocked in 5% BSA-PBS (100 µl of the blocked beads are used for the de-selection and 100 µL for the selection). Using a magnet, the beads are removed from the 5% BSA-PBS and rinsed twice in PBS. To the rinsed beads is added one milliliter of 5% BSA-PBS and the beads are incubated at room temperature for one hour with very gentle rotation. After the incubation, the beads are split into two tubes, with the supernatant removed from one tube for the de-selection. Phage solution is added to the tube with beads designated for the de-selection and resuspended. The phage-bead solution is incubated at room temperature for forty-five minutes with gentle rotation. After the incubation, the phage supernatant (de-selected phage solution) is transferred to a new tube using a magnet. Next, the de-selected phage solution is incubated at room temperature for sixty minutes with one hundred pmols of biotinylated Ep-Cam or IL-1β. The phage-biotinylated Ep-Cam or IL-1β solution is then added to a new aliquot of streptavidin beads (with the supernatant removed) and incubated at room temperature for sixty minutes. After the incubation, the beads are separated from the supernatant using a magnet. Next the beads are washed five times with one ml of 0.5% BSA-PBST by adding the wash to the tube, closing the tube and resuspending the pellet, putting back in the magnet waiting a few seconds until the beads are attached to the magnet side of the tube and removing the wash with a pipetman. Further, the beads are washed five times in 0.5% BSA-PBST for five minutes for each wash, washed five times with one milliliter of 0.5% BSA-PBS, washed five times for five minutes each wash in five milliliters of 0.5% BSA, and washed one time with PBS. Bound phage are eluted by incubating the beads with 500 µL of freshly prepared 100 mM TEA (add 140 µL of 7.18 M Triethylamine stock to 10 ml H₂O) for thirty minutes at room temperature with gentle rotation. The eluate is separated from the beads by using a magnet and transferred to a fifty milliliter falcon tube containing 250 µl of 1 M TRIS pH 7.4 to neutralize the TEA and can be used for infection and/or amplification (see, *e*.*g*., Sambrook and Russell, Molecular Cloning: A Laboratory Manual, 3rd edition, Cold Spring Harbor Laboratory Press, 2001). For example, log phase TG1 cells may be infected with phage eluate at 37°C for one hour at ninety rpm. After infection of the cells, the culture is centrifuged at 2500 rpm for five minutes and the supernatant removed. Next, the cell pellet is resuspended in 700 µl of 2YTAG, plated onto two 15 cm 2YT-ampicillin-2% glucose agar plates and incubated at 30°C overnight.

### Example 4: Screening of an Affinity Matured Protein Using the DELFIA^{®} Competition Assay

Individual proteins including, for example, Fabs obtained from the affinity-based selection of libraries of the ING-1 antibody clone are tested for their ability to inhibit the binding of Ep-Cam to the parent chimeric ING-1 IgG antibody using a competitive screening assay (*e*.*g*., the microplate based competitive screening DELFIA^{®} assay (PERKIN ELMER^{®,} Waltham, Massachusetts)). Ninety-six well plates containing two hundred and fifty milliliters of 2YT media are inoculated with glycerol stock of Fab-expressing E. coli transformed with the pXOMA-Fab vector. The culture is grown at 37°C until cloudy (approximate OD600=0.5) and inoculated with IPTG to a final concentration of 1 mM. The cultures are grown overnight at 30°C. In addition, a Costar plate 3922 (White) is coated with 1.25 ug/mL of parental ING-1 chimeric IgG O/N at 4°C.

Periplasmic extracts (PPE) of the overnight expression constructs are prepared by spinning the overnight expression plates at 3000 rpm for fifteen minutes, discarding supernatant and adding 60 microliters of PPB buffer to each well. The pellets are resuspended, and 90 microliters of cold PPB diluted 1:5 with cold water are added to each well. This mixture is incubated on ice for one hour and subsequently spun down at 3000 rpm for fifteen minutes. This PPE supernatant is transferred to a new plate. The PPE is diluted into 10% PPE in PBS, 5% PPE in PBS, and 1% PPE in PBS. For the coated Costar plate, it is washed three times with PBS-tween and blocked with 350 microliters of 3% BSA in PBS for one hour.

The blocked Costar plate is washed three times with PBS and then biotinylated Ep-Cam is added to the diluted PPE to a final concentration of 3 nM. The diluted PPE and biotinylated Ep-Cam solution is then added to the coated Costar plate and incubated for one and a half hours at room temperature. The plates are washed three times with PBST and fifty microliters of 1:250 dilution of Europrium-Streptavidin in Delfia Assay Buffer (PERKIN ELMER^{®,} Waltham, Massachusetts) is added. The mixture is incubated at room temperature for one hour, and the Time-Resolved Fluorescence Plate reader is setup (Gemini microplate reader, Molecular Devices), interval 200-1600 microseconds, 20 reads/well, excitation 345 nm, emission 618 nm and cutoff 590 nm. The plates are washed seven times with Delfia Wash Buffer (PERKIN ELMER^{®,} Waltham, Massachusetts), followed by the addition of fifty µl of Delfia Enhancement buffer (PERKIN Elver^{®,} Waltham, Massachusetts) and incubated for five minutes. The plates are read on the Gemini plate reader. Plates with decreased signal compared with control parental antibody show greater binding by the affinity matured Fab and can be further characterized by Biacore (e.g., Biacore 2000 or A100) and other affinity measuring techniques (see, *e*.*g*., Tables 4 and 5).

Similarly, XPA23 antibody clones may be tested for their ability to inhibit the binding of IL-1β to the parent chimeric XPA23 IgG using a competitive screening assay as described above.

**Table 4: Delfia Screening of 10% Periplasmic Extract**

| | 1 | 2 | 3 | 4 | 5 | 6 | 7 | 8 | 9 | 10 | 11 | 12 |
|---|---|---|---|---|---|---|---|---|---|---|---|---|
| A | 46.9 | 37.1 | 71.2 | 75.7 | 51.3 | 22.3 | 65.8 | 72.9 | 58.8 | 81.7 | 56.2 | 96.7 |
| B | **2.6** | 55.2 | 39.2 | 54.8 | 31.7 | 41.3 | 57.1 | 56.7 | 21.6 | 77.8 | **1.8** | 102.0 |
| C | 53.2 | 42.3 | 72.5 | 61.2 | 16.2 | 78.0 | 41.2 | 57.2 | 63.8 | 28.6 | 13.6 | 100.7 |
| D | 49.0 | 45.5 | **8.9** | **1.0** | 21.5 | 82.8 | 105.8 | 67.3 | 68.5 | 61.8 | 63.5 | 100.6 |
| E | 49.1 | 72.1 | 68.6 | **0.3** | 91.8 | 57.6 | 53.1 | **8.3** | 58.3 | 60.4 | 82.2 | -0.4 |
| F | 61.7 | 72.1 | 71.8 | 45.6 | 44.6 | 53.1 | 15.3 | 73.2 | 84.7 | 15.1 | 59.0 | 0.1 |
| G | 58.4 | 26.4 | **1.0** | 59.4 | 62.3 | 19.9 | **-0.1** | 49.0 | 52.4 | 76.2 | 46.8 | 0.3 |
| H | 36.1 | 67.7 | 65.2 | 27.4 | 34.3 | 50.3 | 60.0 | 60.1 | 56.8 | 83.0 | 49.3 | -0.4 |

| | | | | | | | | | | | | |
|---|---|---|---|---|---|---|---|---|---|---|---|---|
| Percentage of inhibition is shown in each well using the average signal from wells A12-D12 as positive control, 100% inhibition and the average signal in well E12-H12 as 0% inhibition negative control wells. Wells bolded show strong competition in the Delphia assay. | | | | | | | | | | | | |

**Table 5: Delfia Screening of 5% Periplasmic Extract**

| | 1 | 2 | 3 | 4 | 5 | 6 | 7 | 8 | 9 | 10 | 11 | 12 |
|---|---|---|---|---|---|---|---|---|---|---|---|---|
| A | 58.4 | 59.3 | 86.6 | 87.8 | 74.8 | 52.4 | 71.7 | 84.4 | 68.6 | 84.8 | 74.2 | 99.4 |
| B | **14.5** | 73.0 | 71.5 | 77.5 | 63.1 | 75.4 | 79.2 | 76.4 | 53.2 | 82.6 | **44.9** | 101.8 |
| C | 56.2 | 56.3 | 92.3 | 76.5 | 55.1 | 94.0 | 81.1 | 79.9 | 77.6 | 60.2 | 53.7 | 100.5 |
| D | 46.8 | 57.8 | 43.0 | 33.0 | 52.4 | 92.6 | 115.3 | 91.6 | 74.7 | 77.1 | 74.7 | 98.2 |
| E | 58.8 | 60.1 | 82.5 | **28.5** | 101.3 | 88.5 | 71.6 | 51.9 | 76.2 | 74.9 | 94.8 | 0.2 |
| F | 58.3 | 65.8 | 69.2 | 64.6 | 67.3 | 78.0 | 65.3 | 87.7 | 88.2 | 38.9 | 70.8 | 0.1 |
| G | 52.8 | 47.4 | **22.2** | 72.2 | 65.8 | 55.7 | **10.0** | 68.1 | 69.2 | 73.5 | 64.6 | -0.2 |
| H | 42.2 | 68.8 | 68.5 | 46.3 | 62.7 | 63.6 | 73.7 | 70.9 | 76.2 | 85.1 | 68.7 | -1.1 |

| | | | | | | | | | | | | |
|---|---|---|---|---|---|---|---|---|---|---|---|---|
| Percentage of inhibition is shown in each well using the average signal from wells A12-D12 as positive control, 100% inhibition and the average signal in well E12-H12 as 0% inhibition negative control wells. Wells bolded show strong competition in the Delphia assay. Boxed wells retain strong inhibition and are prioritized for affinity testing. | | | | | | | | | | | | |

### Example 5: Screening of an Affinity Matured Protein Using Kinetic Titration Analysis

Kinetic properties of affinity matured proteins including, antibodies, for example, as represented by XPA23 clones such as Y208L may be determined by kinetic titration analysis. In an exemplary method, an antigen such as IL-1β is amine coupled to a CM5 sensor chip. Each sample (*e*.*g*., from lowest to highest concentration) may be injected for 240 seconds at a flow rate of 30 □l/min at a selected temperature (*e*.*g*., 25°C). Sample are allowed to dissociate for 30 seconds except the highest concentration which may be permitted 300 seconds to dissociate. The assay is run at 25° C.

Biaevaluation software (*e*.*g*., Biacore 2000 evaluation software) is used to calculate dissociation rates of individual samples and the relative amount of sample bound to each test surface. The data is fit to an appropriate kinetic model (*e*.*g*., the kinetic titration model). For example, XPA23 had a ka= 2.5e5 and a kd= 1.2e-2 KD = 4.6e-8, while the modified XPA23 Y208L mutant had a ka=3.57E+05kd= 5.80E-03 KD=1.62E-08.

### Example 6: ELISA Measurement for Fab Expression, EpCam Binding or IL-1 Binding

Additionally or alternatively to the Biacore assay described below in Example 10, an ELISA assay may be used for the identification of modified antibody variable domains that bind its binding partner or for verifying expression of Fab domains.

In an exemplary method, ELISA plates (*e*.*g*., Nunc MAXISORP™) are coated with 1µg/ml EpCam, 1 µg/mL EpCam for EpCam ELISA, 1 µg/mL IL-1 (Peprotech), or anti-human IgG, F(ab')₂ fragment specific antibody (Jackson Immunoresearch) in PBS at 50 µg/ml. The ELISA plates are then covered and incubated at 4°C overnight. After the incubation, the coated ELISA plates are washed three times with PBS. The plates are then filled with 370 µl of 3% milk (*e*.*g*., Carnation, nonfat) and incubated for one hour at room temperature. Separately, 150 µl of periplasmic extract is blocked by adding 50 µl of 15% milk and incubating the extract for one hour at room temperature. The blocked plates are washed three times with PBS and 50 µL of the blocked periplasmic extract is added to each well of the antigen coated ELISA plates. The plates are incubated for two hours at room temperature and then washed four times with TBST.

Secondary antibodies are added to each ELISA plate. For the Ep-Cam or IL-1 ELISA, 50 µl of mouse anti-human c-myc antibody (9E10 Ab, Roche) at 2.5 µg/ml in 3% milk is added to each well. For the anti-Fab ELISA, 50 µl of biotin-SP-conjugated anti-human IgG F(ab')2 fragment specific antibody (Jackson Immunoresearch) at 1:2000 dilution in 3% milk is added to each well. The plates from both ELISAs are incubated at room temperature for one hour. After the incubation, the plates are washed four times with TBST. After the washes, a tertiary antibody may be added to the plates in both ELISAs. For the Ep-Cam or IL-1 ELISA, 50 µl of goat antimouse IgG-HRP (Pierce) diluted 1:10,000 in 3% milk is added to each well. For the anti-Fab ELISA, 50 µl of extravidin-HRP conjugate (Sigma) at a 1:500 dilution in 3% milk is added to each well. Again the plates from both ELISAs are incubated for one hour at room temperature. After the incubation, the plates are washed four times with TBST. Next, 50 µl of the TMB substrate (Calbiochem) is added to each well and incubated until the color develops (do not incubate long enough to see the negative control turn blue). The reaction is stopped by adding 50 µl of 2N H₂SO₄ to each well and the plates are read at 450 nm.

### Example 7: Methods for Off-Rate Ranking of Antibody Fragments

A high-throughput off-rate ranking method is used for rapid prioritization of modified proteins including, for example, modified antibody variable domains that bind to their binding partner by analyzing their relative off-rates (using, *e.g.,* Biacore 2000 or A100).

In an exemplary method, modified antibody variable domains (*e.g.,* Epcam-binding) are produced in ninety-six well plates by inoculating two hundred and fifty microliters of 2YT media with a glycerol stock of Fab-expressing *E*. *coli* transformed with a pXOMA-Fab vector comprising a modified Epcam-binding variable domain. The culture is grown at 37°C until cloudy (*e.g.,* approximate OD₆₀₀=0.5), inoculated with IPTG to a final concentration of 1 mM and grown overnight at 30°C.

Next, periplasmic extracts (PPE) of the overnight expression constructs are prepared by spinning the overnight expression plates at 3000 rpm for fifteen minutes, discarding the supernatant and adding 60 µl of PPB buffer to each well. The pellets are resuspended, and 90 µl of cold PPB diluted 1:5 with cold water is added to each well. This mixture is incubated on ice for one hour and subsequently spun down at 3000 rpm for fifteen minutes. The supernatant is transferred to a new plate and the periplasmic extracts are used for the Biacore (*e.g.,* Biacore 2000 or A100) determination.

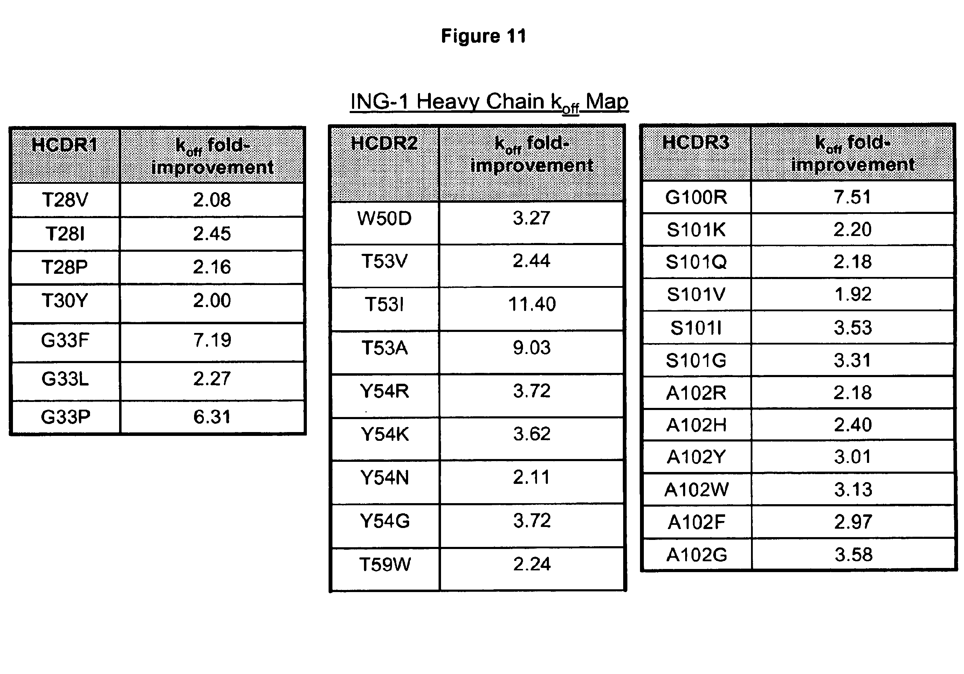

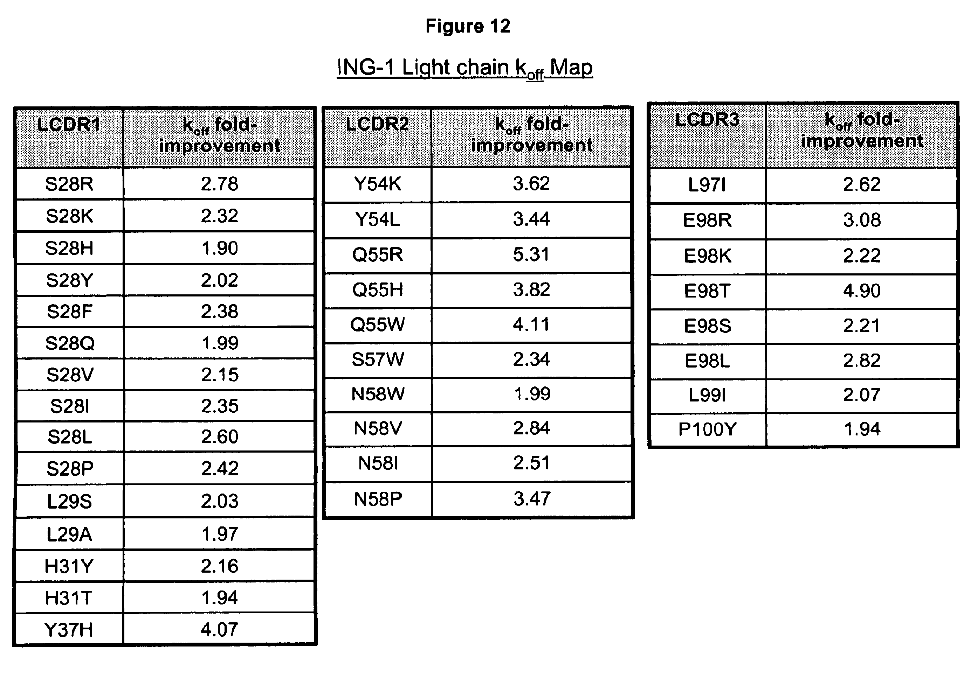

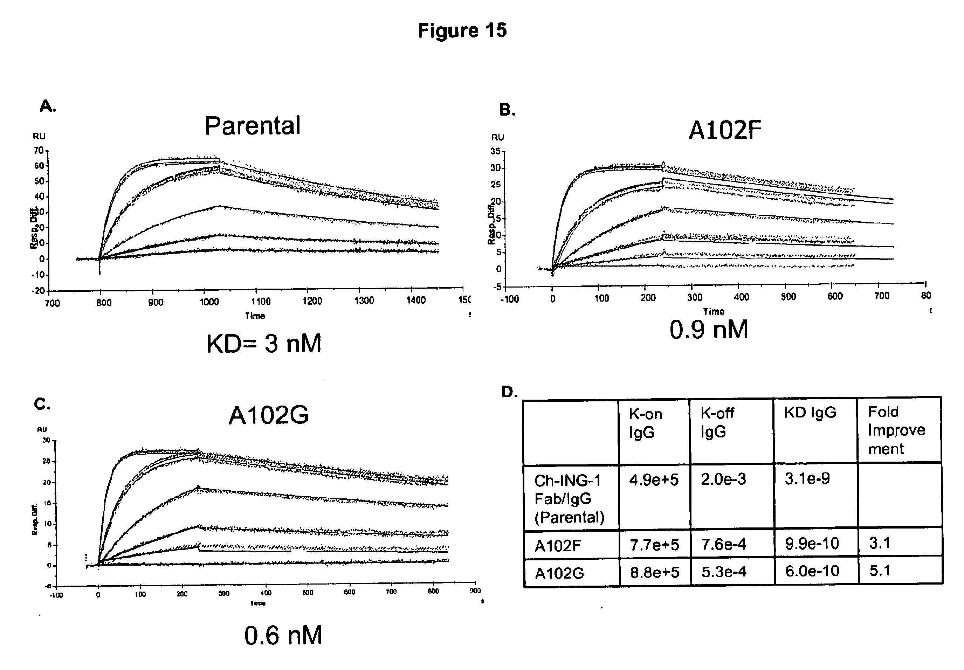

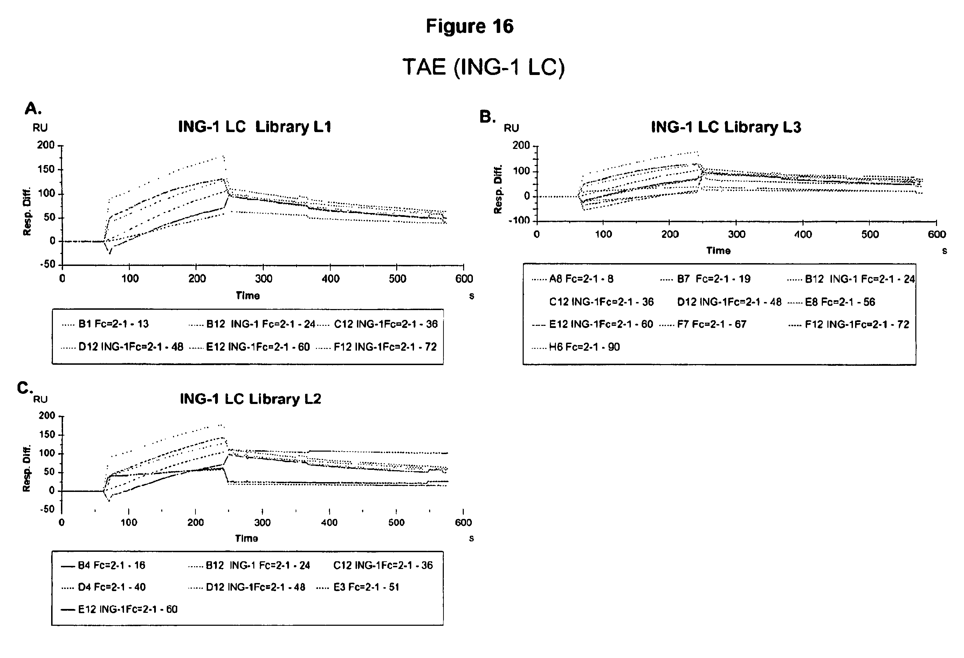

Epcam from the periplasmic extracts is amine coupled (*e.g.,* 10µg/mL Epcam in pH 4.5 acetate, seven minute injection at 5 µl/minute) to a CM5 sensor chip and periplasmic extracts containing the antibody fragments are injected over the sensor, resulting in binding of the Fab to the immobilized Epcam. Non specific binding of the antibody fragment to the sensor surface is corrected by subtracting the interaction of the antibody fragment with a blank flow cell (*e.g.,* having no immobilized Epcam) from the interaction of the antibody fragment with the Epcam immobilized flow cell. The instrument settings are: a flow rate of 20 microliters/minute, an injection time of three minutes, a dissociation time of five minutes and an instrument temperature set to 25°C. Biaevaluation software is used to calculate dissociation rates of individual samples and the relative amount of sample bound to each test surface. Samples are then ranked according to their dissociation rates. Sensograms depicting the off-rates for heavy chains (Figure 15) and light chains (Figure 16) are shown. The off rates for the improved clones are tabulated for the heavy chain (Figure 11) and the light chain (Figure 12).

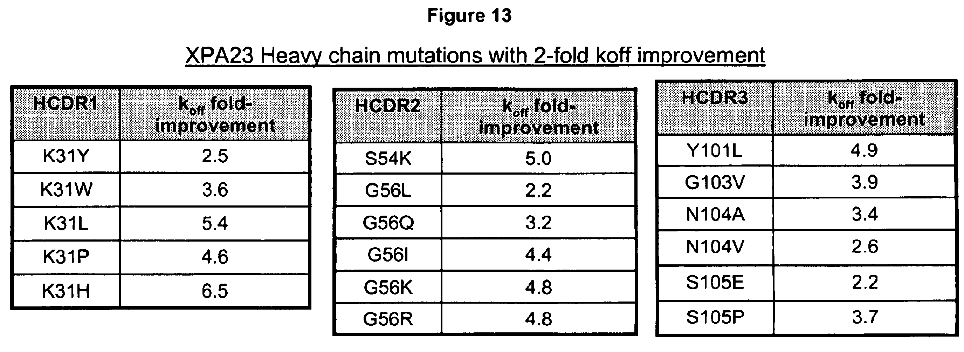

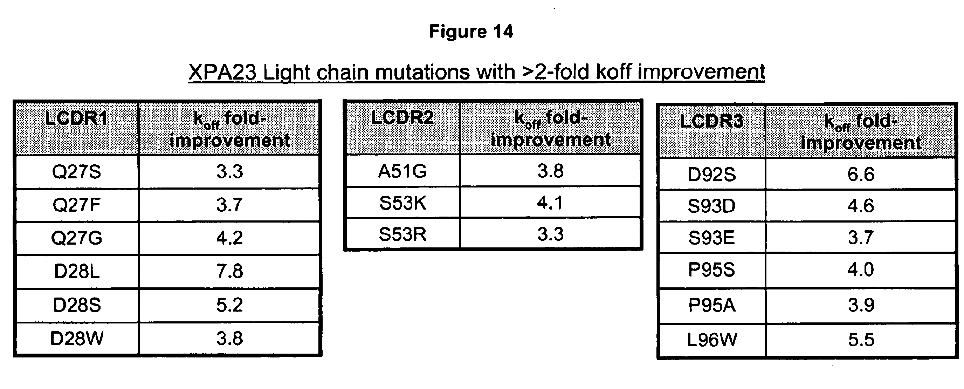

Likewise, modified XPA23 variable domains (*e.g.,* IL-1β -binding) may be ranked according to their dissociation rates using the high-throughput off-rate ranking method described above. The instrument settings are: a flow rate of 30 microliters/minute, an injection time of three minutes, a dissociation time of ten minutes and an instrument temperature set to 25°C. The off rates for the improved clones are tabulated for the heavy chain (Figure 13) and the light chain (Figure 14).

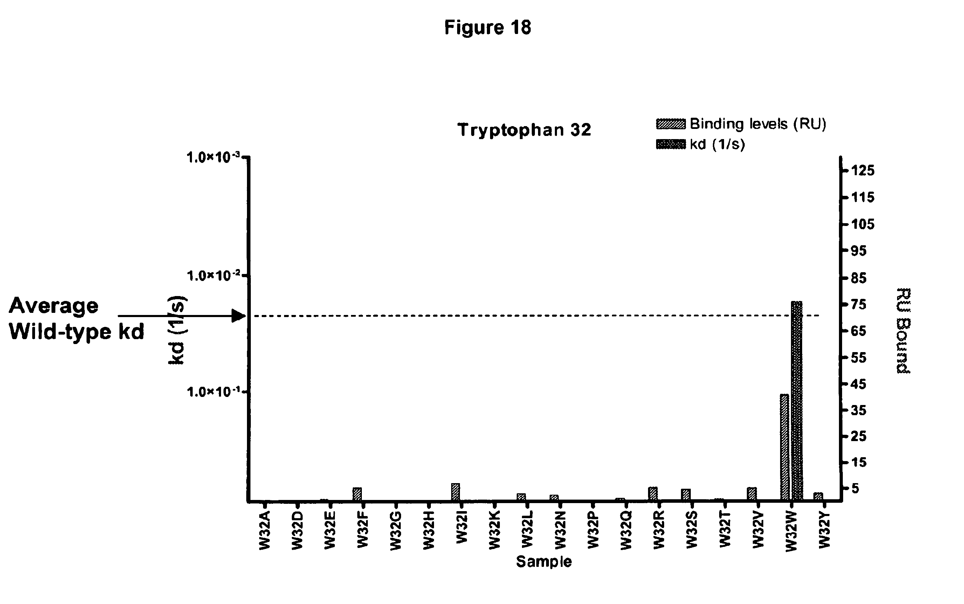

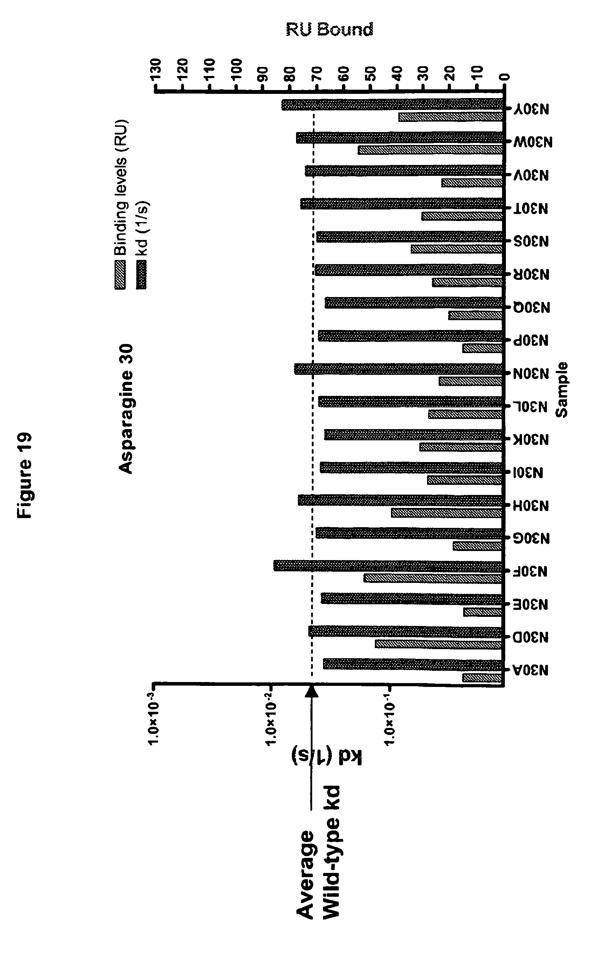

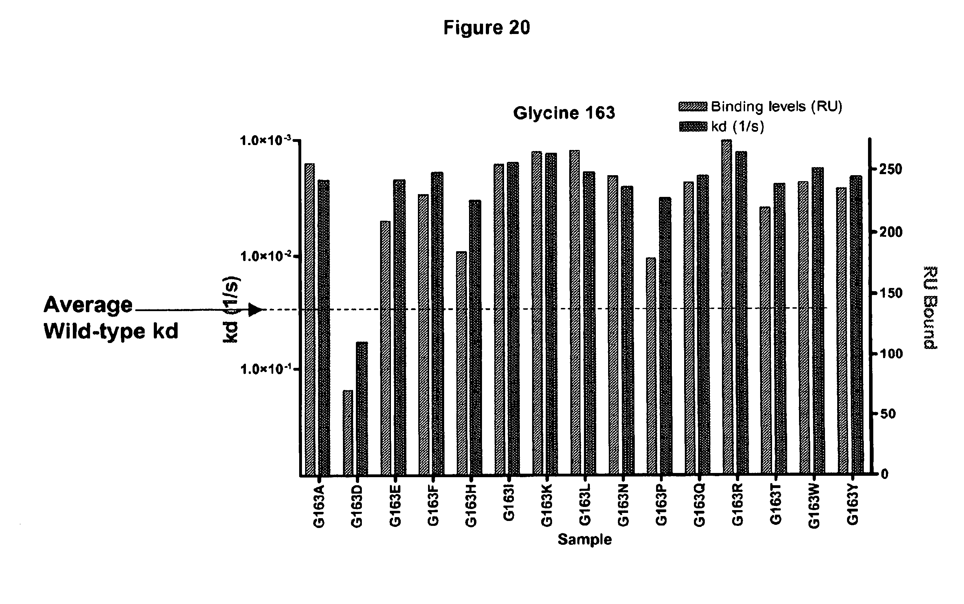

The modified antibody variable domains of the present disclosure may have a k_{off} that is greater than (see, *e.g.,* Figure 20), less than (see, *e.g.,* Figure 18) or equal to (see, *e.g.,* Figure 19) than an unmodified antibody variable domain.

### Example 8: Reformatting of Candidate Clones to IgG

Two of the improved off-rate clones from the k_{off} analysis were reformatted into IgG₁ format by PCR amplification of the heavy and light chain variable domains and cloning the PCR amplified regions into a mammalian expression vector containing the Fc and the light chain constant domain respectively. The heavy chain is cloned into a mammalian expression vector containing a CMV promoter using Bsml and Nhel sites for the 5' and 3' ends respectively and is cloned in frame with the heavy chain secretion signal on the 5' end and the constant CH1,CH2, and CH3 portions of the IgG molecule on the 3' end. The amplification sequences are as follows: (ING-HC-IgGF 5'-ATATATTGCATTCCCAGATCCAGTTGGTGCAGTC-3'), ING-HC-lgGR (5'-ATATATGCTAGCTGAGCTGACGGTGACCGAGGTTCC-3'). The light chain is cloned into a similarly constructed expression vector utilizing a blunt 5' cloning site and the BsiWI site on the 3' end and is cloned in frame with the light chain secretion signal on the 5' end and the light chain constant region on the 3' end. The PCR amplification primer sequences are as follows: (ING-LC-IgGF 5'-CAAATTGTGATGACGCAGGC-3') and (ING-LC-IgGR 5'-ATATATCGTACGTTTCATCTCTAGTTTGGTGCC-3'). The PCRs are performed under standard conditions: see, *e.g.,* Sambrook and Russell, Molecule Cloning: A Laboratory Manual, 3rd edition, Cold Spring Harbor Laboratory Press, 2001. Improved off-rate clones reformatted into IgG₁ vectors are transiently co-transfected in a 2:1 light chain to heavy chain DNA ratio into HEK 292 cells using Lipofectamine 2000 (Invitrogen) using the manufacturer's guidelines. Secreted IgGs secreted from HEK 292 cells are purified using protein A SEPHAROSE^{®} (GE-AMERSHAM^{®} Piscataway, NJ) using the manufacturer's gudielines and tested by BIACORE^{®} (*e.g.,* Biacore 2000 or A100) for affinity (see, *e.g.,* Figures 11 and 15) and Example 8.

### Example 9: Expression and Testing of Modified Proteins with a Combination of Amino Acid Changes

Modified proteins including, for example, antibody variable domains with improved off-rates and affinities as compared to a parent protein may be identified by employing the DELFIA^{®} competition assay and/or B*IACORE^{®} (e.g.,* Biacore 2000 or A100) off-rate ranking. Clones with improved k_{off} are sequenced and aligned by both their light and heavy chain. Identified amino acid changes in the light and heavy chain that increase affinity can be combined in one modified antibody variable domain for potential additive and synergistic combinations. Modifications for combination may utilize the residues that have improved off-rates greater than or equal to 4.9 fold compared with the parental antibodies (*see, e.g.,* Figure 11, 12). For any given amino acid position, the change that leads to the greatest improvement is chosen for study. This compilation is described in Table 6, and will lead to 21 combinations of heavy and light chains (*e.g.,* 7 heavy chains combined in all variations with three light chains).

**Table 6: Heavy and Light Chain CDR1, CDR2 and/or CDR3 Combinations**

| | **Heavy Chain Combinations** | |
|---|---|---|
| **CDR1** | **CDR2** | **CDR3** |
| G33F | wt | wt |
| wt | T53I | wt |
| wt | wt | G100R |
| G33F | T53I | wt |
| wt | T53I | G100R |
| G33F | wt | G100R |
| G33F | T53I | G100R |

| | **Light Chain Combinations** | |
|---|---|---|
| wt | Q55R | wt |
| wt | wt | E98T |
| wt | Q55R | E98T |

Alternatively, the initial modifications for combination may utilize the residues that have improved off-rates greater than or equal to approximately 2.5-fold compared with the parental antibodies (see, *e.g.,* Figure 13 ,14). For any given amino acid change, the change that leads to the greatest improvement is chosen for study. The amino acids with greater than or equal to approximately 2.5 fold improved k_{off} are compiled in Table 7. There are two amino acids in CDR1 (position 28), two amino acids in position 100, three amino acids in position 101, and five amino acids in position 102. In all, there are 60 (2x2x3x5=60) combinations.

**Table 7: Heavy Chain CDR1 and CDR3 Combinations**

| **CDR1** | **CDR3** |
|---|---|
| 28T (wt) | 100G(wt) |
| 28I | 100R |
| | |
| | 101S(wt) |
| | 101I |
| | 101G |
| | |
| | |
| | 102A (wt) |
| | 102Y |
| | 102F |
| | 102W |
| | 102G |

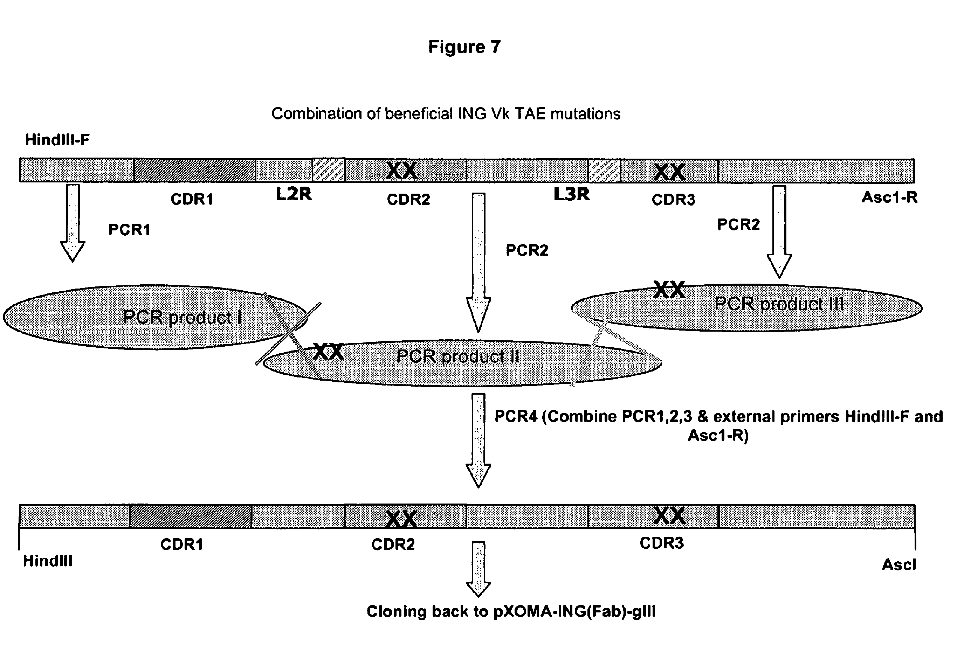

A PCR based strategy may be used to create a modified antibody light chain containing more than one amino acid change (*see, e.g.,* Figure 7). In an exemplary method, PCR may be used to amplify three segments of the Vₖ gene, two of which may be engineered to contain an amino acid change. For example, to create a light chain containing the mutations Q55R and E98T, PCR product 1 may be synthesized using the HindIII-F (SEQ ID NO: 814) and L2R primer (SEQ ID NO: 74), PCR product 2 may be synthesized using L2-Q55R primer (SEQ ID NO: 808) and the L3R primer (SEQ ID NO: 110) and PCR product 3 may be synthesized using L3-E98T primer (SEQ ID NO: 807)and the Ascl-R primer (SEQ ID NO: 812). The PCR products are then melted and re-annealed such that their regions of overlap hybridize. Subsequently, all three PCR products may be joined into one molecule by PCR amplification using the forward primer from PCR product 1 (HindIII-For) (SEQ ID NO: 814) and the reverse primer from PCR product 3 (Ascl-R) (SEQ ID NO: 812). In an exemplary method to create a heavy chain containing the mutations outlined above and described in Figure 7, product 1 may be synthesized using the Ascl-F (SEQ ID NO: 813) and H1R primer (SEQ ID NO: 146), PCR product 2 may be synthesized using H1-28TI primer and the H3R primer (SEQ ID NO: 247) and PCR product 3 may be synthesized using each H3 combination primer (6 primers, 6 rxns) and the Notl-R primer (SEQ ID NO: 285). The PCR products are then melted and re-annealed such that their regions of overlap hybridize. Subsequently, all three PCR products may be joined into one molecule by PCR amplification using the forward primer from PCR product 1 (AscI-F) (SEQ ID NO: 813) and the reverse primer from PCR product 3 (Notl-R) (SEQ ID NO: 285).

In an exemplary method, a 50 µL PCR reaction for the production of PCR product 1, 2 and 3 may be performed with 25 pmol of each of the forward and reverse primers, 10 ng of template DNA, 5 µL PFU buffer, 2.5 µL of 10 µM dNTPs, 1 µL PFU and water to 50 µL. The PCR reaction is heated to 94°C for two minutes, followed by 25 cycles of 30 seconds at 94°C, 30 seconds at 54°C, and one minute at 72°C. After the 25 cycles, a final 72°C incubation may be performed for five minutes.

An equal mass of the three PCR products may be combined in a PCR reaction to produce a modified variable domain with several amino acid changes which enhance affinity. Briefly, the PCR may be conducted by adding approximately 2 µL of each pooled PCR reaction to 5 µL PFU buffer, 25 pmol of both HindIII-f primer (SEQ ID NO: 814)and Ascl-R primers (SEQ ID NO: 812), 2.5 µL of 10 µM dNTPs, 1 µL PFU polymerase and water to 100 µL. Next, the PCR reaction is heated to 94°C for two minutes, followed by twenty-five cycles of thirty seconds at 94°C, 30 seconds at 54°C, and finally one minute at 72°C. After the twenty cycles, a final 72°C incubation is performed for five minutes.

The resulting DNA fragment may be purified (*e.g.,* using the QIAGEN^{®} PCR purification kit (Valencia, CA)) and sequentially digested with *Hind*III (NEB) and then AscI (NEW ENGLAND BIOLABS^{®}, Ipswich, MA) such that it may be cloned into the pXOMA Fab or pXOMA Fab-gIII vector.

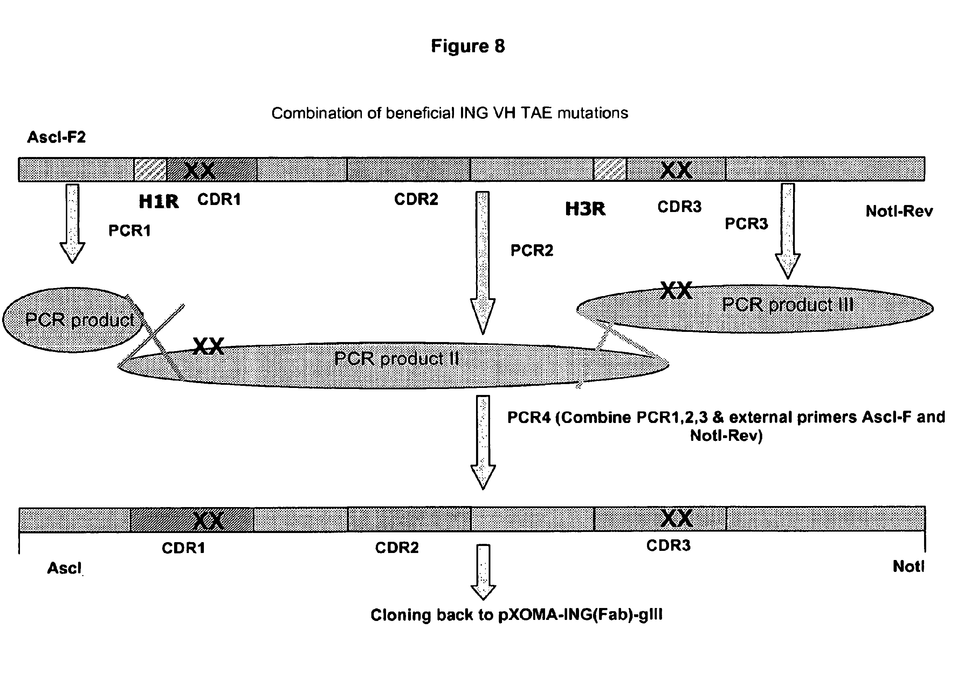

For the heavy chain modifications, a similar PCR based strategy may be used to create a modified antibody heavy chain containing more than one amino acid change (see, *e.g.,* Figure 8). In an exemplary method, PCR may be used to amplify four segments of the V_{H} gene, three of which may be engineered to contain the G33F, T53I and G100R amino acid changes. For example, PCR product 1 may be synthesized using the AscI-F (SEQ ID NO: 813) and H1R primers (SEQ ID NO: 146), PCR product 2 may be synthesized using the H1-G33F primer (SEQ ID NO: 809) and H2R primer (SEQ ID NO: 182), PCR product 3 may be synthesized using H2-T3I primer (SEQ ID NO: 810) and H3R primer (SEQ ID NO: 247) and PCR product 4 may be synthesized using H3-G100R primer (SEQ ID NO: 811) and the NotI-R primer (SEQ ID NO: 285). The PCR products are then melted and re-annealed such that their regions of overlap hybridize. All four PCR products may then be joined into one molecule by PCR amplification using the forward primer from PCR product 1 (AscI-F) (SEQ ID NO: 813) and the reverse primer from PCR product 3 (Notl-R) (SEQ ID NO: 285).

In an exemplary method, a 50 µL PCR reaction for the production of PCR products 1, 2, 3 and 4 may be performed with 25 pmol each of the forward and reverse primers, 10 ng of template DNA, 5 µL PFU buffer, 2.5 µL of 10 µM dNTPs, 1 µL PFU and water to 50 µL. The PCR reaction is heated to 94°C for 2 minutes, followed by 25 cycles of 30 sec at 94°C, 30 seconds at 54°C, and one minute at 72°C. After the 25 cycles, a final 72°C incubation may be performed for five minutes.

An equal mass of the four PCR products may be combined in a PCR reaction to produce a modified variable domain with several amino acid changes which enhance affinity. Briefly, the PCR may be conducted by adding approximately 2 µL of each pooled PCR reaction to 5 µL PFU buffer, 25 pmol of both AscI-F primer (SEQ ID NO: 813) and NotI-R primer (SEQ ID NO: 285), 2.5 µL of 10 µM dNTPs, 1 µL PFU polymerase and water to 100 µL. Next, the PCR reaction is heated to 94°C for two minutes, followed by twenty-five cycles of thirty seconds at 94°C, 30 seconds at 54°C, and finally one minute at 72°C. After the twenty cycles, a final 72°C incubation is performed for five minutes.

The heavy chain PCR fragments and the vector will be digested with *Asc*I (NEW ENGLAND BIOLABS^{®}, Ipswich, MA) and *Not*I (NEW ENGLAND BIOLABS^{®}, Ipswich, MA) such that it may be cloned into the pXOMA Fab or pXOMA Fab-gIII vector.

### Example 10: Biacore Measurement of Protien Affinity

Proteins including, for example, IgGs that bind a binding partner (*e.g.,* Epcam) are tested by BIACORE^{®} for affinity (see, *e.g.,* Figure 15). For example, kinetic analysis of anti-Epcam mAb's are conducted on a Biacore 2000®.

In an exemplary method, the ING1 antibody is diluted to 0.5 µg/mL in HBS-EP running buffer and injected for two minutes at 5 µl/ minute over a high density protein A/G surface. Next, six serial 3 fold dilutions of Epcam are prepared in running buffer and injected in triplicate in random order over the high density protein A/G surface with buffer injections evenly distributed throughout the run. The sample injections are then double referenced against the blank flow cells and buffer injections to correct for any bulk shift or non-specific binding. Data are then analyzed with the Biaevaluation software from Biacore and sensorgrams are fit utilizing the 1:1 langmuir model (see, *e.g.,* Figure 15).

### Example 11: Construction of Arrays of Modified Proteins

Arrays of modified proteins including, for example, antibody variable domains (*e.g.,* modified ING-1 variable domains) with amino acids changes at desired positions (*e.g.,* contacting (C) residues) may be generated and tested for enhanced binding affinity compared to the parent protein (*e.g.,* ING-1). Modified variable domains used in the array may be obtained directly from a library of modified variable domains as described in Example 2 or may first be screened for those modified variable domains that exhibit enhanced binding as compared to the parent variable domain as described in Examples 3, 4 and 5.

In an exemplary method, each contacting (C) residue in the heavy and light chain variable region of ING-1 is separately changed (*e.g.,* by PCR mutagenesis) with alanine, arginine, asparagine, aspartic acid, glutamine, glutamine acid, glycine, histidine, isoleucine, leucine, lysine, phenylalanine, proline, serine, threonine, tryptophan, tyrosine and valine to generate modified ING-1 variable domains. CDNAs encoding the modified ING-1 variable domains are then inserted into a pXOMA vector and used to transform electrocompetent TG1 cells. The clones are plated on 2YT- Amp₁₀₀/2%Glucose plates (Teknova) and the plates filled with 250 µl of 2YT-Amp₁₀₀/well (Teknova). Each well is inoculated with a single colony comprising a single amino acid change at a contacting (C) residue. The colonies are grown by incubating the plates at 37°C for two to four hours with shaking at 450 rpm. After the incubation, the plates are duplicated to sequencing plates by filling new deep-well culture plates (Thomson) with one milliliter of 2YT-Amp₁₀₀Gluc_{2%}/well from the grown cultures. The Genetix 96-pin replicator is used to transfer cells from the master plate to the new sequencing plates. The sequencing plates are grown overnight at 37°C with shaking at 450 rpm. After the incubation, the sequencing plate is spun down at 5000 rpm for ten minutes and the supernatant is discarded. Samples from the plate are sequenced (e.g., samples may be submitted for automated miniprep and automated sequencing (Elim biopharmaceuticals). After the incubation, Master Plates are made by adding glycerol to a final concentration of 15% to the wells on the glycerol plate and storing the plates at -80°C. The unique clones and their well position in the master plate are identified after sequencing results are returned.

Eighteen different clones, each containing an amino acid change at a contacting (C) residue in ING-1, are identified (typically 96 sequenced clones yield all eighteen clones). Unique clones from the master plates are rearrayed to a new 96-well master plate containing 2YT- Amp₁₀₀ by transferring ten microliters of glycerol stock from the master plate to the rearrayed master plate. Alternatively, automation, such as the QPIX II is used to transfer the glycerol stock containing the unique clones to the new master plate. The new rearrayed glycerol master plates are replicated into new expression plates to perform Biacore (*e.g.,* Biacore A100) analysis (*see, e.g.,* Table 8 and Table 9). Arrays may also be constructed for XPA 23 modified antibodies (see, e.g., Table 10 and 11).

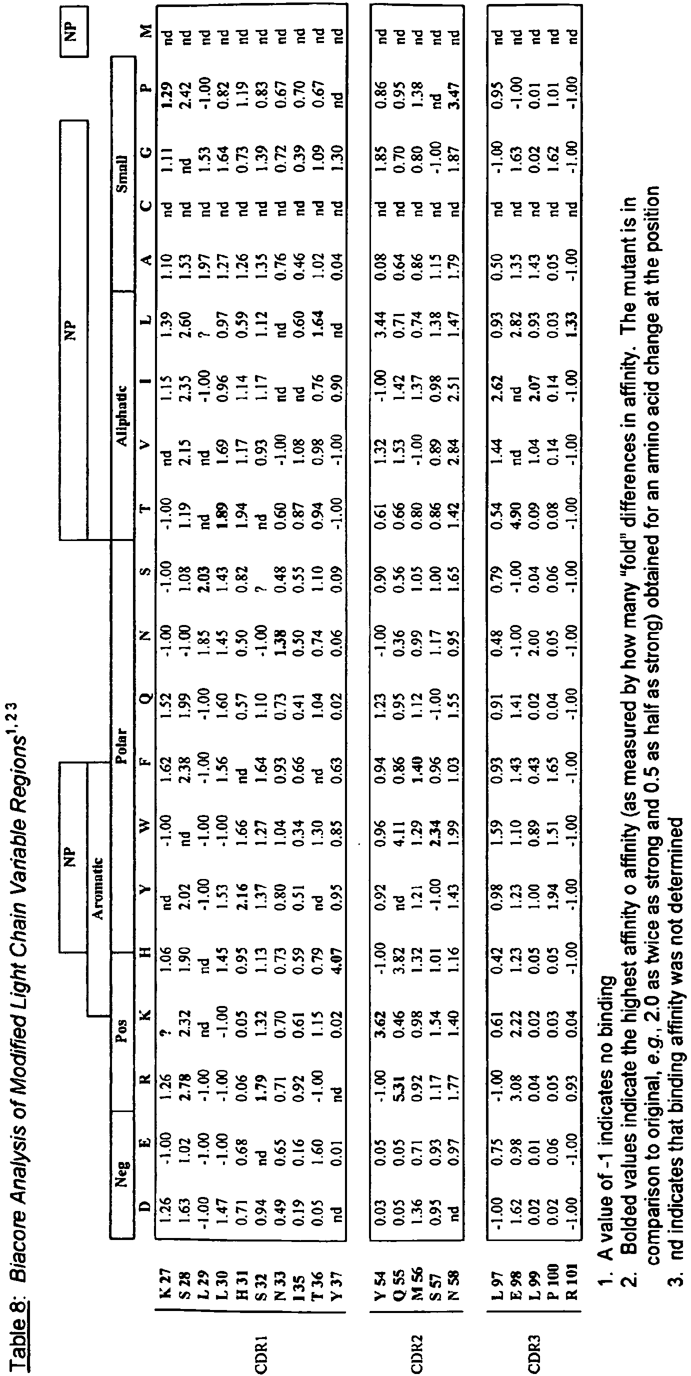

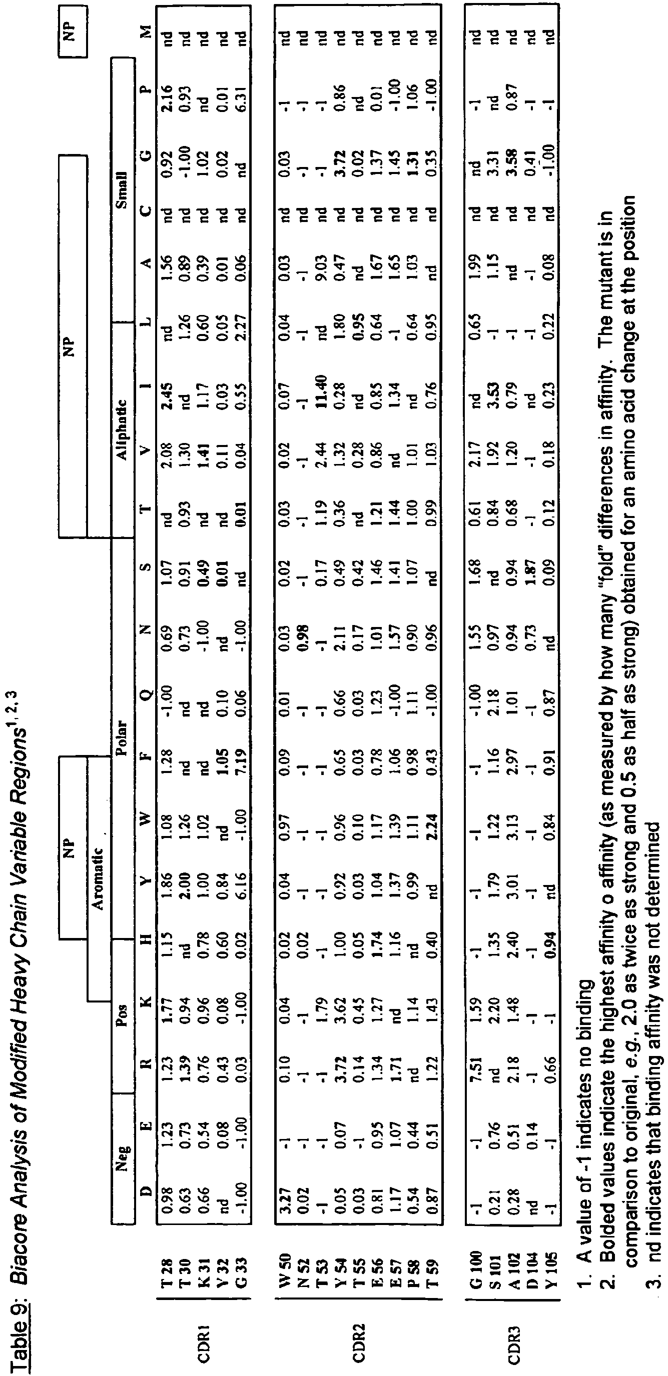

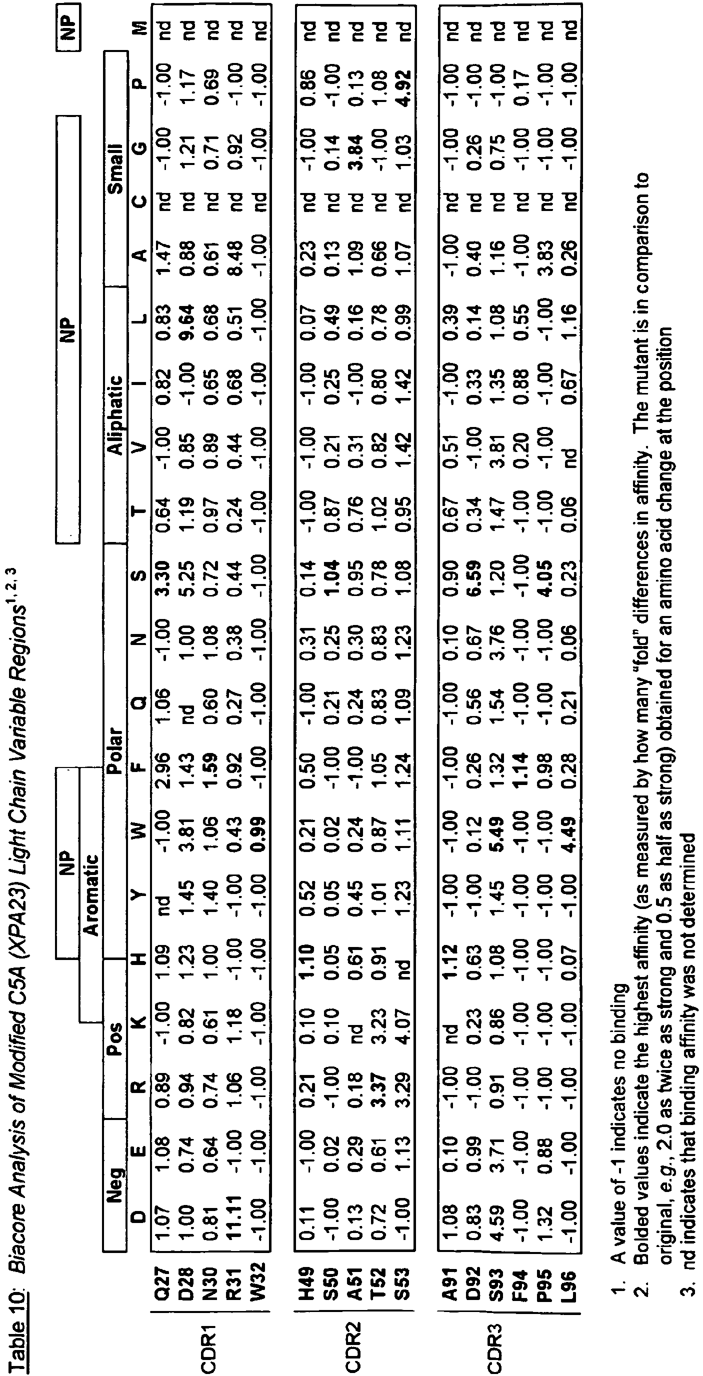

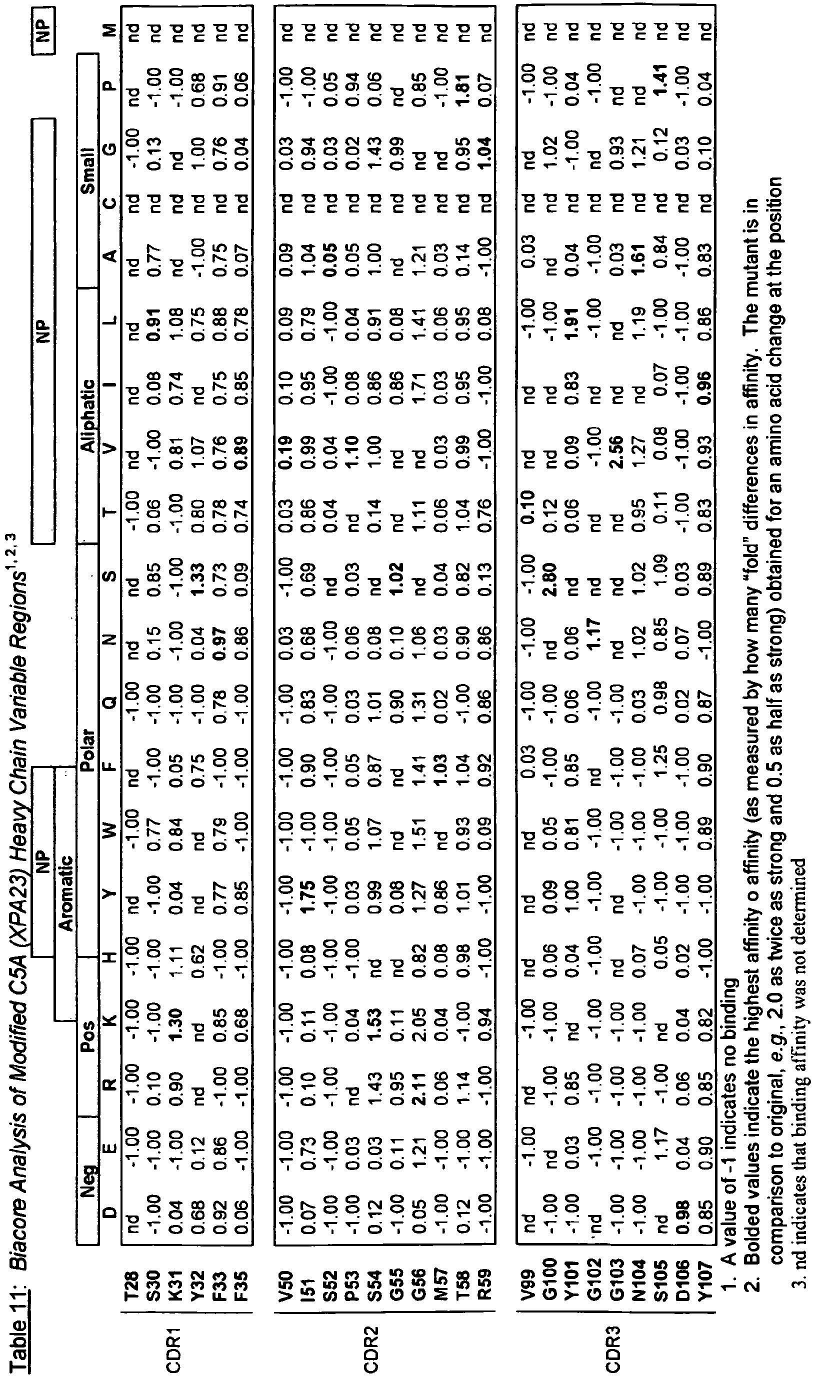

### Example 12: Construction of Arrays of Modified Proteins

Arrays of modified proteins including, for example, antibody variable domains (*e.g.,* modified ING-1 variable domains) with amino acids changes at desired positions (*e.g.,* contacting (C) residues) may be generated and tested for enhanced binding affinity compared to the parent protein (*e.g.,* ING-1). Modified variable domains used in the array may be obtained directly from a library of modified variable domains as described in Example 2 or may first be screened for those modified variable domains that exhibit enhanced binding as compared to the parent variable domain as described in Examples 3, 4 and 5.

In an exemplary method, each contacting (C) residue in the heavy and light chain variable region of ING-1 is separately changed (*e.g.,* by PCR mutagenesis) with alanine, arginine, asparagine, aspartic acid, glutamine, glutamine acid, glycine, histidine, isoleucine, leucine, lysine, phenylalanine, proline, serine, threonine, tryptophan, tyrosine and valine to generate modified ING-1 variable domains. cDNAs encoding the modified ING-1 variable domains are then inserted into a pXOMA vector and used to transform electrocompetent TG1 cells. The clones are plated on 2YT- Amp₁₀₀/2%Glucose plates (Teknova) and the plates filled with 250 µl of 2YT-Amp₁₀₀/well (Teknova). Each well is inoculated with a single colony comprising a single amino acid change at a contacting (C) residue. The colonies are grown by incubating the plates at 37°C for two to four hours with shaking at 450 rpm. After the incubation, the plates are duplicated to expression plates by filling new plates (Costar) with two hundred and fifty microliters of 2YT-Amp100 media (Teknova). The Genetix 96-pin replicator is used to transfer cells from the Master plate to the new expression plates The culture is grown at 37°C until cloudy (e.g., approximate OD₆₀₀=0.5), inoculated with IPTG to a final concentration of 1 mM and grown overnight at 30°C.

Next, periplasmic extracts (PPE) of the overnight expression constructs are prepared by spinning the overnight expression plates at 3000 rpm for fifteen minutes, discarding the supernatant and adding 60 µl of PPB buffer to each well. The pellets are resuspended, and 90 µl of cold PPB diluted 1:5 with cold water is added to each well. This mixture is incubated on ice for one hour and subsequently spun down at 3000 rpm for fifteen minutes. The supernatant is transferred to a new plate and the periplasmic extracts are used for the Biacore (*e.g.,* Biacore A100) determination.

After Biacore determination, wells that contain clones with improved off rates are sequenced and further characterized (*e.g.* IgG reformatting and affinity determination).

### Example 13: Affinity Optimization of Protein by Targeted Mutagenesis of Selected Amino Acid Residues.

Affinity optimized proteins including, for example, antibodies or fragments thereof may be obtained by mutation of one or more selected amino acid residues in a parent protein with other amino acid residues (*e.g.,* alanine, arginine, asparagine, aspartic acid, glutamine, glutamic acid, glycine, histidine, isoleucine, leucine, lysine, phenylalanine, proline, serine, threonine, tryptophan, tyrosine or valine). Methods for optimization of an exemplary antibody variable domain may comprise the stages as set forth below.

### A. Selection of Amino Acid Residues for Mutation

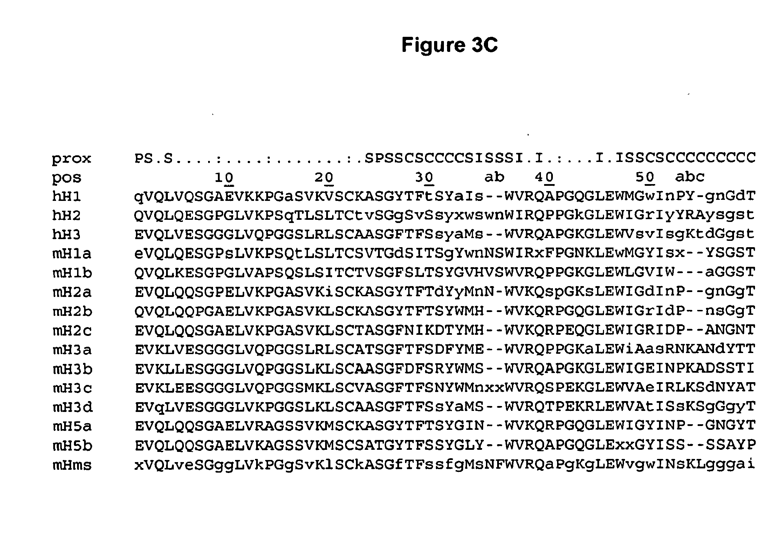

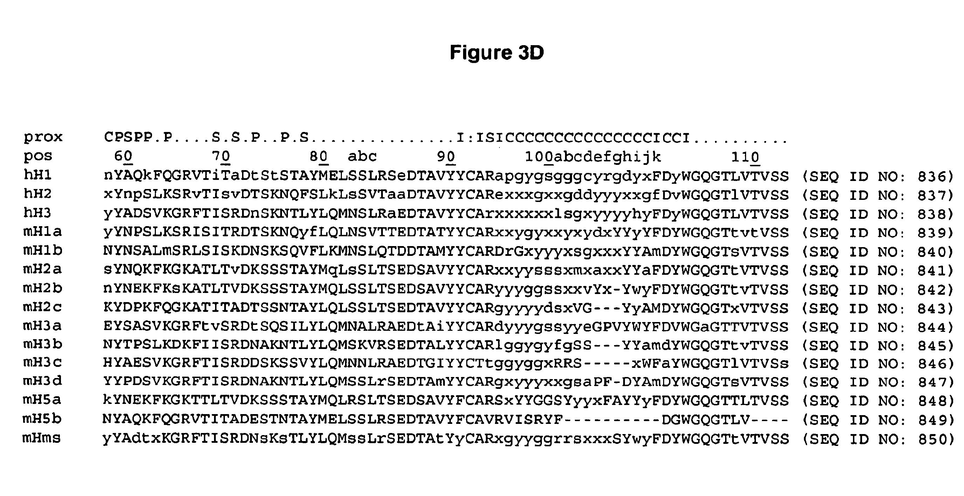

Amino acid residues at one or more positions in a parent antibody or binding fragment thereof are selected for mutagenesis. Such methods may include, for example, identifying the proximity assigned to amino acid positions in the variable domain of the antibody using the "prox" line as shown in Figure 3A, 3B, 3C and/or 3D. One or more amino acid resdies identified as C, P, S and/or I residues may be selected for mutation.

### B. Design of Primers for Mutagenesis

Primers are designed to mutagenize a parent nucleic acid sequence that codes for an antibody or binding fragment thereof.

For a PCR-based mutagenesis method, a primer may be designed such that the forward primer sequence flanks both sides (*e.g.,* 20 base pairs) of the position to be mutated. Additionally, it is preferred that the primer be 70 bases or les in length. A representative CDR comprising amino acid residues 1-8 is shown below.

| aa# | 1 | 2 | 3 | 4 | 5 | 6 | 7 | 8 |
|---|---|---|---|---|---|---|---|---|
| | G | F | T | F | S | K | Y | F |

If the CDR is too long to incorporate all the desired mutations and remain under 70 nucleotides, the mutagenesis region may be broken up into two regions. An example of this process is shown below, where the 8 amino acid CDR as shown above is broken into two 4 amino acid regions (region 1 and region 2, respectively).
Region 1:

| aa# | 1 | 2 | 3 | 4 |
|---|---|---|---|---|
| | G | F | T | F |

Region 2:

| aa# | 5 | 6 | 7 | 8 |
|---|---|---|---|---|
| | S | K | Y | F |

Sets of primers may be constructed to incorporate all 18 amino acid mutations at each positon in region 2. Each codon selected for mutation may be replaced with NHT, VAA or BGG in the sense direction. Exemplary primer sets for mutation of each of positions 5-8 are shown below.

Mutation of the S position (aa5) in region 2 above may be accomplished by the following primers:R2-5-NHT 5'- GCTGCTTCCGGATTCACTTT-CNHTAAGTACTTTATGTTTTGGGTTCGCCAAGC-3'(SEQ ID NO: 970); R2-5-VAA 5'-GCTGCTTCCGGATTCACTTTCVAAAAGTACTTTATGTTTTGGGTTCGCCAAGC-3' (SEQ ID NO: 971); and R2-5-BGG 5'-GCTGCTTCCGGATTCACTTTCBGGAAGTAC-TTTATGT-TTTGGGTTCGCCAAGC-3' (SEQ ID NO: 972).

Mutation of the K position (aa6) in region 2 above may be accomplished by the following primers: R2-6-NHT 5'- GCTGCTTCCGGATTCACTTT CTCTNHTTACTTTATGTTTTGGGTTCGCCAAGC-3' (SEQ ID NO: 973); R2-6-VAA 5'- GCTGCTTCCGGATTCACTTTCTCTVAATACTTTATGTTTTGGGTTCGCCAAGC-3' (SEQ ID NO: 974); and R2-6-BGG 5'-GCTGCTTCCGGATTCACTTTCTCTBGGTAC TTTATGTTTTGGGTTCGCCAAGC-3' (SEQ ID NO: 975).

Mutation of the Y position (aa7) in region 2 above may be accomplished by the following primers: R2-7-NHT 5'- GCTGCTTCCGGATTCACTTT CTCTAAGNHTTTTATGTTTTGGGTTCGCCAAGC-3' (SEQ ID NO: 976); R2-7-VAA 5'-GCTGCTTCCGGATTCACTTTCTCTAAGVAATTTATGTTTTGGGTTCGCCAAGC-3' (SEQ ID NO: 977); and R2-7-BGG 5'- GCTGCTTCCGGATTCACTTTCTCTAAGBGG TTTATGTTTTGGGTTCGCCAAGC-3' (SEQ ID NO: 978).

Mutation of the F position (aa8) in region 2 above may be accomplished by the following primers: R2-8-NHT 5'- GCTGCTTCCGGATTCACTTT CTCTAAGTACNHTATGTTTTGGGTTCGCCAAGC-3' (SEQ ID NO: 979); R2-8-VAA 5'-GCTGCTTCCGGATTCACTTTCTCTAAGTACVAAATGTTTTGGGTTCGCCAAGC-3' (SEQ ID NO: 980); and R2-8-BGG 5'- GCTGCTTCCGGATTCACTTTCTCTAAGTAC BGGATGTTTTGGGTTCGCCAAGC-3' (SEQ ID NO: 981).

Alternatively, modified antibody variable domains containing amino acid changes at one or more contacting (C) residues present within an exemplary antibody may be synthesized by QUIKCHANGE™ site-directed mutagenesis (STRATAGENE, Texas).

In an exemplary method, QUIKCHANGE™ site-directed mutagenesis may be performed to replace one or more codons in an antibody variable region (*e.g.,* a CDR) such as XPA-23. Mutagenic primers are designed to contain the desired mutation and anneal to the same sequence on opposite strands of a plasmid comprising a nucleotide coding for XPA-23. Preferably, the desired mutation in the middle of the primer contains 20 bases of correct sequence on both sides of the nucleic acid flanking the mutation. The XPA-23 CDR1 coding region is shown below.

| aa# | 1 | 2 | 3 | 4 | 5 | 6 | 7 | 8 |
|---|---|---|---|---|---|---|---|---|
| | G | F | T | F | S | K | Y | F |

Primers for QUIKCHANGE™ site-directed mutagenesis are synthesized such that they are complementary to a parent nucleic acid sequence with the exception that they comprise a NHT, a VAA, or a BGG codon in the sense direction, and a ADN, a TTB, or a CCV codon in the antisense direction at the position to be mutagenized in the parent nucleic acid. Exemplary primers for mutagenesis of each of the eight amino acid residues in the XPA-23 heavy chain CDR1 are shown below and comprise a degenerate codon (underlined nucleotide triplet):

Mutation of the G position (aa1) may be accomplished by the following primers: 5'-GTCTTTCTTGCGCTGCTTCCNHTTTCACTTTCTCTAAGTACTTTATG-3' (SEQ ID NO: 853) and 3'-CAGAAAGAACGCGACGAAGGNDAAAGTGAAAG-AGATTCATGAAATAC-5' (SEQ ID NO: 854); 5'-GTCTTTCTTGCGCTGCTTCCVAAT-TCACTTTCTCTAAGTACTTTATG-3' (SEQ ID NO: 855) and 3'-CAGAAAGAACGC-GACGAAGGBTTAAGTGAAAGAGATTCATGAAATAC-5' (SEQ ID NO: 856); and 5'-GTC-TTTCTTGCGCTGCTTCCBGGTTCACTTTCTCTAAGTACTTTATG-3' (SEQ ID NO: 857) and 3'-CAGAAAGAACGCGACGAAGGVCCAAGTGAAAGAGATTCATGAA-ATAC-5' (SEQ ID NO: 858).

Mutation of the F position (aa2) may be accomplished by the following primers: 5'-CTTTCTTGCGCTGCTTCCGGANHTACTTTCTCTAAGTACTTTATG-3' (SEQ ID NO: 859) and 3'-GAAAGAACGCGACGAAGGCCTNDATGAAAGAGATTCAT-GAAATAC-5' (SEQ ID NO: 860); 5'-CTTTCTTGCGCTGCTTCCGGAVAAACTTTC-TCTAAGTACTTTATG-3' (SEQ ID NO: 861) and 3'-GAAAGAACGCGACGAAGGC-CTBTTTGAAAGAGATTCATGAAATAC-5' (SEQ ID NO: 862); and 5'-CTTTCTTGC-GCTGCTTCCGGABGGACTTTCTCTAAGTACTTTATG-3' (SEQ ID NO: 863) and 3'-GAAAGAACGCGACGAAGGCCTVCCTGAAAGAGATTCATGAAATAC-5' (SEQ ID NO: 864).

Mutation of the T (aa3) position may be accomplished by the following primers: 5'-CTTGCGCTGCTTCCGGATTCNHTTTCTCTAAGTACTTTATGTTTTG-3' (SEQ ID NO: 865) and 3'-GAACGCGACGAAGGCCTAAGNDAAAGAGATTCATGA-AATACAAAAC-5' (SEQ ID NO: 866); 5'-CTTGCGCTGCTTCCGGATTCVAATT-CTCTAAGTACTTTATGTTTTG-3' (SEQ ID NO: 867) and 3'-GAACGCGACGAAGGC-CTAAGBTTAAGAGATTCATGAAATACAAAAC-5' (SEQ ID NO: 868); and 5'-CTTGCGCTGCTTCCGGATTCBGGTTCTCTAAGTACTTTATGTTTTG-3' (SEQ ID NO: 869) and 3'-GAACGCGACGAAGGCCTAAGVCCAAGAGATTCATGAAATACAAAAC-5' (SEQ ID NO: 870).

Mutation of the F (aa4) position may be accomplished by the following primers: 5'-CGCTGCTTCCGGATTCACTNHTTCTAAGTACTTTATGTTTTGGG-3' (SEQ ID NO: 871) and 3'-G CGACGAAGGCCTAAGTGANDAAGATTCATGAAA-TACAAAACCC-5' (SEQ ID NO: 872); 5'-CGCTGCTTCCGGATTCACTVAATCTAA-GTACTTTATGTTTTGGG-3' (SEQ ID NO: 873) and 3'-GCGACGAAGGCCTAAGTGA-BTTAGATTCATGAAATACAAAACCC-5' (SEQ ID NO: 874); and 5'-CGCTGCTTCC-GGATTCACTBGGTCTAAGTACTTTATGTTTTGGG-3' (SEQ ID NO: 875) and 3'-G CGACGAAGGCCTAAGTGAVCCAGATTCATGAAATACAAAACCC-5' (SEQ ID NO: 876).

Mutation of the S (aa5) position may be accomplished by the following primers: 5'-CTGCTTCCGGATTCACTTTCNHTAAGTACTTTATGTTTTGGGTTCG-3' (SEQ ID NO: 877) and 3'-GACGAAGGCCTAAGTGAAAGNDATTCATGAAAT-ACAAAACCCAAGC-5' (SEQ ID NO: 878); 5'-CTGCTTCCGGATTCACTTTCVAAAA-GTACTTTATGTTTTGGGTTCG-3'(SEQ ID NO: 879) and 3'-GACGAAGGCCT-AAGTGAAAGBTTTTCATGAAATACAAAACCCAAGC-5'(SEQ ID NO: 880); and 5'-CTGCTTCCGGATTCACTTTCBGGAAGTACTTTATGTTTTGGGTTCG-3'(SEQ ID NO: 881) and 3'-GACGAAGGCCTAAGTGAAAGVCCTTCATGAAATACAAAACCCAAGC-5'(SEQ ID NO: 882).

Mutation of the K (aa6) position may be accomplished by the following primers: 5'-CTTCCGGATTCACTTTCTCTNHTTACTTTATGTTTTGGGTTCGCC-3'(SEQ ID NO: 883) and 3'-GAAGGCCTAAGTGAAAGAGANDAATGAAATACAAAAC-CCAAGCGG-5'(SEQ ID NO: 884); 5'-CTTCCGGATTCACTTTCTCTVAATACTTT-ATGTTTTGGGTTCGCC-3'(SEQ ID NO: 885) and 3'-GAAGGCCTAAGTGAAAGAG-ABTTATGAAATACAAAACCCAAGCGG-5'(SEQ ID NO: 886); and 5'-CTTCCGGA-TTCACTTTCTCTBGGTACTTTATGTTTTGGGTTCGCC-3'(SEQ ID NO: 887) and 3'-GAAGGCCTAAGTGAAAGAGAVCCATGAAATACAAAACCCAAGCGG-5'(SEQ ID NO: 888).

Mutation of the Y (aa7) position may be accomplished by the following primers: 5'-CCGGATTCACTTTCTCTAAGNHTTTTATGTTTTGGGTTCGCCAAG-3'(SEQ ID NO: 889) and 3'-GGCCTAAGTGAAAGAGATTCNDAAAATACAAAA-CCCAAGCGGTTC-5'(SEQ ID NO: 890); 5'-CCGGATTCACTTTCTCTAAGVAATT-TATGTTTTGGGTTCGCCAAG-3'(SEQ ID NO: 891) and 3'-GGCCTAAGTGAAAGA-GATTCBTTAAATACAAAACCCAAGCGGTTC-5'(SEQ ID NO: 892); and 5'-CCGGATTCACTTTCTCTAAGBGGTTTATGTTTTGGGTTCGCCAAG-3'(SEQ ID NO: 893) and 3'-GGCCTAAGTGAAAGAGATTCVCCAAATACAAAACCCAAGCGGTTG 5'(SEQ ID NO: 894).

Mutation of the F (aa8) position may be accomplished by the following primers: 5'-GGATTCACTTTCTCTAAGTACNHTATGTTTTGGGTTCGCCAAGC-3' (SEQ ID NO: 895) and 3'-CCTAAGTGAAAGAGATTCATGNDATACAAAACCCAA-GCGGTTCG-5' (SEQ ID NO: 896); 5'-GGATTCACTT'TCTCTAAGTACVAAATGTTTT-GGGTTCGCCAAGC-3' (SEQ ID NO: 897) and 3'-CCTAAGTGAAAGAGATTCAT-GBTTTACAAAACCCAAGCGGTTCG-5' (SEQ ID NO: 898); and 5'-GGATTCACTTT-CTCTAAGTACBGGATGTTTTGGGTTCGCCAAGC-3' (SEQ ID NO: 899) and 3'-CCTAAGTGAAAGAGATTCATGVCCTACAAAACCCAAGCGGTTCG-5' (SEQ ID NO: 900).

### C. Synthesis of Full-Length Mutagenized Antibody

Full-length mutagenized antibodies may be produced by recombinant DNA technologies.

For the PCR-based method, a first PCR reaction (PCR1) is performed with a R2-rev primer and a 5'-Sfil primer, which incorporates a 5' Sfil restriction site into the amplified fragment. For each library oligonucleotide containing the mutations described above, the PCR2 reaction is performed to create the DNA fragment incorporating the primer mutation and the 3' Sfil restriction site. For the mutations in region 2, twelve PCR2 reactions will be performed with forward primers denoted R2-5 through R2-8 above (denoted primer-F in PCR2 below). The reverse primer for the mutagenic reaction will be 3'-Sfil. An appropriate amount of the following reagents may be used for PCR1: PfuUltra buffer; dNTPs [10□M], template (10 ng total), 5'-Sfil [25 pmol], R2-rev [25 pmol], PfuUltra (2.5 U/L), dH2O to 50 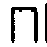 L total. An appropriate amount of the following reagents may be used for PCR2: PfuUltra buffer, dNTPs [10 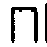 M], template (10 ng total), Primer-F [10 pmol], 3'-Sfil [25 pmol], PfuUltra (2.5 U/ 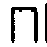 L), dH2O to 50 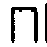 L total. PCR1 and PCR2 may be conduced according to standard protocols includinig an initial denatural step, a number of cycles including a denaturation, annealing and extension step and a final extension step for appropriate times and temperatures.

A full-length antibody fragment may be produced by performing a separate reaction for each PCR2 product. For this step, an approximately equimolar amount of PCR product 1 and 2 is combined (*e.g.,* 0.5 microliters of each PCR is combined). An appropriate amount of the following reagents may be used generation of a full-length antibody fragment: PfuUltra buffer, dNTPs [10 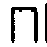 M], PCR1 product, PCR2 product, PfuUltra (2.5 U/ 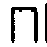 L), dH2O to 50 nL total. PCR may be conduced according to standard protocols includinig an initial denatural step, a number of cycles including a denaturation, annealing and extension step for appropriate times and temperatures.

The full-length fragment may then be amplified by directly adding to the above reaction an appropriate amount of the following reagents: PfuUltra buffer, dNTPs [10µM], 5'-Sfil [25 pmol], 3'-Sfil [25 pmol], PfuUltra (2.5 U/µL), dH2O to 50 µL total. PCR may be conduced according to standard protocols including an initial denaturation step, a number of cycles that comprise a denaturation, annealing and extension step for approapte times and temperatures and a final extension step. The PCR product may be examined on an agarose gel to ensure that the amplified DNA segment is the correct length.

Next, a vector and the DNA inserts obtained from the above PCR are digested with Sfil (NEB) according to the manufacturer's instructions and gel purified. The DNA synthesized fragment may be cloned into a pXOMA Fab or pXOMA Fab-gIII vector. Briefly, the DNA fragment is purified by using the QIAGEN^{®} PCR purification kit and sequentially digesting the fragment with *Not*I (NEW ENGLAND BIOLABS^{®}, Ipswich, MA) and *Asc*I (NEW ENGLAND BIOLABS^{®}, Ipswich, MA) (See, Methods in Molecular Biology, vol. 178: Antibody Phage Display: Methods and Protocols Edited by: P.M. O'Brien and R. Aitken, Humana Press, "Standard Protocols for the Construction of Fab Libraries, Clark, M. A., 39-58) (see, *e.g.,* Figure 6). Next, the vectors may be ligated with the mutagenized insert using T4 Ligase (NEW ENGLAND BIOLABS^{®}, Ipswich, MA) and transformed into TG1 cells by electroporation.

Alternatively, for the DPN-based method, a double-stranded DNA (*e.g*., dsDNA) vector with an antibody insert isolated from a dam+ host is used as template for mutagenesis. DNA isolated from almost all *E*. *coli* strains is dam methylated and therefore susceptible to Dpnl digestion. Two synthetic oligonucleotide primers containing the desired mutation each complementary to opposite strands of the vector, are extended during temperature cycling by DNA polymerase (*e.g.,* PfuTurbo). PCR reactions may comprise an appropriate amout of PfuUltra buffer, dNTPs [10 mM] each dNTP, template (50 ng total), Primer-F [5µM], Primer-R [5µM], PfuUltra (2.5 U/µL), DMSO, and dH2O up to 50 µL total and be conducted with the following cycling parameters: an initial denaturation, subsequent cycles of denaturation, annealing and extension and a final extension step. Incorporation of the mutagenesis primers generates a mutated plasmid containing staggered nicks. Following temperature cycling, the PCR product is treated with Dpnl and incubated at an appropriate temperature (e.g, at 37°C for 4-5 hours). The Dpnl endonuclease (target sequence: 5'-Gm6ATC-3') is specific for methylated and hemimethylated DNA and is used to digest the parental DNA template and to select for mutation-containing synthesized DNA. The nicked vector DNA containing the desired mutations is then transformed into supercompetent cells *(e.g.,* XL1-Blue).

### D. Sequencing of Mutagenized Antibodies

A library of mutagenized antibodies may comprise each of 18 unique amino acid mutations at each postion mutated. To identify all possible unique mutations an appropriate number of clones obtained from each degenerate codon are analyzed. For example, the NHT codon encodes 12 amino acids such that 72 clones from this reaction are sequenced for each mutated position. The VAA codon encodes 3 amino acids such that 12 clones are sequenced from this reaction for each mutated position. The BGG codon encodes 3 amino acids such that 12 clones from this reaction are sequenced for each mutated position. Unique clones are rearrayed into 96-well plates.

### E. Expression of Mutagenized Antibodies

Mutagenized antibodies may be expressed. In an exemplary method, starting cultures may be produced by filling a plate (*e.g.,* a 96 well plate) with an appropriate growth media (*e.g.,* 2YTAG (2YT+ 2% glucose +100 □gs/ml Ampicillin) and inoculating the plate with glycerol stocks of the mutagenized antibodies. The cultures are then grown overnight (*e.g.,* in an ATR plate shaker incubator at 37°C with shaking at 450 rpm). Next, plates are filled with an appropriate growth medium (*e.g.,* 1.2 mL per well of Superbroth + 100 □gs.ml Ampicillin +0.2% glucose). The plates are then Innoculated with an appropriate amout of the overnight culture (*e.g*, 25 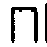 L of overnight culture). The cultures are then grown with incubation (*e.g.,* ATR plate shaker incubator at 37°C) and shaking (*e.g.,* at 700 rpm until Abs600nm = 1.5). Expression in the cultures is then induced (*e.g.,* by adding 12 uL of 100mM IPTG per well to get a final concentration of 1mM IPTG final) and incubated overnight (*e.g.,* in an ATR plate shaker incubator at 30°C with shaking at 700 rpm). Next, the plates are spun (*e.g.,* at 4000 rpm using Beckman Coulter table top centrifuge for 10 minutes) and the supernatant decanted. The cells are then vortexed to disturb and loosen the pellet. The pellets are resuspended (*e.g.,* with 75 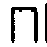 L per well of cold PPB) and incubated one ice (*e.g.,* for 10 mintues). Next, water (*e.g.,* 225 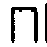 L per well) is added and the cells resuspended. The suspension is incubated on ice (*e.g.,* for 1 hour) and the plates are then spun (*e.g.,* at 4000 rpm using Beckman Coulter table top centrifuge for 20 minutes). Last, the supernatants are collected for use in assays as described in detail below.

### F. ELISA Screening of Mutagenized Antibodies

An assay including, for example, an ELISA may be performed to ensure that the mutagenized antibodies are capable of binding to their respective antigen.

In an exemplary ELISA, plates (*e.g.,* 96-well Nunc Maxisorp plates) are coated with an antibody to the mutagenized antibody (*e.g.,* 50 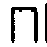 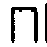 L per well of 1 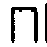 g/ml Goat anti Human IgG (Fab)₂ Jackson immunoresearch, Cat. 109-005-006) and the plates are then incubated overnight at 4°C. After incubation, the plates may be washed (*e.g.,* 3X with PBS-Tween at 350□L/well) and then blocked (*e.g.,* by adding 350□L/well with 5% Milk+ PBS).

Next, periplasmic extracts (PPE) containing the mutagenized antibody are blocked (*e.g.,* by milk(diluted in PBS) to 200□L of PPE to get a final milk percent of 5%). The PPEs are then mixed and incubated (*e.g.,* at room temperature still for 1 hour) before using as samples to screen on ELISA and then washed (*e.g.,* 3X with PBS-Tween at 350□L/well). The blocked PPE samples (*e.g.,* 50□L) are then added to the blocked ELISA plates and incubated (*e.g.,* at room temperature for 1-2 hours). Again the PPEs are washed (*e.g.,* 3X with PBS-Tween at 350 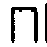 L/well). Next, an antibody specific for the mutagenized antibody is added to the PPEs (*e.g.,* 50 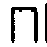 L/well of 1 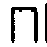 g/ml monoclonal anti-V5 antibody, Sigma Cat.# V8012-50UG) and the PPEs incubated (*e.g.,* at room temperature for 1 hour). Again the PPEs are washed (*e.g.,* 3X with PBS-Tween at 350 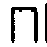 L/well). Next, a secondary antibody conjugated to a enzymatic label is added to the PPEs (*e.g.,* 1:10000 diluted Goat anti mouse HRP conjugated, Biorad, Cat. 170-5047) and incubated wth the PPEs (*e.g.,* for 1 hour at room temperature). Again the PPEs are washed (*e.g.,* 3X with PBS-Tween at 350 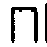 L/well). Next, an appropriate amount of substrate for the enzymatic label is added to the PPEs (*e.g.,* 50□L/well of TMB, soluble, Calbiochem, Cat. 613544) and the enzyme is allowed time to act on the substrate (*e.g.,* until sufficiently blue color develops). The reaction may be stopped by the addition of an agent that sequesters the substrate and/or and agent that inhibits the enzymatic activity of the secondary antibody (*e.g,* 50□L per well of 2N H₂SO₄). Last, absorbance of the samples are read at 450nm.

### G. Ranking of Mutagenized Antibodies

Mutagenized antibodies may be ranked based on their dissociation rate from their respective antigen.

In an exemplary method, a Biacore A100 screening protocol may be used to rank mutagenized antibody clones. For example, a CM5 chip may be docked and normalized using normalization solution (*e.g*., using A100 normalization solution and use and an appropriate running buffer (*e.g.,* HBS-N (0.01 M HEPES pH 7.4, 0.15 M NaCl). After normalization, software is set to immobilize antigen on desired spots of each flow cell. For antigen surface preparation the surface may be activated (*e.g.,* with NHS/EDC mixture from the amine coupling kit for 5 minutes at 10 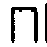 l/min). Antigen is then diluted (*e.g.,* in 10 mM sodium acetate buffer) and the surface of the CM5 chip is blocked (*e.g.,* with 1 M ethanolamine HCl pH 8.5 for 5 min at 10 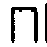 l/min). Next, each sample comprising a mutagenized antibody is injected over the CM5 chip (*e.g.,* for 3 min at 30 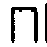 l/min flow rate with 600s dissociation) at an appropriate temperature (*e.g.,* 25°C). Biaevaluation software (*e.g.,* Biacore A100 evaluation software) is then used to calculate dissociation rates of individual samples and the relative amount of sample bound to each test surface. The data is fit to an appropriate kinetic model (*e.g.,* the kinetic titration model).

## Claims

1. A method of mutagenesis of a parent nucleic acid encoding a protein to generate modified proteins, said method comprising:
(a.) obtaining a primer set comprising primers that each comprise at least one 2 to 12 fold degenerate codon, wherein each primer comprises at least two oligonucleotide sequences that are complementary to a sequence in the parent nucleic acid, and wherein the degenerate codons non-redundantly code for an equal representation of eighteen amino acid substitutions with the exception of cysteine or methionine at each of one or more amino acid position(s) encoded by the parent nucleic acid; and
(b.) mutating the parent nucleic acid by replication or polymerase based amplification using the primer set obtained in (a), wherein replication or amplification of the parent nucleic acid with the primers generate mutated nucleic acids that encode said equal representation of eighteen amino acid substitutions in modified proteins.

2. The method of claim 1 , wherein the amino acid substitutions are selected from the group consisting of: alanine, arginine, asparagine, aspartic acid, glutamine, glutamine acid, glycine, histidine, isoleucine, leucine, lysine, phenylalanine, proline, serine, threonine, tryptophan, tyrosine and valine at each position.

3. The method of claim 1 or 2, wherein three primers that each comprise at least one 2 to 12 fold degenerate codon are obtained.

4. The method of claim 1 or 2, wherein seven primers that each comprise at least one 2 to 12 fold degenerate codon are obtained.

5. The method of claim 3, wherein the degenerate codons are selected from the group consisting of: NHT or NHC (where N=A/G/C/T, H=A/C/T), VAG or VAA (where V=A/C/G) and BGG or DGG (where B=C/G/T, D=A/G/T).

6. The method of claim 4, wherein the degenerate codons are selected from the group consisting of: ARG (where R=A/G), WMC (where W=A/T and M=A/C), CAS (where S=C/G), GAS (where S=C/G), NTC (where N=A/G/C/T), KGG (where K=G/T) and SCG (where S=C/G).

7. The method of claim 1, wherein the parent nucleic acid encodes a binding molecule, optionally wherein the binding molecule is an antibody or fragment thereof.

8. The method of claim 1 further comprising selecting the one or more positions in the parent nucleic acid sequence for mutation.

9. The method of claim 1 further comprising transforming the mutated nucleic acid sequences into competent cells.

10. A library/array of mutated nucleic acid sequences generated by the method of claim 1.

11. A set of primers that each comprise at least one 2 to 12 fold degenerate codon, wherein each primer comprises at least two oligonucleotide sequences that are complementary to a sequence in a parent nucleic acid and wherein the degenerate codons non-redundantly code for an equal representation of eighteen amino acid substitutions with the exception of cysteine or methionine at each of one or more amino acid position(s) encoded by the parent nucleic acid.

12. The set of primers of claim 11, wherein the primers each comprise one or more degenerate codons as represented by NHT or NHC (where N=A/G/C/T, H=A/C/T), VAG or VAA (where V=A/C/G) and BGG or DGG (where B=C/G/T, D=A/G/T).

13. The set of primers of claim 11, wherein the primers each comprise one or more degenerate codons as represented by ARG (where R=A/G), WMC (where W=A/T and M=A/C), CAS (where S=C/G), GAS (where S=C/G), NTC (where N=A/G/C/T), KGG (where K=G/T) and SCG (where S=C/G).

14. A kit for mutagenesis of an amino acid residue encoded by a parent nucleic acid, the kit comprising: a set of primers that each comprise at least one 2 to 12 fold degenerate codon, wherein each primer comprises at least two oligonucleotide sequences that are complementary to a sequence in a parent nucleic acid and wherein the degenerate codons non-redundantly code for an equal representation of eighteen amino acid substitutions with the exception of cysteine or methionine at each of one or more amino acid position(s) encoded by the parent nucleic acid.

## Patentansprüche

1. Verfahren zur Mutagenese einer Stammnukleinsäure, die ein Protein kodiert, um modifizierte Proteine herzustellen, wobei das Verfahren umfasst:
(a) Beschaffen eines Primersets umfassend Primer, die jeweils wenigstens ein 2- bis 12-fach degeneriertes Kodon umfassen, wobei jeder Primer wenigstens zwei Oligonukleotidsequenzen, welche komplementär zu einer Sequenz in der Stammnukleinsäure sind, umfasst und wobei die degenerierten Kodons nicht redundant für eine gleiche Repräsentation von achtzehn Aminosäure-Substitutionen mit der Ausnahme von Cystein oder Methionin an jeder der einen oder mehreren durch die Stammnukleinsäure kodierten Aminosäure-Position(en) kodieren; und
(b) Mutieren der Stammnukleinsäure durch Replikation oder Polymerase-basierte Amplifikation unter Verwendung des Primersets aus (a), wobei die Replikation oder Amplifikation der Stammnukleinsäure mit den Primern mutierte Nukleinsäuren, welche die gleichen Repräsentationen von achtzehn Aminosäure-Substitutionen in modifizierten Proteinen kodieren, herstellt.

2. Verfahren gemäß Anspruch 1, wobei die Aminosäure-Substitutionen ausgewählt sind aus der Gruppe bestehend aus Alanin, Arginin, Asparagin, Asparaginsäure, Glutamin, Glutaminsäure, Glycin, Histidin, Isoleucin, Leucin, Lysin, Phenylalanin, Prolin, Serin, Threonin, Tryptophan, Tyrosin und Valin an jeder Position.

3. Verfahren gemäß Anspruch 1 oder 2, wobei drei Primer, die jeweils wenigstens ein 2- bis 12-fach degeneriertes Kodon umfassen, beschaffen werden.

4. Verfahren gemäß Anspruch 1 oder 2, wobei sieben Primer, die jeweils wenigstens ein 2- bis 12-fach degeneriertes Kodon umfassen, beschafft werden.

5. Verfahren gemäß Anspruch 3, wobei die degenerierten Kodons ausgewählt sind aus der Gruppe bestehend aus: NHT oder NHC (wobei N=A/G/C/T, H=A/C/T), VAG oder VAA (wobei V=A/C/G) und BGG oder DGG (wobei B=C/G/T, D=A/G/T).

6. Verfahren gemäß Anspruch 4, wobei die degenerierten Kodons ausgewählt sind aus der Gruppe bestehend aus ARG (wobei R=A/G), WMC (wobei W=A/T und M=A/C), CAS (wobei S=C/G), GAS (wobei S=C/G), NTC (wobei N=A/G/C/T), KGG (wobei K=G/T) und SCG (wobei S=C/G).

7. Verfahren gemäß Anspruch 1, wobei die Stammnukleinsäure ein Bindungsmolekül kodiert, wobei optional das Bindungsmolekül ein Antikörper oder Fragment davon ist.

8. Verfahren gemäß Anspruch 1, ferner umfassend Auswählen der einen oder mehreren Positionen in der Stammnukleinsäuresequenz für die Mutation.

9. Verfahren gemäß Anspruch 1, ferner umfassend Transformieren der mutierten Nukleinsäuresequenz in kompetente Zellen.

10. Bibliothek/Array von mutierten Nukleinsäuresequenzen, hergestellt durch das Verfahren gemäß Anspruch 1.

11. Primerset, das jeweils wenigstens ein 2- bis 12-fach degeneriertes Kodon umfasst, wobei jeder Primer wenigstens zwei Oligonukleotidsequenzen, welche komplementär zu einer Sequenz in einer Stammnukleinsäure sind, umfasst und wobei die degenerierten Kodons nicht redundant für eine gleiche Repräsentation von achtzehn Aminosäure-Substitutionen mit der Ausnahme von Cystein oder Methionin an jeder der einen oder mehreren durch die Stammnukleinsäure kodierten Aminosäure-Position(en) kodieren.

12. Primerset gemäß Anspruch 11, wobei die Primer jeweils ein oder mehrere degenerierte Kodons repräsentiert durch NHT oder NHC (wobei N=A/G/C/T, H=A/C/T), VAG oder VAA (wobei V=A/C/G) und BGG oder DGG (wobei B=C/G/T, D=A/G/T) umfassen.

13. Primerset gemäß Anspruch 11, wobei die Primer jeweils ein oder mehrere degenerierte Kodons dargestellt durch ARG (wobei R=A/G), WMC (wobei W=A/T und M=A/C), CAS (wobei S=C/G), GAS (wobei S=C/G), NTC (wobei N=A/G/C/T), KGG (wobei K=G/T) und SCG (wobei S=C/G) umfassen.

14. Kit für die Mutagenese eines Aminosäurerestes kodiert durch eine Stammaminosäure, wobei das Kit umfasst: ein Primerset, wobei jeder wenigstens ein 2- bis 12-fach degeniertes Kodon umfasst, wobei jeder Primer wenigstens zwei Oligonukleotidsequenzen, welche komplementär zu einer Sequenz in einer Stammnukleinsäure sind, umfasst und wobei die degenerierten Kodons nicht redundant für eine gleiche Repräsentation von achtzehn Aminosäure-Substitutionen mit der Ausnahme von Cystein oder Methionin an jeder der einen oder mehreren durch die Stammnukleinsäure kodierten Aminosäure-Position(en) kodieren.

## Revendications

1. Procédé de mutagenèse d'un acide nucléique parent codant une protéine pour générer des protéines modifiées, ledit procédé comprenant :
(a.) l'obtention d'un ensemble d'amorces comprenant des amorces qui comprennent chacun au moins un codon dégénéré 2 à 12 fois, chaque amorce comprenant au moins deux séquences d'oligonucléotides qui sont complémentaires d'une séquence dans l'acide nucléique parent, et les codons dégénérés codant de manière non redondante pour une représentation égale de dix-huit substitutions d'acide aminé à l'exception de la cystéine ou de la méthionine à chacune d'une ou de plusieurs positions d'acide aminé codées par l'acide nucléique parent ; et
(b.) mutation de l'acide nucléique parent par réplication ou amplification par polymérase au moyen de l'ensemble d'amorces obtenu en (a), la réplication ou l'amplification de l'acide nucléique parent avec les amorces générant des acides nucléiques mutés qui codent ladite représentation égale de dix-huit substitutions d'acide aminé dans des protéines modifiées.

2. Le procédé de la revendication 1, dans lequel les substitutions d'acide aminé sont sélectionnées dans le groupe constitué par : l'alanine, l'arginine, l'asparagine, l'acide aspartique, la glutamine, l'acide glutaminique, la glycine, l'histidine, l'isoleucine, la leucine, la lysine, la phénylalanine, la proline, la sérine, la thréonine, le tryptophane, la tyrosine et la valine à chaque position.

3. Le procédé de la revendication 1 ou 2, dans lequel trois amorces qui comprennent chacun au moins un codon dégénéré 2 à 12 fois sont obtenus.

4. Le procédé de la revendication 1 ou 2, dans lequel sept amorces qui comprennent chacun au moins un codon dégénéré 2 à 12 fois sont obtenus.

5. Le procédé de la revendication 3, dans lequel les codons dégénérés sont sélectionnés dans le groupe constitué par : NHT ou NHC (où N=A/G/C/T, H=A/C/T), VAG ou VAA (où V=A/C/G) et BGG ou DGG (où B=C/G/T, D=A/G/T).

6. Le procédé de la revendication 4, dans lequel les codons dégénérés sont sélectionnés dans le groupe constitué par : ARG (où R=A/G), WMC (où W=A/T et M=A/C), CAS (où S=C/G), GAS (où S=C/G), NTC (où N=A/G/C/T), KGG (où K=G/T) et SCG (où S=C/G).

7. Le procédé de la revendication 1, dans lequel l'acide nucléique parent code une molécule de liaison, facultativement dans lequel la molécule de liaison est un anticorps ou un fragment de celui-ci.

8. Le procédé de la revendication 1, comprenant en outre la sélection de l'une ou des plusieurs positions dans la séquence d'acide nucléique parent pour la mutation.

9. Le procédé de la revendication 1, comprenant en outre la transformation des séquences d'acides nucléiques mutés en cellules compétentes.

10. Bibliothèque/réseau de séquences d'acides nucléiques mutés générées par le procédé de la revendication 1.

11. Ensemble d'amorces qui comprennent chacun au moins un codon dégénéré 2 à 12 fois, chaque amorce comprenant au moins deux séquences d'oligonucléotides qui sont complémentaires d'une séquence dans un acide nucléique parent et les codons dégénérés codant de manière non redondante pour une représentation égale de dix-huit substitutions d'acide aminé à l'exception de la cystéine ou de la méthionine à chacune d'une ou de plusieurs positions d'acide aminé codées par l'acide nucléique parent.

12. Le jeu d'amorces de la revendication 11, dans lequel les amorces comprennent chacun un ou plusieurs codons dégénérés tels que représentés par NHT ou NHC (où N=A/G/C/T, H=A/C/T), VAG ou VAA (où V=A/C/G) et BGG ou DGG (où B=C/G/T, D=A/G/T).

13. Le jeu d'amorces de la revendication 11, dans lequel les amorces comprennent chacun un ou plusieurs codons dégénérés tels que représentés par ARG (où R=A/G), WMC (où W=A/T et M=A/C), CAS (où S=C/G), GAS (où S=C/G), NTC (où N=A/G/C/T), KGG (où K=G/T) et SCG (où S=C/G).

14. Kit pour la mutagenèse d'un résidu d'acide aminé codé par un acide nucléique parent, le kit comprenant : un ensemble d'amorces qui comprennent chacun au moins un codon dégénéré 2 à 12 fois, chaque amorce comprenant au moins deux séquences d'oligonucléotides qui sont complémentaires d'une séquence dans un acide nucléique parent et les codons dégénérés codant de manière non redondante pour une représentation égale de dix-huit substitutions d'acide aminé à l'exception de la cystéine ou de la méthionine à chacune d'une ou de plusieurs positions d'acide aminé codées par l'acide nucléique parent.


### SEQUENCE LISTING

<110> XOMA, Ltd.
<120> METHODS AND MATERIALS FOR TARGETED MUTAGENESIS
<130> XOMA
<160> 981
<170> PatentIn version 3.5
<210> 1
   <211> 20
   <212> DNA
   <213> human
<400> 1
   actagagcgg caggagatgg 20
<210> 2
   <211> 55
   <212> DNA
   <213> artificial
<220>
   <223> L1-1B
<400> 2
   ccatctcctg ccgctctagt argagtctcc tacatagtaa tggcatcact tattt 55
<210> 3
   <211> 55
   <212> DNA
   <213> artificial
<220>
   <223> L1-1P1 (ING-1)
<400> 3
   ccatctcctg ccgctctagt wmcagtctcc tacatagtaa tggcatcact tattt 55
<210> 4
   <211> 55
   <212> DNA
   <213> artificial
<220>
   <223> L1-1P2 (ING-1)
<400> 4
   ccatctcctg ccgctctagt casagtctcc tacatagtaa tggcatcact tattt 55
<210> 5
   <211> 55
   <212> DNA
   <213> artificial
<220>
   <223> L1-1A (ING-1)
<400> 5
   ccatctcctg ccgctctagt gasagtctcc tacatagtaa tggcatcact tattt 55
<210> 6
   <211> 55
   <212> DNA
   <213> artificial
<220>
   <223> L1-1NP1 (ING-1)
<220>
   <221> misc_feature
   <222> (21)..(21)
   <223> n is a, c, g, or t
<400> 6
   ccatctcctg ccgctctagt ntcagtctcc tacatagtaa tggcatcact tattt 55
<210> 7
   <211> 55
   <212> DNA
   <213> artificial
<220>
   <223> L1-1NP2 (ING-1)
<400> 7
   ccatctcctg ccgctctagt kggagtctcc tacatagtaa tggcatcact tattt 55
<210> 8
   <211> 55
   <212> DNA
   <213> artificial
<220>
   <223> L1-1NP3
<400> 8
   ccatctcctg ccgctctagt scgagtctcc tacatagtaa tggcatcact tattt 55
<210> 9
   <211> 55
   <212> DNA
   <213> artificial
<220>
   <223> L1-2B (ING-1)
<400> 9
   ccatctcctg ccgctctagt aagargctcc tacatagtaa tggcatcact tattt 55
<210> 10
   <211> 55
   <212> DNA
   <213> artificial
<220>
   <223> L1-2P1 (ING-1)
<400> 10
   ccatctcctg ccgctctagt aagwmcctcc tacatagtaa tggcatcact tattt 55
<210> 11
   <211> 55
   <212> DNA
   <213> artificial
<220>
   <223> L1-2P2
<400> 11
   ccatctcctg ccgctctagt aagcasctcc tacatagtaa tggcatcact tattt 55
<210> 12
   <211> 55
   <212> DNA
   <213> artificial
<220>
   <223> L1-2A
<400> 12
   ccatctcctg ccgctctagt aaggasctcc tacatagtaa tggcatcact tattt 55
<210> 13
   <211> 55
   <212> DNA
   <213> artificial
<220>
   <223> L1-2NP1 (ING-1)
<220>
   <221> misc_feature
   <222> (24)..(24)
   <223> n is a, c, g, or t
<400> 13
   ccatctcctg ccgctctagt aagntcctcc tacatagtaa tggcatcact tattt 55
<210> 14
   <211> 55
   <212> DNA
   <213> artificial
<220>
   <223> L1-2NP2 (ING-1)
<400> 14
   ccatctcctg ccgctctagt aagkggctcc tacatagtaa tggcatcact tattt 55
<210> 15
   <211> 55
   <212> DNA
   <213> artificial
<220>
   <223> L1-2NP3 (ING-1)
<400> 15
   ccatctcctg ccgctctagt aagscgctcc tacatagtaa tggcatcact tattt 55
<210> 16
   <211> 55
   <212> DNA
   <213> artificial
<220>
   <223> L1-3B (ING-1)
<400> 16
   ccatctcctg ccgctctagt aagagtargc tacatagtaa tggcatcact tattt 55
<210> 17
   <211> 55
   <212> DNA
   <213> artificial
<220>
   <223> L1-3P1 (ING-1)
<400> 17
   ccatctcctg ccgctctagt aagagtwmcc tacatagtaa tggcatcact tattt 55
<210> 18
   <211> 55
   <212> DNA
   <213> artificial
<220>
   <223> L1-3P2 (ING-1)
<400> 18
   ccatctcctg ccgctctagt aagagtcasc tacatagtaa tggcatcact tattt 55
<210> 19
   <211> 55
   <212> DNA
   <213> artificial
<220>
   <223> L1-3A (ING-1)
<400> 19
   ccatctcctg ccgctctagt aagagtgasc tacatagtaa tggcatcact tattt 55
<210> 20
   <211> 55
   <212> DNA
   <213> artificial
<220>
   <223> L1-3NP1 (ING-1)
<220>
   <221> misc_feature
   <222> (27)..(27)
   <223> n is a, c, g, or t
<400> 20
   ccatctcctg ccgctctagt aagagtntcc tacatagtaa tggcatcact tattt 55
<210> 21
   <211> 55
   <212> DNA
   <213> artificial
<220>
   <223> L1-3NP2 (ING-1)
<400> 21
   ccatctcctg ccgctctagt aagagtkggc tacatagtaa tggcatcact tattt 55
<210> 22
   <211> 55
   <212> DNA
   <213> artificial
<220>
   <223> L1-3NP3 (ING-1)
<400> 22
   ccatctcctg ccgctctagt aagagtscgc tacatagtaa tggcatcact tattt 55
<210> 23
   <211> 55
   <212> DNA
   <213> artificial
<220>
   <223> L1-4B (ING-1)
<400> 23
   ccatctcctg ccgctctagt aagagtctca rgcatagtaa tggcatcact tattt 55
<210> 24
   <211> 55
   <212> DNA
   <213> artificial
<220>
   <223> L1-4P1 (ING-1)
<400> 24
   ccatctcctg ccgctctagt aagagtctcw mccatagtaa tggcatcact tattt 55
<210> 25
   <211> 55
   <212> DNA
   <213> artificial
<220>
   <223> L1-4P2 (ING-1)
<400> 25
   ccatctcctg ccgctctagt aagagtctcc ascatagtaa tggcatcact tattt 55
<210> 26
   <211> 55
   <212> DNA
   <213> artificial
<220>
   <223> L1-4A
<400> 26
   ccatctcctg ccgctctagt aagagtctcg ascatagtaa tggcatcact tattt 55
<210> 27
   <211> 55
   <212> DNA
   <213> artificial
<220>
   <223> L1-4NP1 (ING-1)
<220>
   <221> misc_feature
   <222> (30)..(30)
   <223> n is a, c, g, or t
<400> 27
   ccatctcctg ccgctctagt aagagtctcn tccatagtaa tggcatcact tattt 55
<210> 28
   <211> 55
   <212> DNA
   <213> artificial
<220>
   <223> L1-4NP2 (ING-1)
<400> 28
   ccatctcctg ccgctctagt aagagtctck ggcatagtaa tggcatcact tattt 55
<210> 29
   <211> 55
   <212> DNA
   <213> artificial
<220>
   <223> L1-4NP3 (ING-1)
<400> 29
   ccatctcctg ccgctctagt aagagtctcs cgcatagtaa tggcatcact tattt 55
<210> 30
   <211> 21
   <212> DNA
   <213> artificial
<220>
   <223> L12-R (ING-1)
<400> 30
   taggagactc ttactagagc g 21
<210> 31
   <211> 54
   <212> DNA
   <213> artificial
<220>
   <223> L12-1B (ING-1)
<400> 31
   cgctctagta agagtctcct aargagtaat ggcatcactt atttgtattg gtat 54
<210> 32
   <211> 54
   <212> DNA
   <213> artificial
<220>
   <223> L12-1P1 (ING-1)
<400> 32
   cgctctagta agagtctcct awmcagtaat ggcatcactt atttgtattg gtat 54
<210> 33
   <211> 54
   <212> DNA
   <213> artificial
<220>
   <223> L12-1P2 (ING-1)
<400> 33
   cgctctagta agagtctcct acasagtaat ggcatcactt atttgtattg gtat 54
<210> 34
   <211> 54
   <212> DNA
   <213> artificial
<220>
   <223> L12-1A (ING-1)
<400> 34
   cgctctagta agagtctcct agasagtaat ggcatcactt atttgtattg gtat 54
<210> 35
   <211> 54
   <212> DNA
   <213> artificial
<220>
   <223> L12-1NP1 (ING-1)
<220>
   <221> misc_feature
   <222> (22)..(22)
   <223> n is a, c, g, or t
<400> 35
   cgctctagta agagtctcct antcagtaat ggcatcactt atttgtattg gtat 54
<210> 36
   <211> 54
   <212> DNA
   <213> artificial
<220>
   <223> L12-1NP2 (ING-1)
<400> 36
   cgctctagta agagtctcct akggagtaat ggcatcactt atttgtattg gtat 54
<210> 37
   <211> 54
   <212> DNA
   <213> artificial
<220>
   <223> L12-1NP3 (ING-1)
<400> 37
   cgctctagta agagtctcct ascgagtaat ggcatcactt atttgtattg gtat 54
<210> 38
   <211> 54
   <212> DNA
   <213> artificial
<220>
   <223> L12-2B (ING-1)
<400> 38 54
   cgctctagta agagtctcct acatargaat ggcatcactt atttgtattg gtat 54
<210> 39
   <211> 54
   <212> DNA
   <213> artificial
<220>
   <223> L12-2P1 (ING-1)
<400> 39
   cgctctagta agagtctcct acatwmcaat ggcatcactt atttgtattg gtat 54
<210> 40
   <211> 54
   <212> DNA
   <213> artificial
<220>
   <223> L12-2P2 (ING-1)
<400> 40
   cgctctagta agagtctcct acatcasaat ggcatcactt atttgtattg gtat 54
<210> 41
   <211> 54
   <212> DNA
   <213> artificial
<220>
   <223> L12-2A (ING-1)
<400> 41
   cgctctagta agagtctcct acatgasaat ggcatcactt atttgtattg gtat 54
<210> 42
   <211> 54
   <212> DNA
   <213> artificial
<220>
   <223> L12-2NP1 (ING-1)
<220>
   <221> misc_feature
   <222> (25)..(25)
   <223> n is a, c, g, or t
<400> 42
   cgctctagta agagtctcct acatntcaat ggcatcactt atttgtattg gtat 54
<210> 43
   <211> 54
   <212> DNA
   <213> artificial
<220>
   <223> L12-2NP2 (ING-1)
<400> 43
   cgctctagta agagtctcct acatkggaat ggcatcactt atttgtattg gtat 54
<210> 44
   <211> 54
   <212> DNA
   <213> artificial
<220>
   <223> L12-2NP3 (ING-1)
<400> 44
   cgctctagta agagtctcct acatscgaat ggcatcactt atttgtattg gtat 54
<210> 45
   <211> 54
   <212> DNA
   <213> artificial
<220>
   <223> L12-3B (ING-1)
<400> 45
   cgctctagta agagtctcct acatagtarg ggcatcactt atttgtattg gtat 54
<210> 46
   <211> 54
   <212> DNA
   <213> artificial
<220>
   <223> L12-3P1 (ING-1)
<400> 46
   cgctctagta agagtctcct acatagtwmc ggcatcactt atttgtattg gtat 54
<210> 47
   <211> 54
   <212> DNA
   <213> artificial
<220>
   <223> L12-3P2 (ING-1)
<400> 47
   cgctctagta agagtctcct acatagtcas ggcatcactt atttgtattg gtat 54
<210> 48
   <211> 54
   <212> DNA
   <213> artificial
<220>
   <223> L12-3A (ING-1)
<400> 48
   cgctctagta agagtctcct acatagtgas ggcatcactt atttgtattg gtat 54
<210> 49
   <211> 54
   <212> DNA
   <213> artificial
<220>
   <223> L12-3NP1 (ING-1)
<220>
   <221> misc_feature
   <222> (28)..(28)
   <223> n is a, c, g, or t
<400> 49
   cgctctagta agagtctcct acatagtntc ggcatcactt atttgtattg gtat 54
<210> 50
   <211> 54
   <212> DNA
   <213> artificial
<220>
   <223> L12-3NP2 (ING-1)
<400> 50
   cgctctagta agagtctcct acatagtkgg ggcatcactt atttgtattg gtat 54
<210> 51
   <211> 54
   <212> DNA
   <213> artificial
<220>
   <223> L12-3NP3 (ING-1)
<400> 51
   cgctctagta agagtctcct acatagtscg ggcatcactt atttgtattg gtat 54
<210> 52
   <211> 22
   <212> DNA
   <213> artificial
<220>
   <223> L13-R (ING-1)
<400> 52
   gccattacta tgtaggagac tc 22
<210> 53
   <211> 54
   <212> DNA
   <213> artificial
<220>
   <223> L13-1B (ING-1)
<400> 53
   gagtctccta catagtaatg gcargactta tttgtattgg tatttacaga agcc 54
<210> 54
   <211> 54
   <212> DNA
   <213> artificial
<220>
   <223> L13-1P1 (ING-1)
<400> 54
   gagtctccta catagtaatg gcwmcactta tttgtattgg tatttacaga agcc 54
<210> 55
   <211> 54
   <212> DNA
   <213> artificial
<220>
   <223> L13-1P2 (ING-1)
<400> 55
   gagtctccta catagtaatg gccasactta tttgtattgg tatttacaga agcc 54
<210> 56
   <211> 54
   <212> DNA
   <213> artificial
<220>
   <223> L13-1A (ING-1)
<400> 56
   gagtctccta catagtaatg gcgasactta tttgtattgg tatttacaga agcc 54
<210> 57
   <211> 54
   <212> DNA
   <213> artificial
<220>
   <223> L13-1NP1 (ING-1)
<220>
   <221> misc_feature
   <222> (23)..(23)
   <223> n is a, c, g, or t
<400> 57
   gagtctccta catagtaatg gcntcactta tttgtattgg tatttacaga agcc 54
<210> 58
   <211> 54
   <212> DNA
   <213> artificial
<220>
   <223> L13-1NP2 (ING-1)
<400> 58
   gagtctccta catagtaatg gckggactta tttgtattgg tatttacaga agcc 54
<210> 59
   <211> 54
   <212> DNA
   <213> artificial
<220>
   <223> L13-1NP3 (ING-1)
<400> 59
   gagtctccta catagtaatg gcscgactta tttgtattgg tatttacaga agcc 54
<210> 60
   <211> 54
   <212> DNA
   <213> artificial
<220>
   <223> L13-2B (ING-1)
<400> 60
   gagtctccta catagtaatg gcatcargta tttgtattgg tatttacaga agcc 54
<210> 61
   <211> 54
   <212> DNA
   <213> artificial
<220>
   <223> L13-2P1 (ING-1)
<400> 61
   gagtctccta catagtaatg gcatcwmcta tttgtattgg tatttacaga agcc 54
<210> 62
   <211> 54
   <212> DNA
   <213> artificial
<220>
   <223> L13-2P2 (ING-1)
<400> 62
   gagtctccta catagtaatg gcatccasta tttgtattgg tatttacaga agcc 54
<210> 63
   <211> 54
   <212> DNA
   <213> artificial
<220>
   <223> L13-2A (ING-1)
<400> 63
   gagtctccta catagtaatg gcatcgasta tttgtattgg tatttacaga agcc 54
<210> 64
   <211> 54
   <212> DNA
   <213> artificial
<220>
   <223> L13-2NP1 (ING-1)
<220>
   <221> misc_feature
   <222> (26)..(26)
   <223> n is a, c, g, or t
<400> 64
   gagtctccta catagtaatg gcatcntcta tttgtattgg tatttacaga agcc 54
<210> 65
   <211> 54
   <212> DNA
   <213> artificial
<220>
   <223> L13-2NP2 (ING-1)
<400> 65
   gagtctccta catagtaatg gcatckggta tttgtattgg tatttacaga agcc 54
<210> 66
   <211> 54
   <212> DNA
   <213> artificial
<220>
   <223> L13-2NP3 (ING-1)
<400> 66
   gagtctccta catagtaatg gcatcscgta tttgtattgg tatttacaga agcc 54
<210> 67
   <211> 54
   <212> DNA
   <213> artificial
<220>
   <223> L13-3B (ING-1)
<400> 67
   gagtctccta catagtaatg gcatcactar gttgtattgg tatttacaga agcc 54
<210> 68
   <211> 54
   <212> DNA
   <213> artificial
<220>
   <223> L13-3P1 (ING-1)
<400> 68
   gagtctccta catagtaatg gcatcactwm cttgtattgg tatttacaga agcc 54
<210> 69
   <211> 54
   <212> DNA
   <213> artificial
<220>
   <223> L13-3P2 (ING-1)
<400> 69
   gagtctccta catagtaatg gcatcactca sttgtattgg tatttacaga agcc 54
<210> 70
   <211> 54
   <212> DNA
   <213> artificial
<220>
   <223> L13-3A (ING-1)
<400> 70
   gagtctccta catagtaatg gcatcactga sttgtattgg tatttacaga agcc 54
<210> 71
   <211> 54
   <212> DNA
   <213> artificial
<220>
   <223> L13-3NP1 (ING-1)
<220>
   <221> misc_feature
   <222> (29)..(29)
   <223> n is a, c, g, or t
<400> 71
   gagtctccta catagtaatg gcatcactnt cttgtattgg tatttacaga agcc 54
<210> 72
   <211> 54
   <212> DNA
   <213> artificial
<220>
   <223> L13-3NP2 (ING-1)
<400> 72
   gagtctccta catagtaatg gcatcactkg gttgtattgg tatttacaga agcc 54
<210> 73
   <211> 54
   <212> DNA
   <213> artificial
<220>
   <223> L13-3NP3 (ING-1)
<400> 73
   gagtctccta catagtaatg gcatcactsc gttgtattgg tatttacaga agcc 54
<210> 74
   <211> 21
   <212> DNA
   <213> artificial
<220>
   <223> L2R (ING-1)
<220>
   <221> misc_feature
   <223> L2R
<400> 74
   aatcaggagc tgaggagact g 21
<210> 75
   <211> 56
   <212> DNA
   <213> artificial
<220>
   <223> L2-1B (ING-1)
<400> 75
   cagtctcctc agctcctgat targcagatg tccaaccttg cctcaggagt cccaga 56
<210> 76
   <211> 56
   <212> DNA
   <213> artificial
<220>
   <223> L2-1P1 (ING-1)
<400> 76
   cagtctcctc agctcctgat twmccagatg tccaaccttg cctcaggagt cccaga 56
<210> 77
   <211> 56
   <212> DNA
   <213> artificial
<220>
   <223> L2-1P2 (ING-1)
<400> 77
   cagtctcctc agctcctgat tcascagatg tccaaccttg cctcaggagt cccaga 56
<210> 78
   <211> 56
   <212> DNA
   <213> artificial
<220>
   <223> L2-1A (ING-1)
<400> 78
   cagtctcctc agctcctgat tgascagatg tccaaccttg cctcaggagt cccaga 56
<210> 79
   <211> 56
   <212> DNA
   <213> artificial
<220>
   <223> L2-1NP1 (ING-1)
<220>
   <221> misc_feature
   <222> (22)..(22)
   <223> n is a, c, g, or t
<400> 79
   cagtctcctc agctcctgat tntccagatg tccaaccttg cctcaggagt cccaga 56
<210> 80
   <211> 56
   <212> DNA
   <213> artificial
<220>
   <223> L2-1NP2 (ING-1)
<400> 80
   cagtctcctc agctcctgat tkggcagatg tccaaccttg cctcaggagt cccaga 56
<210> 81
   <211> 56
   <212> DNA
   <213> artificial
<220>
   <223> L2-1NP3 (ING-1)
<400> 81
   cagtctcctc agctcctgat tscgcagatg tccaaccttg cctcaggagt cccaga 56
<210> 82
   <211> 56
   <212> DNA
   <213> artificial
<220>
   <223> L2-2B (ING-1)
<400> 82
   cagtctcctc agctcctgat ttatargatg tccaaccttg cctcaggagt cccaga 56
<210> 83
   <211> 56
   <212> DNA
   <213> artificial
<220>
   <223> L2-2P1 (ING-1)
<400> 83
   cagtctcctc agctcctgat ttatwmcatg tccaaccttg cctcaggagt cccaga 56
<210> 84
   <211> 56
   <212> DNA
   <213> artificial
<220>
   <223> L2-2P2 (ING-1)
<400> 84
   cagtctcctc agctcctgat ttatcasatg tccaaccttg cctcaggagt cccaga 56
<210> 85
   <211> 56
   <212> DNA
   <213> artificial
<220>
   <223> L2-2A (ING-1)
<400> 85
   cagtctcctc agctcctgat ttatgasatg tccaaccttg cctcaggagt cccaga 56
<210> 86
   <211> 56
   <212> DNA
   <213> artificial
<220>
   <223> L2-2NP1 (ING-1)
<220>
   <221> misc_feature
   <222> (25)..(25)
   <223> n is a, c, g, or t
<400> 86
   cagtctcctc agctcctgat ttatntcatg tccaaccttg cctcaggagt cccaga 56
<210> 87
   <211> 56
   <212> DNA
   <213> artificial
<220>
   <223> L2-2NP2 (ING-1)
<400> 87
   cagtctcctc agctcctgat ttatkggatg tccaaccttg cctcaggagt cccaga 56
<210> 88
   <211> 56
   <212> DNA
   <213> artificial
<220>
   <223> L2-2NP3 (ING-1)
<400> 88
   cagtctcctc agctcctgat ttatscgatg tccaaccttg cctcaggagt cccaga 56
<210> 89
   <211> 56
   <212> DNA
   <213> artificial
<220>
   <223> L2-3B (ING-1)
<400> 89
   cagtctcctc agctcctgat ttatcagarg tccaaccttg cctcaggagt cccaga 56
<210> 90
   <211> 56
   <212> DNA
   <213> artificial
<220>
   <223> L2-3P1 (ING-1)
<400> 90
   cagtctcctc agctcctgat ttatcagwmc tccaaccttg cctcaggagt cccaga 56
<210> 91
   <211> 56
   <212> DNA
   <213> artificial
<220>
   <223> L2-3P2 (ING-1)
<400> 91
   cagtctcctc agctcctgat ttatcagcas tccaaccttg cctcaggagt cccaga 56
<210> 92
   <211> 56
   <212> DNA
   <213> artificial
<220>
   <223> L2-3A (ING-1)
<400> 92
   cagtctcctc agctcctgat ttatcaggas tccaaccttg cctcaggagt cccaga 56
<210> 93
   <211> 56
   <212> DNA
   <213> artificial
<220>
   <223> L2-3NP1 (ING-1)
<220>
   <221> misc_feature
   <222> (28)..(28)
   <223> n is a, c, g, or t
<400> 93
   cagtctcctc agctcctgat ttatcagntc tccaaccttg cctcaggagt cccaga 56
<210> 94
   <211> 56
   <212> DNA
   <213> artificial
<220>
   <223> L2-3NP2 (ING-1)
<400> 94
   cagtctcctc agctcctgat ttatcagkgg tccaaccttg cctcaggagt cccaga 56
<210> 95
   <211> 56
   <212> DNA
   <213> artificial
<220>
   <223> L2-3NP3 (ING-1)
<400> 95
   cagtctcctc agctcctgat ttatcagscg tccaaccttg cctcaggagt cccaga 56
<210> 96
   <211> 56
   <212> DNA
   <213> artificial
<220>
   <223> L2-4B (ING-1)
<400> 96
   cagtctcctc agctcctgat ttatcagatg argaaccttg cctcaggagt cccaga 56
<210> 97
   <211> 56
   <212> DNA
   <213> artificial
<220>
   <223> L2-4P1 (ING-1)
<400> 97
   cagtctcctc agctcctgat ttatcagatg wmcaaccttg cctcaggagt cccaga 56
<210> 98
   <211> 56
   <212> DNA
   <213> artificial
<220>
   <223> L2-4P2 (ING-1)
<400> 98
   cagtctcctc agctcctgat ttatcagatg casaaccttg cctcaggagt cccaga 56
<210> 99
   <211> 56
   <212> DNA
   <213> artificial
<220>
   <223> L2-4A (ING-1)
<400> 99
   cagtctcctc agctcctgat ttatcagatg gasaaccttg cctcaggagt cccaga 56
<210> 100
   <211> 56
   <212> DNA
   <213> artificial
<220>
   <223> L2-4NP1 (ING-1)
<220>
   <221> misc_feature
   <222> (31)..(31)
   <223> n is a, c, g, or t
<400> 100
   cagtctcctc agctcctgat ttatcagatg ntcaaccttg cctcaggagt cccaga 56
<210> 101
   <211> 56
   <212> DNA
   <213> artificial
<220>
   <223> L2-4NP2 (ING-1)
<400> 101
   cagtctcctc agctcctgat ttatcagatg kggaaccttg cctcaggagt cccaga 56
<210> 102
   <211> 56
   <212> DNA
   <213> artificial
<220>
   <223> L2-4NP3 (ING-1)
<400> 102
   cagtctcctc agctcctgat ttatcagatg scgaaccttg cctcaggagt cccaga 56
<210> 103
   <211> 56
   <212> DNA
   <213> artificial
<220>
   <223> L2-5B (ING-1)
<400> 103
   cagtctcctc agctcctgat ttatcagatg tccargcttg cctcaggagt cccaga 56
<210> 104
   <211> 56
   <212> DNA
   <213> artificial
<220>
   <223> L2-5P1 (ING-1)
<400> 104
   cagtctcctc agctcctgat ttatcagatg tccwmccttg cctcaggagt cccaga 56
<210> 105
   <211> 56
   <212> DNA
   <213> artificial
<220>
   <223> L2-5P2 (ING-1)
<400> 105
   cagtctcctc agctcctgat ttatcagatg tcccascttg cctcaggagt cccaga 56
<210> 106
   <211> 56
   <212> DNA
   <213> artificial
<220>
   <223> L2-5A (ING-1)
<400> 106
   cagtctcctc agctcctgat ttatcagatg tccgascttg cctcaggagt cccaga 56
<210> 107
   <211> 56
   <212> DNA
   <213> artificial
<220>
   <223> L2-5NP1 (ING-1)
<220>
   <221> misc_feature
   <222> (34)..(34)
   <223> n is a, c, g, or t
<400> 107
   cagtctcctc agctcctgat ttatcagatg tccntccttg cctcaggagt cccaga 56
<210> 108
   <211> 56
   <212> DNA
   <213> artificial
<220>
   <223> L2-5NP2 (ING-1)
<400> 108
   cagtctcctc agctcctgat ttatcagatg tcckggcttg cctcaggagt cccaga 56
<210> 109
   <211> 56
   <212> DNA
   <213> artificial
<220>
   <223> L2-5NP3 (ING-1)
<400> 109
   cagtctcctc agctcctgat ttatcagatg tccscgcttg cctcaggagt cccaga 56
<210> 110
   <211> 24
   <212> DNA
   <213> artificial
<220>
   <223> L3R (ING-1)
<400> 110
   attttgagca cagtaataaa cacc 24
<210> 111
   <211> 59
   <212> DNA
   <213> artificial
<220>
   <223> L3-1B (ING-1)
<400> 111
   ggtgtttatt actgtgctca aaatarggaa cttcctcgga cgttcggtgg aggcaccaa 59
<210> 112
   <211> 59
   <212> DNA
   <213> artificial
<220>
   <223> L3-1P1 (ING-1)
<400> 112
   ggtgtttatt actgtgctca aaatwmcgaa cttcctcgga cgttcggtgg aggcaccaa 59
<210> 113
   <211> 59
   <212> DNA
   <213> artificial
<220>
   <223> L3-1P2 (ING-1)
<400> 113
   ggtgtttatt actgtgctca aaatcasgaa cttcctcgga cgttcggtgg aggcaccaa 59
<210> 114
   <211> 59
   <212> DNA
   <213> artificial
<220>
   <223> L3-1A (ING-1)
<400> 114
   ggtgtttatt actgtgctca aaatgasgaa cttcctcgga cgttcggtgg aggcaccaa 59
<210> 115
   <211> 59
   <212> DNA
   <213> artificial
<220>
   <223> L3-1NP1 (ING-1)
<220>
   <221> misc_feature
   <222> (25)..(25)
   <223> n is a, c, g, or t
<400> 115
   ggtgtttatt actgtgctca aaatntcgaa cttcctcgga cgttcggtgg aggcaccaa 59
<210> 116
   <211> 59
   <212> DNA
   <213> artificial
<220>
   <223> L3-1NP2 (ING-1)
<400> 116
   ggtgtttatt actgtgctca aaatkgggaa cttcctcgga cgttcggtgg aggcaccaa 59
<210> 117
   <211> 59
   <212> DNA
   <213> artificial
<220>
   <223> L3-1NP3 (ING-1)
<220>
   <221> misc_feature
   <223> L3-1NP3
<400> 117
   ggtgtttatt actgtgctca aaatscggaa cttcctcgga cgttcggtgg aggcaccaa 59
<210> 118
   <211> 59
   <212> DNA
   <213> artificial
<220>
   <223> L3-2B (ING-1)
<400> 118
   ggtgtttatt actgtgctca aaatctaarg cttcctcgga cgttcggtgg aggcaccaa 59
<210> 119
   <211> 59
   <212> DNA
   <213> artificial
<220>
   <223> L3-2P1 (ING-1)
<400> 119
   ggtgtttatt actgtgctca aaatctawmc cttcctcgga cgttcggtgg aggcaccaa 59
<210> 120
   <211> 59
   <212> DNA
   <213> artificial
<220>
   <223> L3-2P2 (ING-1)
<400> 120
   ggtgtttatt actgtgctca aaatctacas cttcctcgga cgttcggtgg aggcaccaa 59
<210> 121
   <211> 59
   <212> DNA
   <213> artificial
<220>
   <223> L3-2A (ING-1)
<400> 121
   ggtgtttatt actgtgctca aaatctagas cttcctcgga cgttcggtgg aggcaccaa 59
<210> 122
   <211> 59
   <212> DNA
   <213> artificial
<220>
   <223> L3-2NP1 (ING-1)
<220>
   <221> misc_feature
   <222> (28)..(28)
   <223> n is a, c, g, or t
<400> 122
   ggtgtttatt actgtgctca aaatctantc cttcctcgga cgttcggtgg aggcaccaa 59
<210> 123
   <211> 59
   <212> DNA
   <213> artificial
<220>
   <223> L3-2NP2 (ING-1)
<400> 123
   ggtgtttatt actgtgctca aaatctakgg cttcctcgga cgttcggtgg aggcaccaa 59
<210> 124
   <211> 59
   <212> DNA
   <213> artificial
<220>
   <223> L3-2NP3 (ING-1)
<400> 124
   ggtgtttatt actgtgctca aaatctascg cttcctcgga cgttcggtgg aggcaccaa 59
<210> 125
   <211> 59
   <212> DNA
   <213> artificial
<220>
   <223> L3-3B (ING-1)
<400> 125
   ggtgtttatt actgtgctca aaatctagaa argcctcgga cgttcggtgg aggcaccaa 59
<210> 126
   <211> 59
   <212> DNA
   <213> artificial
<220>
   <223> L3-3P1 (ING-1)
<400> 126
   ggtgtttatt actgtgctca aaatctagaa wmccctcgga cgttcggtgg aggcaccaa 59
<210> 127
   <211> 59
   <212> DNA
   <213> artificial
<220>
   <223> L3-3P2 (ING-1)
<400> 127
   ggtgtttatt actgtgctca aaatctagaa cascctcgga cgttcggtgg aggcaccaa 59
<210> 128
   <211> 59
   <212> DNA
   <213> artificial
<220>
   <223> L3-3A (ING-1)
<400> 128
   ggtgtttatt actgtgctca aaatctagaa gascctcgga cgttcggtgg aggcaccaa 59
<210> 129
   <211> 59
   <212> DNA
   <213> artificial
<220>
   <223> L3-3NP1 (ING-1)
<220>
   <221> misc_feature
   <222> (31)..(31)
   <223> n is a, c, g, or t
<400> 129
   ggtgtttatt actgtgctca aaatctagaa ntccctcgga cgttcggtgg aggcaccaa 59
<210> 130
   <211> 59
   <212> DNA
   <213> artificial
<220>
   <223> L3-3NP2 (ING-1)
<400> 130
   ggtgtttatt actgtgctca aaatctagaa kggcctcgga cgttcggtgg aggcaccaa 59
<210> 131
   <211> 59
   <212> DNA
   <213> artificial
<220>
   <223> L3-3NP3 (ING-1)
<400> 131
   ggtgtttatt actgtgctca aaatctagaa scgcctcgga cgttcggtgg aggcaccaa 59
<210> 132
   <211> 59
   <212> DNA
   <213> artificial
<220>
   <223> L3-4B (ING-1)
<400> 132
   ggtgtttatt actgtgctca aaatctagaa cttargcgga cgttcggtgg aggcaccaa 59
<210> 133
   <211> 59
   <212> DNA
   <213> artificial (ING-1)
<220>
   <223> L3-4P1 (ING-1)
<400> 133
   ggtgtttatt actgtgctca aaatctagaa cttwmccgga cgttcggtgg aggcaccaa 59
<210> 134
   <211> 59
   <212> DNA
   <213> artificial
<220>
   <223> L3-4P2 (ING-1)
<400> 134
   ggtgtttatt actgtgctca aaatctagaa cttcascgga cgttcggtgg aggcaccaa 59
<210> 135
   <211> 59
   <212> DNA
   <213> artificial
<220>
   <223> L3-4A (ING-1)
<400> 135
   ggtgtttatt actgtgctca aaatctagaa cttgascgga cgttcggtgg aggcaccaa 59
<210> 136
   <211> 59
   <212> DNA
   <213> artificial
<220>
   <223> L3-4NP1 (ING-1)
<220>
   <221> misc_feature
   <222> (34)..(34)
   <223> n is a, c, g, or t
<400> 136
   ggtgtttatt actgtgctca aaatctagaa cttntccgga cgttcggtgg aggcaccaa 59
<210> 137
   <211> 59
   <212> DNA
   <213> artificial
<220>
   <223> L3-4NP2 (ING-1)
<400> 137
   ggtgtttatt actgtgctca aaatctagaa cttkggcgga cgttcggtgg aggcaccaa 59
<210> 138
   <211> 59
   <212> DNA
   <213> artificial
<220>
   <223> L3-4NP3 (ING-1)
<400> 138
   ggtgtttatt actgtgctca aaatctagaa cttscgcgga cgttcggtgg aggcaccaa 59
<210> 139
   <211> 59
   <212> DNA
   <213> artificial
<220>
   <223> L3-5B (ING-1)
<400> 139
   ggtgtttatt actgtgctca aaatctagaa cttcctarga cgttcggtgg aggcaccaa 59
<210> 140
   <211> 59
   <212> DNA
   <213> artificial
<220>
   <223> L3-5P1 (ING-1)
<400> 140
   ggtgtttatt actgtgctca aaatctagaa cttcctwmca cgttcggtgg aggcaccaa 59
<210> 141
   <211> 59
   <212> DNA
   <213> artificial
<220>
   <223> L3-5P2 (ING-1)
<400> 141
   ggtgtttatt actgtgctca aaatctagaa cttcctcasa cgttcggtgg aggcaccaa 59
<210> 142
   <211> 59
   <212> DNA
   <213> artificial
<220>
   <223> L3-5A (ING-1)
<400> 142
   ggtgtttatt actgtgctca aaatctagaa cttcctgasa cgttcggtgg aggcaccaa 59
<210> 143
   <211> 59
   <212> DNA
   <213> artificial
<220>
   <223> L3-5NP1 (ING-1)
<220>
   <221> misc_feature
   <222> (37)..(37)
   <223> n is a, c, g, or t
<400> 143
   ggtgtttatt actgtgctca aaatctagaa cttcctntca cgttcggtgg aggcaccaa 59
<210> 144
   <211> 59
   <212> DNA
   <213> artificial
<220>
   <223> L3-5NP2 (ING-1)
<400> 144
   ggtgtttatt actgtgctca aaatctagaa cttcctkgga cgttcggtgg aggcaccaa 59
<210> 145
   <211> 59
   <212> DNA
   <213> artificial
<220>
   <223> L3-5NP3 (ING-1)
<400> 145
   ggtgtttatt actgtgctca aaatctagaa cttcctscga cgttcggtgg aggcaccaa 59
<210> 146
   <211> 21
   <212> DNA
   <213> artificial
<220>
   <223> H1R (ING-1)
<400> 146
   atatccagaa gccttgcagg a 21
<210> 147
   <211> 59
   <212> DNA
   <213> artificial
<220>
   <223> H1-1B (ING-1)
<400> 147
   tcctgcaagg cttctggata targttcaca aaatatggaa tgaactgggt gaagcaggc 59
<210> 148
   <211> 59
   <212> DNA
   <213> artificial
<220>
   <223> H1-1P1 (ING-1)
<400> 148
   tcctgcaagg cttctggata twmcttcaca aaatatggaa tgaactgggt gaagcaggc 59
<210> 149
   <211> 59
   <212> DNA
   <213> artificial
<220>
   <223> H1-1P2 (ING-1)
<400> 149
   tcctgcaagg cttctggata tcasttcaca aaatatggaa tgaactgggt gaagcaggc 59
<210> 150
   <211> 59
   <212> DNA
   <213> artificial
<220>
   <223> H1-1A (ING-1)
<400> 150
   tcctgcaagg cttctggata tgasttcaca aaatatggaa tgaactgggt gaagcaggc 59
<210> 151
   <211> 59
   <212> DNA
   <213> artificial
<220>
   <223> H1-1NP1 (ING-1)
<220>
   <221> misc_feature
   <222> (22)..(22)
   <223> n is a, c, g, or t
<400> 151
   tcctgcaagg cttctggata tntcttcaca aaatatggaa tgaactgggt gaagcaggc 59
<210> 152
   <211> 59
   <212> DNA
   <213> artificial
<220>
   <223> H1-1NP2 (ING-1)
<400> 152
   tcctgcaagg cttctggata tkggttcaca aaatatggaa tgaactgggt gaagcaggc 59
<210> 153
   <211> 59
   <212> DNA
   <213> artificial
<220>
   <223> H1-1NP3 (ING-1)
<400> 153
   tcctgcaagg cttctggata tscgttcaca aaatatggaa tgaactgggt gaagcaggc 59
<210> 154
   <211> 59
   <212> DNA
   <213> artificial
<220>
   <223> H1-2B (ING-1)
<400> 154
   tcctgcaagg cttctggata taccttcarg aaatatggaa tgaactgggt gaagcaggc 59
<210> 155
   <211> 59
   <212> DNA
   <213> artificial
<220>
   <223> H1-2P1 (ING-1)
<400> 155
   tcctgcaagg cttctggata taccttcwmc aaatatggaa tgaactgggt gaagcaggc 59
<210> 156
   <211> 59
   <212> DNA
   <213> artificial
<220>
   <223> H1-2P2 (ING-1)
<400> 156
   tcctgcaagg cttctggata taccttccas aaatatggaa tgaactgggt gaagcaggc 59
<210> 157
   <211> 59
   <212> DNA
   <213> artificial
<220>
   <223> H1-2A (ING-1)
<400> 157
   tcctgcaagg cttctggata taccttcgas aaatatggaa tgaactgggt gaagcaggc 59
<210> 158
   <211> 59
   <212> DNA
   <213> artificial
<220>
   <223> H1-2NP1 (ING-1)
<220>
   <221> misc_feature
   <222> (28)..(28)
   <223> n is a, c, g, or t
<400> 158
   tcctgcaagg cttctggata taccttcntc aaatatggaa tgaactgggt gaagcaggc 59
<210> 159
   <211> 59
   <212> DNA
   <213> artificial
<220>
   <223> H1-2NP2 (ING-1)
<400> 159
   tcctgcaagg cttctggata taccttckgg aaatatggaa tgaactgggt gaagcaggc 59
<210> 160
   <211> 59
   <212> DNA
   <213> artificial
<220>
   <223> H1-2NP3 (ING-1)
<400> 160
   tcctgcaagg cttctggata taccttcscg aaatatggaa tgaactgggt gaagcaggc 59
<210> 161
   <211> 59
   <212> DNA
   <213> artificial
<220>
   <223> H1-3B (ING-1)
<400> 161
   tcctgcaagg cttctggata taccttcaca argtatggaa tgaactgggt gaagcaggc 59
<210> 162
   <211> 59
   <212> DNA
   <213> artificial
<220>
   <223> H1-3P1 (ING-1)
<400> 162
   tcctgcaagg cttctggata taccttcaca wmctatggaa tgaactgggt gaagcaggc 59
<210> 163
   <211> 59
   <212> DNA
   <213> artificial
<220>
   <223> H1-3P2 (ING-1)
<400> 163
   tcctgcaagg cttctggata taccttcaca castatggaa tgaactgggt gaagcaggc 59
<210> 164
   <211> 59
   <212> DNA
   <213> artificial
<220>
   <223> H1-3A (ING-1)
<400> 164
   tcctgcaagg cttctggata taccttcaca gastatggaa tgaactgggt gaagcaggc 59
<210> 165
   <211> 59
   <212> DNA
   <213> artificial
<220>
   <223> H1-3NP1 (ING-1)
<220>
   <221> misc_feature
   <222> (31)..(31)
   <223> n is a, c, g, or t
<400> 165
   tcctgcaagg cttctggata taccttcaca ntctatggaa tgaactgggt gaagcaggc 59
<210> 166
   <211> 59
   <212> DNA
   <213> artificial
<220>
   <223> H1-3NP2 (ING-1)
<400> 166
   tcctgcaagg cttctggata taccttcaca kggtatggaa tgaactgggt gaagcaggc 59
<210> 167
   <211> 59
   <212> DNA
   <213> artificial
<220>
   <223> H1-3NP3 (ING-1)
<400> 167
   tcctgcaagg cttctggata taccttcaca scgtatggaa tgaactgggt gaagcaggc 59
<210> 168
   <211> 59
   <212> DNA
   <213> artificial
<220>
   <223> H1-4B (ING-1)
<400> 168
   tcctgcaagg cttctggata taccttcaca aaaargggaa tgaactgggt gaagcaggc 59
<210> 169
   <211> 59
   <212> DNA
   <213> artificial
<220>
   <223> H1-4P1 (ING-1)
<400> 169
   tcctgcaagg cttctggata taccttcaca aaawmcggaa tgaactgggt gaagcaggc 59
<210> 170
   <211> 59
   <212> DNA
   <213> artificial
<220>
   <223> H1-4P2 (ING-1)
<400> 170
   tcctgcaagg cttctggata taccttcaca aaacasggaa tgaactgggt gaagcaggc 59
<210> 171
   <211> 59
   <212> DNA
   <213> artificial
<220>
   <223> H1-4A (ING-1)
<400> 171
   tcctgcaagg cttctggata taccttcaca aaagasggaa tgaactgggt gaagcaggc 59
<210> 172
   <211> 59
   <212> DNA
   <213> artificial
<220>
   <223> H1-4NP1 (ING-1)
<220>
   <221> misc_feature
   <222> (34)..(34)
   <223> n is a, c, g, or t
<400> 172
   tcctgcaagg cttctggata taccttcaca aaantcggaa tgaactgggt gaagcaggc 59
<210> 173
   <211> 59
   <212> DNA
   <213> artificial
<220>
   <223> H1-4NP2 (ING-1)
<400> 173
   tcctgcaagg cttctggata taccttcaca aaakggggaa tgaactgggt gaagcaggc 59
<210> 174
   <211> 59
   <212> DNA
   <213> artificial
<220>
   <223> H1-4NP3 (ING-1)
<400> 174
   tcctgcaagg cttctggata taccttcaca aaascgggaa tgaactgggt gaagcaggc 59
<210> 175
   <211> 59
   <212> DNA
   <213> artificial
<220>
   <223> H1-5B (ING-1)
<400> 175
   tcctgcaagg cttctggata taccttcaca aaatatarga tgaactgggt gaagcaggc 59
<210> 176
   <211> 59
   <212> DNA
   <213> artificial
<220>
   <223> H1-5P1 (ING-1)
<400> 176
   tcctgcaagg cttctggata taccttcaca aaatatwmca tgaactgggt gaagcaggc 59
<210> 177
   <211> 59
   <212> DNA
   <213> artificial
<220>
   <223> H1-5P2 (ING-1)
<400> 177
   tcctgcaagg cttctggata taccttcaca aaatatcasa tgaactgggt gaagcaggc 59
<210> 178
   <211> 59
   <212> DNA
   <213> artificial
<220>
   <223> H1-5A (ING-1)
<400> 178
   tcctgcaagg cttctggata taccttcaca aaatatgasa tgaactgggt gaagcaggc 59
<210> 179
   <211> 59
   <212> DNA
   <213> artificial
<220>
   <223> H1-5NP1 (ING-1)
<220>
   <221> misc_feature
   <222> (37)..(37)
   <223> n is a, c, g, or t
<400> 179
   tcctgcaagg cttctggata taccttcaca aaatatntca tgaactgggt gaagcaggc 59
<210> 180
   <211> 59
   <212> DNA
   <213> artificial
<220>
   <223> H1-5NP2 (ING-1)
<400> 180
   tcctgcaagg cttctggata taccttcaca aaatatkgga tgaactgggt gaagcaggc 59
<210> 181
   <211> 59
   <212> DNA
   <213> artificial
<220>
   <223> H1-5NP3 (ING-1)
<400> 181
   tcctgcaagg cttctggata taccttcaca aaatatscga tgaactgggt gaagcaggc 59
<210> 182
   <211> 21
   <212> DNA
   <213> artificial
<220>
   <223> H2R (ING-1)
<400> 182
   gcccatccac tttaaaccct t 21
<210> 183
   <211> 58
   <212> DNA
   <213> artificial
<220>
   <223> H2-1B (ING-1)
<400> 183
   aagggtttaa agtggatggg cargataaac acctacactg aagagcctac atatggtg 58
<210> 184
   <211> 58
   <212> DNA
   <213> artificial
<220>
   <223> H2-1P1 (ING-1)
<400> 184
   aagggtttaa agtggatggg cwmcataaac acctacactg aagagcctac atatggtg 58
<210> 185
   <211> 58
   <212> DNA
   <213> artificial
<220>
   <223> H2-1P2 (ING-1)
<400> 185
   aagggtttaa agtggatggg ccasataaac acctacactg aagagcctac atatggtg 58
<210> 186
   <211> 58
   <212> DNA
   <213> artificial
<220>
   <223> H2-1A (ING-1)
<400> 186
   aagggtttaa agtggatggg cgasataaac acctacactg aagagcctac atatggtg 58
<210> 187
   <211> 58
   <212> DNA
   <213> artificial
<220>
   <223> H2-1NP1 (ING-1)
<220>
   <221> misc_feature
   <222> (22)..(22)
   <223> n is a, c, g, or t
<400> 187
   aagggtttaa agtggatggg cntcataaac acctacactg aagagcctac atatggtg 58
<210> 188
   <211> 58
   <212> DNA
   <213> artificial
<220>
   <223> H2-1NP2 (ING-1)
<400> 188
   aagggtttaa agtggatggg ckggataaac acctacactg aagagcctac atatggtg 58
<210> 189
   <211> 58
   <212> DNA
   <213> artificial
<220>
   <223> H2-1NP3 (ING-1)
<400> 189
   aagggtttaa agtggatggg cscgataaac acctacactg aagagcctac atatggtg 58
<210> 190
   <211> 58
   <212> DNA
   <213> artificial
<220>
   <223> H2-2B (ING-1)
<400> 190
   aagggtttaa agtggatggg ctggataarg acctacactg aagagcctac atatggtg 58
<210> 191
   <211> 58
   <212> DNA
   <213> artificial
<220>
   <223> H2-2P1 (ING-1)
<400> 191
   aagggtttaa agtggatggg ctggatawmc acctacactg aagagcctac atatggtg 58
<210> 192
   <211> 58
   <212> DNA
   <213> artificial
<220>
   <223> H2-2P2 (ING-1)
<400> 192
   aagggtttaa agtggatggg ctggatacas acctacactg aagagcctac atatggtg 58
<210> 193
   <211> 58
   <212> DNA
   <213> artificial
<220>
   <223> H2-2A (ING-1)
<400> 193
   aagggtttaa agtggatggg ctggatagas acctacactg aagagcctac atatggtg 58
<210> 194
   <211> 58
   <212> DNA
   <213> artificial
<220>
   <223> H2-2NP1 (ING-1)
<220>
   <221> misc_feature
   <222> (28)..(28)
   <223> n is a, c, g, or t
<400> 194
   aagggtttaa agtggatggg ctggatantc acctacactg aagagcctac atatggtg 58
<210> 195
   <211> 58
   <212> DNA
   <213> artificial
<220>
   <223> H2-2NP2 (ING-1)
<400> 195
   aagggtttaa agtggatggg ctggatakgg acctacactg aagagcctac atatggtg 58
<210> 196
   <211> 58
   <212> DNA
   <213> artificial
<220>
   <223> H2-2NP3 (ING-1)
<400> 196
   aagggtttaa agtggatggg ctggatascg acctacactg aagagcctac atatggtg 58
<210> 197
   <211> 58
   <212> DNA
   <213> artificial
<220>
   <223> H2-3B (ING-1)
<400> 197
   aagggtttaa agtggatggg ctggataaac argtacactg aagagcctac atatggtg 58
<210> 198
   <211> 58
   <212> DNA
   <213> artificial
<220>
   <223> H2-3P1 (ING-1)
<400> 198
   aagggtttaa agtggatggg ctggataaac wmctacactg aagagcctac atatggtg 58
<210> 199
   <211> 58
   <212> DNA
   <213> artificial
<220>
   <223> H2-3P2 (ING-1)
<400> 199
   aagggtttaa agtggatggg ctggataaac castacactg aagagcctac atatggtg 58
<210> 200
   <211> 58
   <212> DNA
   <213> artificial
<220>
   <223> H2-3A (ING-1)
<400> 200
   aagggtttaa agtggatggg ctggataaac gastacactg aagagcctac atatggtg 58
<210> 201
   <211> 58
   <212> DNA
   <213> artificial
<220>
   <223> H2-3NP1 (ING-1)
<220>
   <221> misc_feature
   <222> (31)..(31)
   <223> n is a, c, g, or t
<400> 201
   aagggtttaa agtggatggg ctggataaac ntctacactg aagagcctac atatggtg 58
<210> 202
   <211> 58
   <212> DNA
   <213> artificial
<220>
   <223> H2-3NP2 (ING-1)
<400> 202
   aagggtttaa agtggatggg ctggataaac kggtacactg aagagcctac atatggtg 58
<210> 203
   <211> 58
   <212> DNA
   <213> artificial
<220>
   <223> H2-3NP3 (ING-1)
<400> 203
   aagggtttaa agtggatggg ctggataaac scgtacactg aagagcctac atatggtg 58
<210> 204
   <211> 58
   <212> DNA
   <213> artificial
<220>
   <223> H2-4B (ING-1)
<400> 204
   aagggtttaa agtggatggg ctggataaac accargactg aagagcctac atatggtg 58
<210> 205
   <211> 58
   <212> DNA
   <213> artificial
<220>
   <223> H2-4P1 (ING-1)
<400> 205
   aagggtttaa agtggatggg ctggataaac accwmcactg aagagcctac atatggtg 58
<210> 206
   <211> 58
   <212> DNA
   <213> artificial
<220>
   <223> H2-4P2 (ING-1)
<400> 206
   aagggtttaa agtggatggg ctggataaac acccasactg aagagcctac atatggtg 58
<210> 207
   <211> 58
   <212> DNA
   <213> artificial
<220>
   <223> H2-4A (ING-1)
<400> 207
   aagggtttaa agtggatggg ctggataaac accgasactg aagagcctac atatggtg 58
<210> 208
   <211> 58
   <212> DNA
   <213> artificial
<220>
   <223> H2-4NP1 (ING-1)
<220>
   <221> misc_feature
   <222> (34)..(34)
   <223> n is a, c, g, or t
<400> 208
   aagggtttaa agtggatggg ctggataaac accntcactg aagagcctac atatggtg 58
<210> 209
   <211> 58
   <212> DNA
   <213> artificial
<220>
   <223> H2-4NP2 (ING-1)
<400> 209
   aagggtttaa agtggatggg ctggataaac acckggactg aagagcctac atatggtg 58
<210> 210
   <211> 58
   <212> DNA
   <213> artificial
<220>
   <223> H2-4NP3 (ING-1)
<400> 210
   aagggtttaa agtggatggg ctggataaac accscgactg aagagcctac atatggtg 58
<210> 211
   <211> 21
   <212> DNA
   <213> artificial
<220>
   <223> H22-R (ING-1)
<400> 211
   gtaggtgttt atccagccca t 21
<210> 212
   <211> 58
   <212> DNA
   <213> artificial
<220>
   <223> H22-1B (ING-1)
<400> 212
   atgggctgga taaacaccta carggaagag cctacatatg gtgatgactt caagggac 58
<210> 213
   <211> 58
   <212> DNA
   <213> artificial
<220>
   <223> H22-1P1 (ING-1)
<400> 213
   atgggctgga taaacaccta cwmcgaagag cctacatatg gtgatgactt caagggac 58
<210> 214
   <211> 58
   <212> DNA
   <213> artificial
<220>
   <223> H22-1P2 (ING-1)
<400> 214
   atgggctgga taaacaccta ccasgaagag cctacatatg gtgatgactt caagggac 58
<210> 215
   <211> 58
   <212> DNA
   <213> artificial
<220>
   <223> H22-1A (ING-1)
<400> 215
   atgggctgga taaacaccta cgasgaagag cctacatatg gtgatgactt caagggac 58
<210> 216
   <211> 58
   <212> DNA
   <213> artificial
<220>
   <223> H22-1NP1 (ING-1)
<220>
   <221> misc_feature
   <222> (22)..(22)
   <223> n is a, c, g, or t
<400> 216
   atgggctgga taaacaccta cntcgaagag cctacatatg gtgatgactt caagggac 58
<210> 217
   <211> 58
   <212> DNA
   <213> artificial
<220>
   <223> H22-1NP2 (ING-1)
<400> 217
   atgggctgga taaacaccta ckgggaagag cctacatatg gtgatgactt caagggac 58
<210> 218
   <211> 58
   <212> DNA
   <213> artificial
<220>
   <223> H22-lNp3 (ING-1)
<400> 218
   atgggctgga taaacaccta cscggaagag cctacatatg gtgatgactt caagggac 58
<210> 219
   <211> 58
   <212> DNA
   <213> artificial
<220>
   <223> H22-2B (ING-1)
<400> 219
   atgggctgga taaacaccta cactarggag cctacatatg gtgatgactt caagggac 58
<210> 220
   <211> 58
   <212> DNA
   <213> artificial
<220>
   <223> H22-2P1 (ING-1)
<400> 220
   atgggctgga taaacaccta cactwmcgag cctacatatg gtgatgactt caagggac 58
<210> 221
   <211> 58
   <212> DNA
   <213> artificial
<220>
   <223> H22-2P2 (ING-1)
<400> 221
   atgggctgga taaacaccta cactcasgag cctacatatg gtgatgactt caagggac 58
<210> 222
   <211> 58
   <212> DNA
   <213> artificial
<220>
   <223> H22-2A (ING-1)
<400> 222
   atgggctgga taaacaccta cactgasgag cctacatatg gtgatgactt caagggac 58
<210> 223
   <211> 58
   <212> DNA
   <213> artificial
<220>
   <223> H22-2NP1 (ING-1)
<220>
   <221> misc_feature
   <222> (25)..(25)
   <223> n is a, c, g, or t
<400> 223
   atgggctgga taaacaccta cactntcgag cctacatatg gtgatgactt caagggac 58
<210> 224
   <211> 58
   <212> DNA
   <213> artificial
<220>
   <223> H22-2NP2 (ING-1)
<400> 224
   atgggctgga taaacaccta cactkgggag cctacatatg gtgatgactt caagggac 58
<210> 225
   <211> 58
   <212> DNA
   <213> artificial
<220>
   <223> H22-2NP3 (ING-1)
<400> 225
   atgggctgga taaacaccta cactscggag cctacatatg gtgatgactt caagggac 58
<210> 226
   <211> 58
   <212> DNA
   <213> artificial
<220>
   <223> H22-3B (ING-1)
<400> 226
   atgggctgga taaacaccta cactgaaarg cctacatatg gtgatgactt caagggac 58
<210> 227
   <211> 58
   <212> DNA
   <213> artificial
<220>
   <223> H22-3P1 (ING-1)
<400> 227
   atgggctgga taaacaccta cactgaawmc cctacatatg gtgatgactt caagggac 58
<210> 228
   <211> 58
   <212> DNA
   <213> artificial
<220>
   <223> H22-3P2 (ING-1)
<400> 228
   atgggctgga taaacaccta cactgaacas cctacatatg gtgatgactt caagggac 58
<210> 229
   <211> 58
   <212> DNA
   <213> artificial
<220>
   <223> H22-3A (ING-1)
<400> 229
   atgggctgga taaacaccta cactgaagas cctacatatg gtgatgactt caagggac 58
<210> 230
   <211> 58
   <212> DNA
   <213> artificial
<220>
   <223> H22-3NP1 (ING-1)
<220>
   <221> misc_feature
   <222> (28)..(28)
   <223> n is a, c, g, or t
<400> 230
   atgggctgga taaacaccta cactgaantc cctacatatg gtgatgactt caagggac 58
<210> 231
   <211> 58
   <212> DNA
   <213> artificial
<220>
   <223> H22-3NP2 (ING-1)
<400> 231
   atgggctgga taaacaccta cactgaakgg cctacatatg gtgatgactt caagggac 58
<210> 232
   <211> 58
   <212> DNA
   <213> artificial
<220>
   <223> H22-3NP3 (ING-1)
<220>
   <221> misc_feature
   <223> H22-3NP3
<400> 232
   atgggctgga taaacaccta cactgaascg cctacatatg gtgatgactt caagggac 58
<210> 233
   <211> 58
   <212> DNA
   <213> artificial
<220>
   <223> H22-4B (ING-1)
<400> 233
   atgggctgga taaacaccta cactgaagag argacatatg gtgatgactt caagggac 58
<210> 234
   <211> 58
   <212> DNA
   <213> artificial
<220>
   <223> H22-4P1 (ING-1)
<400> 234
   atgggctgga taaacaccta cactgaagag wmcacatatg gtgatgactt caagggac 58
<210> 235
   <211> 58
   <212> DNA
   <213> artificial
<220>
   <223> H22-4P2 (ING-1)
<400> 235
   atgggctgga taaacaccta cactgaagag casacatatg gtgatgactt caagggac 58
<210> 236
   <211> 58
   <212> DNA
   <213> artificial
<220>
   <223> H22-4A (ING-1)
<400> 236
   atgggctgga taaacaccta cactgaagag gasacatatg gtgatgactt caagggac 58
<210> 237
   <211> 58
   <212> DNA
   <213> artificial
<220>
   <223> H22-4NP1 (ING-1)
<400> 237
   atgggctgga taaacaccta cactgaagag gasacatatg gtgatgactt caagggac 58
<210> 238
   <211> 58
   <212> DNA
   <213> artificial
<220>
   <223> H22-4NP2 (ING-1)
<400> 238
   atgggctgga taaacaccta cactgaagag kggacatatg gtgatgactt caagggac 58
<210> 239
   <211> 58
   <212> DNA
   <213> artificial
<220>
   <223> H22-4NP3 (ING-1)
<400> 239
   atgggctgga taaacaccta cactgaagag scgacatatg gtgatgactt caagggac 58
<210> 240
   <211> 58
   <212> DNA
   <213> artificial
<220>
   <223> H22-5B (ING-1)
<400> 240
   atgggctgga taaacaccta cactgaagag cctargtatg gtgatgactt caagggac 58
<210> 241
   <211> 58
   <212> DNA
   <213> artificial
<220>
   <223> H22-5P1 (ING-1)
<400> 241
   atgggctgga taaacaccta cactgaagag cctwmctatg gtgatgactt caagggac 58
<210> 242
   <211> 58
   <212> DNA
   <213> artificial
<220>
   <223> H22-5P2 (ING-1)
<400> 242
   atgggctgga taaacaccta cactgaagag cctcastatg gtgatgactt caagggac 58
<210> 243
   <211> 58
   <212> DNA
   <213> artificial
<220>
   <223> H22-5A (ING-1)
<400> 243
   atgggctgga taaacaccta cactgaagag cctgastatg gtgatgactt caagggac 58
<210> 244
   <211> 58
   <212> DNA
   <213> artificial
<220>
   <223> H22-5NP1 (ING-1)
<220>
   <221> misc_feature
   <222> (34)..(34)
   <223> n is a, c, g, or t
<400> 244
   atgggctgga taaacaccta cactgaagag cctntctatg gtgatgactt caagggac 58
<210> 245
   <211> 58
   <212> DNA
   <213> artificial
<220>
   <223> H22-5NP2 (ING-1)
<400> 245
   atgggctgga taaacaccta cactgaagag cctkggtatg gtgatgactt caagggac 58
<210> 246
   <211> 58
   <212> DNA
   <213> artificial
<220>
   <223> H22-5NP3 (ING-1)
<400> 246
   atgggctgga taaacaccta cactgaagag cctscgtatg gtgatgactt caagggac 58
<210> 247
   <211> 24
   <212> DNA
   <213> artificial
<220>
   <223> H3-R (ING-1)
<400> 247
   aaatcttgca cagaaatatg tagc 24
<210> 248
   <211> 53
   <212> DNA
   <213> artificial
<220>
   <223> H3-1B (ING-1)
<400> 248
   gctacatatt tctgtgcaag atttargtct gctgtggact actggggtca agg 53
<210> 249
   <211> 53
   <212> DNA
   <213> artificial
<220>
   <223> H3-1P1 (ING-1)
<400> 249
   gctacatatt tctgtgcaag atttwmctct gctgtggact actggggtca agg 53
<210> 250
   <211> 53
   <212> DNA
   <213> artificial
<220>
   <223> H3-1P2 (ING-1)
<400> 250
   gctacatatt tctgtgcaag atttcastct gctgtggact actggggtca agg 53
<210> 251
   <211> 53
   <212> DNA
   <213> artificial
<220>
   <223> H3-1A (ING-1)
<400> 251
   gctacatatt tctgtgcaag atttgastct gctgtggact actggggtca agg 53
<210> 252
   <211> 53
   <212> DNA
   <213> artificial
<220>
   <223> H3-1NP1 (ING-1)
<220>
   <221> misc_feature
   <222> (25)..(25)
   <223> n is a, c, g, or t
<400> 252
   gctacatatt tctgtgcaag atttntctct gctgtggact actggggtca agg 53
<210> 253
   <211> 53
   <212> DNA
   <213> artificial
<220>
   <223> H3-1NP2 (ING-1)
<400> 253
   gctacatatt tctgtgcaag atttkggtct gctgtggact actggggtca agg 53
<210> 254
   <211> 53
   <212> DNA
   <213> artificial
<220>
   <223> H3-1NP3 (ING-1)
<400> 254
   gctacatatt tctgtgcaag atttscgtct gctgtggact actggggtca agg 53
<210> 255
   <211> 53
   <212> DNA
   <213> artificial
<220>
   <223> H3-2B (ING-1)
<400> 255
   gctacatatt tctgtgcaag atttggcarg gctgtggact actggggtca agg 53
<210> 256
   <211> 53
   <212> DNA
   <213> artificial
<220>
   <223> H3-2P1 (ING-1)
<400> 256
   gctacatatt tctgtgcaag atttggcwmc gctgtggact actggggtca agg 53
<210> 257
   <211> 53
   <212> DNA
   <213> artificial
<220>
   <223> H3-2P2 (ING-1)
<400> 257
   gctacatatt tctgtgcaag atttggccas gctgtggact actggggtca agg 53
<210> 258
   <211> 53
   <212> DNA
   <213> artificial
<220>
   <223> H3-2A (ING-1)
<400> 258
   gctacatatt tctgtgcaag atttggcgas gctgtggact actggggtca agg 53
<210> 259
   <211> 53
   <212> DNA
   <213> artificial
<220>
   <223> H3-2NP1 (ING-1)
<220>
   <221> misc_feature
   <222> (28)..(28)
   <223> n is a, c, g, or t
<400> 259
   gctacatatt tctgtgcaag atttggcntc gctgtggact actggggtca agg 53
<210> 260
   <211> 53
   <212> DNA
   <213> artificial
<220>
   <223> H3-2NP2 (ING-1)
<400> 260
   gctacatatt tctgtgcaag atttggckgg gctgtggact actggggtca agg 53
<210> 261
   <211> 53
   <212> DNA
   <213> artificial
<220>
   <223> H3-2NP3 (ING-1)
<400> 261
   gctacatatt tctgtgcaag atttggcscg gctgtggact actggggtca agg 53
<210> 262
   <211> 53
   <212> DNA
   <213> artificial
<220>
   <223> H3-3B (ING-1)
<400> 262
   gctacatatt tctgtgcaag atttggctct arggtggact actggggtca agg 53
<210> 263
   <211> 53
   <212> DNA
   <213> artificial
<220>
   <223> H3-3P1 (ING-1)
<400> 263
   gctacatatt tctgtgcaag atttggctct wmcgtggact actggggtca agg 53
<210> 264
   <211> 53
   <212> DNA
   <213> artificial
<220>
   <223> H3-3P2 (ING-1)
<400> 264
   gctacatatt tctgtgcaag atttggctct casgtggact actggggtca agg 53
<210> 265
   <211> 53
   <212> DNA
   <213> artificial
<220>
   <223> H3-3A (ING-1)
<400> 265
   gctacatatt tctgtgcaag atttggctct gasgtggact actggggtca agg 53
<210> 266
   <211> 53
   <212> DNA
   <213> artificial
<220>
   <223> H3-3NP1 (ING-1)
<220>
   <221> misc_feature
   <222> (31)..(31)
   <223> n is a, c, g, or t
<400> 266
   gctacatatt tctgtgcaag atttggctct ntcgtggact actggggtca agg 53
<210> 267
   <211> 53
   <212> DNA
   <213> artificial
<220>
   <223> H3-3NP2 (ING-1)
<400> 267
   gctacatatt tctgtgcaag atttggctct kgggtggact actggggtca agg 53
<210> 268
   <211> 53
   <212> DNA
   <213> artificial
<220>
   <223> H3-3NP3 (ING-1)
<400> 268
   gctacatatt tctgtgcaag atttggctct scggtggact actggggtca agg 53
<210> 269
   <211> 21
   <212> DNA
   <213> artificial
<220>
   <223> H32-R (ING-1)
<400> 269
   cacagcagag ccaaatcttg c 21
<210> 270
   <211> 46
   <212> DNA
   <213> artificial
<220>
   <223> H32-1B (ING-1)
<400> 270
   gcaagatttg gctctgctgt gargtactgg ggtcaaggaa cctcgg 46
<210> 271
   <211> 46
   <212> DNA
   <213> artificial
<220>
   <223> H32-1P1 (ING-1)
<400> 271
   gcaagatttg gctctgctgt gwmctactgg ggtcaaggaa cctcgg 46
<210> 272
   <211> 46
   <212> DNA
   <213> artificial
<220>
   <223> H32-1P2 (ING-1)
<400> 272
   gcaagatttg gctctgctgt gcastactgg ggtcaaggaa cctcgg 46
<210> 273
   <211> 46
   <212> DNA
   <213> artificial
<220>
   <223> H32-1A (ING-1)
<400> 273
   gcaagatttg gctctgctgt ggastactgg ggtcaaggaa cctcgg 46
<210> 274
   <211> 46
   <212> DNA
   <213> artificial
<220>
   <223> H32-1NP1 (ING-1)
<220>
   <221> misc_feature
   <222> (22)..(22)
   <223> n is a, c, g, or t
<400> 274
   gcaagatttg gctctgctgt gntctactgg ggtcaaggaa cctcgg 46
<210> 275
   <211> 46
   <212> DNA
   <213> artificial
<220>
   <223> H32-1NP2 (ING-1)
<400> 275
   gcaagatttg gctctgctgt gkggtactgg ggtcaaggaa cctcgg 46
<210> 276
   <211> 46
   <212> DNA
   <213> artificial
<220>
   <223> H32-1NP3 (ING-1)
<400> 276
   gcaagatttg gctctgctgt gscgtactgg ggtcaaggaa cctcgg 46
<210> 277
   <211> 46
   <212> DNA
   <213> artificial
<220>
   <223> H32-2B (ING-1)
<400> 277
   gcaagatttg gctctgctgt ggacargtgg ggtcaaggaa cctcgg 46
<210> 278
   <211> 46
   <212> DNA
   <213> artificial
<220>
   <223> H32-2P1 (ING-1)
<400> 278
   gcaagatttg gctctgctgt ggacwmctgg ggtcaaggaa cctcgg 46
<210> 279
   <211> 46
   <212> DNA
   <213> artificial
<220>
   <223> H32-2P2 (ING-1)
<400> 279
   gcaagatttg gctctgctgt ggaccastgg ggtcaaggaa cctcgg 46
<210> 280
   <211> 46
   <212> DNA
   <213> artificial
<220>
   <223> H32-2A (ING-1)
<400> 280
   gcaagatttg gctctgctgt ggacgastgg ggtcaaggaa cctcgg 46
<210> 281
   <211> 46
   <212> DNA
   <213> artificial
<220>
   <223> H32-2NP1 (ING-1)
<220>
   <221> misc_feature
   <222> (25)..(25)
   <223> n is a, c, g, or t
<400> 281
   gcaagatttg gctctgctgt ggacntctgg ggtcaaggaa cctcgg 46
<210> 282
   <211> 46
   <212> DNA
   <213> artificial
<220>
   <223> H32-2NP2 (ING-1)
<400> 282
   gcaagatttg gctctgctgt ggackggtgg ggtcaaggaa cctcgg 46
<210> 283
   <211> 46
   <212> DNA
   <213> artificial
<220>
   <223> H32-2NP3 (ING-1)
<400> 283
   gcaagatttg gctctgctgt ggacscgtgg ggtcaaggaa cctcgg 46
<210> 284
   <211> 22
   <212> DNA
   <213> artificial
<220>
   <223> Ascl-F2 (ING-1)
<400> 284
   taataaggcg cgcctaacca tc 22
<210> 285
   <211> 31
   <212> DNA
   <213> artificial
<220>
   <223> Notl-R (ING-1)
<400> 285
   agcggccgca caagatttgg gctcaactct c 31
<210> 286
   <211> 20
   <212> DNA
   <213> Artificial
<220>
   <223> L1R (XPA-23)
<400> 286
   actcgcccga caaatgatgg 20
<210> 287
   <211> 58
   <212> DNA
   <213> artificial
<220>
   <223> L1-1B (XPA-23)
<400> 287
   ccatcatttg tcgggcgagt arggatatta acaggtggtt agcctggtat cagcagac 58
<210> 288
   <211> 58
   <212> DNA
   <213> artificial
<220>
   <223> L1-1P1 (XPA-23)
<400> 288
   ccatcatttg tcgggcgagt wmcgatatta acaggtggtt agcctggtat cagcagac 58
<210> 289
   <211> 58
   <212> DNA
   <213> artificial
<220>
   <223> L1-1P2 (XPA-23)
<400> 289
   ccatcatttg tcgggcgagt casgatatta acaggtggtt agcctggtat cagcagac 58
<210> 290
   <211> 58
   <212> DNA
   <213> artificial
<220>
   <223> L1-1A (XPA-23)
<400> 290
   ccatcatttg tcgggcgagt gasgatatta acaggtggtt agcctggtat cagcagac 58
<210> 291
   <211> 58
   <212> DNA
   <213> artificial
<220>
   <223> L1-1NP1 (XPA-23)
<220>
   <221> misc_feature
   <222> (21)..(21)
   <223> n is a, c, g, or t
<400> 291
   ccatcatttg tcgggcgagt ntcgatatta acaggtggtt agcctggtat cagcagac 58
<210> 292
   <211> 58
   <212> DNA
   <213> artificial
<220>
   <223> L1-1NP2 (XPA-23)
<400> 292
   ccatcatttg tcgggcgagt kgggatatta acaggtggtt agcctggtat cagcagac 58
<210> 293
   <211> 58
   <212> DNA
   <213> artificial
<220>
   <223> L1-1NP3 (XPA-23)
<400> 293
   ccatcatttg tcgggcgagt scggatatta acaggtggtt agcctggtat cagcagac 58
<210> 294
   <211> 58
   <212> DNA
   <213> artificial
<220>
   <223> L1-2B (XPA-23)
<400> 294
   ccatcatttg tcgggcgagt cagargatta acaggtggtt agcctggtat cagcagac 58
<210> 295
   <211> 58
   <212> DNA
   <213> artificial
<220>
   <223> L1-2P1 (XPA-23)
<400> 295
   ccatcatttg tcgggcgagt cagwmcatta acaggtggtt agcctggtat cagcagac 58
<210> 296
   <211> 58
   <212> DNA
   <213> artificial
<220>
   <223> L1-2P2 (XPA-23)
<400> 296
   ccatcatttg tcgggcgagt cagcasatta acaggtggtt agcctggtat cagcagac 58
<210> 297
   <211> 58
   <212> DNA
   <213> artificial
<220>
   <223> L1-2A (XPA-23)
<400> 297
   ccatcatttg tcgggcgagt caggasatta acaggtggtt agcctggtat cagcagac 58
<210> 298
   <211> 58
   <212> DNA
   <213> artificial
<220>
   <223> L1-2NP1 (XPA-23)
<220>
   <221> misc_feature
   <222> (24)..(24)
   <223> n is a, c, g, or t
<400> 298
   ccatcatttg tcgggcgagt cagntcatta acaggtggtt agcctggtat cagcagac 58
<210> 299
   <211> 58
   <212> DNA
   <213> artificial
<220>
   <223> L1-2NP2 (XPA-23)
<400> 299
   ccatcatttg tcgggcgagt cagkggatta acaggtggtt agcctggtat cagcagac 58
<210> 300
   <211> 58
   <212> DNA
   <213> artificial
<220>
   <223> L1-2NP3 (XPA-23)
<400> 300
   ccatcatttg tcgggcgagt cagscgatta acaggtggtt agcctggtat cagcagac 58
<210> 301
   <211> 58
   <212> DNA
   <213> artificial
<220>
   <223> L1-3B (XPA-23)
<400> 301
   ccatcatttg tcgggcgagt caggatatta rgaggtggtt agcctggtat cagcagac 58
<210> 302
   <211> 58
   <212> DNA
   <213> artificial
<220>
   <223> L1-3P1 (XPA-23)
<400> 302
   ccatcatttg tcgggcgagt caggatattw mcaggtggtt agcctggtat cagcagac 58
<210> 303
   <211> 58
   <212> DNA
   <213> artificial
<220>
   <223> L1-3P2 (XPA-23)
<400> 303
   ccatcatttg tcgggcgagt caggatattc asaggtggtt agcctggtat cagcagac 58
<210> 304
   <211> 58
   <212> DNA
   <213> artificial
<220>
   <223> L1-3A (XPA-23)
<400> 304
   ccatcatttg tcgggcgagt caggatattg asaggtggtt agcctggtat cagcagac 58
<210> 305
   <211> 58
   <212> DNA
   <213> artificial
<220>
   <223> L1-3NP1 (XPA-23)
<220>
   <221> misc_feature
   <222> (30)..(30)
   <223> n is a, c, g, or t
<400> 305
   ccatcatttg tcgggcgagt caggatattn tcaggtggtt agcctggtat cagcagac 58
<210> 306
   <211> 58
   <212> DNA
   <213> artificial
<220>
   <223> L1-3NP2 (XPA-23)
<400> 306
   ccatcatttg tcgggcgagt caggatattk ggaggtggtt agcctggtat cagcagac 58
<210> 307
   <211> 58
   <212> DNA
   <213> artificial
<220>
   <223> L1-3NP3 (XPA-23)
<400> 307
   ccatcatttg tcgggcgagt caggatatts cgaggtggtt agcctggtat cagcagac 58
<210> 308
   <211> 58
   <212> DNA
   <213> artificial
<220>
   <223> L1-4B (XPA-23)
<400> 308
   ccatcatttg tcgggcgagt caggatatta acargtggtt agcctggtat cagcagac 58
<210> 309
   <211> 58
   <212> DNA
   <213> artificial
<220>
   <223> L1-4P1 (XPA-23)
<400> 309
   ccatcatttg tcgggcgagt caggatatta acwmctggtt agcctggtat cagcagac 58
<210> 310
   <211> 58
   <212> DNA
   <213> artificial
<220>
   <223> L1-4P2 (XPA-23)
<400> 310
   ccatcatttg tcgggcgagt caggatatta accastggtt agcctggtat cagcagac 58
<210> 311
   <211> 58
   <212> DNA
   <213> artificial
<220>
   <223> L1-4A (XPA-23)
<400> 311
   ccatcatttg tcgggcgagt caggatatta acgastggtt agcctggtat cagcagac 58
<210> 312
   <211> 58
   <212> DNA
   <213> artificial
<220>
   <223> L1-4NP1 (XPA-23)
<220>
   <221> misc_feature
   <222> (33)..(33)
   <223> n is a, c, g, or t
<400> 312
   ccatcatttg tcgggcgagt caggatatta acntctggtt agcctggtat cagcagac 58
<210> 313
   <211> 58
   <212> DNA
   <213> artificial
<220>
   <223> L1-4NP2 (XPA-23)
<400> 313
   ccatcatttg tcgggcgagt caggatatta ackggtggtt agcctggtat cagcagac 58
<210> 314
   <211> 58
   <212> DNA
   <213> artificial
<220>
   <223> L1-4NP3 (XPA-23)
<400> 314
   ccatcatttg tcgggcgagt caggatatta acscgtggtt agcctggtat cagcagac 58
<210> 315
   <211> 58
   <212> DNA
   <213> artificial
<220>
   <223> L1-5B (XPA-23)
<400> 315
   ccatcatttg tcgggcgagt caggatatta acaggargtt agcctggtat cagcagac 58
<210> 316
   <211> 58
   <212> DNA
   <213> artificial
<220>
   <223> L1-5P1 (XPA-23)
<400> 316
   ccatcatttg tcgggcgagt caggatatta acaggwmctt agcctggtat cagcagac 58
<210> 317
   <211> 58
   <212> DNA
   <213> artificial
<220>
   <223> L1-5P2 (XPA-23)
<400> 317
   ccatcatttg tcgggcgagt caggatatta acaggcastt agcctggtat cagcagac 58
<210> 318
   <211> 58
   <212> DNA
   <213> artificial
<220>
   <223> L1-5NP1 (XPA-23)
<220>
   <221> misc_feature
   <222> (36)..(36)
   <223> n is a, c, g, or t
<400> 318
   ccatcatttg tcgggcgagt caggatatta acaggntctt agcctggtat cagcagac 58
<210> 319
   <211> 58
   <212> DNA
   <213> artificial
<220>
   <223> L1-5NP2 (XPA-23)
<400> 319
   ccatcatttg tcgggcgagt caggatatta acaggkggtt agcctggtat cagcagac 58
<210> 320
   <211> 58
   <212> DNA
   <213> artificial
<220>
   <223> L1-5NP3 (XPA-23)
<400> 320
   ccatcatttg tcgggcgagt caggatatta acaggscgtt agcctggtat cagcagac 58
<210> 321
   <211> 20
   <212> DNA
   <213> artificial
<220>
   <223> L2R (XPA-23)
<400> 321
   gatcaggagc ttaggggcat 20
<210> 322
   <211> 55
   <212> DNA
   <213> artificial
<220>
   <223> L2-1B (XPA-23)
<400> 322
   atgcccctaa gctcctgatc argtctgcaa ccagtctgca aagtggggtc ccatc 55
<210> 323
   <211> 55
   <212> DNA
   <213> artificial
<220>
   <223> L2-1P1 (XPA-23)
<400> 323
   atgcccctaa gctcctgatc wmctctgcaa ccagtctgca aagtggggtc ccatc 55
<210> 324
   <211> 55
   <212> DNA
   <213> artificial
<220>
   <223> L2-1P2 (XPA-23)
<400> 324
   atgcccctaa gctcctgatc castctgcaa ccagtctgca aagtggggtc ccatc 55
<210> 325
   <211> 55
   <212> DNA
   <213> artificial
<220>
   <223> L2-1A (XPA-23)
<400> 325
   atgcccctaa gctcctgatc gastctgcaa ccagtctgca aagtggggtc ccatc 55
<210> 326
   <211> 55
   <212> DNA
   <213> artificial
<220>
   <223> L2-1NP1 (XPA-23)
<220>
   <221> misc_feature
   <222> (21)..(21)
   <223> n is a, c, g, or t
<400> 326
   atgcccctaa gctcctgatc ntctctgcaa ccagtctgca aagtggggtc ccatc 55
<210> 327
   <211> 55
   <212> DNA
   <213> artificial
<220>
   <223> L2-1NP2 (XPA-23)
<400> 327
   atgcccctaa gctcctgatc kggtctgcaa ccagtctgca aagtggggtc ccatc 55
<210> 328
   <211> 55
   <212> DNA
   <213> artificial
<220>
   <223> L2-1NP3 (XPA-23)
<400> 328
   atgcccctaa gctcctgatc scgtctgcaa ccagtctgca aagtggggtc ccatc 55
<210> 329
   <211> 55
   <212> DNA
   <213> artificial
<220>
   <223> L2-2B (XPA-23)
<400> 329
   atgcccctaa gctcctgatc catarggcaa ccagtctgca aagtggggtc ccatc 55
<210> 330
   <211> 55
   <212> DNA
   <213> artificial
<220>
   <223> L2-2P1 (XPA-23)
<400> 330
   atgcccctaa gctcctgatc catwmcgcaa ccagtctgca aagtggggtc ccatc 55
<210> 331
   <211> 55
   <212> DNA
   <213> artificial
<220>
   <223> L2-2P2 (XPA-23)
<400> 331
   atgcccctaa gctcctgatc catcasgcaa ccagtctgca aagtggggtc ccatc 55
<210> 332
   <211> 55
   <212> DNA
   <213> artificial
<220>
   <223> L2-2A (XPA-23)
<400> 332
   atgcccctaa gctcctgatc catgasgcaa ccagtctgca aagtggggtc ccatc 55
<210> 333
   <211> 55
   <212> DNA
   <213> artificial
<220>
   <223> L2-2NP1 (XPA-23)
<220>
   <221> misc_feature
   <222> (24)..(24)
   <223> n is a, c, g, or t
<400> 333
   atgcccctaa gctcctgatc catntcgcaa ccagtctgca aagtggggtc ccatc 55
<210> 334
   <211> 55
   <212> DNA
   <213> artificial
<220>
   <223> L2-2NP2 (XPA-23)
<400> 334
   atgcccctaa gctcctgatc catkgggcaa ccagtctgca aagtggggtc ccatc 55
<210> 335
   <211> 55
   <212> DNA
   <213> artificial
<220>
   <223> L2-2NP3 (XPA-23)
<400> 335
   atgcccctaa gctcctgatc catscggcaa ccagtctgca aagtggggtc ccatc 55
<210> 336
   <211> 55
   <212> DNA
   <213> artificial
<220>
   <223> L2-3B (XPA-23)
<400> 336
   atgcccctaa gctcctgatc cattctarga ccagtctgca aagtggggtc ccatc 55
<210> 337
   <211> 55
   <212> DNA
   <213> artificial
<220>
   <223> L2-3P1 (XPA-23)
<400> 337
   atgcccctaa gctcctgatc cattctwmca ccagtctgca aagtggggtc ccatc 55
<210> 338
   <211> 55
   <212> DNA
   <213> artificial
<220>
   <223> L2-3P2 (XPA-23)
<400> 338
   atgcccctaa gctcctgatc cattctcasa ccagtctgca aagtggggtc ccatc 55
<210> 339
   <211> 55
   <212> DNA
   <213> artificial
<220>
   <223> L2-3A (XPA-23)
<400> 339
   atgcccctaa gctcctgatc cattctgasa ccagtctgca aagtggggtc ccatc 55
<210> 340
   <211> 55
   <212> DNA
   <213> artificial
<220>
   <223> L2-3NP1 (XPA-23)
<220>
   <221> misc_feature
   <222> (27)..(27)
   <223> n is a, c, g, or t
<400> 340
   atgcccctaa gctcctgatc cattctntca ccagtctgca aagtggggtc ccatc 55
<210> 341
   <211> 55
   <212> DNA
   <213> artificial
<220>
   <223> L2-3NP2 (XPA-23)
<400> 341
   atgcccctaa gctcctgatc cattctkgga ccagtctgca aagtggggtc ccatc 55
<210> 342
   <211> 55
   <212> DNA
   <213> artificial
<220>
   <223> L2-3NP3 (XPA-23)
<400> 342
   atgcccctaa gctcctgatc cattctscga ccagtctgca aagtggggtc ccatc 55
<210> 343
   <211> 55
   <212> DNA
   <213> artificial
<220>
   <223> L2-4B (XPA-23)
<400> 343
   atgcccctaa gctcctgatc cattctgcaa rgagtctgca aagtggggtc ccatc 55
<210> 344
   <211> 55
   <212> DNA
   <213> artificial
<220>
   <223> L2-4P1 (XPA-23)
<400> 344
   atgcccctaa gctcctgatc cattctgcaw mcagtctgca aagtggggtc ccatc 55
<210> 345
   <211> 55
   <212> DNA
   <213> artificial
<220>
   <223> L2-4P2 (XPA-23)
<400> 345
   atgcccctaa gctcctgatc cattctgcac asagtctgca aagtggggtc ccatc 55
<210> 346
   <211> 55
   <212> DNA
   <213> artificial
<220>
   <223> L2-4A (XPA-23)
<400> 346
   atgcccctaa gctcctgatc cattctgcag asagtctgca aagtggggtc ccatc 55
<210> 347
   <211> 55
   <212> DNA
   <213> artificial
<220>
   <223> L2-4NP1 (XPA-23)
<220>
   <221> misc_feature
   <222> (30)..(30)
   <223> n is a, c, g, or t
<400> 347
   atgcccctaa gctcctgatc cattctgcan tcagtctgca aagtggggtc ccatc 55
<210> 348
   <211> 55
   <212> DNA
   <213> artificial
<220>
   <223> L2-4NP2 (XPA-23)
<400> 348
   atgcccctaa gctcctgatc cattctgcak ggagtctgca aagtggggtc ccatc 55
<210> 349
   <211> 55
   <212> DNA
   <213> artificial
<220>
   <223> L2-4NP3 (XPA-23)
<400> 349
   atgcccctaa gctcctgatc cattctgcas cgagtctgca aagtggggtc ccatc 55
<210> 350
   <211> 55
   <212> DNA
   <213> artificial
<220>
   <223> L2-5B (XPA-23)
<400> 350
   atgcccctaa gctcctgatc cattctgcaa ccargctgca aagtggggtc ccatc 55
<210> 351
   <211> 55
   <212> DNA
   <213> artificial
<220>
   <223> L2-5P1 (XPA-23)
<400> 351
   atgcccctaa gctcctgatc cattctgcaa ccwmcctgca aagtggggtc ccatc 55
<210> 352
   <211> 55
   <212> DNA
   <213> artificial
<220>
   <223> L2-5P2 (XPA-23)
<400> 352
   atgcccctaa gctcctgatc cattctgcaa cccasctgca aagtggggtc ccatc 55
<210> 353
   <211> 55
   <212> DNA
   <213> artificial
<220>
   <223> L2-5A (XPA-23)
<400> 353
   atgcccctaa gctcctgatc cattctgcaa ccgasctgca aagtggggtc ccatc 55
<210> 354
   <211> 55
   <212> DNA
   <213> artificial
<220>
   <223> L2-5NP1 (XPA-23)
<220>
   <221> misc_feature
   <222> (33)..(33)
   <223> n is a, c, g, or t
<400> 354
   atgcccctaa gctcctgatc cattctgcaa ccntcctgca aagtggggtc ccatc 55
<210> 355
   <211> 55
   <212> DNA
   <213> artificial
<220>
   <223> L2-5NP2 (XPA-23)
<400> 355
   atgcccctaa gctcctgatc cattctgcaa cckggctgca aagtggggtc ccatc 55
<210> 356
   <211> 55
   <212> DNA
   <213> artificial
<220>
   <223> L2-5NP3 (XPA-23)
<400> 356
   atgcccctaa gctcctgatc cattctgcaa ccscgctgca aagtggggtc ccatc 55
<210> 357
   <211> 21
   <212> DNA
   <213> artificial
<220>
   <223> L3R (XPA-23)
<400> 357
   ctgctgacaa tagtaagttg c 21
<210> 358
   <211> 57
   <212> DNA
   <213> artificial
<220>
   <223> L3-1B (XPA-23)
<400> 358
   gcaacttact attgtcagca garggacagt ttcccgctca ctttcggcgg agggacc 57
<210> 359
   <211> 57
   <212> DNA
   <213> artificial
<220>
   <223> L3-1P1 (XPA-23)
<400> 359
   gcaacttact attgtcagca gwmcgacagt ttcccgctca ctttcggcgg agggacc 57
<210> 360
   <211> 57
   <212> DNA
   <213> artificial
<220>
   <223> L3-1P2 (XPA-23)
<400> 360
   gcaacttact attgtcagca gcasgacagt ttcccgctca ctttcggcgg agggacc 57
<210> 361
   <211> 57
   <212> DNA
   <213> artificial
<220>
   <223> L3-1A (XPA-23)
<400> 361
   gcaacttact attgtcagca ggasgacagt ttcccgctca ctttcggcgg agggacc 57
<210> 362
   <211> 57
   <212> DNA
   <213> artificial
<220>
   <223> L3-1NP1 (XPA-23)
<220>
   <221> misc_feature
   <222> (22)..(22)
   <223> n is a, c, g, or t
<400> 362
   gcaacttact attgtcagca gntcgacagt ttcccgctca ctttcggcgg agggacc 57
<210> 363
   <211> 57
   <212> DNA
   <213> artificial
<220>
   <223> L3-1NP2 (XPA-23)
<400> 363
   gcaacttact attgtcagca gkgggacagt ttcccgctca ctttcggcgg agggacc 57
<210> 364
   <211> 57
   <212> DNA
   <213> artificial
<220>
   <223> L3-1NP3 (XPA-23)
<400> 364
   gcaacttact attgtcagca gscggacagt ttcccgctca ctttcggcgg agggacc 57
<210> 365
   <211> 57
   <212> DNA
   <213> artificial
<220>
   <223> L3-2B (XPA-23)
<400> 365
   gcaacttact attgtcagca ggctargagt ttcccgctca ctttcggcgg agggacc 57
<210> 366
   <211> 57
   <212> DNA
   <213> artificial
<220>
   <223> L3-2P1 (XPA-23)
<400> 366
   gcaacttact attgtcagca ggctwmcagt ttcccgctca ctttcggcgg agggacc 57
<210> 367
   <211> 57
   <212> DNA
   <213> artificial
<220>
   <223> L3-2P2 (XPA-23)
<400> 367
   gcaacttact attgtcagca ggctcasagt ttcccgctca ctttcggcgg agggacc 57
<210> 368
   <211> 57
   <212> DNA
   <213> artificial
<220>
   <223> L3-2A (XPA-23)
<400> 368
   gcaacttact attgtcagca ggctgasagt ttcccgctca ctttcggcgg agggacc 57
<210> 369
   <211> 57
   <212> DNA
   <213> artificial
<220>
   <223> L3-2NP1 (XPA-23)
<220>
   <221> misc_feature
   <222> (25)..(25)
   <223> n is a, c, g, or t
<400> 369
   gcaacttact attgtcagca ggctntcagt ttcccgctca ctttcggcgg agggacc 57
<210> 370
   <211> 57
   <212> DNA
   <213> artificial
<220>
   <223> L3-2NP2 (XPA-23)
<400> 370
   gcaacttact attgtcagca ggctkggagt ttcccgctca ctttcggcgg agggacc 57
<210> 371
   <211> 57
   <212> DNA
   <213> artificial
<220>
   <223> L3-2NP3 (XPA-23)
<400> 371
   gcaacttact attgtcagca ggctscgagt ttcccgctca ctttcggcgg agggacc 57
<210> 372
   <211> 57
   <212> DNA
   <213> artificial
<220>
   <223> L3-3B (XPA-23)
<400> 372
   gcaacttact attgtcagca ggctgacarg ttcccgctca ctttcggcgg agggacc 57
<210> 373
   <211> 57
   <212> DNA
   <213> artificial
<220>
   <223> L3-3P1 (XPA-23)
<400> 373
   gcaacttact attgtcagca ggctgacwmc ttcccgctca ctttcggcgg agggacc 57
<210> 374
   <211> 57
   <212> DNA
   <213> artificial
<220>
   <223> L3-3P2 (XPA-23)
<400> 374
   gcaacttact attgtcagca ggctgaccas ttcccgctca ctttcggcgg agggacc 57
<210> 375
   <211> 57
   <212> DNA
   <213> artificial
<220>
   <223> L3-3A (XPA-23)
<400> 375
   gcaacttact attgtcagca ggctgacgas ttcccgctca ctttcggcgg agggacc 57
<210> 376
   <211> 57
   <212> DNA
   <213> artificial
<220>
   <223> L3-3NP1 (XPA-23)
<220>
   <221> misc_feature
   <222> (28)..(28)
   <223> n is a, c, g, or t
<400> 376
   gcaacttact attgtcagca ggctgacntc ttcccgctca ctttcggcgg agggacc 57
<210> 377
   <211> 57
   <212> DNA
   <213> artificial
<220>
   <223> L3-3NP2 (XPA-23)
<400> 377
   gcaacttact attgtcagca ggctgackgg ttcccgctca ctttcggcgg agggacc 57
<210> 378
   <211> 57
   <212> DNA
   <213> artificial
<220>
   <223> L3-3NP3 (XPA-23)
<400> 378
   gcaacttact attgtcagca ggctgacscg ttcccgctca ctttcggcgg agggacc 57
<210> 379
   <211> 57
   <212> DNA
   <213> artificial
<220>
   <223> L3-4B (XPA-23)
<400> 379
   gcaacttact attgtcagca ggctgacagt argccgctca ctttcggcgg agggacc 57
<210> 380
   <211> 57
   <212> DNA
   <213> artificial
<220>
   <223> L3-4P1 (XPA-23)
<400> 380
   gcaacttact attgtcagca ggctgacagt wmcccgctca ctttcggcgg agggacc 57
<210> 381
   <211> 57
   <212> DNA
   <213> artificial
<220>
   <223> L3-4P2 (XPA-23)
<400> 381
   gcaacttact attgtcagca ggctgacagt casccgctca ctttcggcgg agggacc 57
<210> 382
   <211> 57
   <212> DNA
   <213> artificial
<220>
   <223> L3-4A (XPA-23)
<400> 382
   gcaacttact attgtcagca ggctgacagt gasccgctca ctttcggcgg agggacc 57
<210> 383
   <211> 57
   <212> DNA
   <213> artificial
<220>
   <223> L3-4NP1 (XPA-23)
<220>
   <221> misc_feature
   <222> (31)..(31)
   <223> n is a, c, g, or t
<400> 383
   gcaacttact attgtcagca ggctgacagt ntcccgctca ctttcggcgg agggacc 57
<210> 384
   <211> 57
   <212> DNA
   <213> artificial
<220>
   <223> L3-4NP2 (XPA-23)
<400> 384
   gcaacttact attgtcagca ggctgacagt kggccgctca ctttcggcgg agggacc 57
<210> 385
   <211> 57
   <212> DNA
   <213> artificial
<220>
   <223> L3-4NP3 (XPA-23)
<400> 385
   gcaacttact attgtcagca ggctgacagt scgccgctca ctttcggcgg agggacc 57
<210> 386
   <211> 57
   <212> DNA
   <213> artificial
<220>
   <223> L3-5B (XPA-23)
<400> 386
   gcaacttact attgtcagca ggctgacagt ttcargctca ctttcggcgg agggacc 57
<210> 387
   <211> 57
   <212> DNA
   <213> artificial
<220>
   <223> L3-5P1 (XPA-23)
<400> 387
   gcaacttact attgtcagca ggctgacagt ttcwmcctca ctttcggcgg agggacc 57
<210> 388
   <211> 57
   <212> DNA
   <213> artificial
<220>
   <223> L3-5P2 (XPA-23)
<400> 388
   gcaacttact attgtcagca ggctgacagt ttccasctca ctttcggcgg agggacc 57
<210> 389
   <211> 57
   <212> DNA
   <213> artificial
<220>
   <223> L3-5A (XPA-23)
<400> 389
   gcaacttact attgtcagca ggctgacagt ttcgasctca ctttcggcgg agggacc 57
<210> 390
   <211> 57
   <212> DNA
   <213> artificial
<220>
   <223> L3-5NP1 (XPA-23)
<220>
   <221> misc_feature
   <222> (34)..(34)
   <223> n is a, c, g, or t
<400> 390
   gcaacttact attgtcagca ggctgacagt ttcntcctca ctttcggcgg agggacc 57
<210> 391
   <211> 57
   <212> DNA
   <213> artificial
<220>
   <223> L3-5NP2 (XPA-23)
<400> 391
   gcaacttact attgtcagca ggctgacagt ttckggctca ctttcggcgg agggacc 57
<210> 392
   <211> 57
   <212> DNA
   <213> artificial
<220>
   <223> L3-5NP3 (XPA-23)
<400> 392
   gcaacttact attgtcagca ggctgacagt ttcscgctca ctttcggcgg agggacc 57
<210> 393
   <211> 57
   <212> DNA
   <213> artificial
<220>
   <223> L3-6B (XPA-23)
<400> 393
   gcaacttact attgtcagca ggctgacagt ttcccgarga ctttcggcgg agggacc 57
<210> 394
   <211> 57
   <212> DNA
   <213> artificial
<220>
   <223> L3-6P1 (XPA-23)
<400> 394
   gcaacttact attgtcagca ggctgacagt ttcccgwmca ctttcggcgg agggacc 57
<210> 395
   <211> 57
   <212> DNA
   <213> artificial
<220>
   <223> L3-6P2 (XPA-23)
<400> 395
   gcaacttact attgtcagca ggctgacagt ttcccgcasa ctttcggcgg agggacc 57
<210> 396
   <211> 57
   <212> DNA
   <213> artificial
<220>
   <223> L3-6A (XPA-23)
<400> 396
   gcaacttact attgtcagca ggctgacagt ttcccggasa ctttcggcgg agggacc 57
<210> 397
   <211> 57
   <212> DNA
   <213> artificial
<220>
   <223> L3-6NP1 (XPA-23)
<220>
   <221> misc_feature
   <222> (37)..(37)
   <223> n is a, c, g, or t
<400> 397
   gcaacttact attgtcagca ggctgacagt ttcccgntca ctttcggcgg agggacc 57
<210> 398
   <211> 57
   <212> DNA
   <213> artificial
<220>
   <223> L3-6NP2 (XPA-23)
<400> 398
   gcaacttact attgtcagca ggctgacagt ttcccgkgga ctttcggcgg agggacc 57
<210> 399
   <211> 57
   <212> DNA
   <213> artificial
<220>
   <223> L3-6NP3 (XPA-23)
<400> 399
   gcaacttact attgtcagca ggctgacagt ttcccgscga ctttcggcgg agggacc 57
<210> 400
   <211> 20
   <212> DNA
   <213> artificial
<220>
   <223> H1R (XPA-23)
<400> 400
   gaatccggaa gcagcgcaag 20
<210> 401
   <211> 54
   <212> DNA
   <213> artificial
<220>
   <223> H1-1B (XPA-23)
<400> 401
   cttgcgctgc ttccggattc argttctcta agtactttat gttttgggtt cgcc 54
<210> 402
   <211> 54
   <212> DNA
   <213> artificial
<220>
   <223> H1-1P1 (XPA-23)
<400> 402
   cttgcgctgc ttccggattc wmcttctcta agtactttat gttttgggtt cgcc 54
<210> 403
   <211> 54
   <212> DNA
   <213> artificial
<220>
   <223> H1-1P2 (XPA-23)
<400> 403
   cttgcgctgc ttccggattc casttctcta agtactttat gttttgggtt cgcc 54
<210> 404
   <211> 54
   <212> DNA
   <213> artificial
<220>
   <223> H1-1A (XPA-23)
<400> 404
   cttgcgctgc ttccggattc gasttctcta agtactttat gttttgggtt cgcc 54
<210> 405
   <211> 54
   <212> DNA
   <213> artificial
<220>
   <223> H1-1NP1 (XPA-23)
<220>
   <221> misc_feature
   <222> (21)..(21)
   <223> n is a, c, g, or t
<400> 405
   cttgcgctgc ttccggattc ntcttctcta agtactttat gttttgggtt cgcc 54
<210> 406
   <211> 54
   <212> DNA
   <213> artificial
<220>
   <223> H1-1NP3 (XPA-23)
<400> 406
   cttgcgctgc ttccggattc scgttctcta agtactttat gttttgggtt cgcc 54
<210> 407
   <211> 54
   <212> DNA
   <213> artificial
<220>
   <223> H1-2B (XPA-23)
<400> 407
   cttgcgctgc ttccggattc actttcarga agtactttat gttttgggtt cgcc 54
<210> 408
   <211> 54
   <212> DNA
   <213> artificial
<220>
   <223> H1-2P1 (XPA-23)
<400> 408
   cttgcgctgc ttccggattc actttcwmca agtactttat gttttgggtt cgcc 54
<210> 409
   <211> 54
   <212> DNA
   <213> artificial
<220>
   <223> H1-2P2 (XPA-23)
<400> 409
   cttgcgctgc ttccggattc actttccasa agtactttat gttttgggtt cgcc 54
<210> 410
   <211> 54
   <212> DNA
   <213> artificial
<220>
   <223> H1-2A (XPA-23)
<400> 410
   cttgcgctgc ttccggattc actttcgasa agtactttat gttttgggtt cgcc 54
<210> 411
   <211> 54
   <212> DNA
   <213> artificial
<220>
   <223> H1-2NP1 (XPA-23)
<220>
   <221> misc_feature
   <222> (27)..(27)
   <223> n is a, c, g, or t
<400> 411
   cttgcgctgc ttccggattc actttcntca agtactttat gttttgggtt cgcc 54
<210> 412
   <211> 54
   <212> DNA
   <213> artificial
<220>
   <223> H1-2NP2 (XPA-23)
<400> 412
   cttgcgctgc ttccggattc actttckgga agtactttat gttttgggtt cgcc 54
<210> 413
   <211> 54
   <212> DNA
   <213> artificial
<220>
   <223> H1-2NP3 (XPA-23)
<400> 413
   cttgcgctgc ttccggattc actttcscga agtactttat gttttgggtt cgcc 54
<210> 414
   <211> 54
   <212> DNA
   <213> artificial
<220>
   <223> H1-3B (XPA-23)
<400> 414
   cttgcgctgc ttccggattc actttctcta rgtactttat gttttgggtt cgcc 54
<210> 415
   <211> 54
   <212> DNA
   <213> artificial
<220>
   <223> H1-3P1 (XPA-23)
<400> 415
   cttgcgctgc ttccggattc actttctctw mctactttat gttttgggtt cgcc 54
<210> 416
   <211> 54
   <212> DNA
   <213> artificial
<220>
   <223> H1-3P2 (XPA-23)
<400> 416
   cttgcgctgc ttccggattc actttctctc astactttat gttttgggtt cgcc 54
<210> 417
   <211> 54
   <212> DNA
   <213> artificial
<220>
   <223> H1-3A (XPA-23)
<400> 417
   cttgcgctgc ttccggattc actttctctg astactttat gttttgggtt cgcc 54
<210> 418
   <211> 54
   <212> DNA
   <213> artificial
<220>
   <223> H1-3NP1 (XPA-23)
<220>
   <221> misc_feature
   <222> (30)..(30)
   <223> n is a, c, g, or t
<400> 418
   cttgcgctgc ttccggattc actttctctn tctactttat gttttgggtt cgcc 54
<210> 419
   <211> 54
   <212> DNA
   <213> artificial
<220>
   <223> H1-3NP2 (XPA-23)
<400> 419
   cttgcgctgc ttccggattc actttctctk ggtactttat gttttgggtt cgcc 54
<210> 420
   <211> 54
   <212> DNA
   <213> artificial
<220>
   <223> H1-3NP3 (XPA-23)
<400> 420
   cttgcgctgc ttccggattc actttctcts cgtactttat gttttgggtt cgcc 54
<210> 421
   <211> 22
   <212> DNA
   <213> artificial
<220>
   <223> H12R (XPA-23)
<400> 421
   cttagagaaa gtgaatccgg aa 22
<210> 422
   <211> 53
   <212> DNA
   <213> artificial
<220>
   <223> H12-1B (XPA-23)
<400> 422
   ttccggattc actttctcta agargtttat gttttgggtt cgccaagctc ctg 53
<210> 423
   <211> 53
   <212> DNA
   <213> artificial
<220>
   <223> H12-1P1 (XPA-23)
<400> 423
   ttccggattc actttctcta agwmctttat gttttgggtt cgccaagctc ctg 53
<210> 424
   <211> 53
   <212> DNA
   <213> artificial
<220>
   <223> H12-1P2 (XPA-23)
<400> 424
   ttccggattc actttctcta agcastttat gttttgggtt cgccaagctc ctg 53
<210> 425
   <211> 53
   <212> DNA
   <213> artificial
<220>
   <223> H12-1A (XPA-23)
<400> 425
   ttccggattc actttctcta aggastttat gttttgggtt cgccaagctc ctg 53
<210> 426
   <211> 53
   <212> DNA
   <213> artificial
<220>
   <223> H12-1NP1 (XPA-23)_
<220>
   <221> misc_feature
   <222> (23)..(23)
   <223> n is a, c, g, or t
<400> 426
   ttccggattc actttctcta agntctttat gttttgggtt cgccaagctc ctg 53
<210> 427
   <211> 53
   <212> DNA
   <213> artificial
<220>
   <223> H12-1NP2 (XPA-23)
<400> 427
   ttccggattc actttctcta agkggtttat gttttgggtt cgccaagctc ctg 53
<210> 428
   <211> 53
   <212> DNA
   <213> artificial
<220>
   <223> H12-1NP3 (XPA-23)
<400> 428
   ttccggattc actttctcta agscgtttat gttttgggtt cgccaagctc ctg 53
<210> 429
   <211> 53
   <212> DNA
   <213> artificial
<220>
   <223> H12-2B (XPA-23)
<400> 429
   ttccggattc actttctcta agtacargat gttttgggtt cgccaagctc ctg 53
<210> 430
   <211> 53
   <212> DNA
   <213> artificial
<220>
   <223> H12-2P1 (XPA-23)
<400> 430
   ttccggattc actttctcta agtacwmcat gttttgggtt cgccaagctc ctg 53
<210> 431
   <211> 53
   <212> DNA
   <213> artificial
<220>
   <223> H12-2P2 (XPA- 23)
<400> 431
   ttccggattc actttctcta agtaccasat gttttgggtt cgccaagctc ctg 53
<210> 432
   <211> 53
   <212> DNA
   <213> artificial
<220>
   <223> H12-2A (XPA-23)
<400> 432
   ttccggattc actttctcta agtacgasat gttttgggtt cgccaagctc ctg 53
<210> 433
   <211> 53
   <212> DNA
   <213> artificial
<220>
   <223> H12-2NP1 (XPA-23)
<220>
   <221> misc_feature
   <222> (26)..(26)
   <223> n is a, c, g, or t
<400> 433
   ttccggattc actttctcta agtacntcat gttttgggtt cgccaagctc ctg 53
<210> 434
   <211> 53
   <212> DNA
   <213> artificial
<220>
   <223> H12-2NP2 (XPA-23)
<400> 434
   ttccggattc actttctcta agtackggat gttttgggtt cgccaagctc ctg 53
<210> 435
   <211> 53
   <212> DNA
   <213> artificial
<220>
   <223> H12-2NP3 (XPA-23)
<400> 435
   ttccggattc actttctcta agtacscgat gttttgggtt cgccaagctc ctg 53
<210> 436
   <211> 53
   <212> DNA
   <213> artificial
<220>
   <223> H12-3B (XPA-23)
<400> 436
   ttccggattc actttctcta agtactttat gargtgggtt cgccaagctc ctg 53
<210> 437
   <211> 53
   <212> DNA
   <213> artificial
<220>
   <223> H12-3P1 (XPA-23)
<400> 437
   ttccggattc actttctcta agtactttat gwmctgggtt cgccaagctc ctg 53
<210> 438
   <211> 53
   <212> DNA
   <213> artificial
<220>
   <223> H12-3P2 (XPA-23)
<400> 438
   ttccggattc actttctcta agtactttat gcastgggtt cgccaagctc ctg 53
<210> 439
   <211> 53
   <212> DNA
   <213> artificial
<220>
   <223> H12-3A (XPA-23)
<400> 439
   ttccggattc actttctcta agtactttat ggastgggtt cgccaagctc ctg 53
<210> 440
   <211> 53
   <212> DNA
   <213> artificial
<220>
   <223> H12-3NP1 (XPA-23)
<220>
   <221> misc_feature
   <222> (32)..(32)
   <223> n is a, c, g, or t
<400> 440
   ttccggattc actttctcta agtactttat gntctgggtt cgccaagctc ctg 53
<210> 441
   <211> 53
   <212> DNA
   <213> aritificial
<400> 441
   ttccggattc actttctcta agtactttat gkggtgggtt cgccaagctc ctg 53
<210> 442
   <211> 53
   <212> DNA
   <213> artificial
<220>
   <223> H12-3NP3 (XPA-23)
<400> 442
   ttccggattc actttctcta agtactttat gscgtgggtt cgccaagctc ctg 53
<210> 443
   <211> 22
   <212> DNA
   <213> artificial
<220>
   <223> H2R (XPA-23)
<400> 443
   agaaacccac tccaaacctt ta 22
<210> 444
   <211> 57
   <212> DNA
   <213> artificial
<220>
   <223> H2-1B (XPA-23)
<400> 444
   taaaggtttg gagtgggttt ctargatctc tccttctggt ggcatgactc gttatgc 57
<210> 445
   <211> 57
   <212> DNA
   <213> artificial
<220>
   <223> H2-1P1 (XPA-23)
<400> 445
   taaaggtttg gagtgggttt ctwmcatctc tccttctggt ggcatgactc gttatgc 57
<210> 446
   <211> 57
   <212> DNA
   <213> artificial
<220>
   <223> H2-1P2 (XPA-23)
<400> 446
   taaaggtttg gagtgggttt ctcasatctc tccttctggt ggcatgactc gttatgc 57
<210> 447
   <211> 57
   <212> DNA
   <213> artificial
<220>
   <223> H2-1A (XPA-23)
<400> 447
   taaaggtttg gagtgggttt ctgasatctc tccttctggt ggcatgactc gttatgc 57
<210> 448
   <211> 57
   <212> DNA
   <213> artificial
<220>
   <223> H2-1NP1 (XPA-23)
<220>
   <221> misc_feature
   <222> (23)..(23)
   <223> n is a, c, g, or t
<400> 448
   taaaggtttg gagtgggttt ctntcatctc tccttctggt ggcatgactc gttatgc 57
<210> 449
   <211> 57
   <212> DNA
   <213> artificial
<220>
   <223> H2-1NP2 (XPA-23)
<400> 449
   taaaggtttg gagtgggttt ctkggatctc tccttctggt ggcatgactc gttatgc 57
<210> 450
   <211> 57
   <212> DNA
   <213> artificial
<220>
   <223> H2-1NP3 (XPA-23)
<400> 450
   taaaggtttg gagtgggttt ctscgatctc tccttctggt ggcatgactc gttatgc 57
<210> 451
   <211> 57
   <212> DNA
   <213> artificial
<220>
   <223> H2-2B (XPA-23)
<400> 451
   taaaggtttg gagtgggttt ctgttargtc tccttctggt ggcatgactc gttatgc 57
<210> 452
   <211> 57
   <212> DNA
   <213> artificial
<220>
   <223> H2-2P1 (XPA-23)
<400> 452
   taaaggtttg gagtgggttt ctgttwmctc tccttctggt ggcatgactc gttatgc 57
<210> 453
   <211> 57
   <212> DNA
   <213> artificial
<220>
   <223> H2-2P2 (XPA-23)
<400> 453
   taaaggtttg gagtgggttt ctgttcastc tccttctggt ggcatgactc gttatgc 57
<210> 454
   <211> 57
   <212> DNA
   <213> artificial
<220>
   <223> H2-2A (XPA-23)
<400> 454
   taaaggtttg gagtgggttt ctgttgastc tccttctggt ggcatgactc gttatgc 57
<210> 455
   <211> 57
   <212> DNA
   <213> artificial
<220>
   <223> H2-2NP1 (XPA-23)
<220>
   <221> misc_feature
   <222> (26)..(26)
   <223> n is a, c, g, or t
<400> 455
   taaaggtttg gagtgggttt ctgttntctc tccttctggt ggcatgactc gttatgc 57
<210> 456
   <211> 57
   <212> DNA
   <213> artificial
<220>
   <223> H2-2NP2 (XPA-23)
<400> 456
   taaaggtttg gagtgggttt ctgttkggtc tccttctggt ggcatgactc gttatgc 57
<210> 457
   <211> 57
   <212> DNA
   <213> artificial
<220>
   <223> H2-2NP3 (XPA-23)
<400> 457
   taaaggtttg gagtgggttt ctgttscgtc tccttctggt ggcatgactc gttatgc 57
<210> 458
   <211> 57
   <212> DNA
   <213> artificial
<220>
   <223> H2-3B (XPA-23)
<400> 458
   taaaggtttg gagtgggttt ctgttatcar gccttctggt ggcatgactc gttatgc 57
<210> 459
   <211> 57
   <212> DNA
   <213> artificial
<220>
   <223> H2-3P1 (XPA-23)
<400> 459
   taaaggtttg gagtgggttt ctgttatcwm cccttctggt ggcatgactc gttatgc 57
<210> 460
   <211> 57
   <212> DNA
   <213> artificial
<220>
   <223> H2-3P2 (XPA-23)
<400> 460
   taaaggtttg gagtgggttt ctgttatcca sccttctggt ggcatgactc gttatgc 57
<210> 461
   <211> 57
   <212> DNA
   <213> artificial
<220>
   <223> H2-3A (XPA-23)
<400> 461
   taaaggtttg gagtgggttt ctgttatcga sccttctggt ggcatgactc gttatgc 57
<210> 462
   <211> 57
   <212> DNA
   <213> artificial
<220>
   <223> H2-3NP1 (XPA-23)
<220>
   <221> misc_feature
   <222> (29)..(29)
   <223> n is a, c, g, or t
<400> 462
   taaaggtttg gagtgggttt ctgttatcnt cccttctggt ggcatgactc gttatgc 57
<210> 463
   <211> 57
   <212> DNA
   <213> artificial
<220>
   <223> H2-3NP2 (XPA-23)
<400> 463
   taaaggtttg gagtgggttt ctgttatckg gccttctggt ggcatgactc gttatgc 57
<210> 464
   <211> 57
   <212> DNA
   <213> artificial
<220>
   <223> H2-3NP3 (XPA-23)
<400> 464
   taaaggtttg gagtgggttt ctgttatcsc gccttctggt ggcatgactc gttatgc 57
<210> 465
   <211> 57
   <212> DNA
   <213> artificial
<220>
   <223> H2-4B (XPA-23)
<400> 465
   taaaggtttg gagtgggttt ctgttatctc targtctggt ggcatgactc gttatgc 57
<210> 466
   <211> 57
   <212> DNA
   <213> artificial
<220>
   <223> H2-4P1 (XPA-23)
<400> 466
   taaaggtttg gagtgggttt ctgttatctc twmctctggt ggcatgactc gttatgc 57
<210> 467
   <211> 57
   <212> DNA
   <213> artificial
<220>
   <223> H2-4P2 (XPA-23)
<400> 467
   taaaggtttg gagtgggttt ctgttatctc tcastctggt ggcatgactc gttatgc 57
<210> 468
   <211> 57
   <212> DNA
   <213> artificial
<220>
   <223> H2-4A (XPA-23)
<400> 468
   taaaggtttg gagtgggttt ctgttatctc tgastctggt ggcatgactc gttatgc 57
<210> 469
   <211> 57
   <212> DNA
   <213> artificial
<220>
   <223> H2-4NP1 (XPA-23)
<220>
   <221> misc_feature
   <222> (32)..(32)
   <223> n is a, c, g, or t
<400> 469
   taaaggtttg gagtgggttt ctgttatctc tntctctggt ggcatgactc gttatgc 57
<210> 470
   <211> 57
   <212> DNA
   <213> artificial
<220>
   <223> H2-4NP2 (XPA-23)
<400> 470
   taaaggtttg gagtgggttt ctgttatctc tkggtctggt ggcatgactc gttatgc 57
<210> 471
   <211> 57
   <212> DNA
   <213> artificial
<220>
   <223> H2-4NP3 (XPA-23)
<400> 471
   taaaggtttg gagtgggttt ctgttatctc tscgtctggt ggcatgactc gttatgc 57
<210> 472
   <211> 57
   <212> DNA
   <213> artificial
<220>
   <223> H2-5B (XPA-23)
<400> 472
   taaaggtttg gagtgggttt ctgttatctc tcctargggt ggcatgactc gttatgc 57
<210> 473
   <211> 57
   <212> DNA
   <213> artificial
<220>
   <223> H2-5P1 (XPA-23)
<400> 473
   taaaggtttg gagtgggttt ctgttatctc tcctwmcggt ggcatgactc gttatgc 57
<210> 474
   <211> 57
   <212> DNA
   <213> artificial
<220>
   <223> H2-5P2 (XPA-23)
<400> 474
   taaaggtttg gagtgggttt ctgttatctc tcctcasggt ggcatgactc gttatgc 57
<210> 475
   <211> 57
   <212> DNA
   <213> artificial
<220>
   <223> H2-5A (XPA-23)
<400> 475
   taaaggtttg gagtgggttt ctgttatctc tcctgasggt ggcatgactc gttatgc 57
<210> 476
   <211> 57
   <212> DNA
   <213> artificial
<220>
   <223> H2-5NP1 (XPA-23)
<220>
   <221> misc_feature
   <222> (35)..(35)
   <223> n is a, c, g, or t
<400> 476
   taaaggtttg gagtgggttt ctgttatctc tcctntcggt ggcatgactc gttatgc 57
<210> 477
   <211> 57
   <212> DNA
   <213> artificial
<220>
   <223> H2-5NP2 (XPA-23)
<400> 477
   taaaggtttg gagtgggttt ctgttatctc tcctkggggt ggcatgactc gttatgc 57
<210> 478
   <211> 57
   <212> DNA
   <213> artificial
<220>
   <223> H2-5NP3 (XPA-23)
<400> 478
   taaaggtttg gagtgggttt ctgttatctc tcctscgggt ggcatgactc gttatgc 57
<210> 479
   <211> 22
   <212> DNA
   <213> artificial
<220>
   <223> H22-R (XPA-23)
<400> 479
   agaaggagag ataacagaaa cc 22
<210> 480
   <211> 59
   <212> DNA
   <213> artificial
<220>
   <223> H22-1P1 (XPA-23)
<400> 480
   ggtttctgtt atctctcctt ctwmcggcat gactcgttat gctgactccg ttaaaggtc 59
<210> 481
   <211> 59
   <212> DNA
   <213> artificial
<220>
   <223> H22-1P2 (XPA-23)
<400> 481
   ggtttctgtt atctctcctt ctcasggcat gactcgttat gctgactccg ttaaaggtc 59
<210> 482
   <211> 59
   <212> DNA
   <213> artificial
<220>
   <223> H22-1A (XPA-23)
<400> 482
   ggtttctgtt atctctcctt ctgasggcat gactcgttat gctgactccg ttaaaggtc 59
<210> 483
   <211> 59
   <212> DNA
   <213> artificial
<220>
   <223> H22-1NP1 (XPA-23)
<220>
   <221> misc_feature
   <222> (23)..(23)
   <223> n is a, c, g, or t
<400> 483
   ggtttctgtt atctctcctt ctntcggcat gactcgttat gctgactccg ttaaaggtc 59
<210> 484
   <211> 59
   <212> DNA
   <213> artificial
<220>
   <223> H22-1NP2 (XPA-23)
<400> 484
   ggtttctgtt atctctcctt ctkggggcat gactcgttat gctgactccg ttaaaggtc 59
<210> 485
   <211> 59
   <212> DNA
   <213> artificial
<220>
   <223> H22-1NP3 (XPA-23)
<400> 485
   ggtttctgtt atctctcctt ctscgggcat gactcgttat gctgactccg ttaaaggtc 59
<210> 486
   <211> 59
   <212> DNA
   <213> artificial
<220>
   <223> H22-2B (XPA-23)
<400> 486
   ggtttctgtt atctctcctt ctggtargat gactcgttat gctgactccg ttaaaggtc 59
<210> 487
   <211> 59
   <212> DNA
   <213> artificial
<220>
   <223> H22-2P1 (XPA-23)
<400> 487
   ggtttctgtt atctctcctt ctggtwmcat gactcgttat gctgactccg ttaaaggtc 59
<210> 488
   <211> 59
   <212> DNA
   <213> artificial
<220>
   <223> H22-2P2 (XPA-23)
<400> 488
   ggtttctgtt atctctcctt ctggtcasat gactcgttat gctgactccg ttaaaggtc 59
<210> 489
   <211> 59
   <212> DNA
   <213> artificial
<220>
   <223> H22-2A (XPA-23)
<400> 489
   ggtttctgtt atctctcctt ctggtgasat gactcgttat gctgactccg ttaaaggtc 59
<210> 490
   <211> 59
   <212> DNA
   <213> artificial
<220>
   <223> H22-2NP1 (XPA-23)
<220>
   <221> misc_feature
   <222> (26)..(26)
   <223> n is a, c, g, or t
<400> 490
   ggtttctgtt atctctcctt ctggtntcat gactcgttat gctgactccg ttaaaggtc 59
<210> 491
   <211> 59
   <212> DNA
   <213> artificial
<220>
   <223> H22-2NP2 (XPA-23)
<400> 491
   ggtttctgtt atctctcctt ctggtkggat gactcgttat gctgactccg ttaaaggtc 59
<210> 492
   <211> 59
   <212> DNA
   <213> artificial
<220>
   <223> H22-2NP3 (XPA-23)
<400> 492
   ggtttctgtt atctctcctt ctggtscgat gactcgttat gctgactccg ttaaaggtc 59
<210> 493
   <211> 59
   <212> DNA
   <213> artificial
<220>
   <223> H22-3B (XPA-23)
<400> 493
   ggtttctgtt atctctcctt ctggtggcar gactcgttat gctgactccg ttaaaggtc 59
<210> 494
   <211> 59
   <212> DNA
   <213> artificial
<220>
   <223> H22-3P1 (XPA-23)
<400> 494
   ggtttctgtt atctctcctt ctggtggcwm cactcgttat gctgactccg ttaaaggtc 59
<210> 495
   <211> 59
   <212> DNA
   <213> artificial
<220>
   <223> H22-3P2 (XPA-23)
<400> 495
   ggtttctgtt atctctcctt ctggtggcca sactcgttat gctgactccg ttaaaggtc 59
<210> 496
   <211> 59
   <212> DNA
   <213> artificial
<220>
   <223> H22-3A (XPA-23)
<400> 496
   ggtttctgtt atctctcctt ctggtggcga sactcgttat gctgactccg ttaaaggtc 59
<210> 497
   <211> 59
   <212> DNA
   <213> artificial
<220>
   <223> H22-3NP1 (XPA-23)
<220>
   <221> misc_feature
   <222> (29)..(29)
   <223> n is a, c, g, or t
<400> 497
   ggtttctgtt atctctcctt ctggtggcnt cactcgttat gctgactccg ttaaaggtc 59
<210> 498
   <211> 59
   <212> DNA
   <213> artificial
<220>
   <223> H22-3NP2 (XPA-23)
<400> 498
   ggtttctgtt atctctcctt ctggtggckg gactcgttat gctgactccg ttaaaggtc 59
<210> 499
   <211> 59
   <212> DNA
   <213> artificial
<220>
   <223> H22-3NP3 (XPA-23)
<400> 499
   ggtttctgtt atctctcctt ctggtggcsc gactcgttat gctgactccg ttaaaggtc 59
<210> 500
   <211> 59
   <212> DNA
   <213> artificial
<220>
   <223> H22-4B (XPA-23)
<400> 500
   ggtttctgtt atctctcctt ctggtggcat gargcgttat gctgactccg ttaaaggtc 59
<210> 501
   <211> 59
   <212> DNA
   <213> artificial
<220>
   <223> H22-4P1 (XPA-23)
<400> 501
   ggtttctgtt atctctcctt ctggtggcat gwmccgttat gctgactccg ttaaaggtc 59
<210> 502
   <211> 59
   <212> DNA
   <213> artificial
<220>
   <223> H22-4P2 (XPA-23)
<400> 502
   ggtttctgtt atctctcctt ctggtggcat gcascgttat gctgactccg ttaaaggtc 59
<210> 503
   <211> 59
   <212> DNA
   <213> artificial
<220>
   <223> H22-4A (XPA-23)
<400> 503
   ggtttctgtt atctctcctt ctggtggcat ggascgttat gctgactccg ttaaaggtc 59
<210> 504
   <211> 59
   <212> DNA
   <213> artificial
<220>
   <223> H22-4NP1 (XPA-23)
<220>
   <221> misc_feature
   <222> (32)..(32)
   <223> n is a, c, g, or t
<400> 504
   ggtttctgtt atctctcctt ctggtggcat gntccgttat gctgactccg ttaaaggtc 59
<210> 505
   <211> 59
   <212> DNA
   <213> artificial
<220>
   <223> H22-4NP2 (XPA-23)
<400> 505
   ggtttctgtt atctctcctt ctggtggcat gkggcgttat gctgactccg ttaaaggtc 59
<210> 506
   <211> 59
   <212> DNA
   <213> artificial
<220>
   <223> H22-4NP3 (XPA-23)
<400> 506
   ggtttctgtt atctctcctt ctggtggcat gscgcgttat gctgactccg ttaaaggtc 59
<210> 507
   <211> 59
   <212> DNA
   <213> artificial
<220>
   <223> H22-5B (XPA-23)
<400> 507
   ggtttctgtt atctctcctt ctggtggcat gactargtat gctgactccg ttaaaggtc 59
<210> 508
   <211> 59
   <212> DNA
   <213> artificial
<220>
   <223> H22-5P1 (XPA-23)
<400> 508
   ggtttctgtt atctctcctt ctggtggcat gactwmctat gctgactccg ttaaaggtc 59
<210> 509
   <211> 59
   <212> DNA
   <213> artificial
<220>
   <223> H22-5P2 (XPA-23)
<400> 509
   ggtttctgtt atctctcctt ctggtggcat gactcastat gctgactccg ttaaaggtc 59
<210> 510
   <211> 59
   <212> DNA
   <213> artificial
<220>
   <223> H22-5A (XPA-23)
<400> 510
   ggtttctgtt atctctcctt ctggtggcat gactgastat gctgactccg ttaaaggtc 59
<210> 511
   <211> 59
   <212> DNA
   <213> artificial
<220>
   <223> H22-5NP1 (XPA-23)
<220>
   <221> misc_feature
   <222> (35)..(35)
   <223> n is a, c, g, or t
<400> 511
   ggtttctgtt atctctcctt ctggtggcat gactntctat gctgactccg ttaaaggtc 59
<210> 512
   <211> 59
   <212> DNA
   <213> artificial
<220>
   <223> H22-5NP2 (XPA-23)
<400> 512
   ggtttctgtt atctctcctt ctggtggcat gactkggtat gctgactccg ttaaaggtc 59
<210> 513
   <211> 59
   <212> DNA
   <213> artificial
<220>
   <223> H22-5NP3 (XPA-23)
<400> 513
   ggtttctgtt atctctcctt ctggtggcat gactscgtat gctgactccg ttaaaggtc 59
<210> 514
   <211> 21
   <212> DNA
   <213> artificial
<220>
   <223> H3-R (XPA-23)
<400> 514
   tctcgcacaa tagtagactg c 21
<210> 515
   <211> 56
   <212> DNA
   <213> artificial
<220>
   <223> H3-1B (XPA-23)
<400> 515
   gcagtctact attgtgcgag aargggctac ggtggtaact ctgactactg gggcca 56
<210> 516
   <211> 56
   <212> DNA
   <213> artificial
<220>
   <223> H3-1P1 (XPA-23)
<400> 516
   gcagtctact attgtgcgag awmcggctac ggtggtaact ctgactactg gggcca 56
<210> 517
   <211> 56
   <212> DNA
   <213> artificial
<220>
   <223> H3-1P2 (XPA-23)
<400> 517
   gcagtctact attgtgcgag acasggctac ggtggtaact ctgactactg gggcca 56
<210> 518
   <211> 56
   <212> DNA
   <213> artificial
<220>
   <223> H3-1A (XPA-23)
<400> 518
   gcagtctact attgtgcgag agasggctac ggtggtaact ctgactactg gggcca 56
<210> 519
   <211> 56
   <212> DNA
   <213> artificial
<220>
   <223> H3-1NP1 (XPA-23)
<220>
   <221> misc_feature
   <222> (22)..(22)
   <223> n is a, c, g, or t
<400> 519
   gcagtctact attgtgcgag antcggctac ggtggtaact ctgactactg gggcca 56
<210> 520
   <211> 56
   <212> DNA
   <213> artificial
<220>
   <223> H3-1NP2 (XPA-23)
<400> 520
   gcagtctact attgtgcgag akggggctac ggtggtaact ctgactactg gggcca 56
<210> 521
   <211> 56
   <212> DNA
   <213> artificial
<220>
   <223> H3-1NP3 (XPA-23)
<400> 521
   gcagtctact attgtgcgag ascgggctac ggtggtaact ctgactactg gggcca 56
<210> 522
   <211> 56
   <212> DNA
   <213> artificial
<220>
   <223> H3-2B (XPA-23)
<400> 522
   gcagtctact attgtgcgag agtcargtac ggtggtaact ctgactactg gggcca 56
<210> 523
   <211> 56
   <212> DNA
   <213> artificial
<220>
   <223> H3-2P1 (XPA-23)
<400> 523
   gcagtctact attgtgcgag agtcwmctac ggtggtaact ctgactactg gggcca 56
<210> 524
   <211> 56
   <212> DNA
   <213> artificial
<220>
   <223> H3-2P2 (XPA-23)
<400> 524
   gcagtctact attgtgcgag agtccastac ggtggtaact ctgactactg gggcca 56
<210> 525
   <211> 56
   <212> DNA
   <213> artificial
<220>
   <223> H3-2A (XPA-23)
<400> 525
   gcagtctact attgtgcgag agtcgastac ggtggtaact ctgactactg gggcca 56
<210> 526
   <211> 56
   <212> DNA
   <213> artificial
<220>
   <223> H3-2NP1 (XPA-23)
<220>
   <221> misc_feature
   <222> (25)..(25)
   <223> n is a, c, g, or t
<400> 526
   gcagtctact attgtgcgag agtcntctac ggtggtaact ctgactactg gggcca 56
<210> 527
   <211> 56
   <212> DNA
   <213> artificial
<220>
   <223> H3-2NP2 (XPA-23)
<400> 527
   gcagtctact attgtgcgag agtckggtac ggtggtaact ctgactactg gggcca 56
<210> 528
   <211> 56
   <212> DNA
   <213> artificial
<220>
   <223> H3-2NP3 (XPA-23)
<400> 528
   gcagtctact attgtgcgag agtcscgtac ggtggtaact ctgactactg gggcca 56
<210> 529
   <211> 56
   <212> DNA
   <213> artificial
<220>
   <223> H3-3B (XPA-23)
<400> 529
   gcagtctact attgtgcgag agtcggcarg ggtggtaact ctgactactg gggcca 56
<210> 530
   <211> 56
   <212> DNA
   <213> artificial
<220>
   <223> H3-3P1 (XPA-23)
<400> 530
   gcagtctact attgtgcgag agtcggcwmc ggtggtaact ctgactactg gggcca 56
<210> 531
   <211> 56
   <212> DNA
   <213> artificial
<220>
   <223> H3-3P2 (XPA-23)
<400> 531
   gcagtctact attgtgcgag agtcggccas ggtggtaact ctgactactg gggcca 56
<210> 532
   <211> 56
   <212> DNA
   <213> artificial
<220>
   <223> H3-3A (XPA-23)
<400> 532
   gcagtctact attgtgcgag agtcggcgas ggtggtaact ctgactactg gggcca 56
<210> 533
   <211> 56
   <212> DNA
   <213> artificial
<220>
   <223> H3-3NP1 (XPA-23)
<220>
   <221> misc_feature
   <222> (28)..(28)
   <223> n is a, c, g, or t
<400> 533
   gcagtctact attgtgcgag agtcggcntc ggtggtaact ctgactactg gggcca 56
<210> 534
   <211> 56
   <212> DNA
   <213> artificial
<220>
   <223> H3-3NP2 (XPA-23)
<400> 534
   gcagtctact attgtgcgag agtcggckgg ggtggtaact ctgactactg gggcca 56
<210> 535
   <211> 56
   <212> DNA
   <213> artificial
<220>
   <223> H3-3NP3 (XPA-23)
<400> 535
   gcagtctact attgtgcgag agtcggcscg ggtggtaact ctgactactg gggcca 56
<210> 536
   <211> 56
   <212> DNA
   <213> artificial
<220>
   <223> H3-4B (XPA-23)
<400> 536
   gcagtctact attgtgcgag agtcggctac argggtaact ctgactactg gggcca 56
<210> 537
   <211> 56
   <212> DNA
   <213> artificial
<220>
   <223> H3-4P1 (XPA-23)
<400> 537
   gcagtctact attgtgcgag agtcggctac wmcggtaact ctgactactg gggcca 56
<210> 538
   <211> 56
   <212> DNA
   <213> artificial
<220>
   <223> H3-4P2 (XPA-23)
<400> 538
   gcagtctact attgtgcgag agtcggctac casggtaact ctgactactg gggcca 56
<210> 539
   <211> 56
   <212> DNA
   <213> artificial
<220>
   <223> H3-4A (XPA-23)
<400> 539
   gcagtctact attgtgcgag agtcggctac gasggtaact ctgactactg gggcca 56
<210> 540
   <211> 56
   <212> DNA
   <213> artificial
<220>
   <223> H3-4NP1 (XPA-23)
<220>
   <221> misc_feature
   <222> (31)..(31)
   <223> n is a, c, g, or t
<400> 540
   gcagtctact attgtgcgag agtcggctac ntcggtaact ctgactactg gggcca 56
<210> 541
   <211> 56
   <212> DNA
   <213> artificial
<220>
   <223> H3-4NP2 (XPA-23)
<400> 541
   gcagtctact attgtgcgag agtcggctac kggggtaact ctgactactg gggcca 56
<210> 542
   <211> 56
   <212> DNA
   <213> artificial
<220>
   <223> H3-4NP3 (XPA-23)
<400> 542
   gcagtctact attgtgcgag agtcggctac scgggtaact ctgactactg gggcca 56
<210> 543
   <211> 56
   <212> DNA
   <213> artificial
<220>
   <223> H3-5B (XPA-23)
<400> 543
   gcagtctact attgtgcgag agtcggctac ggtargaact ctgactactg gggcca 56
<210> 544
   <211> 56
   <212> DNA
   <213> artificial
<220>
   <223> H3-5P1 (XPA-23)
<400> 544
   gcagtctact attgtgcgag agtcggctac ggtwmcaact ctgactactg gggcca 56
<210> 545
   <211> 56
   <212> DNA
   <213> artificial
<220>
   <223> H3-5P2 (XPA-23)
<400> 545
   gcagtctact attgtgcgag agtcggctac ggtcasaact ctgactactg gggcca 56
<210> 546
   <211> 56
   <212> DNA
   <213> artificial
<220>
   <223> H3-5A (XPA-23)
<400> 546
   gcagtctact attgtgcgag agtcggctac ggtgasaact ctgactactg gggcca 56
<210> 547
   <211> 56
   <212> DNA
   <213> artificial
<220>
   <223> H3-5NP1 (XPA-23)
<220>
   <221> misc_feature
   <222> (34)..(34)
   <223> n is a, c, g, or t
<400> 547
   gcagtctact attgtgcgag agtcggctac ggtntcaact ctgactactg gggcca 56
<210> 548
   <211> 56
   <212> DNA
   <213> artificial
<220>
   <223> H3-5NP2 (XPA-23)
<400> 548
   gcagtctact attgtgcgag agtcggctac ggtkggaact ctgactactg gggcca 56
<210> 549
   <211> 56
   <212> DNA
   <213> artificial
<220>
   <223> H3-5NP3 (XPA-23)
<400> 549
   gcagtctact attgtgcgag agtcggctac ggtscgaact ctgactactg gggcca 56
<210> 550
   <211> 19
   <212> DNA
   <213> artificial
<220>
   <223> H32R (XPA-23)
<400> 550
   accaccgtag ccgactctc 19
<210> 551
   <211> 49
   <212> DNA
   <213> artificial
<220>
   <223> H32-1B (XPA-23)
<400> 551
   gagagtcggc tacggtggta rgtctgacta ctggggccag ggaaccctg 49
<210> 552
   <211> 49
   <212> DNA
   <213> artificial
<220>
   <223> H32-1P1 (XPA-23)
<400> 552
   gagagtcggc tacggtggtw mctctgacta ctggggccag ggaaccctg 49
<210> 553
   <211> 49
   <212> DNA
   <213> artificial
<220>
   <223> H32-1P2 (XPA-23)
<400> 553
   gagagtcggc tacggtggtc astctgacta ctggggccag ggaaccctg 49
<210> 554
   <211> 49
   <212> DNA
   <213> artificial
<220>
   <223> H32-1A (XPA-23)
<400> 554
   gagagtcggc tacggtggtg astctgacta ctggggccag ggaaccctg 49
<210> 555
   <211> 49
   <212> DNA
   <213> artificial
<220>
   <223> H32-1NP1 (XPA-23)
<220>
   <221> misc_feature
   <222> (20)..(20)
   <223> n is a, c, g, or t
<400> 555
   gagagtcggc tacggtggtn tctctgacta ctggggccag ggaaccctg 49
<210> 556
   <211> 49
   <212> DNA
   <213> artificial
<220>
   <223> H32-1NP2 (XPA-23)
<400> 556
   gagagtcggc tacggtggtk ggtctgacta ctggggccag ggaaccctg 49
<210> 557
   <211> 49
   <212> DNA
   <213> artificial
<220>
   <223> H32-1NP3 (XPA-23)
<400> 557
   gagagtcggc tacggtggts cgtctgacta ctggggccag ggaaccctg 49
<210> 558
   <211> 49
   <212> DNA
   <213> artificial
<220>
   <223> H32-2B (XPA-23)
<400> 558
   gagagtcggc tacggtggta acarggacta ctggggccag ggaaccctg 49
<210> 559
   <211> 49
   <212> DNA
   <213> artificial
<220>
   <223> H32-2P1 (XPA-23)
<400> 559
   gagagtcggc tacggtggta acwmcgacta ctggggccag ggaaccctg 49
<210> 560
   <211> 49
   <212> DNA
   <213> artificial
<220>
   <223> H32-2P2 (XPA-23)
<400> 560
   gagagtcggc tacggtggta accasgacta ctggggccag ggaaccctg 49
<210> 561
   <211> 49
   <212> DNA
   <213> artificial
<220>
   <223> H32-2A (XPA-23)
<400> 561
   gagagtcggc tacggtggta acgasgacta ctggggccag ggaaccctg 49
<210> 562
   <211> 49
   <212> DNA
   <213> artificial
<220>
   <223> H32-2NP1 (XPA-23)
<220>
   <221> misc_feature
   <222> (23)..(23)
   <223> n is a, c, g, or t
<400> 562
   gagagtcggc tacggtggta acntcgacta ctggggccag ggaaccctg 49
<210> 563
   <211> 49
   <212> DNA
   <213> artificial
<220>
   <223> H32-2NP2 (XPA-23)
<400> 563
   gagagtcggc tacggtggta ackgggacta ctggggccag ggaaccctg 49
<210> 564
   <211> 49
   <212> DNA
   <213> artificial
<220>
   <223> H32-2NP3 (XPA-23)
<400> 564
   gagagtcggc tacggtggta acscggacta ctggggccag ggaaccctg 49
<210> 565
   <211> 49
   <212> DNA
   <213> artificial
<220>
   <223> H32-3B (XPA-23)
<400> 565
   gagagtcggc tacggtggta actctargta ctggggccag ggaaccctg 49
<210> 566
   <211> 49
   <212> DNA
   <213> artificial
<220>
   <223> H32-3P1 (XPA-23)
<400> 566
   gagagtcggc tacggtggta actctwmcta ctggggccag ggaaccctg 49
<210> 567
   <211> 49
   <212> DNA
   <213> artificial
<220>
   <223> H32-3P2 (XPA-23)
<400> 567
   gagagtcggc tacggtggta actctcasta ctggggccag ggaaccctg 49
<210> 568
   <211> 49
   <212> DNA
   <213> artificial
<220>
   <223> H32-3A (XPA-23)
<400> 568
   gagagtcggc tacggtggta actctgasta ctggggccag ggaaccctg 49
<210> 569
   <211> 49
   <212> DNA
   <213> artificial
<220>
   <223> H32-3NP1 (XPA-23)
<220>
   <221> misc_feature
   <222> (26)..(26)
   <223> n is a, c, g, or t
<400> 569
   gagagtcggc tacggtggta actctntcta ctggggccag ggaaccctg 49
<210> 570
   <211> 49
   <212> DNA
   <213> artificial
<220>
   <223> H32-3NP2 (XPA-23)
<400> 570
   gagagtcggc tacggtggta actctkggta ctggggccag ggaaccctg 49
<210> 571
   <211> 49
   <212> DNA
   <213> artificial
<220>
   <223> H32-3NP3 (XPA-23)
<400> 571
   gagagtcggc tacggtggta actctscgta ctggggccag ggaaccctg 49
<210> 572
   <211> 49
   <212> DNA
   <213> artificial
<220>
   <223> H32-4B (XPA-23)
<400> 572
   gagagtcggc tacggtggta actctgacar gtggggccag ggaaccctg 49
<210> 573
   <211> 49
   <212> DNA
   <213> artificial
<220>
   <223> H32-4P1 (XPA-23)
<400> 573
   gagagtcggc tacggtggta actctgacwm ctggggccag ggaaccctg 49
<210> 574
   <211> 49
   <212> DNA
   <213> artificial
<220>
   <223> H32-4P2 (XPA-23)
<400> 574
   gagagtcggc tacggtggta actctgacca stggggccag ggaaccctg 49
<210> 575
   <211> 49
   <212> DNA
   <213> artificial
<220>
   <223> H32-4A (XPA-23)
<400> 575
   gagagtcggc tacggtggta actctgacga stggggccag ggaaccctg 49
<210> 576
   <211> 49
   <212> DNA
   <213> artificial
<220>
   <223> H32-4NP1 (XPA-23)
<220>
   <221> misc_feature
   <222> (29)..(29)
   <223> n is a, c, g, or t
<400> 576
   gagagtcggc tacggtggta actctgacnt ctggggccag ggaaccctg 49
<210> 577
   <211> 49
   <212> DNA
   <213> artificial
<220>
   <223> H32-4NP2 (XPA-23)
<400> 577
   gagagtcggc tacggtggta actctgackg gtggggccag ggaaccctg 49
<210> 578
   <211> 49
   <212> DNA
   <213> artificial
<220>
   <223> H32-4NP3 (XPA-23)
<400> 578
   gagagtcggc tacggtggta actctgacsc gtggggccag ggaaccctg 49
<210> 579
   <211> 675
   <212> DNA
   <213> artificial
<220>
   <223> ING-1 (heavy chain)
<400> 579
   cagatccagt tggtgcagtc tggacctgag ctgaagaagc ctggagagac agtcaagatc 60
   tcctgcaagg cttctggata taccttcaca aaatatggaa tgaactgggt gaagcaggct 120
   ccaggaaagg gtttaaagtg gatgggctgg ataaacacct acactgaaga gccaacatat 180
   ggtgatgact tcaagggacg gtttgccttc tctttggaaa cctctgccag cactgccaat 240
   ttgcagatca acaacctcaa aagtgaggac acggctacat atttctgtgc aagatttggc 300
   tctgctgtgg actactgggg tcaaggaacc tcggtcaccg tctcctcagc cagcacaaag 360
   ggcccatcgg tcttccccct ggcaccctcc tccaagagca cctctggggg cacagcggcc 420
   ctgggctgcc tggtcaagga ctacttcccc gaaccggtga cggtgtcgtg gaactcaggc 480
   gccctgacca gcggcgtgca caccttcccg gctgtcctac agtcctcagg actctactcc 540
   ctcagcagcg tggtgaccgt gccctccagc agcttgggca cccagaccta catctgcaac 600
   gtgaatcaca agcccagcaa caccaaggtg gacaagagag ttgagcccaa atcttgtgac 660
   aaaactcaca catga 675
<210> 580
   <211> 675
   <212> DNA
   <213> artificial
<220>
   <223> ING-1 (light chain)
<400> 580
   caaccagcga tggcggatat tgtgatgacg caggctgcat tctccaatcc agtcactctt        60
   ggaacatcag gttccatctc ctgcaggtct agtaagagtc tcctacatag taatggcatc       120
   acttatttgt attggtatct gcagaagcca ggccagtctc ctcagctcct gatttatcag       180
   atgtccaacc ttgcctcagg agtcccagac aggttcagta gcagtgggtc aggaactgat       240
   ttcacactga gaatcagcag agtggaggct gaggatgtgg gtgtttatta ctgtgctcaa       300
   aatctagaac ttcctcggac gttcggtgga ggcaccaagc ttgagatgaa acgaactgtg       360
   gctgcaccat ctgtcttcat cttcccgcca tctgatgagc agttgaaatc tggaactgcc       420
   tctgttgtgt gcctgctgaa taacttctat cccagagagg ccaaagtaca gtggaaggtg       480
   gataacgccc tccaatcggg taactcccag gagagtgtca cagagcagga cagcaaggac       540
   agcacctaca gcctcagcag caccctgacg ctgagcaaag cagactacga gaaacacaaa       600
   gtctacgcct gcgaagtcac ccatcagggc ctgagctcgc ccgtcacaaa gagcttcaac       660
   aggggagagt gttag                                                         675
<210> 581
   <211> 354
   <212> DNA
   <213> artificial
<220>
   <223> IL-1 (XPA-23, heavy chain)
<400> 581
   gaagttcaat tgttagagtc tggtggcggt cttgttcagc ctggtggttc tttacgtctt        60
   tcttgcgctg cttccggatt cactttctct aagtacttta tgttttgggt tcgccaagct       120
   cctggtaaag gtttggagtg ggtttctgtt atctctcctt ctggtggcat gactcgttat       180
   gctgactccg ttaaaggtcg cttcactatc tctagagaca actctaagaa tactctctac       240
   ttgcagatga acagcttaag ggctgaggac actgcagtct actattgtgc gagagtcggc       300
   tacggtggta actctgacta ctggggccag ggaaccctgg tcaccgtctc aagc             354
<210> 582
   <211> 324
   <212> DNA
   <213> artificial
<220>
   <223> IL-1 (XPA-23, kappa chain)
<400> 582
   caagacatcc agatgaccca gtctccctct tccgtgtctg catctgtagg agacagactc 60
   accatcattt gtcgggcgag tcaggatatt aacaggtggt tagcctggta tcagcagaca 120
   ccagggaatg cccctaagct cctgatccat tctgcaacca gtctgcaaag tggggtccca 180
   tcaaggttta gcggcagtgg atctgggaca gatttcactc tcaccatcaa cagcctgcag 240
   cctgaagatt ttgcaactta ctattgtcag caggctgaca gtttcccgct cactttcggc 300
   ggagggacca aggtggagat caaa 324
<210> 583
   <211> 59
   <212> DNA
   <213> artificial
<220>
   <223> L3-1NHT (ING1)
<220>
   <221> misc_feature
   <222> (25)..(25)
   <223> n is a, c, g, or t
<400> 583
   ggtgtttatt actgtgctca aaatnhtgaa cttcctcgga cgttcggtgg aggcaccaa 59
<210> 584
   <211> 59
   <212> DNA
   <213> artificial
<220>
   <223> L3-1VAA (ING1)
<400> 584
   ggtgtttatt actgtgctca aaatvaagaa cttcctcgga cgttcggtgg aggcaccaa 59
<210> 585
   <211> 59
   <212> DNA
   <213> artificial
<220>
   <223> L3-1BGG (ING1)
<400> 585
   ggtgtttatt actgtgctca aaatbgggaa cttcctcgga cgttcggtgg aggcaccaa 59
<210> 586
   <211> 59
   <212> DNA
   <213> artificial
<220>
   <223> L3-2NHT (ING1)
<220>
   <221> misc_feature
   <222> (28)..(28)
   <223> n is a, c, g, or t
<400> 586
   ggtgtttatt actgtgctca aaatctanht cttcctcgga cgttcggtgg aggcaccaa 59
<210> 587
   <211> 59
   <212> DNA
   <213> artificial
<220>
   <223> L3-2VAA (ING1)
<400> 587
   ggtgtttatt actgtgctca aaatctavaa cttcctcgga cgttcggtgg aggcaccaa 59
<210> 588
   <211> 59
   <212> DNA
   <213> artificial
<220>
   <223> L3-2BGG (ING1)
<400> 588
   ggtgtttatt actgtgctca aaatctabgg cttcctcgga cgttcggtgg aggcaccaa 59
<210> 589
   <211> 59
   <212> DNA
   <213> artificial
<220>
   <223> L3-3NHT (ING1)
<220>
   <221> misc_feature
   <222> (31)..(31)
   <223> n is a, c, g, or t
<400> 589
   ggtgtttatt actgtgctca aaatctagaa nhtcctcgga cgttcggtgg aggcaccaa 59
<210> 590
   <211> 59
   <212> DNA
   <213> artificial
<220>
   <223> L3-3VAA (ING1)
<400> 590
   ggtgtttatt actgtgctca aaatctagaa vaacctcgga cgttcggtgg aggcaccaa 59
<210> 591
   <211> 59
   <212> DNA
   <213> artificial
<220>
   <223> L3-3BGG (ING1)
<400> 591
   ggtgtttatt actgtgctca aaatctagaa bggcctcgga cgttcggtgg aggcaccaa 59
<210> 592
   <211> 59
   <212> DNA
   <213> artificial
<220>
   <223> L3-4NHT (ING1)
<220>
   <221> misc_feature
   <222> (34)..(34)
   <223> n is a, c, g, or t
<400> 592
   ggtgtttatt actgtgctca aaatctagaa cttnhtcgga cgttcggtgg aggcaccaa 59
<210> 593
   <211> 59
   <212> DNA
   <213> artificial
<220>
   <223> L3-4VAA (ING1)
<400> 593
   ggtgtttatt actgtgctca aaatctagaa cttvaacgga cgttcggtgg aggcaccaa 59
<210> 594
   <211> 59
   <212> DNA
   <213> artificial
<220>
   <223> L3-4BGG (ING1)
<400> 594
   ggtgtttatt actgtgctca aaatctagaa cttbggcgga cgttcggtgg aggcaccaa 59
<210> 595
   <211> 59
   <212> DNA
   <213> artificial
<220>
   <223> L3-5NHT (ING1)
<220>
   <221> misc_feature
   <222> (37)..(37)
   <223> n is a, c, g, or t
<400> 595
   ggtgtttatt actgtgctca aaatctagaa cttcctnhta cgttcggtgg aggcaccaa 59
<210> 596
   <211> 59
   <212> DNA
   <213> artificial
<220>
   <223> L3-5VAA (ING1)
<400> 596
   ggtgtttatt actgtgctca aaatctagaa cttcctvaaa cgttcggtgg aggcaccaa 59
<210> 597
   <211> 59
   <212> DNA
   <213> artificial
<220>
   <223> L3-5BGG (ING1)
<400> 597
   ggtgtttatt actgtgctca aaatctagaa cttcctbgga cgttcggtgg aggcaccaa 59
<210> 598
   <211> 55
   <212> DNA
   <213> artificial
<220>
   <223> L1-1NHT (ING1)
<220>
   <221> misc_feature
   <222> (21)..(21)
   <223> n is a, c, g, or t
<400> 598
   ccatctcctg ccgctctagt nhtagtctcc tacatagtaa tggcatcact tattt 55
<210> 599
   <211> 55
   <212> DNA
   <213> artificial
<220>
   <223> L1-1VAA (ING1)
<400> 599
   ccatctcctg ccgctctagt vaaagtctcc tacatagtaa tggcatcact tattt 55
<210> 600
   <211> 55
   <212> DNA
   <213> artificial
<220>
   <223> L1-1BGG (ING1)
<400> 600
   ccatctcctg ccgctctagt bggagtctcc tacatagtaa tggcatcact tattt 55
<210> 601
   <211> 55
   <212> DNA
   <213> artificial
<220>
   <223> L1-2NHT (ING1)
<220>
   <221> misc_feature
   <222> (24)..(24)
   <223> n is a, c, g, or t
<400> 601
   ccatctcctg ccgctctagt aagnhtctcc tacatagtaa tggcatcact tattt 55
<210> 602
   <211> 55
   <212> DNA
   <213> artificial
<220>
   <223> L1-2VAA (ING1)
<400> 602
   ccatctcctg ccgctctagt aagvaactcc tacatagtaa tggcatcact tattt 55
<210> 603
   <211> 55
   <212> DNA
   <213> artificial
<220>
   <223> L1-2BGG (ING1)
<400> 603
   ccatctcctg ccgctctagt aagbggctcc tacatagtaa tggcatcact tattt 55
<210> 604
   <211> 55
   <212> DNA
   <213> artificial
<220>
   <223> L1-3NHT (ING1)
<220>
   <221> misc_feature
   <222> (27)..(27)
   <223> n is a, c, g, or t
<400> 604
   ccatctcctg ccgctctagt aagagtnhtc tacatagtaa tggcatcact tattt 55
<210> 605
   <211> 55
   <212> DNA
   <213> artificial
<220>
   <223> L1-3VAA (ING1)
<400> 605
   ccatctcctg ccgctctagt aagagtvaac tacatagtaa tggcatcact tattt 55
<210> 606
   <211> 55
   <212> DNA
   <213> artificial
<220>
   <223> L1-3BGG (ING1)
<400> 606
   ccatctcctg ccgctctagt aagagtbggc tacatagtaa tggcatcact tattt 55
<210> 607
   <211> 55
   <212> DNA
   <213> artificial
<220>
   <223> L1-4NHT (ING1)
<220>
   <221> misc_feature
   <222> (30)..(30)
   <223> n is a, c, g, or t
<400> 607
   ccatctcctg ccgctctagt aagagtctcn htcatagtaa tggcatcact tattt 55
<210> 608
   <211> 55
   <212> DNA
   <213> artificial
<220>
   <223> L1-4VAA (ING1)
<400> 608
   ccatctcctg ccgctctagt aagagtctcv aacatagtaa tggcatcact tattt 55
<210> 609
   <211> 55
   <212> DNA
   <213> artificial
<220>
   <223> L1-4BGG (ING1)
<400> 609
   ccatctcctg ccgctctagt aagagtctcb ggcatagtaa tggcatcact tattt 55
<210> 610
   <211> 54
   <212> DNA
   <213> artificial
<220>
   <223> L12-1NHT (ING1)
<220>
   <221> misc_feature
   <222> (22)..(22)
   <223> n is a, c, g, or t
<400> 610
   cgctctagta agagtctcct anhtagtaat ggcatcactt atttgtattg gtat 54
<210> 611
   <211> 54
   <212> DNA
   <213> artificial
<220>
   <223> L12-1VAA (ING1)
<400> 611
   cgctctagta agagtctcct avaaagtaat ggcatcactt atttgtattg gtat 54
<210> 612
   <211> 54
   <212> DNA
   <213> artificial
<220>
   <223> L12-1BGG (ING1)
<400> 612
   cgctctagta agagtctcct abggagtaat ggcatcactt atttgtattg gtat 54
<210> 613
   <211> 54
   <212> DNA
   <213> artificial
<220>
   <223> L12-2NHT (ING1)
<220>
   <221> misc_feature
   <222> (25)..(25)
   <223> n is a, c, g, or t
<400> 613
   cgctctagta agagtctcct acatnhtaat ggcatcactt atttgtattg gtat 54
<210> 614
   <211> 54
   <212> DNA
   <213> artificial
<220>
   <223> L12-2VAA (ING1)
<400> 614
   cgctctagta agagtctcct acatvaaaat ggcatcactt atttgtattg gtat 54
<210> 615
   <211> 54
   <212> DNA
   <213> artificial
<220>
   <223> L12-2BGG (ING1)
<400> 615
   cgctctagta agagtctcct acatbggaat ggcatcactt atttgtattg gtat 54
<210> 616
   <211> 54
   <212> DNA
   <213> artificial
<220>
   <223> L12-3NHT (ING1)
<220>
   <221> misc_feature
   <222> (28)..(28)
   <223> n is a, c, g, or t
<400> 616
   cgctctagta agagtctcct acatagtnht ggcatcactt atttgtattg gtat 54
<210> 617
   <211> 54
   <212> DNA
   <213> artificial
<220>
   <223> L12-3VAA (ING1)
<400> 617
   cgctctagta agagtctcct acatagtvaa ggcatcactt atttgtattg gtat 54
<210> 618
   <211> 54
   <212> DNA
   <213> artificial
<220>
   <223> L12-3BGG (ING1)
<400> 618
   cgctctagta agagtctcct acatagtbgg ggcatcactt atttgtattg gtat 54
<210> 619
   <211> 54
   <212> DNA
   <213> artificial
<220>
   <223> L13-1NHT (ING1)
<220>
   <221> misc_feature
   <222> (23)..(23)
   <223> n is a, c, g, or t
<400> 619
   gagtctccta catagtaatg gcnhtactta tttgtattgg tatttacaga agcc 54
<210> 620
   <211> 54
   <212> DNA
   <213> artificial
<220>
   <223> L13-1VAA (ING1)
<400> 620
   gagtctccta catagtaatg gcvaaactta tttgtattgg tatttacaga agcc 54
<210> 621
   <211> 54
   <212> DNA
   <213> artificial
<220>
   <223> L13-1BGG (ING1)
<400> 621
   gagtctccta catagtaatg gcbggactta tttgtattgg tatttacaga agcc 54
<210> 622
   <211> 54
   <212> DNA
   <213> artificial
<220>
   <223> L13-2NHT (ING1)
<220>
   <221> misc_feature
   <222> (26)..(26)
   <223> n is a, c, g, or t
<400> 622
   gagtctccta catagtaatg gcatcnhtta tttgtattgg tatttacaga agcc 54
<210> 623
   <211> 54
   <212> DNA
   <213> artificial
<220>
   <223> L13-2VAA (ING1)
<400> 623
   gagtctccta catagtaatg gcatcvaata tttgtattgg tatttacaga agcc 54
<210> 624
   <211> 54
   <212> DNA
   <213> artificial
<220>
   <223> L13-2BGG (ING1)
<400> 624
   gagtctccta catagtaatg gcatcbggta tttgtattgg tatttacaga agcc 54
<210> 625
   <211> 54
   <212> DNA
   <213> artificial
<220>
   <223> L13-3NHT (ING1)
<220>
   <221> misc_feature
   <222> (29)..(29)
   <223> n is a, c, g, or t
<400> 625
   gagtctccta catagtaatg gcatcactnh tttgtattgg tatttacaga agcc 54
<210> 626
   <211> 54
   <212> DNA
   <213> artificial
<220>
   <223> L13-3VAA (ING1)
<400> 626
   gagtctccta catagtaatg gcatcactva attgtattgg tatttacaga agcc 54
<210> 627
   <211> 54
   <212> DNA
   <213> artificial
<220>
   <223> L13-3BGG (ING1)
<400> 627
   gagtctccta catagtaatg gcatcactbg gttgtattgg tatttacaga agcc 54
<210> 628
   <211> 56
   <212> DNA
   <213> artificial
<220>
   <223> L2-1NHT (ING1)
<220>
   <221> misc_feature
   <222> (22)..(22)
   <223> n is a, c, g, or t
<400> 628
   cagtctcctc agctcctgat tnhtcagatg tccaaccttg cctcaggagt cccaga 56
<210> 629
   <211> 56
   <212> DNA
   <213> artificial
<220>
   <223> L2-1VAA (ING1)
<400> 629
   cagtctcctc agctcctgat tvaacagatg tccaaccttg cctcaggagt cccaga 56
<210> 630
   <211> 56
   <212> DNA
   <213> artificial
<220>
   <223> L2-1BGG (ING1)
<400> 630
   cagtctcctc agctcctgat tbggcagatg tccaaccttg cctcaggagt cccaga 56
<210> 631
   <211> 56
   <212> DNA
   <213> artificial
<220>
   <223> L2-2NHT (ING1)
<220>
   <221> misc_feature
   <222> (25)..(25)
   <223> n is a, c, g, or t
<400> 631
   cagtctcctc agctcctgat ttatnhtatg tccaaccttg cctcaggagt cccaga 56
<210> 632
   <211> 56
   <212> DNA
   <213> artificial
<220>
   <223> L2-2VAA (ING1)
<400> 632
   cagtctcctc agctcctgat ttatvaaatg tccaaccttg cctcaggagt cccaga 56
<210> 633
   <211> 56
   <212> DNA
   <213> artificial
<220>
   <223> L2-2BGG (ING1)
<400> 633
   cagtctcctc agctcctgat ttatbggatg tccaaccttg cctcaggagt cccaga 56
<210> 634
   <211> 56
   <212> DNA
   <213> artificial
<220>
   <223> L2-3NHT (ING1)
<220>
   <221> misc_feature
   <222> (28)..(28)
   <223> n is a, c, g, or t
<400> 634
   cagtctcctc agctcctgat ttatcagnht tccaaccttg cctcaggagt cccaga 56
<210> 635
   <211> 56
   <212> DNA
   <213> artificial
<220>
   <223> L2-3VAA (ING1)
<400> 635
   cagtctcctc agctcctgat ttatcagvaa tccaaccttg cctcaggagt cccaga 56
<210> 636
   <211> 56
   <212> DNA
   <213> artificial
<220>
   <223> L2-3BGG (ING1)
<400> 636
   cagtctcctc agctcctgat ttatcagbgg tccaaccttg cctcaggagt cccaga 56
<210> 637
   <211> 56
   <212> DNA
   <213> artificial
<220>
   <223> L2-4NHT (ING1)
<220>
   <221> misc_feature
   <222> (31)..(31)
   <223> n is a, c, g, or t
<400> 637
   cagtctcctc agctcctgat ttatcagatg nhtaaccttg cctcaggagt cccaga 56
<210> 638
   <211> 56
   <212> DNA
   <213> artificial
<220>
   <223> L2-4VAA (ING1)
<400> 638
   cagtctcctc agctcctgat ttatcagatg vaaaaccttg cctcaggagt cccaga 56
<210> 639
   <211> 56
   <212> DNA
   <213> artificial
<220>
   <223> L2-4BGG (ING1)
<400> 639
   cagtctcctc agctcctgat ttatcagatg bggaaccttg cctcaggagt cccaga 56
<210> 640
   <211> 56
   <212> DNA
   <213> artificial
<220>
   <223> L2-5NHT (ING1)
<220>
   <221> misc_feature
   <222> (34)..(34)
   <223> n is a, c, g, or t
<400> 640
   cagtctcctc agctcctgat ttatcagatg tccnhtcttg cctcaggagt cccaga 56
<210> 641
   <211> 56
   <212> DNA
   <213> artificial
<220>
   <223> L2-5VAA (ING1)
<400> 641
   cagtctcctc agctcctgat ttatcagatg tccvaacttg cctcaggagt cccaga 56
<210> 642
   <211> 56
   <212> DNA
   <213> artificial
<220>
   <223> L2-5BGG (ING1)
<400> 642
   cagtctcctc agctcctgat ttatcagatg tccbggcttg cctcaggagt cccaga 56
<210> 643
   <211> 59
   <212> DNA
   <213> artificial
<220>
   <223> H1-1NHT (ING1)
<220>
   <221> misc_feature
   <222> (22)..(22)
   <223> n is a, c, g, or t
<400> 643
   tcctgcaagg cttctggata tnhtttcaca aaatatggaa tgaactgggt gaagcaggc 59
<210> 644
   <211> 59
   <212> DNA
   <213> artificial
<220>
   <223> H1-1VAA (ING1)
<400> 644
   tcctgcaagg cttctggata tvaattcaca aaatatggaa tgaactgggt gaagcaggc 59
<210> 645
   <211> 59
   <212> DNA
   <213> artificial
<220>
   <223> H1-1BGG (ING1)
<400> 645
   tcctgcaagg cttctggata tbggttcaca aaatatggaa tgaactgggt gaagcaggc 59
<210> 646
   <211> 59
   <212> DNA
   <213> artificial
<220>
   <223> H1-2NHT (ING1)
<220>
   <221> misc_feature
   <222> (28)..(28)
   <223> n is a, c, g, or t
<400> 646
   tcctgcaagg cttctggata taccttcnht aaatatggaa tgaactgggt gaagcaggc 59
<210> 647
   <211> 59
   <212> DNA
   <213> artificial
<220>
   <223> H1-2VAA (ING1)
<400> 647
   tcctgcaagg cttctggata taccttcvaa aaatatggaa tgaactgggt gaagcaggc 59
<210> 648
   <211> 59
   <212> DNA
   <213> artificial
<220>
   <223> H1-2BGG (ING1)
<400> 648
   tcctgcaagg cttctggata taccttcbgg aaatatggaa tgaactgggt gaagcaggc 59
<210> 649
   <211> 59
   <212> DNA
   <213> artificial
<220>
   <223> H1-3NHT (ING1)
<220>
   <221> misc_feature
   <222> (31)..(31)
   <223> n is a, c, g, or t
<400> 649
   tcctgcaagg cttctggata taccttcaca nhttatggaa tgaactgggt gaagcaggc 59
<210> 650
   <211> 59
   <212> DNA
   <213> artificial
<220>
   <223> H1-3VAA (ING1)
<400> 650
   tcctgcaagg cttctggata taccttcaca vaatatggaa tgaactgggt gaagcaggc 59
<210> 651
   <211> 59
   <212> DNA
   <213> artificial
<220>
   <223> H1-3BGG (ING1)
<400> 651
   tcctgcaagg cttctggata taccttcaca bggtatggaa tgaactgggt gaagcaggc 59
<210> 652
   <211> 59
   <212> DNA
   <213> artificial
<220>
   <223> H1-4NHT (ING1)
<220>
   <221> misc_feature
   <222> (34)..(34)
   <223> n is a, c, g, or t
<400> 652
   tcctgcaagg cttctggata taccttcaca aaanhtggaa tgaactgggt gaagcaggc 59
<210> 653
   <211> 59
   <212> DNA
   <213> artificial
<220>
   <223> H1-4VAA (ING1)
<400> 653
   tcctgcaagg cttctggata taccttcaca aaavaaggaa tgaactgggt gaagcaggc 59
<210> 654
   <211> 59
   <212> DNA
   <213> artificial
<220>
   <223> H1-4BGG (ING1)
<400> 654
   tcctgcaagg cttctggata taccttcaca aaabggggaa tgaactgggt gaagcaggc 59
<210> 655
   <211> 59
   <212> DNA
   <213> artificial
<220>
   <223> H1-5NHT (ING1)
<220>
   <221> misc_feature
   <222> (37)..(37)
   <223> n is a, c, g, or t
<400> 655
   tcctgcaagg cttctggata taccttcaca aaatatnhta tgaactgggt gaagcaggc 59
<210> 656
   <211> 59
   <212> DNA
   <213> artificial
<220>
   <223> H1-5VAA (ING1)
<400> 656
   tcctgcaagg cttctggata taccttcaca aaatatvaaa tgaactgggt gaagcaggc 59
<210> 657
   <211> 59
   <212> DNA
   <213> artificial
<220>
   <223> H1-5BGG (ING1)
<400> 657
   tcctgcaagg cttctggata taccttcaca aaatatbgga tgaactgggt gaagcaggc 59
<210> 658
   <211> 58
   <212> DNA
   <213> artificial
<220>
   <223> H2-1NHT (ING1)
<220>
   <221> misc_feature
   <222> (22)..(22)
   <223> n is a, c, g, or t
<400> 658
   aagggtttaa agtggatggg cnhtataaac acctacactg aagagcctac atatggtg 58
<210> 659
   <211> 58
   <212> DNA
   <213> artificial
<220>
   <223> H2-1VAA (ING1)
<400> 659
   aagggtttaa agtggatggg cvaaataaac acctacactg aagagcctac atatggtg 58
<210> 660
   <211> 58
   <212> DNA
   <213> artificial
<220>
   <223> H2-1BGG (ING1)
<400> 660
   aagggtttaa agtggatggg cbggataaac acctacactg aagagcctac atatggtg 58
<210> 661
   <211> 58
   <212> DNA
   <213> artificial
<220>
   <223> H2-2NHT (ING1)
<220>
   <221> misc_feature
   <222> (28)..(28)
   <223> n is a, c, g, or t
<400> 661
   aagggtttaa agtggatggg ctggatanht acctacactg aagagcctac atatggtg 58
<210> 662
   <211> 58
   <212> DNA
   <213> artificial
<220>
   <223> H2-2VAA (ING1)
<400> 662
   aagggtttaa agtggatggg ctggatavaa acctacactg aagagcctac atatggtg 58
<210> 663
   <211> 58
   <212> DNA
   <213> artificial
<220>
   <223> H2-2BGG (ING1)
<400> 663
   aagggtttaa agtggatggg ctggatabgg acctacactg aagagcctac atatggtg 58
<210> 664
   <211> 58
   <212> DNA
   <213> artificial
<220>
   <223> H2-3NHT (ING1)
<220>
   <221> misc_feature
   <222> (31)..(31)
   <223> n is a, c, g, or t
<400> 664
   aagggtttaa agtggatggg ctggataaac nhttacactg aagagcctac atatggtg 58
<210> 665
   <211> 58
   <212> DNA
   <213> artificial
<220>
   <223> H2-3VAA (ING1)
<400> 665
   aagggtttaa agtggatggg ctggataaac vaatacactg aagagcctac atatggtg 58
<210> 666
   <211> 58
   <212> DNA
   <213> artificial
<220>
   <223> H2-3BGG (ING1)
<400> 666
   aagggtttaa agtggatggg ctggataaac bggtacactg aagagcctac atatggtg 58
<210> 667
   <211> 58
   <212> DNA
   <213> artificial
<220>
   <223> H2-4NHT (ING1)
<220>
   <221> misc_feature
   <222> (34)..(34)
   <223> n is a, c, g, or t
<400> 667
   aagggtttaa agtggatggg ctggataaac accnhtactg aagagcctac atatggtg 58
<210> 668
   <211> 58
   <212> DNA
   <213> artificial
<220>
   <223> H2-4VAA (ING1)
<400> 668
   aagggtttaa agtggatggg ctggataaac accvaaactg aagagcctac atatggtg 58
<210> 669
   <211> 58
   <212> DNA
   <213> artificial
<220>
   <223> H2-4BGG (ING1)
<400> 669
   aagggtttaa agtggatggg ctggataaac accbggactg aagagcctac atatggtg 58
<210> 670
   <211> 58
   <212> DNA
   <213> artificial
<220>
   <223> H22-1NHT (ING1)
<220>
   <221> misc_feature
   <222> (22)..(22)
   <223> n is a, c, g, or t
<400> 670
   atgggctgga taaacaccta cnhtgaagag cctacatatg gtgatgactt caagggac 58
<210> 671
   <211> 58
   <212> DNA
   <213> artificial
<220>
   <223> H22-1VAA (ING1)
<400> 671
   atgggctgga taaacaccta cvaagaagag cctacatatg gtgatgactt caagggac 58
<210> 672
   <211> 58
   <212> DNA
   <213> artificial
<220>
   <223> H22-1BGG (ING1)
<400> 672
   atgggctgga taaacaccta cbgggaagag cctacatatg gtgatgactt caagggac 58
<210> 673
   <211> 58
   <212> DNA
   <213> artificial
<220>
   <223> H22-2NHT (ING1)
<220>
   <221> misc_feature
   <222> (25)..(25)
   <223> n is a, c, g, or t
<400> 673
   atgggctgga taaacaccta cactnhtgag cctacatatg gtgatgactt caagggac 58
<210> 674
   <211> 58
   <212> DNA
   <213> artificial
<220>
   <223> H22-2VAA (ING1)
<400> 674
   atgggctgga taaacaccta cactvaagag cctacatatg gtgatgactt caagggac 58
<210> 675
   <211> 58
   <212> DNA
   <213> artificial
<220>
   <223> H22-2BGG (ING1)
<400> 675
   atgggctgga taaacaccta cactbgggag cctacatatg gtgatgactt caagggac 58
<210> 676
   <211> 58
   <212> DNA
   <213> artificial
<220>
   <223> H22-3NHT (ING1)
<220>
   <221> misc_feature
   <222> (28)..(28)
   <223> n is a, c, g, or t
<400> 676
   atgggctgga taaacaccta cactgaanht cctacatatg gtgatgactt caagggac 58
<210> 677
   <211> 58
   <212> DNA
   <213> artificial
<220>
   <223> H22-3VAA (ING1)
<400> 677
   atgggctgga taaacaccta cactgaavaa cctacatatg gtgatgactt caagggac 58
<210> 678
   <211> 58
   <212> DNA
   <213> artificial
<220>
   <223> H22-3BGG (ING1)
<400> 678
   atgggctgga taaacaccta cactgaabgg cctacatatg gtgatgactt caagggac 58
<210> 679
   <211> 58
   <212> DNA
   <213> artificial
<220>
   <223> H22-4NHT (ING1)
<220>
   <221> misc_feature
   <222> (31)..(31)
   <223> n is a, c, g, or t
<400> 679
   atgggctgga taaacaccta cactgaagag nhtacatatg gtgatgactt caagggac 58
<210> 680
   <211> 58
   <212> DNA
   <213> artificial
<220>
   <223> H22-4VAA (ING1)
<400> 680
   atgggctgga taaacaccta cactgaagag vaaacatatg gtgatgactt caagggac 58
<210> 681
   <211> 58
   <212> DNA
   <213> artificial
<220>
   <223> H22-4BGG (ING1)
<400> 681
   atgggctgga taaacaccta cactgaagag bggacatatg gtgatgactt caagggac 58
<210> 682
   <211> 58
   <212> DNA
   <213> artificial
<220>
   <223> H22-5NHT (ING1)
<220>
   <221> misc_feature
   <222> (34)..(34)
   <223> n is a, c, g, or t
<400> 682
   atgggctgga taaacaccta cactgaagag cctnhttatg gtgatgactt caagggac 58
<210> 683
   <211> 58
   <212> DNA
   <213> artificial
<220>
   <223> H22-5VAA (ING1)
<400> 683
   atgggctgga taaacaccta cactgaagag cctvaatatg gtgatgactt caagggac 58
<210> 684
   <211> 58
   <212> DNA
   <213> artificial
<220>
   <223> H22-5BGG (ING1)
<400> 684
   atgggctgga taaacaccta cactgaagag cctbggtatg gtgatgactt caagggac 58
<210> 685
   <211> 53
   <212> DNA
   <213> artificial
<220>
   <223> H3-1NHT (ING1)
<220>
   <221> misc_feature
   <222> (25)..(25)
   <223> n is a, c, g, or t
<400> 685
   gctacatatt tctgtgcaag atttnhttct gctgtggact actggggtca agg 53
<210> 686
   <211> 53
   <212> DNA
   <213> artificial
<220>
   <223> H3-1VAA (ING1)
<400> 686
   gctacatatt tctgtgcaag atttvaatct gctgtggact actggggtca agg 53
<210> 687
   <211> 53
   <212> DNA
   <213> artificial
<220>
   <223> H3-1BGG (ING1)
<400> 687
   gctacatatt tctgtgcaag atttbggtct gctgtggact actggggtca agg 53
<210> 688
   <211> 53
   <212> DNA
   <213> artificial
<220>
   <223> H3-2NHT (ING1)
<220>
   <221> misc_feature
   <222> (28)..(28)
   <223> n is a, c, g, or t
<400> 688
   gctacatatt tctgtgcaag atttggcnht gctgtggact actggggtca agg 53
<210> 689
   <211> 53
   <212> DNA
   <213> artificial
<220>
   <223> H3-2VAA (ING1)
<400> 689
   gctacatatt tctgtgcaag atttggcvaa gctgtggact actggggtca agg 53
<210> 690
   <211> 53
   <212> DNA
   <213> artificial
<220>
   <223> H3-2BGG (ING1)
<400> 690
   gctacatatt tctgtgcaag atttggcbgg gctgtggact actggggtca agg 53
<210> 691
   <211> 53
   <212> DNA
   <213> artificial
<220>
   <223> H3-3NHT (ING1)
<220>
   <221> misc_feature
   <222> (31)..(31)
   <223> n is a, c, g, or t
<400> 691
   gctacatatt tctgtgcaag atttggctct nhtgtggact actggggtca agg 53
<210> 692
   <211> 53
   <212> DNA
   <213> artificial
<220>
   <223> H3-3VAA (ING1)
<400> 692
   gctacatatt tctgtgcaag atttggctct vaagtggact actggggtca agg 53
<210> 693
   <211> 53
   <212> DNA
   <213> artificial
<220>
   <223> H3-3BGG (ING1)
<400> 693
   gctacatatt tctgtgcaag atttggctct bgggtggact actggggtca agg 53
<210> 694
   <211> 46
   <212> DNA
   <213> artificial
<220>
   <223> H32-1NHT (ING1)
<220>
   <221> misc_feature
   <222> (22)..(22)
   <223> n is a, c, g, or t
<400> 694
   gcaagatttg gctctgctgt gnhttactgg ggtcaaggaa cctcgg 46
<210> 695
   <211> 46
   <212> DNA
   <213> artificial
<220>
   <223> H32-1VAA (ING1)
<400> 695
   gcaagatttg gctctgctgt gvaatactgg ggtcaaggaa cctcgg 46
<210> 696
   <211> 46
   <212> DNA
   <213> artificial
<220>
   <223> H32-1BGG (ING1)
<400> 696
   gcaagatttg gctctgctgt gbggtactgg ggtcaaggaa cctcgg 46
<210> 697
   <211> 46
   <212> DNA
   <213> artificial
<220>
   <223> H32-2NHT (ING1)
<220>
   <221> misc_feature
   <222> (25)..(25)
   <223> n is a, c, g, or t
<400> 697
   gcaagatttg gctctgctgt ggacnhttgg ggtcaaggaa cctcgg 46
<210> 698
   <211> 46
   <212> DNA
   <213> artificial
<220>
   <223> H32-2VAA (ING1)
<400> 698
   gcaagatttg gctctgctgt ggacvaatgg ggtcaaggaa cctcgg 46
<210> 699
   <211> 46
   <212> DNA
   <213> artificial
<220>
   <223> H32-2BGG (ING1)
<400> 699
   gcaagatttg gctctgctgt ggacbggtgg ggtcaaggaa cctcgg 46
<210> 700
   <211> 54
   <212> DNA
   <213> artificial
<220>
   <223> H1-1NHT (IL1B)
<220>
   <221> misc_feature
   <222> (21)..(21)
   <223> n is a, c, g, or t
<400> 700
   cttgcgctgc ttccggattc nhtttctcta agtactttat gttttgggtt cgcc 54
<210> 701
   <211> 54
   <212> DNA
   <213> artificial
<220>
   <223> H1-1VAA (IL1B)
<400> 701
   cttgcgctgc ttccggattc vaattctcta agtactttat gttttgggtt cgcc 54
<210> 702
   <211> 54
   <212> DNA
   <213> artificial
<220>
   <223> H1-1BGG (IL1B)
<400> 702
   cttgcgctgc ttccggattc bggttctcta agtactttat gttttgggtt cgcc 54
<210> 703
   <211> 54
   <212> DNA
   <213> artificial
<220>
   <223> H1-2NHT (IL1B)
<220>
   <221> misc_feature
   <222> (27)..(27)
   <223> n is a, c, g, or t
<400> 703
   cttgcgctgc ttccggattc actttcnhta agtactttat gttttgggtt cgcc 54
<210> 704
   <211> 54
   <212> DNA
   <213> artificial
<220>
   <223> H1-2VAA (IL1B)
<400> 704
   cttgcgctgc ttccggattc actttcvaaa agtactttat gttttgggtt cgcc 54
<210> 705
   <211> 54
   <212> DNA
   <213> artificial
<220>
   <223> H1-2BGG (IL1B)
<400> 705
   cttgcgctgc ttccggattc actttcbgga agtactttat gttttgggtt cgcc 54
<210> 706
   <211> 54
   <212> DNA
   <213> artificial
<220>
   <223> H1-3NHT (IL1B)
<220>
   <221> misc_feature
   <222> (30)..(30)
   <223> n is a, c, g, or t
<400> 706
   cttgcgctgc ttccggattc actttctctn httactttat gttttgggtt cgcc 54
<210> 707
   <211> 54
   <212> DNA
   <213> artificial
<220>
   <223> H1-3VAA (IL1B)
<400> 707
   cttgcgctgc ttccggattc actttctctv aatactttat gttttgggtt cgcc 54
<210> 708
   <211> 54
   <212> DNA
   <213> artificial
<220>
   <223> H1-3BGG (IL1B)
<400> 708
   cttgcgctgc ttccggattc actttctctb ggtactttat gttttgggtt cgcc 54
<210> 709
   <211> 53
   <212> DNA
   <213> artificial
<220>
   <223> H12-1NHT (IL1B)
<220>
   <221> misc_feature
   <222> (23)..(23)
   <223> n is a, c, g, or t
<400> 709
   ttccggattc actttctcta agnhttttat gttttgggtt cgccaagctc ctg 53
<210> 710
   <211> 53
   <212> DNA
   <213> artificial
<220>
   <223> H12-1VAA (IL1B)
<400> 710
   ttccggattc actttctcta agvaatttat gttttgggtt cgccaagctc ctg 53
<210> 711
   <211> 53
   <212> DNA
   <213> artificial
<220>
   <223> H12-1BGG (IL1B)
<400> 711
   ttccggattc actttctcta agbggtttat gttttgggtt cgccaagctc ctg 53
<210> 712
   <211> 53
   <212> DNA
   <213> artificial
<220>
   <223> H12-2NHT (IL1B)
<220>
   <221> misc_feature
   <222> (26)..(26)
   <223> n is a, c, g, or t
<400> 712
   ttccggattc actttctcta agtacnhtat gttttgggtt cgccaagctc ctg 53
<210> 713
   <211> 53
   <212> DNA
   <213> artificial
<220>
   <223> H12-2VAA (IL1B)
<400> 713
   ttccggattc actttctcta agtacvaaat gttttgggtt cgccaagctc ctg 53
<210> 714
   <211> 53
   <212> DNA
   <213> artificial
<220>
   <223> H12-2BGG (IL1B)
<400> 714
   ttccggattc actttctcta agtacbggat gttttgggtt cgccaagctc ctg 53
<210> 715
   <211> 53
   <212> DNA
   <213> artificial
<220>
   <223> H12-3NHT (IL1B)
<220>
   <221> misc_feature
   <222> (32)..(32)
   <223> n is a, c, g, or t
<400> 715
   ttccggattc actttctcta agtactttat gnhttgggtt cgccaagctc ctg 53
<210> 716
   <211> 53
   <212> DNA
   <213> artificial
<220>
   <223> H12-3VAA (IL1B)
<400> 716
   ttccggattc actttctcta agtactttat gvaatgggtt cgccaagctc ctg 53
<210> 717
   <211> 53
   <212> DNA
   <213> artificial
<220>
   <223> H12-3BGG (IL1B)
<400> 717
   ttccggattc actttctcta agtactttat gbggtgggtt cgccaagctc ctg 53
<210> 718
   <211> 57
   <212> DNA
   <213> artificial
<220>
   <223> H2-1NHT (IL1B)
<220>
   <221> misc_feature
   <222> (23)..(23)
   <223> n is a, c, g, or t
<400> 718
   taaaggtttg gagtgggttt ctnhtatctc tccttctggt ggcatgactc gttatgc 57
<210> 719
   <211> 57
   <212> DNA
   <213> artificial
<220>
   <223> H2-1VAA (IL1B)
<400> 719
   taaaggtttg gagtgggttt ctvaaatctc tccttctggt ggcatgactc gttatgc 57
<210> 720
   <211> 57
   <212> DNA
   <213> artificial
<220>
   <223> H2-1BGG (IL1B)
<400> 720
   taaaggtttg gagtgggttt ctbggatctc tccttctggt ggcatgactc gttatgc 57
<210> 721
   <211> 57
   <212> DNA
   <213> artificial
<220>
   <223> H2-2NHT (IL1B)
<220>
   <221> misc_feature
   <222> (26)..(26)
   <223> n is a, c, g, or t
<400> 721
   taaaggtttg gagtgggttt ctgttnhttc tccttctggt ggcatgactc gttatgc 57
<210> 722
   <211> 57
   <212> DNA
   <213> artificial
<220>
   <223> H2-2VAA (IL1B)
<400> 722
   taaaggtttg gagtgggttt ctgttvaatc tccttctggt ggcatgactc gttatgc 57
<210> 723
   <211> 57
   <212> DNA
   <213> artificial
<220>
   <223> H2-2BGG (IL1B)
<400> 723
   taaaggtttg gagtgggttt ctgttbggtc tccttctggt ggcatgactc gttatgc 57
<210> 724
   <211> 57
   <212> DNA
   <213> artificial
<220>
   <223> H2-3NHT (IL1B)
<220>
   <221> misc_feature
   <222> (29)..(29)
   <223> n is a, c, g, or t
<400> 724
   taaaggtttg gagtgggttt ctgttatcnh tccttctggt ggcatgactc gttatgc 57
<210> 725
   <211> 57
   <212> DNA
   <213> artificial
<220>
   <223> H2-3VAA (IL1B)
<400> 725
   taaaggtttg gagtgggttt ctgttatcva accttctggt ggcatgactc gttatgc 57
<210> 726
   <211> 57
   <212> DNA
   <213> artificial
<220>
   <223> H2-3BGG (IL1B)
<400> 726
   taaaggtttg gagtgggttt ctgttatcbg gccttctggt ggcatgactc gttatgc 57
<210> 727
   <211> 57
   <212> DNA
   <213> artificial
<220>
   <223> H2-4NHT (IL1B)
<220>
   <221> misc_feature
   <222> (32)..(32)
   <223> n is a, c, g, or t
<400> 727
   taaaggtttg gagtgggttt ctgttatctc tnhttctggt ggcatgactc gttatgc 57
<210> 728
   <211> 57
   <212> DNA
   <213> artificial
<220>
   <223> H2-4VAA (IL1B)
<400> 728
   taaaggtttg gagtgggttt ctgttatctc tvaatctggt ggcatgactc gttatgc 57
<210> 729
   <211> 57
   <212> DNA
   <213> artificial
<220>
   <223> H2-4BGG (IL1B)
<400> 729
   taaaggtttg gagtgggttt ctgttatctc tbggtctggt ggcatgactc gttatgc 57
<210> 730
   <211> 57
   <212> DNA
   <213> artificial
<220>
   <223> H2-5NHT (IL1B)
<220>
   <221> misc_feature
   <222> (35)..(35)
   <223> n is a, c, g, or t
<400> 730
   taaaggtttg gagtgggttt ctgttatctc tcctnhtggt ggcatgactc gttatgc 57
<210> 731
   <211> 57
   <212> DNA
   <213> artificial
<220>
   <223> H2-5VAA (IL1B)
<400> 731
   taaaggtttg gagtgggttt ctgttatctc tcctvaaggt ggcatgactc gttatgc 57
<210> 732
   <211> 57
   <212> DNA
   <213> artificial
<220>
   <223> H2-5BGG (IL1B)
<400> 732
   taaaggtttg gagtgggttt ctgttatctc tcctbggggt ggcatgactc gttatgc 57
<210> 733
   <211> 59
   <212> DNA
   <213> artificial
<220>
   <223> H22-1NHT (IL1B)
<220>
   <221> misc_feature
   <222> (23)..(23)
   <223> n is a, c, g, or t
<400> 733
   ggtttctgtt atctctcctt ctnhtggcat gactcgttat gctgactccg ttaaaggtc 59
<210> 734
   <211> 59
   <212> DNA
   <213> artificial
<220>
   <223> H22-1VAA (IL1B)
<400> 734
   ggtttctgtt atctctcctt ctvaaggcat gactcgttat gctgactccg ttaaaggtc 59
<210> 735
   <211> 59
   <212> DNA
   <213> artificial
<220>
   <223> H22-1BGG (IL1B)
<400> 735
   ggtttctgtt atctctcctt ctbggggcat gactcgttat gctgactccg ttaaaggtc 59
<210> 736
   <211> 59
   <212> DNA
   <213> artificial
<220>
   <223> H22-2NHT (IL1B)
<220>
   <221> misc_feature
   <222> (26)..(26)
   <223> n is a, c, g, or t
<400> 736
   ggtttctgtt atctctcctt ctggtnhtat gactcgttat gctgactccg ttaaaggtc 59
<210> 737
   <211> 59
   <212> DNA
   <213> artificial
<220>
   <223> H22-2VAA (IL1B)
<400> 737
   ggtttctgtt atctctcctt ctggtvaaat gactcgttat gctgactccg ttaaaggtc 59
<210> 738
   <211> 59
   <212> DNA
   <213> artificial
<220>
   <223> H22-2BGG (IL1B)
<400> 738
   ggtttctgtt atctctcctt ctggtbggat gactcgttat gctgactccg ttaaaggtc 59
<210> 739
   <211> 59
   <212> DNA
   <213> artificial
<220>
   <223> H22-3NHT (IL1B)
<220>
   <221> misc_feature
   <222> (29)..(29)
   <223> n is a, c, g, or t
<400> 739
   ggtttctgtt atctctcctt ctggtggcnh tactcgttat gctgactccg ttaaaggtc 59
<210> 740
   <211> 59
   <212> DNA
   <213> artificial
<220>
   <223> H22-3VAA (IL1B)
<400> 740
   ggtttctgtt atctctcctt ctggtggcva aactcgttat gctgactccg ttaaaggtc 59
<210> 741
   <211> 59
   <212> DNA
   <213> artificial
<220>
   <223> H22-3BGG (IL1B)
<400> 741
   ggtttctgtt atctctcctt ctggtggcbg gactcgttat gctgactccg ttaaaggtc 59
<210> 742
   <211> 59
   <212> DNA
   <213> artificial
<220>
   <223> H22-4NHT (IL1B)
<220>
   <221> misc_feature
   <222> (32)..(32)
   <223> n is a, c, g, or t
<400> 742
   ggtttctgtt atctctcctt ctggtggcat gnhtcgttat gctgactccg ttaaaggtc 59
<210> 743
   <211> 59
   <212> DNA
   <213> artificial
<220>
   <223> H22-4VAA (IL1B)
<400> 743
   ggtttctgtt atctctcctt ctggtggcat gvaacgttat gctgactccg ttaaaggtc 59
<210> 744
   <211> 59
   <212> DNA
   <213> artificial
<220>
   <223> H22-4BGG (IL1B)
<400> 744
   ggtttctgtt atctctcctt ctggtggcat gbggcgttat gctgactccg ttaaaggtc 59
<210> 745
   <211> 59
   <212> DNA
   <213> artificial
<220>
   <223> H22-5NHT (IL1B)
<220>
   <221> misc_feature
   <222> (35)..(35)
   <223> n is a, c, g, or t
<400> 745
   ggtttctgtt atctctcctt ctggtggcat gactnhttat gctgactccg ttaaaggtc 59
<210> 746
   <211> 59
   <212> DNA
   <213> artificial
<220>
   <223> H22-5VAA (IL1B)
<400> 746
   ggtttctgtt atctctcctt ctggtggcat gactvaatat gctgactccg ttaaaggtc 59
<210> 747
   <211> 59
   <212> DNA
   <213> artificial
<220>
   <223> H22-5BGG (IL1B)
<400> 747
   ggtttctgtt atctctcctt ctggtggcat gactbggtat gctgactccg ttaaaggtc 59
<210> 748
   <211> 56
   <212> DNA
   <213> artificial
<220>
   <223> H3-1NHT (IL1B)
<220>
   <221> misc_feature
   <222> (22)..(22)
   <223> n is a, c, g, or t
<400> 748
   gcagtctact attgtgcgag anhtggctac ggtggtaact ctgactactg gggcca 56
<210> 749
   <211> 56
   <212> DNA
   <213> artificial
<220>
   <223> H3-1VAA (IL1B)
<400> 749
   gcagtctact attgtgcgag avaaggctac ggtggtaact ctgactactg gggcca 56
<210> 750
   <211> 56
   <212> DNA
   <213> artificial
<220>
   <223> H3-1BGG (IL1B)
<400> 750
   gcagtctact attgtgcgag abggggctac ggtggtaact ctgactactg gggcca 56
<210> 751
   <211> 56
   <212> DNA
   <213> artificial
<220>
   <223> H3-2NHT (IL1B)
<220>
   <221> misc_feature
   <222> (25)..(25)
   <223> n is a, c, g, or t
<400> 751
   gcagtctact attgtgcgag agtcnhttac ggtggtaact ctgactactg gggcca 56
<210> 752
   <211> 56
   <212> DNA
   <213> artificial
<220>
   <223> H3-2VAA (IL1B)
<400> 752
   gcagtctact attgtgcgag agtcvaatac ggtggtaact ctgactactg gggcca 56
<210> 753
   <211> 56
   <212> DNA
   <213> artificial
<220>
   <223> H3-2BGG (IL1B)
<400> 753
   gcagtctact attgtgcgag agtcbggtac ggtggtaact ctgactactg gggcca 56
<210> 754
   <211> 56
   <212> DNA
   <213> artificial
<220>
   <223> H3-3NHT (IL1B)
<220>
   <221> misc_feature
   <222> (28)..(28)
   <223> n is a, c, g, or t
<400> 754
   gcagtctact attgtgcgag agtcggcnht ggtggtaact ctgactactg gggcca 56
<210> 755
   <211> 56
   <212> DNA
   <213> artificial
<220>
   <223> H3-3VAA (IL1B)
<400> 755
   gcagtctact attgtgcgag agtcggcvaa ggtggtaact ctgactactg gggcca 56
<210> 756
   <211> 56
   <212> DNA
   <213> artificial
<220>
   <223> H3-3BGG (IL1B)
<400> 756
   gcagtctact attgtgcgag agtcggcbgg ggtggtaact ctgactactg gggcca 56
<210> 757
   <211> 56
   <212> DNA
   <213> artificial
<220>
   <223> H3-4NHT (IL1B)
<220>
   <221> misc_feature
   <222> (31)..(31)
   <223> n is a, c, g, or t
<400> 757
   gcagtctact attgtgcgag agtcggctac nhtggtaact ctgactactg gggcca 56
<210> 758
   <211> 56
   <212> DNA
   <213> artificial
<220>
   <223> H3-4VAA (IL1B)
<400> 758
   gcagtctact attgtgcgag agtcggctac vaaggtaact ctgactactg gggcca 56
<210> 759
   <211> 56
   <212> DNA
   <213> artificial
<220>
   <223> H3-4BGG (IL1B)
<400> 759
   gcagtctact attgtgcgag agtcggctac bggggtaact ctgactactg gggcca 56
<210> 760
   <211> 56
   <212> DNA
   <213> artificial
<220>
   <223> H3-5NHT (IL1B)
<220>
   <221> misc_feature
   <222> (34)..(34)
   <223> n is a, c, g, or t
<400> 760
   gcagtctact attgtgcgag agtcggctac ggtnhtaact ctgactactg gggcca 56
<210> 761
   <211> 56
   <212> DNA
   <213> artificial
<220>
   <223> H3-5VAA (IL1B)
<400> 761
   gcagtctact attgtgcgag agtcggctac ggtvaaaact ctgactactg gggcca 56
<210> 762
   <211> 56
   <212> DNA
   <213> artificial
<220>
   <223> H3-5BGG (IL1B)
<400> 762
   gcagtctact attgtgcgag agtcggctac ggtbggaact ctgactactg gggcca 56
<210> 763
   <211> 49
   <212> DNA
   <213> artificial
<220>
   <223> H32-1NHT (IL1B)
<220>
   <221> misc_feature
   <222> (20)..(20)
   <223> n is a, c, g, or t
<400> 763
   gagagtcggc tacggtggtn httctgacta ctggggccag ggaaccctg 49
<210> 764
   <211> 49
   <212> DNA
   <213> artificial
<220>
   <223> H32-1VAA (IL1B)
<400> 764
   gagagtcggc tacggtggtv aatctgacta ctggggccag ggaaccctg 49
<210> 765
   <211> 49
   <212> DNA
   <213> artificial
<220>
   <223> H32-1BGG (IL1B)
<400> 765
   gagagtcggc tacggtggtb ggtctgacta ctggggccag ggaaccctg 49
<210> 766
   <211> 49
   <212> DNA
   <213> artificial
<220>
   <223> H32-2NHT (IL1B)
<220>
   <221> misc_feature
   <222> (23)..(23)
   <223> n is a, c, g, or t
<400> 766
   gagagtcggc tacggtggta acnhtgacta ctggggccag ggaaccctg 49
<210> 767
   <211> 49
   <212> DNA
   <213> artificial
<220>
   <223> H32-2VAA (IL1B)
<400> 767
   gagagtcggc tacggtggta acvaagacta ctggggccag ggaaccctg 49
<210> 768
   <211> 49
   <212> DNA
   <213> artificial
<220>
   <223> H32-2BGG (IL1B)
<400> 768
   gagagtcggc tacggtggta acbgggacta ctggggccag ggaaccctg 49
<210> 769
   <211> 49
   <212> DNA
   <213> artificial
<220>
   <223> H32-3VAA (IL1B)
<400> 769
   gagagtcggc tacggtggta actctvaata ctggggccag ggaaccctg 49
<210> 770
   <211> 49
   <212> DNA
   <213> artificial
<220>
   <223> H32-3BGG (IL1B)
<400> 770
   gagagtcggc tacggtggta actctbggta ctggggccag ggaaccctg 49
<210> 771
   <211> 49
   <212> DNA
   <213> artificial
<220>
   <223> H32-4NHT (IL1B)
<220>
   <221> misc_feature
   <222> (29)..(29)
   <223> n is a, c, g, or t
<400> 771
   gagagtcggc tacggtggta actctgacnh ttggggccag ggaaccctg 49
<210> 772
   <211> 49
   <212> DNA
   <213> artificial
<220>
   <223> H32-4VAA (IL1B)
<400> 772
   gagagtcggc tacggtggta actctgacva atggggccag ggaaccctg 49
<210> 773
   <211> 49
   <212> DNA
   <213> artificial
<220>
   <223> H32-4BGG (IL1B)
<400> 773
   gagagtcggc tacggtggta actctgacbg gtggggccag ggaaccctg 49
<210> 774
   <211> 58
   <212> DNA
   <213> artificial
<220>
   <223> L1-1NHT (IL1B)
<220>
   <221> misc_feature
   <222> (21)..(21)
   <223> n is a, c, g, or t
<400> 774
   ccatcatttg tcgggcgagt nhtgatatta acaggtggtt agcctggtat cagcagac 58
<210> 775
   <211> 58
   <212> DNA
   <213> artificial
<220>
   <223> L1-1VAA (IL1B)
<400> 775
   ccatcatttg tcgggcgagt vaagatatta acaggtggtt agcctggtat cagcagac 58
<210> 776
   <211> 58
   <212> DNA
   <213> artificial
<220>
   <223> L1-1BGG (IL1B)
<400> 776
   ccatcatttg tcgggcgagt bgggatatta acaggtggtt agcctggtat cagcagac 58
<210> 777
   <211> 58
   <212> DNA
   <213> artificial
<220>
   <223> L1-2NHT (IL1B)
<220>
   <221> misc_feature
   <222> (24)..(24)
   <223> n is a, c, g, or t
<400> 777
   ccatcatttg tcgggcgagt cagnhtatta acaggtggtt agcctggtat cagcagac 58
<210> 778
   <211> 58
   <212> DNA
   <213> artificial
<220>
   <223> L1-2VAA (IL1B)
<400> 778
   ccatcatttg tcgggcgagt cagvaaatta acaggtggtt agcctggtat cagcagac 58
<210> 779
   <211> 58
   <212> DNA
   <213> artificial
<220>
   <223> L1-2BGG (IL1B)
<400> 779
   ccatcatttg tcgggcgagt cagbggatta acaggtggtt agcctggtat cagcagac 58
<210> 780
   <211> 58
   <212> DNA
   <213> artificial
<220>
   <223> L1-3NHT (IL1B)
<220>
   <221> misc_feature
   <222> (30)..(30)
   <223> n is a, c, g, or t
<400> 780
   ccatcatttg tcgggcgagt caggatattn htaggtggtt agcctggtat cagcagac 58
<210> 781
   <211> 58
   <212> DNA
   <213> artificial
<220>
   <223> L1-3VAA (IL1B)
<400> 781
   ccatcatttg tcgggcgagt caggatattv aaaggtggtt agcctggtat cagcagac 58
<210> 782
   <211> 58
   <212> DNA
   <213> artificial
<220>
   <223> L1-3BGG (IL1B)
<400> 782
   ccatcatttg tcgggcgagt caggatattb ggaggtggtt agcctggtat cagcagac 58
<210> 783
   <211> 58
   <212> DNA
   <213> artificial
<220>
   <223> L1-4NHT (IL1B)
<220>
   <221> misc_feature
   <222> (33)..(33)
   <223> n is a, c, g, or t
<400> 783
   ccatcatttg tcgggcgagt caggatatta acnhttggtt agcctggtat cagcagac 58
<210> 784
   <211> 58
   <212> DNA
   <213> artificial
<220>
   <223> L1-4VAA (IL1B)
<400> 784
   ccatcatttg tcgggcgagt caggatatta acvaatggtt agcctggtat cagcagac 58
<210> 785
   <211> 58
   <212> DNA
   <213> artificial
<220>
   <223> L1-4BGG (IL1B)
<400> 785
   ccatcatttg tcgggcgagt caggatatta acbggtggtt agcctggtat cagcagac 58
<210> 786
   <211> 58
   <212> DNA
   <213> artificial
<220>
   <223> L1-5NHT (IL1B)
<220>
   <221> misc_feature
   <222> (36)..(36)
   <223> n is a, c, g, or t
<400> 786
   ccatcatttg tcgggcgagt caggatatta acaggnhttt agcctggtat cagcagac 58
<210> 787
   <211> 58
   <212> DNA
   <213> artificial
<220>
   <223> L1-5VAA (IL1B)
<400> 787
   ccatcatttg tcgggcgagt caggatatta acaggvaatt agcctggtat cagcagac 58
<210> 788
   <211> 55
   <212> DNA
   <213> artificial
<220>
   <223> L1-5BGG (IL1B)
<400> 788
   atgcccctaa gctcctgatc cattctgcaa ccbggctgca aagtggggtc ccatc 55
<210> 789
   <211> 57
   <212> DNA
   <213> artificial
<220>
   <223> L3-1NHT (IL1B)
<220>
   <221> misc_feature
   <222> (22)..(22)
   <223> n is a, c, g, or t
<400> 789
   gcaacttact attgtcagca gnhtgacagt ttcccgctca ctttcggcgg agggacc 57
<210> 790
   <211> 57
   <212> DNA
   <213> artificial
<220>
   <223> L3-1VAA (IL1B)
<400> 790
   gcaacttact attgtcagca gvaagacagt ttcccgctca ctttcggcgg agggacc 57
<210> 791
   <211> 57
   <212> DNA
   <213> artificial
<220>
   <223> L3-1BGG (IL1B)
<400> 791
   gcaacttact attgtcagca gbgggacagt ttcccgctca ctttcggcgg agggacc 57
<210> 792
   <211> 57
   <212> DNA
   <213> artificial
<220>
   <223> L3-2NHT (IL1B)
<220>
   <221> misc_feature
   <222> (25)..(25)
   <223> n is a, c, g, or t
<400> 792
   gcaacttact attgtcagca ggctnhtagt ttcccgctca ctttcggcgg agggacc 57
<210> 793
   <211> 57
   <212> DNA
   <213> artificial
<220>
   <223> L3-2VAA (IL1B)
<400> 793
   gcaacttact attgtcagca ggctvaaagt ttcccgctca ctttcggcgg agggacc 57
<210> 794
   <211> 57
   <212> DNA
   <213> artificial
<220>
   <223> L3-2BGG (IL1B)
<400> 794
   gcaacttact attgtcagca ggctbggagt ttcccgctca ctttcggcgg agggacc 57
<210> 795
   <211> 57
   <212> DNA
   <213> artificial
<220>
   <223> L3-3NHT (IL1B)
<220>
   <221> misc_feature
   <222> (28)..(28)
   <223> n is a, c, g, or t
<400> 795
   gcaacttact attgtcagca ggctgacnht ttcccgctca ctttcggcgg agggacc 57
<210> 796
   <211> 57
   <212> DNA
   <213> artificial
<220>
   <223> L3-3VAA (IL1B)
<400> 796
   gcaacttact attgtcagca ggctgacvaa ttcccgctca ctttcggcgg agggacc 57
<210> 797
   <211> 57
   <212> DNA
   <213> artificial
<220>
   <223> L3-3BGG (IL1B)
<400> 797
   gcaacttact attgtcagca ggctgacbgg ttcccgctca ctttcggcgg agggacc 57
<210> 798
   <211> 57
   <212> DNA
   <213> artificial
<220>
   <223> L3-4NHT (IL1B)
<220>
   <221> misc_feature
   <222> (31)..(31)
   <223> n is a, c, g, or t
<400> 798
   gcaacttact attgtcagca ggctgacagt nhtccgctca ctttcggcgg agggacc 57
<210> 799
   <211> 57
   <212> DNA
   <213> artificial
<220>
   <223> L3-4VAA (IL1B)
<400> 799
   gcaacttact attgtcagca ggctgacagt vaaccgctca ctttcggcgg agggacc 57
<210> 800
   <211> 57
   <212> DNA
   <213> artificial
<220>
   <223> L3-4BGG (IL1B)
<400> 800
   gcaacttact attgtcagca ggctgacagt bggccgctca ctttcggcgg agggacc 57
<210> 801
   <211> 57
   <212> DNA
   <213> artificial
<220>
   <223> L3-5NHT (IL1B)
<220>
   <221> misc_feature
   <222> (34)..(34)
   <223> n is a, c, g, or t
<400> 801
   gcaacttact attgtcagca ggctgacagt ttcnhtctca ctttcggcgg agggacc 57
<210> 802
   <211> 57
   <212> DNA
   <213> artificial
<220>
   <223> L3-5VAA (IL1B)
<400> 802
   gcaacttact attgtcagca ggctgacagt ttcvaactca ctttcggcgg agggacc 57
<210> 803
   <211> 57
   <212> DNA
   <213> artificial
<220>
   <223> L3-5BGG (IL1B)
<400> 803
   gcaacttact attgtcagca ggctgacagt ttcbggctca ctttcggcgg agggacc 57
<210> 804
   <211> 57
   <212> DNA
   <213> artificial
<220>
   <223> L3-6NHT (IL1B)
<220>
   <221> misc_feature
   <222> (37)..(37)
   <223> n is a, c, g, or t
<400> 804
   gcaacttact attgtcagca ggctgacagt ttcccgnhta ctttcggcgg agggacc 57
<210> 805
   <211> 57
   <212> DNA
   <213> artificial
<220>
   <223> L3-6VAA (IL1B)
<400> 805
   gcaacttact attgtcagca ggctgacagt ttcccgvaaa ctttcggcgg agggacc 57
<210> 806
   <211> 57
   <212> DNA
   <213> artificial
<220>
   <223> L3-6BGG (IL1B)
<400> 806
   gcaacttact attgtcagca ggctgacagt ttcccgbgga ctttcggcgg agggacc 57
<210> 807
   <211> 59
   <212> DNA
   <213> artificial
<220>
   <223> L3-E98T
<400> 807
   ggtgtttatt actgtgctca aaatctaact cttcctcgga cgttcggtgg aggcaccaa 59
<210> 808
   <211> 56
   <212> DNA
   <213> artificial
<220>
   <223> L2-Q55R
<400> 808
   cagtctcctc agctcctgat ttatcgaatg tccaaccttg cctcaggagt cccaga 56
<210> 809
   <211> 59
   <212> DNA
   <213> artificial
<220>
   <223> H1-G33F
<400> 809
   tcctgcaagg cttctggata taccttcaca aaatatttca tgaactgggt gaagcaggc 59
<210> 810
   <211> 58
   <212> DNA
   <213> artificial
<220>
   <223> H2-T53I
<400> 810
   aagggtttaa agtggatggg ctggataaac atctacactg aagagcctac atatggtg 58
<210> 811
   <211> 53
   <212> DNA
   <213> artificial
<220>
   <223> H3-G100R
<400> 811
   gctacatatt tctgtgcaag atttcgttct gctgtggact actggggtca agg 53
<210> 812
   <211> 40
   <212> DNA
   <213> artificial
<220>
   <223> AscIR
<400> 812
   atatatggcg cgccttatta acactctccc ctgttgaagc 40
<210> 813
   <211> 21
   <212> DNA
   <213> artificial
<220>
   <223> AscIF
<400> 813
   taataaggcg cgcctaacca t 21
<210> 814
   <211> 20
   <212> DNA
   <213> artificial
<220>
   <223> HindIII-F
<400> 814
   ttacgccaag ctttggagcc 20
<210> 815
   <211> 112
   <212> PRT
   <213> artificial
<220>
   <223> murING1 (light chain)
<400> 815
   Asp Ile Val Met Thr Gln Ala Ala Phe Ser Asn Pro Val Thr Leu Gly
   1               5                   10                  15
   Thr Ser Gly Ser Ile Ser Cys Arg Ser Ser Lys Ser Leu Leu His Ser
               20                  25                  30
   Asn Gly Ile Thr Tyr Leu Tyr Trp Tyr Leu Gln Lys Pro Gly Gln Ser
           35                  40                  45
   Pro Gln Leu Leu Ile Tyr Gln Met Ser Asn Leu Ala Ser Gly Val Pro
       50                  55                  60
   Asp Arg Phe Ser Ser Ser Gly Ser Gly Thr Asp Phe Thr Leu Arg Ile
   65                  70                  75                  80
   Ser Arg Val Glu Ala Glu Asp Val Gly Val Tyr Tyr Cys Ala Gln Asn
                   85                  90                  95
   Leu Glu Leu Pro Arg Thr Phe Gly Gly Gly Thr Lys Leu Glu Ile Lys
               100                 105                 110
<210> 816
   <211> 115
   <212> PRT
   <213> artificial
<220>
   <223> mK2 (light chain)
<400> 816
   Asp Val Val Met Thr Gln Thr Pro Leu Ser Leu Pro Val Ser Leu Gly
   1               5                   10                  15
   Asp Gln Ala Ser Ile Ser Cys Arg Ser Ser Gln Ser Leu Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Thr His Val Pro Pro Tyr Thr Phe Gly Gly Gly Thr Lys Leu Glu Ile
               100                 105                 110
   Lys Arg Ala
           115
<210> 817
   <211> 116
   <212> PRT
   <213> artificial
<220>
   <223> murING1 (heavy chain)
<400> 817
   Gln Ile Gln Leu Val Gln Ser Gly Pro Glu Leu Lys Lys Pro Gly Glu
   1               5                   10                  15
   Thr Val Lys Ile Ser Cys Lys Ala Ser Gly Tyr Thr Phe Thr Lys Tyr
               20                  25                  30
   Gly Met Asn Trp Val Lys Gln Ala Pro Gly Lys Gly Leu Lys Trp Met
           35                  40                  45
   Gly Trp Ile Asn Thr Tyr Thr Glu Glu Pro Thr Tyr Gly Asp Asp Phe
       50                  55                  60
   Lys Gly Arg Phe Ala Phe Ser Leu Glu Thr Ser Ala Ser Thr Ala Asn
   65                  70                  75                  80
   Leu Gln Ile Asn Asn Leu Lys Ser Glu Asp Thr Ala Thr Tyr Phe Cys
                   85                  90                  95
   Ala Arg Phe Gly Ser Ala Val Asp Tyr Trp Gly Gln Gly Thr Ser Val
               100                 105                 110
   Thr Val Ser Ser
           115
<210> 818
   <211> 129
   <212> PRT
   <213> artificial
<220>
   <223> mH2a (heavy chain)
<220>
   <221> misc_feature
   <222> (100)..(101)
   <223> Xaa can be any naturally occurring amino acid
<220>
   <221> misc_feature
   <222> (107)..(107)
   <223> Xaa can be any naturally occurring amino acid
<220>
   <221> misc_feature
   <222> (109)..(109)
   <223> Xaa can be any naturally occurring amino acid
<220>
   <221> misc_feature
   <222> (111)..(112)
   <223> Xaa can be any naturally occurring amino acid
<400> 818
   Glu Val Gln Leu Gln Gln Ser Gly Pro Glu Leu Val Lys Pro Gly Ala
   1               5                   10                  15
   Ser Val Lys Ile Ser Cys Lys Ala Ser Gly Tyr Thr Phe Thr Asp Tyr
               20                  25                  30
   Tyr Met Asn Asn Trp Val Lys Gln Ser Pro Gly Lys Ser Leu Glu Trp
           35                  40                  45
   Ile Gly Asp Ile Asn Pro Gly Asn Gly Gly Thr Ser Tyr Asn Gln Lys
       50                  55                  60
   Phe Lys Gly Lys Ala Thr Leu Thr Val Asp Lys Ser Ser Ser Thr Ala
   65                  70                  75                  80
   Tyr Met Gln Leu Ser Ser Leu Thr Ser Glu Asp Ser Ala Val Tyr Tyr
                   85                  90                  95
   Cys Ala Arg Xaa Xaa Tyr Tyr Ser Ser Ser Xaa Met Xaa Ala Xaa Xaa
               100                 105                 110
   Tyr Tyr Ala Phe Asp Tyr Trp Gly Gln Gly Thr Thr Val Thr Val Ser
           115                 120                 125
   Ser
<210> 819
   <211> 113
   <212> PRT
   <213> artificial
<220>
   <223> hK1
<400> 819
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Leu Val Ser Ile
               20                  25                  30
   Ser Asn Tyr Leu Ala Trp Tyr Gln Gln Lys Pro Gly Lys Ala Pro Lys
           35                  40                  45
   Leu Leu Ile Tyr Ala Ala Ser Ser Leu Glu Ser Gly Val Pro Ser Arg
       50                  55                  60
   Phe Ser Gly Ser Gly Ser Gly Thr Asp Phe Thr Leu Thr Ile Ser Ser
   65                  70                  75                  80
   Leu Gln Pro Glu Asp Phe Ala Thr Tyr Tyr Cys Gln Gln Tyr Asn Ser
                   85                  90                  95
   Leu Pro Glu Trp Thr Phe Gly Gln Gly Thr Lys Val Glu Ile Lys Arg
               100                 105                 110
   Thr
<210> 820
   <211> 113
   <212> PRT
   <213> artificial
<220>
   <223> hK2
<400> 820
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asp Gly Asn Asn Tyr Leu Asn Trp Tyr Leu Gln Lys Pro Gly Gln Ser
           35                  40                  45
   Pro Gln Leu Leu Ile Tyr Leu Val Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Pro Arg Thr Phe Gly Gln Gly Thr Lys Val Glu Ile Lys Arg
               100                 105                 110
   Thr
<210> 821
   <211> 111
   <212> PRT
   <213> artificial
<220>
   <223> hK3
<400> 821
   Glu Ile Val Leu Thr Gln Ser Pro Gly Thr Leu Ser Leu Ser Pro Gly
   1               5                   10                  15
   Glu Arg Ala Thr Leu Ser Cys Arg Ala Ser Gln Ser Val Ser Ser Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Gly Ser Ser Pro
                   85                  90                  95
   Pro Leu Thr Phe Gly Gln Gly Thr Lys Val Glu Ile Lys Arg Thr
               100                 105                 110
<210> 822
   <211> 114
   <212> PRT
   <213> artificial
<220>
   <223> hK4
<400> 822
   Asp Ile Val Met Thr Gln Ser Pro Asp Ser Leu Ala Val Ser Leu Gly
   1               5                   10                  15
   Glu Arg Ala Thr Ile Asn Cys Lys Ser Ser Gln Ser Val Leu Tyr Ser
               20                  25                  30
   Ser Asn Asn Lys Asn Tyr Leu Ala Trp Tyr Gln Gln Lys Pro Gly Gln
           35                  40                  45
   Pro Pro Lys Leu Leu Ile Tyr Trp Ala Ser Thr Arg Glu Ser Gly Val
   50 55 60
   Pro Asp Arg Phe Ser Gly Ser Gly Ser Gly Thr Asp Phe Thr Leu Thr
   65 70 75 80
   Ile Ser Ser Leu Gln Ala Glu Asp Val Ala Val Tyr Tyr Cys Gln Gln
   85 90 95
   Tyr Tyr Ser Thr Pro Thr Phe Gly Gln Gly Thr Lys Val Glu Ile Lys
   100 105 110
   Arg Thr
<210> 823
   <211> 115
   <212> PRT
   <213> artificial
<220>
   <223> mK1
<400> 823
   Asp Ile Val Met Thr Gln Ser Pro Ser Ser Leu Ala Val Ser Ala Gly
   1 5 10 15
   Glu Lys Val Thr Met Ser Cys Lys Ser Ser Gln Ser Leu Leu Asn Ser
   20 25 30
   Gly Asn Gln Lys Asn Tyr Leu Ala Trp Tyr Gln Gln Lys Pro Gly Gln
   35 40 45
   Ser Pro Lys Leu Leu Ile Tyr Trp Ala Ser Thr Arg Glu Ser Gly Val
   50 55 60
   Pro Asp Arg Phe Thr Gly Ser Gly Ser Gly Thr Asp Phe Thr Leu Thr
   65 70 75 80
   Ile Ser Ser Val Gln Ala Glu Asp Leu Ala Val Tyr Tyr Cys Gln Asn
   85 90 95
   Asp Tyr Ser Tyr Pro Leu Thr Phe Gly Ala Gly Thr Lys Leu Glu Leu
   100 105 110
   Lys Arg Ala
   115
<210> 824
   <211> 115
   <212> PRT
   <213> artificial
<220>
   <223> mK2
<400> 824
   Asp Val Val Met Thr Gln Thr Pro Leu Ser Leu Pro Val Ser Leu Gly
   1               5                   10                  15
   Asp Gln Ala Ser Ile Ser Cys Arg Ser Ser Gln Ser Leu Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Thr His Val Pro Pro Tyr Thr Phe Gly Gly Gly Thr Lys Leu Glu Ile
               100                 105                 110
   Lys Arg Ala
           115
<210> 825
   <211> 114
   <212> PRT
   <213> artificial
<220>
   <223> mK3
<400> 825
   Asp Ile Val Leu Thr Gln Ser Pro Ala Ser Leu Ala Val Ser Leu Gly
   1               5                   10                  15
   Gln Arg Ala Thr Ile Ser Cys Arg Ala Ser Glu Ser Val Asp Ser Tyr
               20                  25                  30
   Gly Asn Ser Phe Met His Trp Tyr Gln Gln Lys Pro Gly Gln Pro Pro
           35                  40                  45
   Lys Leu Leu Ile Tyr Ala Ala Ser Asn Leu Glu Ser Gly Val Pro Ala
       50                  55                  60
   Arg Phe Ser Gly Ser Gly Ser Gly Thr Asp Phe Thr Leu Asn Ile His
   65                  70                  75                  80
   Pro Val Glu Glu Asp Asp Ala Ala Thr Tyr Tyr Cys Gln Gln Ser Asn
                   85                  90                  95
   Glu Asp Pro Pro Trp Thr Phe Gly Gly Gly Thr Lys Leu Glu Ile Lys
               100                 105                 110
   Arg Ala
<210> 826
   <211> 112
   <212> PRT
   <213> artificial
<220>
   <223> mK4
<220>
   <221> misc_feature
   <222> (43)..(43)
   <223> Xaa can be any naturally occurring amino acid
<220>
   <221> misc_feature
   <222> (97)..(97)
   <223> Xaa can be any naturally occurring amino acid
<400> 826
   Glu Ile Val Leu Thr Gln Ser Pro Ala Ile Met Ser Ala Ser Pro Gly
   1 5 10 15
   Glu Lys Val Thr Met Thr Cys Ser Ala Ser Ser Ser Val Ser Ser Ser
   20 25 30
   Tyr Leu His Trp Tyr Gln Gln Lys Pro Gly Xaa Ser Pro Lys Leu Trp
   35 40 45
   Ile Tyr Arg Thr Ser Asn Leu Ala Ser Gly Val Pro Ala Arg Phe Ser
   50 55 60
   Gly Ser Gly Ser Gly Thr Ser Tyr Ser Leu Thr Ile Ser Ser Met Glu
   65 70 75 80
   Ala Glu Asp Ala Ala Thr Tyr Tyr Cys Gln Gln Trp Ser Ser Tyr Pro
   85 90 95
   Xaa Gly Leu Thr Phe Gly Ala Gly Thr Lys Leu Glu Ile Lys Arg Ala
   100 105 110
<210> 827
   <211> 111
   <212> PRT
   <213> artificial
<220>
   <223> mK5
<400> 827
   Asp Ile Gln Met Thr Gln Ser Pro Ser Ser Leu Ser Ala Ser Leu Gly
   1               5                   10                  15
   Asp Arg Val Thr Ile Thr Cys Arg Ala Ser Gln Asp Asp Ile Ser Asn
               20                  25                  30
   Tyr Leu Asn Trp Tyr Gln Gln Lys Pro Gly Gly Ser Pro Lys Leu Leu
           35                  40                  45
   Ile Tyr Tyr Ala Ser Arg Leu His Ser Gly Val Pro Ser Arg Phe Ser
       50                  55                  60
   Gly Ser Gly Ser Gly Thr Asp Tyr Ser Leu Thr Ile Ser Asn Leu Glu
   65                  70                  75                  80
   Gln Glu Asp Ile Ala Thr Tyr Phe Cys Gln Gln Gly Asn Thr Leu Pro
                   85                  90                  95
   Pro Arg Thr Phe Gly Gly Gly Thr Lys Leu Glu Ile Lys Arg Ala
               100                 105                 110
<210> 828
   <211> 111
   <212> PRT
   <213> artificial
<220>
   <223> mK6
<400> 828
   Gln Ile Val Leu Thr Gln Ser Pro Ala Ile Met Ser Ala Ser Pro Gly
   1 5 10 15
   Glu Lys Val Thr Met Thr Cys Ser Ala Ser Ser Ser Val Ser Tyr Met
   20 25 30
   His Trp Tyr Gln Gln Lys Ser Gly Thr Ser Pro Lys Arg Trp Ile Tyr
   35 40 45
   Asp Thr Ser Lys Leu Ala Ser Gly Val Pro Ala Arg Phe Ser Gly Ser
   50 55 60
   Gly Ser Gly Thr Ser Tyr Ser Leu Thr Ile Ser Ser Met Glu Ala Glu
   65 70 75 80
   Asp Ala Ala Thr Tyr Tyr Cys Gln Gln Trp Ser Ser Asn Pro Pro Met
   85 90 95
   Pro Leu Thr Phe Gly Ala Gly Thr Lys Leu Glu Leu Lys Arg Ala
   100 105 110
<210> 829
   <211> 118
   <212> PRT
   <213> artificial
<220>
   <223> hL1
<220>
   <221> misc_feature
   <222> (78)..(78)
   <223> Xaa can be any naturally occurring amino acid
<220>
   <221> misc_feature
   <222> (100)..(100)
   <223> Xaa can be any naturally occurring amino acid
<400> 829
   Glx Ser Val Leu Thr Gln Pro Pro Ser Val Ser Gly Ala Pro Gly Gln
   1 5 10 15
   Arg Val Thr Ile Ser Cys Ser Gly Ser Ser Ser Asn Ile Ile Gly Asn
   20 25 30
   Asn Tyr Val Ser Trp Tyr Gln Gln Leu Pro Gly Thr Ala Pro Lys Leu
   35 40 45
   Leu Ile Tyr Asp Asn Asn Lys Arg Pro Ser Gly Val Pro Asp Arg Phe
       50                  55                  60
   Ser Gly Ser Lys Ser Gly Thr Ser Ala Ser Leu Ala Ile Xaa Gly Leu
   65                  70                  75                  80
   Gln Ser Glu Asp Glu Ala Asp Tyr Tyr Cys Ala Thr Trp Asp Asp Ser
                   85                  90                  95
   Leu Ser Ala Xaa Asn Ser Ala Pro Val Phe Gly Gly Gly Thr Lys Leu
               100                 105                 110
   Thr Val Leu Gly Gln Pro
           115
<210> 830
   <211> 114
   <212> PRT
   <213> artificial
<220>
   <223> hL2
<220>
   <221> misc_feature
   <222> (98)..(99)
   <223> Xaa can be any naturally occurring amino acid
<220>
   <221> misc_feature
   <222> (108)..(108)
   <223> Xaa can be any naturally occurring amino acid
<400> 830
   Glx Ser Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Gly Tyr
               20                  25                  30
   Asn Ala Val Ser Trp Tyr Gln Gln His Pro Gly Lys Ala Pro Lys Leu
           35                  40                  45
   Leu Ile Tyr Asp Val Thr Asp Arg Pro Ser Gly Val Pro Asp Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Ser Tyr Gly Gly Gly
   85 90 95
   Ser Xaa Xaa Asn Val Phe Gly Gly Gly Thr Lys Xaa Thr Val Leu Gly
   100 105 110
   Gln Pro
<210> 831
   <211> 113
   <212> PRT
   <213> artificial
<220>
   <223> hL3
<220>
   <221> misc_feature
   <222> (51)..(51)
   <223> Xaa can be any naturally occurring amino acid
<400> 831
   Ser Tyr Glu Leu Thr Gln Pro Pro Ser Val Ser Val Ser Pro Gly Gln
   1 5 10 15
   Thr Ala Arg Ile Thr Cys Ser Gly Asp Asn Leu Gly Asp Lys Tyr Val
   20 25 30
   His Trp Tyr Gln Gln Lys Pro Gly Gln Ala Pro Val Leu Val Ile Tyr
   35 40 45
   Asp Asp Xaa Lys Arg Pro Ser Gly Ile Pro Glu Arg Phe Ser Gly Ser
   50 55 60
   Asn Ser Gly Asn Thr Ala Thr Leu Thr Ile Ser Gly Val Gln Ala Gly
   65 70 75 80
   Asp Glu Ala Asp Tyr Tyr Cys Gln Ala Trp Asp Ser Ser Ser Asp His
   85 90 95
   Pro Gly Val Val Phe Gly Gly Gly Thr Lys Leu Thr Val Leu Gly Gln
   100 105 110
   Pro
<210> 832
   <211> 112
   <212> PRT
   <213> artificial
<220>
   <223> hL4
<220>
   <221> misc_feature
   <222> (9)..(9)
   <223> Xaa can be any naturally occurring amino acid
<220>
   <221> misc_feature
   <222> (14)..(14)
   <223> Xaa can be any naturally occurring amino acid
<220>
   <221> misc_feature
   <222> (23)..(23)
   <223> Xaa can be any naturally occurring amino acid
<220>
   <221> misc_feature
   <222> (30)..(30)
   <223> Xaa can be any naturally occurring amino acid
<220>
   <221> misc_feature
   <222> (41)..(41)
   <223> Xaa can be any naturally occurring amino acid
<220>
   <221> misc_feature
   <222> (45)..(45)
   <223> Xaa can be any naturally occurring amino acid
<220>
   <221> misc_feature
   <222> (51)..(51)
   <223> Xaa can be any naturally occurring amino acid
<220>
   <221> misc_feature
   <222> (69)..(69)
   <223> Xaa can be any naturally occurring amino acid
<220>
   <221> misc_feature
   <222> (96)..(99)
   <223> Xaa can be any naturally occurring amino acid
<400> 832
   Ser Glu Leu Thr Gln Pro Pro Ser Xaa Val Ser Val Ala Xaa Gly Gln
   1               5                   10                  15
   Thr Val Arg Ile Thr Cys Xaa Gly Asp Ser Asn Leu Gly Xaa Tyr Asp
   20 25 30
   Ala Ser Trp Tyr Gln Gln Lys Pro Xaa Gln Ala Pro Xaa Leu Val Ile
   35 40 45
   Tyr Gly Xaa Asn Asn Arg Pro Ser Gly Ile Pro Asp Arg Phe Ser Gly
   50 55 60
   Ser Ser Ser Gly Xaa Thr Ala Ser Leu Thr Ile Thr Gly Ala Gln Ala
   65 70 75 80
   Glu Asp Glu Ala Asp Tyr Tyr Cys Asn Ser Arg Asp Ser Ser Gly Xaa
   85 90 95
   Xaa Xaa Xaa Phe Gly Gly Gly Thr Lys Leu Thr Val Leu Gly Gln Pro
   100 105 110
<210> 833
   <211> 113
   <212> PRT
   <213> artificial
<220>
   <223> hL5
<220>
   <221> misc_feature
   <222> (1)..(1)
   <223> Xaa can be any naturally occurring amino acid
<400> 833
   Xaa Ser Ala Leu Thr Gln Pro Pro Ser Ala Ser Gly Ser Pro Gly Gln
   1 5 10 15
   Ser Val Thr Ile Ser Cys Thr Gly Thr Ser Ser Asp Val Gly Gly Tyr
   20 25 30
   Asn Tyr Val Ser Trp Tyr Gln Gln His Ala Gly Lys Ala Pro Lys Val
   35 40 45
   Ile Ile Tyr Glu Val Asn Lys Arg Pro Ser Gly Val Pro Asp Arg Phe
   50 55 60
   Ser Gly Ser Lys Ser Gly Asn Thr Ala Ser Leu Thr Val Ser Gly Leu
   65 70 75 80
   Gln Ala Glu Asp Glu Ala Asp Tyr Tyr Cys Ser Ser Tyr Glu Gly Ser
   85 90 95
   Asp Asn Phe Val Phe Gly Thr Gly Thr Lys Val Thr Val Leu Gly Gln
   100 105 110
   Pro
<210> 834
   <211> 111
   <212> PRT
   <213> artificial
<220>
   <223> hL6
<400> 834
   Asn Phe Met Leu Thr Gln Pro His Ser Val Ser Glu Ser Pro Gly Lys
   1 5 10 15
   Thr Val Thr Ile Ser Cys Thr Gly Ser Ser Gly Ser Ile Ala Ser Asn
   20 25 30
   Tyr Val Gln Trp Tyr Gln Gln Arg Pro Gly Ser Ala Pro Thr Thr Val
   35 40 45
   Ile Tyr Glu Asp Asn Gln Arg Pro Ser Gly Val Pro Asp Arg Phe Ser
   50 55 60
   Gly Ser Ser Ser Asn Ser Ala Ser Leu Thr Ile Ser Gly Leu Lys Thr
   65 70 75 80
   Glu Asp Glu Ala Asp Tyr Tyr Cys Gln Ser Tyr Asp Ser Asn Asn His
   85 90 95
   Trp Val Phe Gly Gly Gly Thr Lys Leu Thr Val Leu Gly Gln Pro
   100 105 110
<210> 835
   <211> 116
   <212> PRT
   <213> artificial
<220>
   <223> mL
<400> 835
   Gln Ala Val Val Thr Gln Glu Ser Ala Leu Thr Thr Ser Pro Gly Glu
   1 5 10 15
   Thr Val Thr Leu Thr Cys Arg Ser Ser Thr Gly Ala Val Thr Thr Ser
   20 25 30
   Asn Tyr Ala Asn Trp Val Gln Glu Lys Pro Asp His Leu Phe Thr Gly
   35 40 45
   Leu Ile Gly Gly Thr Asn Asn Arg Ala Pro Gly Val Pro Ala Arg Phe
   50 55 60
   Ser Gly Ser Leu Ile Gly Asp Lys Ala Ala Leu Thr Ile Thr Gly Ala
   65 70 75 80
   Gln Thr Glu Asp Glu Ala Ile Tyr Phe Cys Ala Leu Trp Tyr Ser Asn
   85 90 95
   His Glu Gln Phe Val Trp Val Phe Gly Gly Gly Thr Lys Leu Thr Val
   100 105 110
   Leu Gly Gln Pro
   115
<210> 836
   <211> 129
   <212> PRT
   <213> artificial
<220>
   <223> hH1
<220>
   <221> misc_feature
   <222> (115)..(115)
   <223> Xaa can be any naturally occurring amino acid
<400> 836
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Ser Tyr
   20 25 30
   Ala Ile Ser Trp Val Arg Gln Ala Pro Gly Gln Gly Leu Glu Trp Met
   35 40 45
   Gly Trp Ile Asn Pro Tyr Gly Asn Gly Asp Thr Asn Tyr Ala Gln Lys
   50 55 60
   Phe Gln Gly Arg Val Thr Ile Thr Ala Asp Thr Ser Thr Ser Thr Ala
   65 70 75 80
   Tyr Met Glu Leu Ser Ser Leu Arg Ser Glu Asp Thr Ala Val Tyr Tyr
   85 90 95
   Cys Ala Arg Ala Pro Gly Tyr Gly Ser Gly Gly Gly Cys Tyr Arg Gly
   100 105 110
   Asp Tyr Xaa Phe Asp Tyr Trp Gly Gln Gly Thr Leu Val Thr Val Ser
   115 120 125
   Ser
<210> 837
   <211> 132
   <212> PRT
   <213> artificial
<220>
   <223> hH2
<220>
   <221> misc_feature
   <222> (33)..(33)
   <223> Xaa can be any naturally occurring amino acid
<220>
   <221> misc_feature
   <222> (63)..(63)
   <223> Xaa can be any naturally occurring amino acid
<220>
   <221> misc_feature
   <222> (104)..(106)
   <223> Xaa can be any naturally occurring amino acid
<220>
   <221> misc_feature
   <222> (108)..(109)
   <223> Xaa can be any naturally occurring amino acid
<220>
   <221> misc_feature
   <222> (116)..(117)
   <223> Xaa can be any naturally occurring amino acid
<400> 837
   Gln Val Gln Leu Gln Glu Ser Gly Pro Gly Leu Val Lys Pro Ser Gln
   1 5 10 15
   Thr Leu Ser Leu Thr Cys Thr Val Ser Gly Gly Ser Val Ser Ser Tyr
   20 25 30
   Xaa Trp Ser Trp Asn Trp Ile Arg Gln Pro Pro Gly Lys Gly Leu Glu
   35 40 45
   Trp Ile Gly Arg Ile Tyr Tyr Arg Ala Tyr Ser Gly Ser Thr Xaa Tyr
   50 55 60
   Asn Pro Ser Leu Lys Ser Arg Val Thr Ile Ser Val Asp Thr Ser Lys
   65 70 75 80
   Asn Gln Phe Ser Leu Lys Leu Ser Ser Val Thr Ala Ala Asp Thr Ala
   85 90 95
   Val Tyr Tyr Cys Ala Arg Glu Xaa Xaa Xaa Gly Xaa Xaa Gly Asp Asp
   100 105 110
   Tyr Tyr Tyr Xaa Xaa Gly Phe Asp Val Trp Gly Gln Gly Thr Leu Val
   115 120 125
   Thr Val Ser Ser
   130
<210> 838
   <211> 130
   <212> PRT
   <213> artificial
<220>
   <223> hH3
<220>
   <221> misc_feature
   <222> (101)..(106)
   <223> Xaa can be any naturally occurring amino acid
<220>
   <221> misc_feature
   <222> (110)..(110)
   <223> Xaa can be any naturally occurring amino acid
<400> 838
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
                   35                  40                  45
   Ser Val Ile Ser Gly Lys Thr Asp Gly Gly Ser Thr Tyr Tyr Ala Asp
       50                  55                  60
   Ser Val Lys Gly Arg Phe Thr Ile Ser Arg Asp Asn Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Ala Arg Xaa Xaa Xaa Xaa Xaa Xaa Leu Ser Gly Xaa Tyr Tyr
               100                 105                 110
   Tyr Tyr His Tyr Phe Asp Tyr Trp Gly Gln Gly Thr Leu Val Thr Val
           115                 120                 125
   Ser Ser
       130
<210> 839
   <211> 130
   <212> PRT
   <213> artificial
<220>
   <223> mH1a
<220>
   <221> misc_feature
   <222> (41)..(41)
   <223> Xaa can be any naturally occurring amino acid
<220>
   <221> misc_feature
   <222> (55)..(55)
   <223> Xaa can be any naturally occurring amino acid
<220>
   <221> misc_feature
   <222> (101)..(102)
   <223> Xaa can be any naturally occurring amino acid
<220>
   <221> misc_feature
   <222> (106)..(107)
   <223> Xaa can be any naturally occurring amino acid
<220>
   <221> misc_feature
   <222> (109)..(109)
   <223> Xaa can be any naturally occurring amino acid
<220>
   <221> misc_feature
   <222> (112)..(112)
   <223> Xaa can be any naturally occurring amino acid
<400> 839
   Glu Val Gln Leu Gln Glu Ser Gly Pro Ser Leu Val Lys Pro Ser Gln
   1               5                   10                  15
   Thr Leu Ser Leu Thr Cys Ser Val Thr Gly Asp Ser Ile Thr Ser Gly
               20                  25                  30
   Tyr Trp Asn Asn Ser Trp Ile Arg Xaa Phe Pro Gly Asn Lys Leu Glu
           35                  40                  45
   Trp Met Gly Tyr Ile Ser Xaa Tyr Ser Gly Ser Thr Tyr Tyr Asn Pro
       50                  55                  60
   Ser Leu Lys Ser Arg Ile Ser Ile Thr Arg Asp Thr Ser Lys Asn Gln
   65                  70                  75                  80
   Tyr Phe Leu Gln Leu Asn Ser Val Thr Thr Glu Asp Thr Ala Thr Tyr
                   85                  90                  95
   Tyr Cys Ala Arg Xaa Xaa Tyr Gly Tyr Xaa Xaa Tyr Xaa Tyr Asp Xaa
               100                 105                 110
   Tyr Tyr Tyr Tyr Phe Asp Tyr Trp Gly Gln Gly Thr Thr Val Thr Val
           115                 120                 125
   Ser Ser
       130
<210> 840
   <211> 129
   <212> PRT
   <213> artificial
<220>
   <223> mH1b
<220>
   <221> misc_feature
   <222> (103)..(103)
   <223> Xaa can be any naturally occurring amino acid
<220>
   <221> misc_feature
   <222> (107)..(107)
   <223> Xaa can be any naturally occurring amino acid
<220>
   <221> misc_feature
   <222> (110)..(112)
   <223> Xaa can be any naturally occurring amino acid
<400> 840
   Gln Val Gln Leu Lys Glu Ser Gly Pro Gly Leu Val Ala Pro Ser Gln
   1               5                   10                  15
   Ser Leu Ser Ile Thr Cys Thr Val Ser Gly Phe Ser Leu Thr Ser Tyr
               20                  25                  30
   Gly Val His Val Ser Trp Val Arg Gln Pro Pro Gly Lys Gly Leu Glu
           35                  40                  45
   Trp Leu Gly Val Ile Trp Ala Gly Gly Ser Thr Asn Tyr Asn Ser Ala
       50                  55                  60
   Leu Met Ser Arg Leu Ser Ile Ser Lys Asp Asn Ser Lys Ser Gln Val
   65                  70                  75                  80
   Phe Leu Lys Met Asn Ser Leu Gln Thr Asp Asp Thr Ala Met Tyr Tyr
                   85                  90                  95
   Cys Ala Arg Asp Arg Gly Xaa Tyr Tyr Tyr Xaa Ser Gly Xaa Xaa Xaa
               100                 105                 110
   Tyr Tyr Ala Met Asp Tyr Trp Gly Gln Gly Thr Ser Val Thr Val Ser
           115                 120                 125
   Ser
<210> 841
   <211> 129
   <212> PRT
   <213> artificial
<220>
   <223> mH2a
<220>
   <221> misc_feature
   <222> (100)..(101)
   <223> Xaa can be any naturally occurring amino acid
<220>
   <221> misc_feature
   <222> (107)..(107)
   <223> Xaa can be any naturally occurring amino acid
<220>
   <221> misc_feature
   <222> (109)..(109)
   <223> Xaa can be any naturally occurring amino acid
<220>
   <221> misc_feature
   <222> (111)..(112)
   <223> Xaa can be any naturally occurring amino acid
<400> 841
   Glu Val Gln Leu Gln Gln Ser Gly Pro Glu Leu Val Lys Pro Gly Ala
   1               5                   10                  15
   Ser Val Lys Ile Ser Cys Lys Ala Ser Gly Tyr Thr Phe Thr Asp Tyr
               20                  25                  30
   Tyr Met Asn Asn Trp Val Lys Gln Ser Pro Gly Lys Ser Leu Glu Trp
           35                  40                  45
   Ile Gly Asp Ile Asn Pro Gly Asn Gly Gly Thr Ser Tyr Asn Gln Lys
       50                  55                  60
   Phe Lys Gly Lys Ala Thr Leu Thr Val Asp Lys Ser Ser Ser Thr Ala
   65                  70                  75                  80
   Tyr Met Gln Leu Ser Ser Leu Thr Ser Glu Asp Ser Ala Val Tyr Tyr
                   85                  90                  95
   Cys Ala Arg Xaa Xaa Tyr Tyr Ser Ser Ser Xaa Met Xaa Ala Xaa Xaa
               100                 105                 110
   Tyr Tyr Ala Phe Asp Tyr Trp Gly Gln Gly Thr Thr Val Thr Val Ser
           115                 120                 125
   Ser
<210> 842
   <211> 127
   <212> PRT
   <213> artificial
<220>
   <223> mH2b
<220>
   <221> misc_feature
   <222> (106)..(107)
   <223> Xaa can be any naturally occurring amino acid
<220>
   <221> misc_feature
   <222> (110)..(110)
   <223> Xaa can be any naturally occurring amino acid
<400> 842
   Gln Val Gln Leu Gln Gln Pro Gly Ala Glu Leu Val Lys Pro Gly Ala
   1               5                   10                  15
   Ser Val Lys Leu Ser Cys Lys Ala Ser Gly Tyr Thr Phe Thr Ser Tyr
               20                  25                  30
   Trp Met His Trp Val Lys Gln Arg Pro Gly Gln Gly Leu Glu Trp Ile
           35                  40                  45
   Gly Arg Ile Asp Pro Asn Ser Gly Gly Thr Asn Tyr Asn Glu Lys Phe
       50                  55                  60
   Lys Ser Lys Ala Thr Leu Thr Val Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Tyr Tyr Tyr Gly Gly Ser Ser Xaa Xaa Val Tyr Xaa Tyr Trp
               100                 105                 110
   Tyr Phe Asp Tyr Trp Gly Gln Gly Thr Thr Val Thr Val Ser Ser
           115                 120                 125
<210> 843
   <211> 125
   <212> PRT
   <213> artificial
<220>
   <223> mH2c
<220>
   <221> misc_feature
   <222> (106)..(106)
   <223> Xaa can be any naturally occurring amino acid
<220>
   <221> misc_feature
   <222> (120)..(120)
   <223> Xaa can be any naturally occurring amino acid
<400> 843
   Glu Val Gln Leu Gln Gln Ser Gly Ala Glu Leu Val Lys Pro Gly Ala
   1 5 10 15
   Ser Val Lys Leu Ser Cys Thr Ala Ser Gly Phe Asn Ile Lys Asp Thr
   20 25 30
   Tyr Met His Trp Val Lys Gln Arg Pro Glu Gln Gly Leu Glu Trp Ile
   35 40 45
   Gly Arg Ile Asp Pro Ala Asn Gly Asn Thr Lys Tyr Asp Pro Lys Phe
   50 55 60
   Gln Gly Lys Ala Thr Ile Thr Ala Asp Thr Ser Ser Asn Thr Ala Tyr
   65 70 75 80
   Leu Gln Leu Ser Ser Leu Thr Ser Glu Asp Thr Ala Val Tyr Tyr Cys
   85 90 95
   Ala Arg Gly Tyr Tyr Tyr Tyr Asp Ser Xaa Val Gly Tyr Tyr Ala Met
   100 105 110
   Asp Tyr Trp Gly Gln Gly Thr Xaa Val Thr Val Ser Ser
   115 120 125
<210> 844
   <211> 130
   <212> PRT
   <213> artificial
<220>
   <223> mH3a
<400> 844
   Glu Val Lys Leu Val Glu Ser Gly Gly Gly Leu Val Gln Pro Gly Gly
   1 5 10 15
   Ser Leu Arg Leu Ser Cys Ala Thr Ser Gly Phe Thr Phe Ser Asp Phe
   20 25 30
   Tyr Met Glu Trp Val Arg Gln Pro Pro Gly Lys Ala Leu Glu Trp Ile
   35 40 45
   Ala Ala Ser Arg Asn Lys Ala Asn Asp Tyr Thr Thr Glu Tyr Ser Ala
   50 55 60
   Ser Val Lys Gly Arg Phe Thr Val Ser Arg Asp Thr Ser Gln Ser Ile
   65 70 75 80
   Leu Tyr Leu Gln Met Asn Ala Leu Arg Ala Glu Asp Thr Ala Ile Tyr
   85 90 95
   Tyr Cys Ala Arg Asp Tyr Tyr Tyr Gly Ser Ser Tyr Tyr Glu Gly Pro
   100 105 110
   Val Tyr Trp Tyr Phe Asp Val Trp Gly Ala Gly Thr Thr Val Thr Val
   115 120 125
   Ser Ser
   130
<210> 845
   <211> 127
   <212> PRT
   <213> artificial
<220>
   <223> mH3b
<400> 845
   Glu Val Lys Leu Leu Glu Ser Gly Gly Gly Leu Val Gln Pro Gly Gly
   1 5 10 15
   Ser Leu Lys Leu Ser Cys Ala Ala Ser Gly Phe Asp Phe Ser Arg Tyr
   20 25 30
   Trp Met Ser Trp Val Arg Gln Ala Pro Gly Lys Gly Leu Glu Trp Ile
   35 40 45
   Gly Glu Ile Asn Pro Lys Ala Asp Ser Ser Thr Ile Asn Tyr Thr Pro
   50 55 60
   Ser Leu Lys Asp Lys Phe Ile Ile Ser Arg Asp Asn Ala Lys Asn Thr
   65 70 75 80
   Leu Tyr Leu Gln Met Ser Lys Val Arg Ser Glu Asp Thr Ala Leu Tyr
   85 90 95
   Tyr Cys Ala Arg Leu Gly Gly Tyr Gly Tyr Phe Gly Ser Ser Tyr Tyr
   100 105 110
   Ala Met Asp Tyr Trp Gly Gln Gly Thr Thr Val Thr Val Ser Ser
   115 120 125
<210> 846
   <211> 127
   <212> PRT
   <213> artificial
<220>
   <223> mH3c
<220>
   <221> misc_feature
   <222> (36)..(37)
   <223> Xaa can be any naturally occurring amino acid
<220>
   <221> misc_feature
   <222> (108)..(108)
   <223> Xaa can be any naturally occurring amino acid
<220>
   <221> misc_feature
   <222> (112)..(112)
   <223> Xaa can be any naturally occurring amino acid
<400> 846
   Glu Val Lys Leu Glu Glu Ser Gly Gly Gly Leu Val Gln Pro Gly Gly
   1               5                   10                  15
   Ser Met Lys Leu Ser Cys Val Ala Ser Gly Phe Thr Phe Ser Asn Tyr
               20                  25                  30
   Trp Met Asn Xaa Xaa Trp Val Arg Gln Ser Pro Glu Lys Gly Leu Glu
           35                  40                  45
   Trp Val Ala Glu Ile Arg Leu Lys Ser Asp Asn Tyr Ala Thr His Tyr
       50                  55                  60
   Ala Glu Ser Val Lys Gly Arg Phe Thr Ile Ser Arg Asp Asp Ser Lys
   65                  70                  75                  80
   Ser Ser Val Tyr Leu Gln Met Asn Asn Leu Arg Ala Glu Asp Thr Gly
                   85                  90                  95
   Ile Tyr Tyr Cys Thr Thr Gly Gly Tyr Gly Gly Xaa Arg Arg Ser Xaa
               100                 105                 110
   Trp Phe Ala Tyr Trp Gly Gln Gly Thr Leu Val Thr Val Ser Ser
           115                 120                 125
<210> 847
   <211> 129
   <212> PRT
   <213> artificial
<220>
   <223> mH3d
<220>
   <221> misc_feature
   <222> (102)..(102)
   <223> Xaa can be any naturally occurring amino acid
<220>
   <221> misc_feature
   <222> (106)..(107)
   <223> Xaa can be any naturally occurring amino acid
<400> 847
   Glu Val Gln Leu Val Glu Ser Gly Gly Gly Leu Val Lys Pro Gly Gly
   1               5                   10                  15
   Ser Leu Lys Leu Ser Cys Ala Ala Ser Gly Phe Thr Phe Ser Ser Tyr
               20                  25                  30
   Ala Met Ser Trp Val Arg Gln Thr Pro Glu Lys Arg Leu Glu Trp Val
           35                  40                  45
   Ala Thr Ile Ser Ser Lys Ser Gly Gly Gly Tyr Thr Tyr Tyr Pro Asp
       50                  55                  60
   Ser Val Lys Gly Arg Phe Thr Ile Ser Arg Asp Asn Ala Lys Asn Thr
   65                  70                  75                  80
   Leu Tyr Leu Gln Met Ser Ser Leu Arg Ser Glu Asp Thr Ala Met Tyr
                   85                  90                  95
   Tyr Cys Ala Arg Gly Xaa Tyr Tyr Tyr Xaa Xaa Gly Ser Ala Pro Phe
               100                 105                 110
   Asp Tyr Ala Met Asp Tyr Trp Gly Gln Gly Thr Ser Val Thr Val Ser
           115                 120                 125
   Ser
<210> 848
   <211> 128
   <212> PRT
   <213> artificial
<220>
   <223> mH5a
<220>
   <221> misc_feature
   <222> (100)..(100)
   <223> Xaa can be any naturally occurring amino acid
<220>
   <221> misc_feature
   <222> (109)..(109)
   <223> Xaa can be any naturally occurring amino acid
<400> 848
   Glu Val Gln Leu Gln Gln Ser Gly Ala Glu Leu Val Arg Ala Gly Ser
   1               5                   10                  15
   Ser Val Lys Met Ser Cys Lys Ala Ser Gly Tyr Thr Phe Thr Ser Tyr
               20                  25                  30
   Gly Ile Asn Trp Val Lys Gln Arg Pro Gly Gln Gly Leu Glu Trp Ile
           35                  40                  45
   Gly Tyr Ile Asn Pro Gly Asn Gly Tyr Thr Lys Tyr Asn Glu Lys Phe
       50                  55                  60
   Lys Gly Lys Thr Thr Leu Thr Val Asp Lys Ser Ser Ser Thr Ala Tyr
   65                  70                  75                  80
   Met Gln Leu Arg Ser Leu Thr Ser Glu Asp Ser Ala Val Tyr Phe Cys
                   85                  90                  95
   Ala Arg Ser Xaa Tyr Tyr Gly Gly Ser Tyr Tyr Tyr Xaa Phe Ala Tyr
               100                 105                 110
   Tyr Tyr Phe Asp Tyr Trp Gly Gln Gly Thr Thr Leu Thr Val Ser Ser
           115                 120                 125
<210> 849
   <211> 114
   <212> PRT
   <213> artificial
<220>
   <223> mH5b
<220>
   <221> misc_feature
   <222> (47)..(48)
   <223> Xaa can be any naturally occurring amino acid
<400> 849
   Glu Val Gln Leu Gln Gln Ser Gly Ala Glu Leu Val Lys Ala Gly Ser
   1               5                   10                  15
   Ser Val Lys Met Ser Cys Ser Ala Thr Gly Tyr Thr Phe Ser Ser Tyr
               20                  25                  30
   Gly Leu Tyr Trp Val Arg Gln Ala Pro Gly Gln Gly Leu Glu Xaa Xaa
           35                  40                  45
   Gly Tyr Ile Ser Ser Ser Ser Ala Tyr Pro Asn Tyr Ala Gln Lys Phe
       50                  55                  60
   Gln Gly Arg Val Thr Ile Thr Ala Asp Glu Ser Thr Asn Thr Ala Tyr
   65                  70                  75                  80
   Met Glu Leu Ser Ser Leu Arg Ser Glu Asp Thr Ala Val Tyr Phe Cys
                   85                  90                  95
   Ala Val Arg Val Ile Ser Arg Tyr Phe Asp Gly Trp Gly Gln Gly Thr
               100                 105                 110
   Leu Val
<210> 850
   <211> 132
   <212> PRT
   <213> artificial
<220>
   <223> mHms
<220>
   <221> misc_feature
   <222> (1)..(1)
   <223> Xaa can be any naturally occurring amino acid
<220>
   <221> misc_feature
   <222> (68)..(68)
   <223> Xaa can be any naturally occurring amino acid
<220>
   <221> misc_feature
   <222> (103)..(103)
   <223> Xaa can be any naturally occurring amino acid
<220>
   <221> misc_feature
   <222> (112)..(114)
   <223> Xaa can be any naturally occurring amino acid
<400> 850
   Xaa Val Gln Leu Val Glu Ser Gly Gly Gly Leu Val Lys Pro Gly Gly
   1               5                   10                  15
   Ser Val Lys Leu Ser Cys Lys Ala Ser Gly Phe Thr Phe Ser Ser Phe
               20                  25                  30
   Gly Met Ser Asn Phe Trp Val Arg Gln Ala Pro Gly Lys Gly Leu Glu
           35                  40                  45
   Trp Val Gly Trp Ile Asn Ser Lys Leu Gly Gly Gly Ala Ile Tyr Tyr
       50                  55                  60
   Ala Asp Thr Xaa Lys Gly Arg Phe Thr Ile Ser Arg Asp Asn Ser Lys
   65                  70                  75                  80
   Ser Thr Leu Tyr Leu Gln Met Ser Ser Leu Arg Ser Glu Asp Thr Ala
                   85                  90                  95
   Thr Tyr Tyr Cys Ala Arg Xaa Gly Tyr Tyr Gly Gly Arg Arg Ser Xaa
               100                 105                 110
   Xaa Xaa Ser Tyr Trp Tyr Phe Asp Tyr Trp Gly Gln Gly Thr Thr Val
           115                 120                 125
   Thr Val Ser Ser
       130
<210> 851
   <211> 63
   <212> DNA
   <213> artificial
<220>
   <223> XPA CDR1 coding sequence
<400> 851
   tctttcttgc gctgcttccg gattcacttt ctctaagtac tttatgtttt gggttcgcca     60
   agc                                                                   63
<210> 852
   <211> 63
   <212> DNA
   <213> artificial
<220>
   <223> XPA CDR1 coding sequence (antisense)
<400> 852
   agaaagaacg cgacgaaggc ctaagtgaaa gagattcatg aaatacaaaa cccaagcggt 60
   tcg 63
<210> 853
   <211> 47
   <212> DNA
   <213> artificial
<220>
   <223> primer 1 for mutation of G position in XPA CDR1
<220>
   <221> misc_feature
   <222> (21)..(21)
   <223> n is a, c, g, or t
<400> 853
   gtctttcttg cgctgcttcc nhtttcactt tctctaagta ctttatg 47
<210> 854
   <211> 47
   <212> DNA
   <213> artificial
<220>
   <223> primer 1 for mutation of G position in XPA CDR1 (antisense)
<220>
   <221> misc_feature
   <222> (21)..(21)
   <223> n is a, c, g, or t
<400> 854
   cagaaagaac gcgacgaagg ndaaagtgaa agagattcat gaaatac 47
<210> 855
   <211> 47
   <212> DNA
   <213> artificial
<220>
   <223> primer 2 for mutation of G position in XPA CDR1
<400> 855
   gtctttcttg cgctgcttcc vaattcactt tctctaagta ctttatg 47
<210> 856
   <211> 47
   <212> DNA
   <213> artificial
<220>
   <223> primer 2 for mutation of G position in XPA CDR1(antisense)
<400> 856
   cagaaagaac gcgacgaagg bttaagtgaa agagattcat gaaatac 47
<210> 857
   <211> 47
   <212> DNA
   <213> artificial
<220>
   <223> primer 3 for mutation of G position in XPA CDR1
<400> 857
   gtctttcttg cgctgcttcc bggttcactt tctctaagta ctttatg 47
<210> 858
   <211> 47
   <212> DNA
   <213> artificial
<220>
   <223> primer 3 for mutation of G position in XPA CDR1 (antisense)
<400> 858
   cagaaagaac gcgacgaagg vccaagtgaa agagattcat gaaatac 47
<210> 859
   <211> 45
   <212> DNA
   <213> artificial
<220>
   <223> primer 1 for mutation of F position in XPA CDR1
<220>
   <221> misc_feature
   <222> (22)..(22)
   <223> n is a, c, g, or t
<400> 859
   ctttcttgcg ctgcttccgg anhtactttc tctaagtact ttatg 45
<210> 860
   <211> 45
   <212> DNA
   <213> artificial
<220>
   <223> primer 1 for mutation of F position in XPA CDR1 (antisense)
<220>
   <221> misc_feature
   <222> (22)..(22)
   <223> n is a, c, g, or t
<400> 860
   gaaagaacgc gacgaaggcc tndatgaaag agattcatga aatac 45
<210> 861
   <211> 45
   <212> DNA
   <213> artificial
<220>
   <223> primer 2 for mutation of F position in XPA CDR1
<400> 861
   ctttcttgcg ctgcttccgg avaaactttc tctaagtact ttatg 45
<210> 862
   <211> 45
   <212> DNA
   <213> artificial
<220>
   <223> primer 2 for mutation of F position in XPA CDR1 (antisense)
<400> 862
   gaaagaacgc gacgaaggcc tbtttgaaag agattcatga aatac 45
<210> 863
   <211> 45
   <212> DNA
   <213> artificial
<220>
   <223> primer 3 for mutation of F position in XPA CDR1
<400> 863
   ctttcttgcg ctgcttccgg abggactttc tctaagtact ttatg 45
<210> 864
   <211> 45
   <212> DNA
   <213> artificial
<220>
   <223> primer 3 for mutation of F position in XPA CDR1 (antisense)
<400> 864
   gaaagaacgc gacgaaggcc tvcctgaaag agattcatga aatac 45
<210> 865
   <211> 46
   <212> DNA
   <213> artificial
<220>
   <223> primer 1 for mutation of T position in XPA CDR1
<220>
   <221> misc_feature
   <222> (21)..(21)
   <223> n is a, c, g, or t
<400> 865
   cttgcgctgc ttccggattc nhtttctcta agtactttat gttttg 46
<210> 866
   <211> 46
   <212> DNA
   <213> artificial
<220>
   <223> primer 1 for mutation of T position in XPA CDR1 (antisense)
<220>
   <221> misc_feature
   <222> (21)..(21)
   <223> n is a, c, g, or t
<400> 866
   gaacgcgacg aaggcctaag ndaaagagat tcatgaaata caaaac 46
<210> 867
   <211> 46
   <212> DNA
   <213> artificial
<220>
   <223> primer 2 for mutation of T position in XPA CDR1
<400> 867
   cttgcgctgc ttccggattc vaattctcta agtactttat gttttg 46
<210> 868
   <211> 46
   <212> DNA
   <213> artificial
<220>
   <223> primer 2 for mutation of T position in XPA CDR1 (antisense)
<400> 868
   gaacgcgacg aaggcctaag bttaagagat tcatgaaata caaaac 46
<210> 869
   <211> 46
   <212> DNA
   <213> artificial
<220>
   <223> primer 3 for mutation of T position in XPA CDR1
<400> 869
   cttgcgctgc ttccggattc bggttctcta agtactttat gttttg 46
<210> 870
   <211> 46
   <212> DNA
   <213> artificial
<220>
   <223> primer 3 for mutation of T position in XPA CDR1 (antisense)
<400> 870
   gaacgcgacg aaggcctaag vccaagagat tcatgaaata caaaac 46
<210> 871
   <211> 43
   <212> DNA
   <213> artificial
<220>
   <223> primer 1 for mutation of F4 position in XPA CDR1
<220>
   <221> misc_feature
   <222> (19)..(19)
   <223> n is a, c, g, or t
<400> 871
   gctgcttccg gattcactnh ttctaagtac tttatgtttt ggg 43
<210> 872
   <211> 43
   <212> DNA
   <213> artificial
<220>
   <223> primer 1 for mutation of F4 position in XPA CDR1 (antisense)
<220>
   <221> misc_feature
   <222> (19)..(19)
   <223> n is a, c, g, or t
<400> 872
   cgacgaaggc ctaagtgand aagattcatg aaatacaaaa ccc 43
<210> 873
   <211> 43
   <212> DNA
   <213> artificial
<220>
   <223> primer 2 for mutation of F4 position in XPA CDR1
<400> 873
   gctgcttccg gattcactva atctaagtac tttatgtttt ggg 43
<210> 874
   <211> 43
   <212> DNA
   <213> artificial
<220>
   <223> primer 2 for mutation of F4 position in XPA CDR1 (antisense)
<400> 874
   cgacgaaggc ctaagtgabt tagattcatg aaatacaaaa ccc 43
<210> 875
   <211> 43
   <212> DNA
   <213> artificial
<220>
   <223> primer 3 for mutation of F4 position in XPA CDR1
<400> 875
   gctgcttccg gattcactbg gtctaagtac tttatgtttt ggg 43
<210> 876
   <211> 43
   <212> DNA
   <213> artificial
<220>
   <223> primer 3 for mutation of F4 position in XPA CDR1 (antisense)
<400> 876
   cgacgaaggc ctaagtgavc cagattcatg aaatacaaaa ccc 43
<210> 877
   <211> 46
   <212> DNA
   <213> artificial
<220>
   <223> primer 1 for mutation of S position in XPA CDR1
<220>
   <221> misc_feature
   <222> (21)..(21)
   <223> n is a, c, g, or t
<400> 877
   ctgcttccgg attcactttc nhtaagtact ttatgttttg ggttcg 46
<210> 878
   <211> 46
   <212> DNA
   <213> artificial
<220>
   <223> primer 1 for mutation of S position in XPA CDR1 (antisense)
<220>
   <221> misc_feature
   <222> (21)..(21)
   <223> n is a, c, g, or t
<400> 878
   gacgaaggcc taagtgaaag ndattcatga aatacaaaac ccaagc 46
<210> 879
   <211> 46
   <212> DNA
   <213> artificial
<220>
   <223> primer 2 for mutation of S position in XPA CDR1
<400> 879
   ctgcttccgg attcactttc vaaaagtact ttatgttttg ggttcg 46
<210> 880
   <211> 46
   <212> DNA
   <213> artificial
<220>
   <223> primer 2 for mutation of S position in XPA CDR1 (antisense)
<400> 880
   gacgaaggcc taagtgaaag bttttcatga aatacaaaac ccaagc 46
<210> 881
   <211> 46
   <212> DNA
   <213> artificial
<220>
   <223> primer 3 for mutation of S position in XPA CDR1
<400> 881
   ctgcttccgg attcactttc bggaagtact ttatgttttg ggttcg 46
<210> 882
   <211> 46
   <212> DNA
   <213> artificial
<220>
   <223> primer 3 for mutation of S position in XPA CDR1 (antisense)
<400> 882
   gacgaaggcc taagtgaaag vccttcatga aatacaaaac ccaagc 46
<210> 883
   <211> 45
   <212> DNA
   <213> artificial
<220>
   <223> primer 1 for mutation of K position in XPA CDR1
<220>
   <221> misc_feature
   <222> (21)..(21)
   <223> n is a, c, g, or t
<400> 883
   cttccggatt cactttctct nhttacttta tgttttgggt tcgcc 45
<210> 884
   <211> 45
   <212> DNA
   <213> artificial
<220>
   <223> primer 1 for mutation of K position in XPA CDR1 (antisense)
<220>
   <221> misc_feature
   <222> (21)..(21)
   <223> n is a, c, g, or t
<400> 884
   gaaggcctaa gtgaaagaga ndaatgaaat acaaaaccca agcgg 45
<210> 885
   <211> 45
   <212> DNA
   <213> artificial
<220>
   <223> primer 2 for mutation of K position in XPA CDR1
<400> 885
   cttccggatt cactttctct vaatacttta tgttttgggt tcgcc 45
<210> 886
   <211> 45
   <212> DNA
   <213> artificial
<220>
   <223> primer 2 for mutation of K position in XPA CDR1 (antisense(
<400> 886
   gaaggcctaa gtgaaagaga bttatgaaat acaaaaccca agcgg 45
<210> 887
   <211> 45
   <212> DNA
   <213> artificial
<220>
   <223> primer 3 for mutation of K position in XPA CDR1
<400> 887
   cttccggatt cactttctct bggtacttta tgttttgggt tcgcc 45
<210> 888
   <211> 45
   <212> DNA
   <213> artificial
<220>
   <223> primer 3 for mutation of K position in XPA CDR1 (antisense)
<400> 888
   gaaggcctaa gtgaaagaga vccatgaaat acaaaaccca agcgg 45
<210> 889
   <211> 45
   <212> DNA
   <213> artificial
<220>
   <223> primer 1 for mutation of Y position in XPA CDR1
<220>
   <221> misc_feature
   <222> (21)..(21)
   <223> n is a, c, g, or t
<400> 889
   ccggattcac tttctctaag nhttttatgt tttgggttcg ccaag 45
<210> 890
   <211> 45
   <212> DNA
   <213> artificial
<220>
   <223> primer 1 for mutation of Y position in XPA CDR1 (antisense)
<220>
   <221> misc_feature
   <222> (21)..(21)
   <223> n is a, c, g, or t
<400> 890
   ggcctaagtg aaagagattc ndaaaataca aaacccaagc ggttc 45
<210> 891
   <211> 45
   <212> DNA
   <213> artificial
<220>
   <223> primer 2 for mutation of Y position in XPA CDR1
<400> 891
   ccggattcac tttctctaag vaatttatgt tttgggttcg ccaag 45
<210> 892
   <211> 45
   <212> DNA
   <213> artificial
<220>
   <223> primer 2 for mutation of Y position in XPA CDR1 (artificial)
<400> 892
   ggcctaagtg aaagagattc bttaaataca aaacccaagc ggttc 45
<210> 893
   <211> 45
   <212> DNA
   <213> artificial
<220>
   <223> primer 3 for mutation of Y position in XPA CDR1
<400> 893
   ccggattcac tttctctaag bggtttatgt tttgggttcg ccaag 45
<210> 894
   <211> 45
   <212> DNA
   <213> artificial
<220>
   <223> primer 3 for mutation of Y position in XPA CDR1 (antisense)
<400> 894
   ggcctaagtg aaagagattc vccaaataca aaacccaagc ggttc 45
<210> 895
   <211> 44
   <212> DNA
   <213> artificial
<220>
   <223> primer 1 for mutation of F8 position in XPA CDR1
<220>
   <221> misc_feature
   <222> (22)..(22)
   <223> n is a, c, g, or t
<400> 895
   ggattcactt tctctaagta cnhtatgttt tgggttcgcc aagc 44
<210> 896
   <211> 44
   <212> DNA
   <213> artificial
<220>
   <223> primer 1 for mutation of F8 position in XPA CDR1 (antisense)
<220>
   <221> misc_feature
   <222> (22)..(22)
   <223> n is a, c, g, or t
<400> 896
   cctaagtgaa agagattcat gndatacaaa acccaagcgg ttcg 44
<210> 897
   <211> 44
   <212> DNA
   <213> artificial
<220>
   <223> primer 2 for mutation of F8 position in XPA CDR1
<400> 897
   ggattcactt tctctaagta cvaaatgttt tgggttcgcc aagc 44
<210> 898
   <211> 44
   <212> DNA
   <213> artificial
<220>
   <223> primer 2 for mutation of F8 position in XPA CDR1 (antisense)
<400> 898
   cctaagtgaa agagattcat gbtttacaaa acccaagcgg ttcg 44
<210> 899
   <211> 44
   <212> DNA
   <213> artificial
<220>
   <223> primer 3 for mutation of F8 position in XPA CDR1
<400> 899
   ggattcactt tctctaagta cbggatgttt tgggttcgcc aagc 44
<210> 900
   <211> 44
   <212> DNA
   <213> artificial
<220>
   <223> primer 3 for mutation of F8 position in XPA CDR1 (artificial)
<400> 900
   cctaagtgaa agagattcat gvcctacaaa acccaagcgg ttcg 44
<210> 901
   <211> 54
   <212> DNA
   <213> artificial
<220>
   <223> H1-A combination primer
<400> 901
   cttgcgctgc ttccggattc actttctcta aatactttat gttttgggtt cgcc 54
<210> 902
   <211> 54
   <212> DNA
   <213> artificial
<220>
   <223> H1-B combination primer
<400> 902
   cttgcgctgc ttccggattc actttctctc ygtactttat gttttgggtt cgcc 54
<210> 903
   <211> 54
   <212> DNA
   <213> artificial
<220>
   <223> H1-C combination primer
<400> 903
   cttgcgctgc ttccggattc actttctcty attactttat gttttgggtt cgcc 54
<210> 904
   <211> 54
   <212> DNA
   <213> artificial
<220>
   <223> H1-D combination primer
<400> 904
   cttgcgctgc ttccggattc actttctctt ggtactttat gttttgggtt cgcc 54
<210> 905
   <211> 59
   <212> DNA
   <213> artificial
<220>
   <223> H2-A combination primer
<400> 905
   gtgggtttct gttatctctc ctaaaggtmt catgactcgt tatgctgact ccgttaaag 59
<210> 906
   <211> 59
   <212> DNA
   <213> artificial
<220>
   <223> H2-B combination primer
<400> 906
   gtgggtttct gttatctctc ctaaaggtma aatgactcgt tatgctgact ccgttaaag 59
<210> 907
   <211> 59
   <212> DNA
   <213> artificial
<220>
   <223> H2-C combination primer
<400> 907
   gtgggtttct gttatctctc ctaaaggtsg tatgactcgt tatgctgact ccgttaaag 59
<210> 908
   <211> 59
   <212> DNA
   <213> artificial
<220>
   <223> H2-D combination primer
<400> 908
   gtgggtttct gttatctctc cttctggtmt catgactcgt tatgctgact ccgttaaag 59
<210> 909
   <211> 59
   <212> DNA
   <213> artificial
<220>
   <223> H2-E combination primer
<400> 909
   gtgggtttct gttatctctc cttctggtma aatgactcgt tatgctgact ccgttaaag 59
<210> 910
   <211> 59
   <212> DNA
   <213> artificial
<220>
   <223> H2-F combination primer
<400> 910
   gtgggtttct gttatctctc cttctggtsg tatgactcgt tatgctgact ccgttaaag 59
<210> 911
   <211> 54
   <212> DNA
   <213> artificial
<220>
   <223> H3-A combination primer
<400> 911
   ctattgtgcg agagtcggcc tgggtgkgaa tycagactac tggggccagg gaac 54
<210> 912
   <211> 54
   <212> DNA
   <213> artificial
<220>
   <223> H3-B combination primer
<400> 912
   ctattgtgcg agagtcggcc tgggtgkgaa tgaggactac tggggccagg gaac 54
<210> 913
   <211> 54
   <212> DNA
   <213> artificial
<220>
   <223> H3-C combination primer
<400> 913
   ctattgtgcg agagtcggcc tgggtgkggy gycagactac tggggccagg gaac 54
<210> 914
   <211> 54
   <212> DNA
   <213> artificial
<220>
   <223> H3-D combination primer
<400> 914
   ctattgtgcg agagtcggcc tgggtgkggy ggaggactac tggggccagg gaac 54
<210> 915
   <211> 54
   <212> DNA
   <213> artificial
<220>
   <223> H3-E combination primer
<400> 915
   ctattgtgcg agagtcggct atggtgkgaa tycagactac tggggccagg gaac 54
<210> 916
   <211> 54
   <212> DNA
   <213> artificial
<220>
   <223> H3-F combination primer
<400> 916
   ctattgtgcg agagtcggct atggtgkgaa tgaggactac tggggccagg gaac 54
<210> 917
   <211> 54
   <212> DNA
   <213> artificial
<220>
   <223> H3-G combination primer
<400> 917
   ctattgtgcg agagtcggct atggtgkggy gycagactac tggggccagg gaac 54
<210> 918
   <211> 54
   <212> DNA
   <213> artificial
<220>
   <223> H3-H combination primer
<400> 918
   ctattgtgcg agagtcggct atggtgkggy ggaggactac tggggccagg gaac 54
<210> 919
   <211> 21
   <212> DNA
   <213> artificial
<220>
   <223> H1 fwd combination primer
<400> 919
   gcttccggat tcactttctc t 21
<210> 920
   <211> 21
   <212> DNA
   <213> artificial
<220>
   <223> H1- rev combination primer
<400> 920
   agagaaagtg aatccggaag c 21
<210> 921
   <211> 20
   <212> DNA
   <213> artificial
<220>
   <223> H2- fwd combination primer
<400> 921
   gggtttctgt tatctctcct 20
<210> 922
   <211> 20
   <212> DNA
   <213> artificial
<220>
   <223> H2- rev combination primer
<400> 922
   aggagagata acagaaaccc 20
<210> 923
   <211> 19
   <212> DNA
   <213> artificial
<220>
   <223> H3- fwd combination primer
<400> 923
   ctattgtgcg agagtcggc 19
<210> 924
   <211> 20
   <212> DNA
   <213> artificial
<220>
   <223> H3- rev combination primer
<400> 924
   gccgactctc gcacaatagt 20
<210> 925
   <211> 20
   <212> DNA
   <213> artificial
<220>
   <223> L1R combination primer
<400> 925
   actcgcccga caaatgatgg 20
<210> 926
   <211> 20
   <212> DNA
   <213> artificial
<220>
   <223> L2R combination primer
<400> 926
   gatcaggagc ttaggggcat 20
<210> 927
   <211> 21
   <212> DNA
   <213> artificial
<220>
   <223> L3R combination primer
<400> 927
   ctgctgacaa tagtaagttg c 21
<210> 928
   <211> 58
   <212> DNA
   <213> artificial
<220>
   <223> L127Q28SLW30N combination primer
<400> 928
   ccatcatttg tcgggcgagt cagtbgatta ataggtggtt agcctggtat cagcagac 58
<210> 929
   <211> 58
   <212> DNA
   <213> artificial
<220>
   <223> L127Q28D30N combination primer
<400> 929
   ccatcatttg tcgggcgagt caggacatta ataggtggtt agcctggtat cagcagac 58
<210> 930
   <211> 58
   <212> DNA
   <213> artificial
<220>
   <223> L127Q28SLW30F combination primer
<400> 930
   ccatcatttg tcgggcgagt cagtbgattt ttaggtggtt agcctggtat cagcagac 58
<210> 931
   <211> 58
   <212> DNA
   <213> artificial
<220>
   <223> L127Q28D30F combination primer
<400> 931
   ccatcatttg tcgggcgagt caggacattt ttaggtggtt agcctggtat cagcagac 58
<210> 932
   <211> 58
   <212> DNA
   <213> artificial
<220>
   <223> L127SF28SLW30N combination primer
<400> 932
   ccatcatttg tcgggcgagt tyttbgatta ataggtggtt agcctggtat cagcagac 58
<210> 933
   <211> 58
   <212> DNA
   <213> artificial
<220>
   <223> L127SF28D30N combination primer
<400> 933
   ccatcatttg tcgggcgagt tytgacatta ataggtggtt agcctggtat cagcagac 58
<210> 934
   <211> 58
   <212> DNA
   <213> artificial
<220>
   <223> L127SF28SLW30F combination primer
<400> 934
   ccatcatttg tcgggcgagt tyttbgattt ttaggtggtt agcctggtat cagcagac 58
<210> 935
   <211> 58
   <212> DNA
   <213> artificial
<220>
   <223> L127SF28D30F combination primer
<400> 935
   ccatcatttg tcgggcgagt tytgacattt ttaggtggtt agcctggtat cagcagac 58
<210> 936
   <211> 58
   <212> DNA
   <213> artificial
<220>
   <223> L127G28SLW30N combination primer
<400> 936
   ccatcatttg tcgggcgagt ggatbgatta ataggtggtt agcctggtat cagcagac 58
<210> 937
   <211> 58
   <212> DNA
   <213> artificial
<220>
   <223> L127G28D30N combination primer
<400> 937
   ccatcatttg tcgggcgagt ggagacatta ataggtggtt agcctggtat cagcagac 58
<210> 938
   <211> 58
   <212> DNA
   <213> artificial
<220>
   <223> L127G28SLW30F combination primer
<400> 938
   ccatcatttg tcgggcgagt ggatbgattt ttaggtggtt agcctggtat cagcagac 58
<210> 939
   <211> 58
   <212> DNA
   <213> artificial
<220>
   <223> L127G28D30F combination primer
<400> 939
   ccatcatttg tcgggcgagt ggagacattt ttaggtggtt agcctggtat cagcagac 58
<210> 940
   <211> 55
   <212> DNA
   <213> artificial
<220>
   <223> L251GA53S combination primer
<400> 940
   atgcccctaa gctcctgatc cattctgsta cctctctgca aagtggggtc ccatc 55
<210> 941
   <211> 55
   <212> DNA
   <213> artificial
<220>
   <223> L251GA53KR combination primer
<400> 941
   atgcccctaa gctcctgatc cattctgsta ccargctgca aagtggggtc ccatc 55
<210> 942
   <211> 57
   <212> DNA
   <213> artificial
<220>
   <223> L392S93ED combination primer
<400> 942
   gcaacttact attgtcagca ggcttcagak ttcbcatkga ctttcggcgg agggacc 57
<210> 943
   <211> 57
   <212> DNA
   <213> artificial
<220>
   <223> L392S93S combination primer
<400> 943
   gcaacttact attgtcagca ggcttcatcg ttcbcatkga ctttcggcgg agggacc 57
<210> 944
   <211> 57
   <212> DNA
   <213> artificial
<220>
   <223> L392D93ED combination primer
<400> 944
   gcaacttact attgtcagca ggctgatgak ttcbcatkga ctttcggcgg agggacc 57
<210> 945
   <211> 57
   <212> DNA
   <213> artificial
<220>
   <223> L392D93S combination primer
<400> 945
   gcaacttact attgtcagca ggctgattcg ttcbcatkga ctttcggcgg agggacc 57
<210> 946
   <211> 104
   <212> PRT
   <213> artificial
<220>
   <223> Ca5 light chain variable region
<220>
   <221> misc_feature
   <222> (21)..(21)
   <223> Xaa can be any naturally occurring amino acid
<400> 946
   Asp Ile Gln Met Thr Gln Ser Pro Ser Ser Val Ser Ala Ser Val Gly
   1               5                   10                  15
   Asp Arg Leu Thr Xaa Ile Cys Arg Ala Ser Gln Asp Ile Asn Arg Trp
               20                  25                  30
   Leu Ala Trp Gln Gln Thr Pro Gly Asn Ala Pro Lys Leu Leu Ile His
           35                  40                  45
   Ser Ala Thr Ser Leu Gln Ser Gly Val Pro Ser Arg Phe Ser Gly Ser
       50                  55                  60
   Gly Ser Gly Thr Asp Phe Thr Leu Thr Ile Asn Ser Leu Met Asp Phe
   65                  70                  75                  80
   Ala Thr Tyr Tyr Cys Gln Gln Ala Asp Ser Phe Pro Leu Thr Phe Gly
                   85                  90                  95
   Gly Gly Thr Lys Val Glu Ile Lys
               100
<210> 947
   <211> 106
   <212> PRT
   <213> artificial
<220>
   <223> A8.2 light chain variable region
<400> 947
   Asp Ile Gln Met Thr Gln Ser Pro Ser Ser Val Ser Ala Ser Ala Gly
   1               5                   10                  15
   Asp Arg Val Thr Ile Thr Cys Arg Ala Ser Gln Gly Ile Ser Thr Trp
               20                  25                  30
   Leu Ala Trp Tyr Gln Gln Lys Pro Gly Ala Pro Lys Leu Leu Ile Tyr
           35                  40                  45
   Ala Ala Ser Ser Leu Gln Ser Gly Val Pro Ser Arg Phe Ser Gly Ser
       50                  55                  60
   Gly Ser Gly Thr Asp Phe Thr Leu Thr Ile Ser Ser Leu Gln Pro Glu
   65                  70                  75                  80
   Asp Phe Ala Thr Tyr Phe Cys Gln Gln Gly Tyr Arg Phe Pro Phe Thr
                   85                  90                  95
   Phe Gly Pro Gly Thr Lys Val Glu Ile Lys
               100                 105
<210> 948
   <211> 118
   <212> PRT
   <213> artificial
<220>
   <223> Ca5 heavy chain variable region
<400> 948
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Lys Tyr
               20                  25                  30
   Phe Met Phe Trp Val Arg Gln Ala Pro Gly Lys Gly Leu Glu Trp Val
           35                  40                  45
   Ser Val Ile Ser Pro Ser Gly Gly Met Thr Arg Tyr Ala Asp Ser Val
       50                  55                  60
   Lys Gly Arg Pro Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Val Gly Phe Gly Gly Asn Ser Asp Tyr Trp Gly Gln Gly Thr
               100                 105                 110
   Leu Val Thr Val Ser Ser
           115
<210> 949
   <211> 118
   <212> PRT
   <213> artificial
<220>
   <223> A8.2 heavy chain variable region
<400> 949
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Met Tyr
               20                  25                  30
   Asp Met His Trp Val Arg Gln Ala Pro Gly Lys Gly Leu Glu Trp Val
           35                  40                  45
   Ser Tyr Ile Tyr Pro Ser Gly Gly Ile Thr Trp Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gln Gly Gly Pro Asn Trp Phe Asp Pro Trp Gly Gln Gly Thr
               100                 105                 110
   Leu Val Thr Val Ser Ser
           115
<210> 950
   <211> 768
   <212> DNA
   <213> artificial
<220>
   <223> ING-1 variable region DNA
<400> 950
   ggcgcgccta accatctatt tcaaggagac agtcataatg aaatacctat tgcctacggc      60
   agccgctgga ttgttattac tcgctgccca accagcgatg gcgcagatcc agttggtgca     120
   gtctggacct gagctgaaga agcctggaga gacagtcaag atctcctgca aggcttctgg     180
   atataccttc acaaaatatg gaatgaactg ggtgaagcag gctccaggaa agggtttaaa     240
   gtggatgggc tggataaaca cctacactga agagcctaca tatggtgatg acttcaaggg     300
   acggtttgcc ttctctttgg aaacctctgc cagcactgcc aatttgcaga tcaacaacct 360
   caaaagtgag gacacggcta catatttctg tgcaagattt ggctctgctg tggactactg 420
   gggtcaagga acctcggtca ccgtctcctc agcctccacc aagggcccat cggtcttccc 480
   cctggcaccc tcctccaaga gcacctctgg gggcacagcg gccctgggct gcctggtcaa 540
   ggactacttc cccgaaccgg tgacggtgtc gtggaactca ggcgccctga ccagcggcgt 600
   tcataccttc ccggctgtcc tacagtcctc aggactctac tccctcagca gcgtggtgac 660
   cgtgccctcc agcagcttgg gcacccagac ctacatctgc aacgtgaatc acaagcccag 720
   caacaccaag gtggacaaga gagttgagcc caaatcttgt gcggccgc 768
<210> 951
   <211> 768
   <212> DNA
   <213> artificial
<220>
   <223> ING-1 variable region DNA (antisense)
<400> 951
   ccgcgcggat tggtagataa agttcctctg tcagtattac tttatggata acggatgccg 60
   tcggcgacct aacaataatg agcgacgggt tggtcgctac cgcgtctagg tcaaccacgt 120
   cagacctgga ctcgacttct tcggacctct ctgtcagttc tagaggacgt tccgaagacc 180
   tatatggaag tgttttatac cttacttgac ccacttcgtc cgaggtcctt tcccaaattt 240
   cacctacccg acctatttgt ggatgtgact tctcggatgt ataccactac tgaagttccc 300
   tgccaaacgg aagagaaacc tttggagacg gtcgtgacgg ttaaacgtct agttgttgga 360
   gttttcactc ctgtgccgat gtataaagac acgttctaaa ccgagacgac acctgatgac 420
   cccagttcct tggagccagt ggcagaggag tcggaggtgg ttcccgggta gccagaaggg 480
   ggaccgtggg aggaggttct cgtggagacc cccgtgtcgc cgggacccga cggaccagtt 540
   cctgatgaag gggcttggcc actgccacag caccttgagt ccgcgggact ggtcgccgca 600
   agtatggaag ggccgacagg atgtcaggag tcctgagatg agggagtcgt cgcaccactg 660
   gcacgggagg tcgtcgaacc cgtgggtctg gatgtagacg ttgcacttag tgttcgggtc 720
   gttgtggttc cacctgttct ctcaactcgg gtttagaaca cgccggcg 768
<210> 952
   <211> 116
   <212> PRT
   <213> artificial
<220>
   <223> ING-1 KABAT HEAVY CHAIN (KABAT NUMBERING)
<400> 952
   Gln Ile Gln Leu Val Gln Ser Gly Pro Glu Leu Lys Lys Pro Gly Glu
   1               5                   10                  15
   Thr Val Lys Ile Ser Cys Lys Ala Ser Gly Tyr Thr Phe Thr Lys Tyr
               20                  25                  30
   Gly Met Asn Trp Val Lys Gln Ala Pro Gly Lys Gly Leu Lys Trp Met
           35                  40                  45
   Gly Trp Ile Asn Thr Tyr Thr Glu Glu Pro Thr Tyr Gly Asp Asp Phe
       50                  55                  60
   Lys Gly Arg Phe Ala Phe Ser Leu Glu Thr Ser Ala Ser Thr Ala Asn
   65                  70                  75                  80
   Leu Gln Ile Asn Asn Leu Lys Ser Glu Asp Thr Ala Thr Tyr Phe Cys
                   85                  90                  95
   Ala Arg Phe Gly Ser Ala Val Asp Tyr Trp Gly Gln Gly Thr Ser Val
               100                 105                 110
   Thr Val Ser Ser
           115
<210> 953
   <211> 116
   <212> PRT
   <213> artificial
<220>
   <223> ING-1 CHOTHIA HEAVY CHAIN
<400> 953
   Gln Ile Gln Leu Val Gln Ser Gly Pro Glu Leu Lys Lys Pro Gly Glu
   1 5 10 15
   Thr Val Lys Ile Ser Cys Lys Ala Ser Gly Tyr Thr Phe Thr Lys Tyr
   20 25 30
   Gly Met Asn Trp Val Lys Gln Ala Pro Gly Lys Gly Leu Lys Trp Met
   35 40 45
   Gly Trp Ile Asn Thr Tyr Thr Glu Glu Pro Thr Tyr Gly Asp Asp Phe
   50 55 60
   Lys Gly Arg Phe Ala Phe Ser Leu Glu Thr Ser Ala Ser Thr Ala Asn
   65 70 75 80
   Leu Gln Ile Asn Asn Leu Lys Ser Glu Asp Thr Ala Thr Tyr Phe Cys
   85 90 95
   Ala Arg Phe Gly Ser Ala Val Asp Tyr Trp Gly Gln Gly Thr Ser Val
   100 105 110
   Thr Val Ser Ser
   115
<210> 954
   <211> 116
   <212> PRT
   <213> artificial
<220>
   <223> ING-1 IMGT HEAVY CHAIN
<400> 954
   Gln Ile Gln Leu Val Gln Ser Gly Pro Glu Leu Lys Lys Pro Gly Glu
   1               5                   10                  15
   Thr Val Lys Ile Ser Cys Lys Ala Ser Gly Tyr Thr Phe Thr Lys Tyr
               20                  25                  30
   Gly Met Asn Trp Val Lys Gln Ala Pro Gly Lys Gly Leu Lys Trp Met
           35                  40                  45
   Gly Trp Ile Asn Thr Tyr Thr Glu Glu Pro Thr Tyr Gly Asp Asp Phe
       50                  55                  60
   Lys Gly Arg Phe Ala Phe Ser Leu Glu Thr Ser Ala Ser Thr Ala Asn
   65                  70                  75                  80
   Leu Gln Ile Asn Asn Leu Lys Ser Glu Asp Thr Ala Thr Tyr Phe Cys
                   85                  90                  95
   Ala Arg Phe Gly Ser Ala Val Asp Tyr Trp Gly Gln Gly Thr Ser Val
               100                 105                 110
   Thr Val Ser Ser
           115
<210> 955
   <211> 113
   <212> PRT
   <213> artificial
<220>
   <223> ING-1 KABAT LIGHT CHAIN (KABAT NUMBERING)
<400> 955
   Gln Ile Val Met Thr Gln Ala Ala Phe Ser Asn Pro Val Thr Leu Gly
   1 5 10 15
   Thr Ser Gly Ser Ile Ser Cys Arg Ser Ser Lys Ser Leu Leu His Ser
   20 25 30
   Asn Gly Ile Thr Tyr Leu Tyr Trp Tyr Leu Gln Lys Pro Gly Gln Ser
   35 40 45
   Pro Gln Leu Leu Ile Tyr Gln Met Ser Asn Leu Ala Ser Gly Val Pro
   50 55 60
   Asp Arg Phe Ser Ser Ser Gly Ser Gly Thr Asp Phe Thr Leu Arg Ile
   65 70 75 80
   Ser Arg Val Glu Ala Glu Asp Val Gly Val Tyr Tyr Cys Ala Gln Asn
   85 90 95
   Leu Glu Leu Pro Arg Thr Phe Gly Gly Gly Thr Lys Leu Glu Met Lys
   100 105 110
   Arg
<210> 956
   <211> 113
   <212> PRT
   <213> artificial
<220>
   <223> ING-1 CHOTHIA LIGHT CHAIN
<400> 956
   Gln Ile Val Met Thr Gln Ala Ala Phe Ser Asn Pro Val Thr Leu Gly
   1 5 10 15
   Thr Ser Gly Ser Ile Ser Cys Arg Ser Ser Lys Ser Leu Leu His Ser
   20 25 30
   Asn Gly Ile Thr Tyr Leu Tyr Trp Tyr Leu Gln Lys Pro Gly Gln Ser
   35 40 45
   Pro Gln Leu Leu Ile Tyr Gln Met Ser Asn Leu Ala Ser Gly Val Pro
   50 55 60
   Asp Arg Phe Ser Ser Ser Gly Ser Gly Thr Asp Phe Thr Leu Arg Ile
   65 70 75 80
   Ser Arg Val Glu Ala Glu Asp Val Gly Val Tyr Tyr Cys Ala Gln Asn
   85 90 95
   Leu Glu Leu Pro Arg Thr Phe Gly Gly Gly Thr Lys Leu Glu Met Lys
   100 105 110
   Arg
<210> 957
   <211> 113
   <212> PRT
   <213> artificial
<220>
   <223> ING-1 IMGT LIGHT CHAIN
<400> 957
   Gln Ile Val Met Thr Gln Ala Ala Phe Ser Asn Pro Val Thr Leu Gly
   1 5 10 15
   Thr Ser Gly Ser Ile Ser Cys Arg Ser Ser Lys Ser Leu Leu His Ser
   20 25 30
   Asn Gly Ile Thr Tyr Leu Tyr Trp Tyr Leu Gln Lys Pro Gly Gln Ser
   35 40 45
   Pro Gln Leu Leu Ile Tyr Gln Met Ser Asn Leu Ala Ser Gly Val Pro
   50 55 60
   Asp Arg Phe Ser Ser Ser Gly Ser Gly Thr Asp Phe Thr Leu Arg Ile
   65 70 75 80
   Ser Arg Val Glu Ala Glu Asp Val Gly Val Tyr Tyr Cys Ala Gln Asn
   85 90 95
   Leu Glu Leu Pro Arg Thr Phe Gly Gly Gly Thr Lys Leu Glu Met Lys
   100 105 110
   Arg
<210> 958
   <211> 118
   <212> PRT
   <213> artificial
<220>
   <223> XPA23 KABAT HEAVY CHAIN (KABAT NUMBERING)
<400> 958
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Lys Tyr
               20                  25                  30
   Phe Met Phe Trp Val Arg Gln Ala Pro Gly Lys Gly Leu Glu Trp Val
           35                  40                  45
   Ser Val Ile Ser Pro Ser Gly Gly Met Thr Arg Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Val Gly Tyr Gly Gly Asn Ser Asp Tyr Trp Gly Gln Gly Thr
               100                 105                 110
   Leu Val Thr Val Ser Ser
           115
<210> 959
   <211> 118
   <212> PRT
   <213> artificial
<220>
   <223> XPA23 CHOTHIA HEAVY CHAIN
<400> 959
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Lys Tyr
               20                  25                  30
   Phe Met Phe Trp Val Arg Gln Ala Pro Gly Lys Gly Leu Glu Trp Val
   35 40 45
   Ser Val Ile Ser Pro Ser Gly Gly Met Thr Arg Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Val Gly Tyr Gly Gly Asn Ser Asp Tyr Trp Gly Gln Gly Thr
   100 105 110
   Leu Val Thr Val Ser Ser
   115
<210> 960
   <211> 118
   <212> PRT
   <213> artificial
<220>
   <223> XPA23 IMGT HEAVY CHAIN
<400> 960
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Lys Tyr
   20 25 30
   Phe Met Phe Trp Val Arg Gln Ala Pro Gly Lys Gly Leu Glu Trp Val
   35 40 45
   Ser Val Ile Ser Pro Ser Gly Gly Met Thr Arg Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Val Gly Tyr Gly Gly Asn Ser Asp Tyr Trp Gly Gln Gly Thr
   100 105 110
   Leu Val Thr Val Ser Ser
   115
<210> 961
   <211> 108
   <212> PRT
   <213> artificial
<220>
   <223> XPA23 KABAT LIGHT CHAIN (KABAT NUMBERING)
<400> 961
   Asp Ile Gln Met Thr Gln Ser Pro Ser Ser Val Ser Ala Ser Val Gly
   1 5 10 15
   Asp Arg Leu Thr Ile Ile Cys Arg Ala Ser Gln Asp Ile Asn Arg Trp
   20 25 30
   Leu Ala Trp Tyr Gln Gln Thr Pro Gly Asn Ala Pro Lys Leu Leu Ile
   35 40 45
   His Ser Ala Thr Ser Leu Gln Ser Gly Val Pro Ser Arg Phe Ser Gly
   50 55 60
   Ser Gly Ser Gly Thr Asp Phe Thr Leu Thr Ile Asn Ser Leu Gln Pro
   65 70 75 80
   Glu Asp Phe Ala Thr Tyr Tyr Cys Gln Gln Ala Asp Ser Phe Pro Leu
   85 90 95
   Thr Phe Gly Gly Gly Thr Lys Val Glu Ile Lys Arg
   100 105
<210> 962
   <211> 108
   <212> PRT
   <213> artificial
<220>
   <223> XPA23 CHOTHIA LIGHT CHAIN
<400> 962
   Asp Ile Gln Met Thr Gln Ser Pro Ser Ser Val Ser Ala Ser Val Gly
   1 5 10 15
   Asp Arg Leu Thr Ile Ile Cys Arg Ala Ser Gln Asp Ile Asn Arg Trp
   20 25 30
   Leu Ala Trp Tyr Gln Gln Thr Pro Gly Asn Ala Pro Lys Leu Leu Ile
   35 40 45
   His Ser Ala Thr Ser Leu Gln Ser Gly Val Pro Ser Arg Phe Ser Gly
   50 55 60
   Ser Gly Ser Gly Thr Asp Phe Thr Leu Thr Ile Asn Ser Leu Gln Pro
   65 70 75 80
   Glu Asp Phe Ala Thr Tyr Tyr Cys Gln Gln Ala Asp Ser Phe Pro Leu
   85 90 95
   Thr Phe Gly Gly Gly Thr Lys Val Glu Ile Lys Arg
   100 105
<210> 963
   <211> 108
   <212> PRT
   <213> artificial
<220>
   <223> XPA23 IMGT LIGHT CHAIN
<400> 963
   Asp Ile Gln Met Thr Gln Ser Pro Ser Ser Val Ser Ala Ser Val Gly
   1 5 10 15
   Asp Arg Leu Thr Ile Ile Cys Arg Ala Ser Gln Asp Ile Asn Arg Trp
   20 25 30
   Leu Ala Trp Tyr Gln Gln Thr Pro Gly Asn Ala Pro Lys Leu Leu Ile
   35 40 45
   His Ser Ala Thr Ser Leu Gln Ser Gly Val Pro Ser Arg Phe Ser Gly
   50 55 60
   Ser Gly Ser Gly Thr Asp Phe Thr Leu Thr Ile Asn Ser Leu Gln Pro
   65 70 75 80
   Glu Asp Phe Ala Thr Tyr Tyr Cys Gln Gln Ala Asp Ser Phe Pro Leu
   85 90 95
   Thr Phe Gly Gly Gly Thr Lys Val Glu Ile Lys Arg
   100 105
<210> 964
   <211> 64
   <212> DNA
   <213> artificial
<220>
   <223> Representative CDR region
<400> 964
   gtctttcttg cgctgcttcc ggattcactt tctctaagta ctttatgttt tgggttcgcc 60
   aagc 64
<210> 965
   <211> 64
   <212> DNA
   <213> artificial
<220>
   <223> Representative CDR region (antisense)
<400> 965
   cagaaagaac gcgacgaagg cctaagtgaa agagattcat gaaatacaaa acccaagcgg 60
   ttcg 64
<210> 966
   <211> 57
   <212> DNA
   <213> artificial
<220>
   <223> CDR region 1
<400> 966
   gtctttcttg cgctgcttcc ggattcactt tctctaagta ctttatgttt tgggttc 57
<210> 967
   <211> 57
   <212> DNA
   <213> artificial
<220>
   <223> CDR region 1 (antisense)
<400> 967
   cagaaagaac gcgacgaagg cctaagtgaa agagattcat gaaatacaaa acccaag 57
<210> 968
   <211> 53
   <212> DNA
   <213> artificial
<220>
   <223> CDR region 2
<400> 968
   gctgcttccg gattcacttt ctctaagtac tttatgtttt gggttcgcca agc 53
<210> 969
   <211> 53
   <212> DNA
   <213> artificial
<220>
   <223> CDR region 2 (antisense)
<400> 969
   cgacgaaggc ctaagtgaaa gagattcatg aaatacaaaa cccaagcggt tcg 53
<210> 970
   <211> 53
   <212> DNA
   <213> artificial
<220>
   <223> R2-5-NHT primer
<220>
   <221> misc_feature
   <222> (22)..()
   <223> N may be A, G, C or T
<220>
   <221> misc_feature
   <222> (22)..(22)
   <223> n is a, c, g, or t
<220>
   <221> misc_feature
   <222> (23)..()
   <223> H may be A, C or T
<400> 970
   gctgcttccg gattcacttt cnhtaagtac tttatgtttt gggttcgcca agc 53
<210> 971
   <211> 53
   <212> DNA
   <213> artificial
<220>
   <223> R2-5-VAA primer
<220>
   <221> misc_feature
   <222> (22)..()
   <223> V may be A, C or G
<400> 971
   gctgcttccg gattcacttt cvaaaagtac tttatgtttt gggttcgcca agc 53
<210> 972
   <211> 53
   <212> DNA
   <213> artificial
<220>
   <223> R2-5-BGG primer
<220>
   <221> misc_feature
   <222> (22)..()
   <223> B may be C, G or T
<400> 972
   gctgcttccg gattcacttt cbggaagtac tttatgtttt gggttcgcca agc 53
<210> 973
   <211> 53
   <212> DNA
   <213> artificial
<220>
   <223> R2-6-NHT primer
<220>
   <221> misc_feature
   <222> (25)..()
   <223> N may be A, G, C or T
<220>
   <221> misc_feature
   <222> (25)..(25)
   <223> n is a, c, g, or t
<220>
   <221> misc_feature
   <222> (26)..()
   <223> H may be A, C or T
<400> 973
   gctgcttccg gattcacttt ctctnhttac tttatgtttt gggttcgcca agc 53
<210> 974
   <211> 53
   <212> DNA
   <213> artificial
<220>
   <223> R2-6-VAA primer
<220>
   <221> misc_feature
   <222> (25)..()
   <223> V may be A, C or G
<400> 974
   gctgcttccg gattcacttt ctctvaatac tttatgtttt gggttcgcca agc 53
<210> 975
   <211> 53
   <212> DNA
   <213> artificial
<220>
   <223> R2-6-BGG primer
<220>
   <221> misc_feature
   <222> (25)..()
   <223> B may be C, G or T
<400> 975
   gctgcttccg gattcacttt ctctbggtac tttatgtttt gggttcgcca agc 53
<210> 976
   <211> 53
   <212> DNA
   <213> artificial
<220>
   <223> R2-7-NHT primer
<220>
   <221> misc_feature
   <222> (28)..()
   <223> N may be A, C, G or T
<220>
   <221> misc_feature
   <222> (28)..(28)
   <223> n is a, c, g, or t
<220>
   <221> misc_feature
   <222> (29)..()
   <223> H may be A, C or T
<400> 976
   gctgcttccg gattcacttt ctctaagnht tttatgtttt gggttcgcca agc 53
<210> 977
   <211> 53
   <212> DNA
   <213> artificial
<220>
   <223> R2-7-VAA primer
<220>
   <221> misc_feature
   <222> (28)..()
   <223> V may be A, C or G
<400> 977
   gctgcttccg gattcacttt ctctaagvaa tttatgtttt gggttcgcca agc 53
<210> 978
   <211> 53
   <212> DNA
   <213> artificial
<220>
   <223> R2-7-BGG primer
<220>
   <221> misc_feature
   <222> (28)..()
   <223> B may be C, G or T
<400> 978
   gctgcttccg gattcacttt ctctaagbgg tttatgtttt gggttcgcca agc 53
<210> 979
   <211> 53
   <212> DNA
   <213> artificial
<220>
   <223> R2-8-NHT primer
<220>
   <221> misc_feature
   <222> (31)..()
   <223> N may be A, G, C or T
<220>
   <221> misc_feature
   <222> (31)..(31)
   <223> n is a, c, g, or t
<220>
   <221> misc_feature
   <222> (32)..()
   <223> H may be A, C or T
<400> 979
   gctgcttccg gattcacttt ctctaagtac nhtatgtttt gggttcgcca agc 53
<210> 980
   <211> 53
   <212> DNA
   <213> artificial
<220>
   <223> R2-8-VAA primer
<220>
   <221> misc_feature
   <222> (31)..()
   <223> V may be A, C or G
<400> 980
   gctgcttccg gattcacttt ctctaagtac vaaatgtttt gggttcgcca agc 53
<210> 981
   <211> 53
   <212> DNA
   <213> artificial
<220>
   <223> R2-8-BGG primer
<220>
   <221> misc_feature
   <222> (31)..()
   <223> B may be C, G or T
<400> 981
   gctgcttccg gattcacttt ctctaagtac bggatgtttt gggttcgcca agc 53